# EUROPEAN PATENT SPECIFICATION

(11) **EP 4 367 103 B1**
(45) Date of publication and mention of the grant of the patent: **03.09.2025**
(21) Application number: 22744729.9
(22) Date of filing: 07.07.2022
(51) Int. Cl.: C07D 241/44, C07D 401/12, C07D 403/12, C07D 405/12, C07D 409/12, C07D 515/04, A61K 31/4985, A61P 31/04

(54) **INHIBITORS OF ALPHA-HEMOLYSIN OF STAPHYLOCOCCUS AUREUS**
INHIBITOREN VON ALPHA-HEMOLYSIN VON STAPHYLOCOCCUS AUREUS
INHIBITEURS D'ALPHA-HÉMOLYSINE DE STAPHYLOCOCCUS AUREUS

(30) Priority: 08.07.2021 EP 21184557
(43) Date of publication of application: 15.05.2024
(73) Proprietor: Helmholtz-Zentrum für Infektionsforschung GmbH, 38124 Braunschweig (DE); Lead Discovery Center GmbH, 44227 Dortmund (DE)
(72) Inventor: DILUCREZIA, Raffaella, 44227 Dortmund (DE); BRÖNSTRUP, Mark, 38124 Braunschweig (DE); BILITEWSKI, Ursula, 38124 Braunschweig (DE); DEGENHART, Carsten, 44227 Dortmund (DE); DINKEL, Klaus, 44227 Dortmund (DE); JERYE, Karoline, 38124 Braunschweig (DE); KALAWY FANSA, Eyad, 44227 Dortmund (DE); KOROTKOV, Vadim, 38124 Braunschweig (DE); MEDINA, Eva, 38124 Braunschweig (DE); ROX, Katharina, 38124 Braunschweig (DE); SHEKHAR, Aditya, 38124 Braunschweig (DE); WEICH, Herbert, 38124 Braunschweig (DE)
(74) Representative: Forstmeyer, Dietmar
(86) International application number: PCT/EP2022/068862
(87) International publication number: WO 2023/280970

(56) References cited:
- WO-A1-2019/084271
- WO-A2-02/09648
- US-A1- 2005 032 805
- US-A1- 2006 205 719
- US-A1- 2017 174 639
- US-A1- 2018 162 867
- JEFFERSON ELIZABETH A. ET AL: "New Inhibitors of Bacterial Protein Synthesis from a Combinatorial Library of Macrocycles", JOURNAL OF MEDICINAL CHEMISTRY, vol. 45, no. 16, 27 June 2002 (2002-06-27), US, pages 3430 - 3439, XP055864589, ISSN: 0022-2623, DOI: 10.1021/jm010437x
- TOYAMA MASAAKI ET AL: "Pyrimidotriazine derivatives as selective inhibitors of HBV capsid assembly", VIRUS RESEARCH, AMSTERDAM, NL, vol. 271, 31 July 2019 (2019-07-31), XP085765479, ISSN: 0168-1702, [retrieved on 20190731], DOI: 10.1016/J.VIRUSRES.2019.197677

## Description

The present invention provides novel inhibitors of α-hemolysin and the use thereof for the prophylaxis and treatment of infections caused by *Staphylococcus aureus*; especially *S*. *aureus* lung infections.

Hospital-acquired bacterial pneumonia is the most frequent nosocomial infection. It is classified into two categories: HAP, which develops in hospitalized patients after 48 h of admission, and does not require artificial ventilation at the time of diagnosis, and VAP, which occurs in patients who have received mechanical ventilation for at least 48 h. Both types have a high mortality rate (>20%) in spite of adequate antibiotic therapy and require considerable health resources. *S. aureus* is among the most common pathogens associated with hospital acquired pneumonia worldwide. Treatment of these infections has become more challenging because of the global emergence of S. *aureus* strains resistant to commonly used antibiotics. In developed countries such as the USA, MRSA strains are a major problem in hospitals with up to one half of staphylococcal pneumonia isolates classified as MRSA, resulting in mortality as high as 56%.

The Infectious Diseases Society of America (IDSA) and American Thoracic Society (ATS) recommended vancomycin or linezolid in patients with hospital- and ventilation-acquired pneumonia (HAPNAP) when empiric coverage of MRSA is indicated. Vancomycin poorly penetrates into the lung parenchyma, and high serum levels are often required to achieve adequate lung levels for bacterial killing. Unfortunately, increasing serum vancomycin levels comes with the risk of nephrotoxicity. Furthermore, the increasing prevalence of *S*. *aureus* strains with elevated vancomycin MIC (1-2 µg/mL) is associated with significantly treatment failure. Linezolid is not bactericidal against *S*. *aureus* and is not suitable for all patients due to drug interactions and hematologic effects.

The limited effectiveness of available standard-of-care treatments poses increasing public health risks. Thus, an improvement of current treatment regimens is critically needed for patients with HAP/VAP caused by *S*. *aureus.* An improvement cannot be achieved by bacterial killing with antibiotics alone, but require pre-emptive or adjunctive therapies that prevent or ameliorate the disease pathology on the host side. A highly promising approach, validated by preclinical and clinical data, is to block *S*. *aureus'* key virulence factor Hla and hence interfere with the capacity of *S*. *aureus* to colonize the lungs, thereby halting pathogenesis until the host immune response or antibiotics kill the bacteria.

It has therefore been an object of the present invention to provide novel inhibitors of virulence factor Hla.

The present invention provides compounds of formula (I): 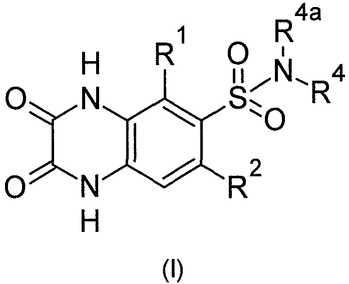 wherein
R¹ is hydrogen, fluorine or a methyl group;
R² is halogen, OH, NO₂, CN or NH₂; or a C₁₋₄ alkyl group, a C₂₋₄ alkenyl group, a C₂₋₄ alkynyl group, a C₃₋₅ cycloalkyl group, an -O-C₃₋₅ cycloalkyl group, a C₄₋₈ alkylcycloalkyl group, or a C₁₋₄ heteroalkyl group;
R⁴ is an optionally substituted phenyl group; an optionally substituted naphthyl group; an optionally substituted heteroaryl group containing 1 or 2 rings and 5 to 10 ring atoms selected from O, S, N and C; an optionally substituted cycloalkyl aryl group comprising a phenyl group and a cycloalkyl group containing 5 or 6 ring atoms; an optionally substituted heterocycloalkyl aryl group comprising a phenyl group and a heterocycloalkyl group containing 5 or 6 ring atoms selected from O, S, B, N and C; an optionally substituted cycloalkyl heteroaryl group comprising a heteroaryl group comprising 5 or 6 ring atoms selected from O, S, N and C and a cycloalkyl group containing 5 or 6 ring atoms; or an optionally substituted heterocycloalkyl heteroaryl group comprising a heteroaryl group comprising 5 or 6 ring atoms selected from O, S, N and C and a heterocycloalkyl group containing 5 or 6 ring atoms selected from O, S, N and C; or an optionally substituted cycloalkyl group containing 1 or 2 rings and 3 to 10 ring atoms; and
R^{4a} is hydrogen; or
R² and R^{4a} together are a group of formula -O-(CH₂)ₙ-, wherein n is 1, 2 or 3, wherein the oxygen is bound to the phenyl ring;
or a solvate, a hydrate or a salt thereof, wherein the compounds listed on the following pages 11 and 12 are excluded.

The present invention moreover provides compounds of formula (I): 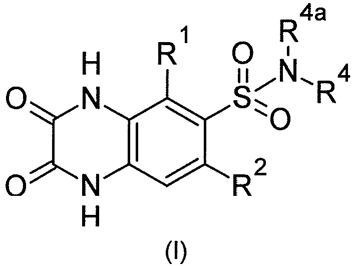 wherein
R¹ is hydrogen, fluorine or a methyl group;
R² is halogen, OH, NO₂, CN or NH₂; or a C₁₋₄ alkyl group, a C₂₋₄ alkenyl group, a C₂₋₄ alkynyl group, a C₃₋₅ cycloalkyl group, a C₄₋₈ alkylcycloalkyl group or a C₁₋₄ heteroalkyl group;
R⁴ is an optionally substituted phenyl group; an optionally substituted naphthyl group; an optionally substituted heteroaryl group containing 1 or 2 rings and 5 to 10 ring atoms selected from O, S, N and C; an optionally substituted cycloalkyl aryl group comprising a phenyl group and a cycloalkyl group containing 5 or 6 ring atoms; an optionally substituted heterocycloalkyl aryl group comprising a phenyl group and a heterocycloalkyl group containing 5 or 6 ring atoms selected from O, S, N and C; an optionally substituted cycloalkyl heteroaryl group comprising a heteroaryl group comprising 5 or 6 ring atoms selected from O, S, N and C and a cycloalkyl group containing 5 or 6 ring atoms; or an optionally substituted heterocycloalkyl heteroaryl group comprising a heteroaryl group comprising 5 or 6 ring atoms selected from O, S, N and C and a heterocycloalkyl group containing 5 or 6 ring atoms selected from O, S, N and C; or an optionally substituted cycloalkyl group containing 1 or 2 rings and 3 to 10 ring atoms; and
R^{4a} is hydrogen; or
R² and R^{4a} together are a group of formula -O-(CH₂)ₙ-, wherein n is 1, 2 or 3 (especially a group of formula -O-CH₂-CH₂-), wherein the oxygen is bound to the phenyl ring;
or a solvate, a hydrate or a salt thereof.

Preferably, R¹ is hydrogen or fluorine; especially preferably, R¹ is hydrogen.

Moreover preferably, R^{4a} is hydrogen.

Further preferably, R² is F, Cl, Br, a methyl group, an ethyl group, an iso-propyl group, a NO₂ group, a -CF₃ group, a methoxy group, a -O-CF₃ group, a cyclopropyl group, a CN group, a CD₃ group, a -CHF₂ group, a -CH₂F group, a -CH₂OH group, a -NHMe group, an -O-cyclopropyl group, an -O-CH₂CF₃ group, an ethoxy group, an - NHCH₂CH₂OH group, or a -NMe₂ group.

Moreover preferably, R² is F, Cl, Br, a methyl group, an ethyl group, an iso-propyl group, a NO₂ group, a -CF₃ group, a methoxy group, a -O-CF₃ group, a cyclopropyl group, a CN group, a CD₃ group, a -CHF₂ group, a -CH₂F group, a -CH₂OH group or a -NMe₂ group.

More preferably, R² is F, Cl, Br, a methyl group, an ethyl group, iso-propyl group, a methoxy group, a trifluoromethoxy group, a nitro group, a cyclopropyl group or a dimethylamino group.

Especially preferably, R² is a methyl group.

Further preferably, R⁴ is an optionally substituted phenyl group; an optionally substituted naphthyl group; an optionally substituted heteroaryl group containing 1 or 2 rings and 5 to 10 ring atoms selected from O, S, N and C; an optionally substituted cycloalkyl aryl group comprising a phenyl group and a cycloalkyl group containing 5 or 6 ring atoms; an optionally substituted heterocycloalkyl aryl group comprising a phenyl group and a heterocycloalkyl group containing 5 or 6 ring atoms selected from O, S, N and C; an optionally substituted cycloalkyl heteroaryl group comprising a heteroaryl group comprising 5 or 6 ring atoms selected from O, S, N and C and a cycloalkyl group containing 5 or 6 ring atoms; or an optionally substituted heterocycloalkyl heteroaryl group comprising a heteroaryl group comprising 5 or 6 ring atoms selected from O, S, N and C and a heterocycloalkyl group containing 5 or 6 ring atoms selected from O, S, N and C.

Moreover preferably, R⁴ is an optionally substituted phenyl group; an optionally substituted naphthyl group; an optionally substituted heteroaryl group containing 1 or 2 rings and 5 to 10 ring atoms selected from O, S, N and C or an optionally substituted cycloalkyl aryl group comprising a phenyl group and a cycloalkyl group containing 5 or 6 ring atoms.

Further preferably, R⁴ is an optionally substituted phenyl group; an optionally substituted naphthyl group; or an optionally substituted heteroaryl group containing 1 or 2 rings and 5 to 10 ring atoms selected from O, S, N and C.

Moreover preferably, R⁴ is an optionally substituted phenyl group; or an optionally substituted heteroaryl group containing 5 or 6 ring atoms selected from O, S, N and C.

Further preferably, R⁴ has the following formula: 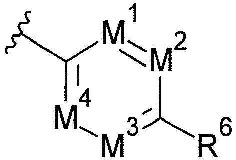 wherein
M¹ is N or CR⁷; M² is N or CR⁵; M³ is N or CR^{5a}; and M⁴ is N or CR^{7a};
R⁵, R^{5a}, R⁷ and R^{7a} are independently selected from hydrogen, halogen, CN, a C₁₋₄ alkyl group, a C₂₋₄ alkenyl group, a C₂₋₄ alkynyl group, or a C₁₋₄ heteroalkyl group; and R⁶ is halogen, CN, an alkyl group, an alkenyl group, an alkynyl group, a heteroalkyl group, a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁶ is a group of formula -OR^{6a} or -NHR^{6a}, wherein R^{6a} is a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁵ and R⁶ together are part of an optionally substituted phenyl group, an optionally substituted heteroaryl group containing 5 or 6 ring atoms selected from O, S, N and C, an optionally substituted cycloalkyl group containing 5 or 6 ring atoms or an optionally substituted heterocycloalkyl group containing 5 or 6 ring atoms selected from O, S, B, N and C (especially from O, S, N and C).

Preferably, only one of M¹, M², M³ and M⁴ is N.

Moreover preferably, R⁷ is hydrogen or methyl; especially preferably, R⁷ is hydrogen.

Further preferably, R^{7a} is hydrogen.

Moreover preferably, R⁴ is an optionally substituted phenyl group.

Further preferably, R⁴ has the following formula: 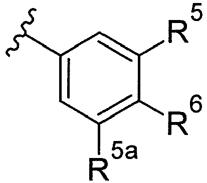 wherein
R⁵ and R^{5a} are independently selected from hydrogen, halogen, CN, a C₁₋₄ alkyl group, a C₂₋₄ alkenyl group, a C₂₋₄ alkynyl group, or a C₁₋₄ heteroalkyl group; and
R⁶ is halogen, CN, an alkyl group, an alkenyl group, an alkynyl group, a heteroalkyl group, a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁶ is a group of formula -OR^{6a} or -NHR^{6a}, wherein R^{6a} is a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁵ and R⁶ together are part of an optionally substituted phenyl group, an optionally substituted heteroaryl group containing 5 or 6 ring atoms selected from O, S, N and C, an optionally substituted cycloalkyl group containing 5 or 6 ring atoms or an optionally substituted heterocycloalkyl group containing 5 or 6 ring atoms selected from O, S, B, N and C (especially from O, S, N and C).

Moreover preferably, R⁵ is hydrogen or methyl; especially hydrogen.

Further preferably, R^{5a} is hydrogen, Cl, Br, -CN, methyl, methoxy, -CF₃, -OCF₃, -NMe₂, -C=CH, or -SO₂Me.

Moreover preferably, R^{5a} is hydrogen, Cl, Br, methyl or methoxy.

Further preferably, R⁶ is F, Cl, Br, CN, a C₁₋₆ alkyl group, a C₂₋₆ alkenyl group, a C₂₋₆ alkynyl group, a C₁₋₆ heteroalkyl group, an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

Moreover preferably, R⁶ is CN, a C₂₋₆ alkenyl group, a C₂₋₆ alkynyl group, a C₂₋₆ heteroalkyl group, an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

Further preferably, R⁶ is an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

Moreover preferably, R⁶ is F, Cl, Br, CN, a C₁₋₆ alkyl group, a C₂₋₆ alkenyl group, a C₂₋₆ alkynyl group, a C₁₋₆ heteroalkyl group, an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted heteroaryl group containing 5 or 6 ring atoms selected from O, S, N and C or an optionally substituted heterocycloalkyl aryl group comprising a phenyl group and a heterocycloalkyl group containing 5 or 6 ring atoms selected from O, S, N and C.

Further preferably, R⁶ is a group of formula OR^{6a}, wherein R^{6a} is an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted heteroaryl group containing 5 or 6 ring atoms selected from O, S, N and C or an optionally substituted heterocycloalkyl aryl group comprising a phenyl group and a heterocycloalkyl group containing 5 or 6 ring atoms selected from O, S, N and C.

Moreover preferably, R⁶ is a group of formula NHR^{6a}, wherein R^{6a} is an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted heteroaryl group containing 5 or 6 ring atoms selected from O, S, N and C or an optionally substituted heterocycloalkyl aryl group comprising a phenyl group and a heterocycloalkyl group containing 5 or 6 ring atoms selected from O, S, N and C.

Further preferably, R⁵ and R⁶ together are a group of formula -O-CH₂-O-, -O-CF₂-O- or -O-CH₂-CH₂-O-.

Moreover preferably, R⁶ is OCF₃.

Further preferably, R⁶ is an optionally substituted phenyl group or an optionally substituted heteroaryl group containing 5 or 6 to 10 ring atoms selected from O, S, N and C or an optionally substituted heterocycloalkyl aryl group comprising a phenyl group and a heterocycloalkyl group containing 5 or 6 ring atoms selected from O, S, N and C.

Moreover preferably, R⁶ is an optionally substituted phenyl group or an optionally substituted heteroaryl group containing 5 or 6 ring atoms selected from O, S, N and C.

According to a preferred embodiment, R⁶ is unsubstituted or substituted by 1, 2 or 3 substituents that are independently selected from halogen, CN, OH, NH₂, =O, - P(=O)Me₂, COOH, CONH₂, a C₁₋₄ alkyl group, a C₂₋₄ alkenyl group, a C₂₋₄ alkynyl group, a C₁₋₄ heteroalkyl group, a C₃₋₇ cycloalkyl group, an -O-C₃₋₇ cycloalkyl group or a heterocycloalkyl group containing from 3 to 7 ring atoms selected from O, S, C and N; especially wherein R⁶ is unsubstituted or substituted by 1, 2 or 3 substituents that are independently selected from halogen, CN, COOH, CONH₂, a C₁₋₄ alkyl group, a C₂₋₄ alkenyl group, a C₂₋₄ alkynyl group, a C₁₋₄ heteroalkyl group, a C₃₋₇ cycloalkyl group or a heterocycloalkyl group containing from 3 to 7 ring atoms selected from O, S, C and N.

Especially preferably, the optionally substituted phenyl group or the optionally substituted heteroaryl group containing 5 or 6 ring atoms selected from O, S, N and C at R⁶ is unsubstituted or substituted by 1, 2 or 3 substituents that are independently selected from halogen, CN, COOH, a C₁₋₄ alkyl group, a C₂₋₄ alkenyl group, a C₂₋₄ alkynyl group, a C₁₋₄ heteroalkyl group, a C₃₋₇ cycloalkyl group or a heterocycloalkyl group containing from 3 to 7 ring atoms selected from O, S, C and N.

The most preferred compounds of the present invention are the compounds disclosed in the examples, or a salt, solvate or a hydrate thereof.

It is further preferred to combine the preferred embodiments of the present invention in any desired manner.

According to one embodiment of the present invention, compounds of formula (I) as such, wherein R¹ is H, R² is Me, R^{4a} is hydrogen and R⁴ is selected from the following groups: 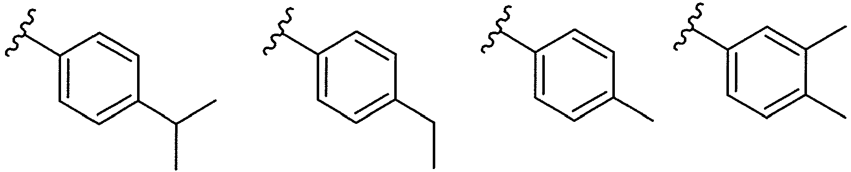 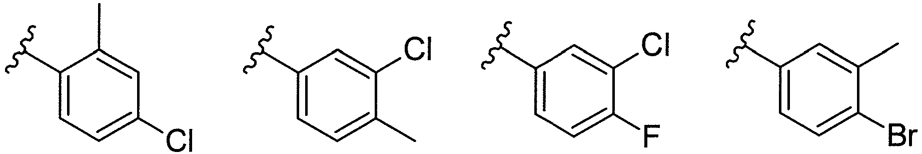 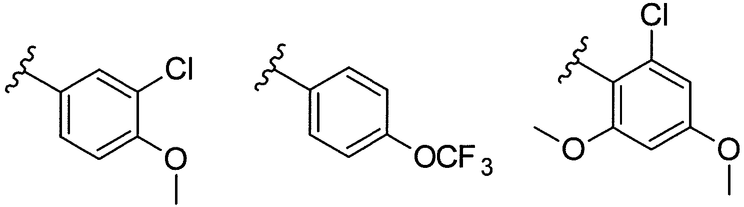 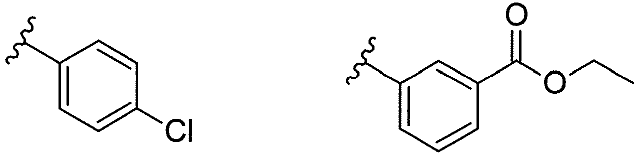 are excluded from the present invention. According to another embodiment, the use of these compounds in the prophylaxis, decolonization and treatment of a *Staphylococcus aureus* infection; especially for use in the prophylaxis and treatment of pneumonia caused by *Staphylococcus aureus* is encompassed by the present invention.

According to a further embodiment of the present invention, compound No. 16 disclosed in Jefferson et al. Journal of Medicinal Chemistry, 2002, Vol. 45, No. 16, pages 3430-3439 is excluded from the present invention.

According to a further embodiment of the present invention, the following compound is excluded from the present invention: 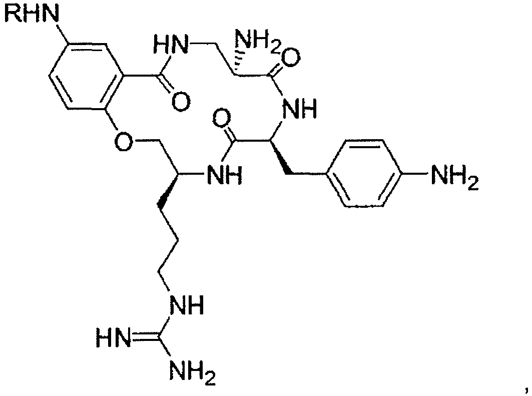 wherein R is a group having the following structure:

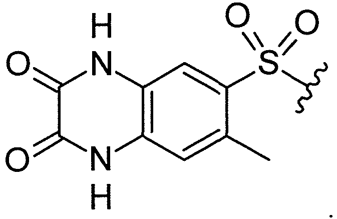

The expression alkyl refers to a saturated, straight-chain or branched hydrocarbon group that contains from 1 to 20 carbon atoms, preferably from 1 to 15 carbon atoms, especially from 1 to 10 (e.g. 1, 2, 3 or 4) carbon atoms, for example a methyl (Me, CH₃), ethyl, propyl, iso-propyl, n-butyl, iso-butyl, sec-butyl, tert-butyl, n-pentyl, isopentyl, n-hexyl, 2,2-dimethylbutyl or n-octyl group.

The expression C₁₋₆ alkyl refers to a saturated, straight-chain or branched hydrocarbon group that contains from 1 to 6 carbon atoms. The expression C₁₋₄ alkyl refers to a saturated, straight-chain or branched hydrocarbon group that contains from 1 to 4 carbon atoms. Examples are a methyl (Me), CF₃, ethyl, propyl, iso-propyl, n-butyl, iso-butyl, sec-butyl or tert-butyl group.

The expressions alkenyl and alkynyl refer to at least partially unsaturated, straight-chain or branched hydrocarbon groups that contain from 2 to 20 carbon atoms, preferably from 2 to 15 carbon atoms, especially from 2 to 10 (e.g. 2, 3 or 4) carbon atoms, for example an ethenyl (vinyl), propenyl (allyl), iso-propenyl, butenyl, ethinyl, propinyl, butinyl, acetylenyl, propargyl, isoprenyl or hex-2-enyl group. Preferably, alkenyl groups have one or two (especially preferably one) double bond(s), and alkynyl groups have one or two (especially preferably one) triple bond(s).

Furthermore, the terms alkyl, alkenyl and alkynyl refer to groups in which one or more hydrogen atoms have been replaced by a halogen atom (preferably F or Cl) such as, for example, a 2,2,2-trichloroethyl, difluoromethyl, fluoromethyl or a trifluoromethyl group.

The expression heteroalkyl refers to an alkyl, alkenyl or alkynyl group as defined above in which one or more (preferably 1 to 8; especially preferably 1, 2, 3 or 4) carbon atoms have been replaced by an oxygen, nitrogen, phosphorus, boron, selenium, silicon or sulfur atom (preferably by an oxygen, sulfur or nitrogen atom) or by a SO or a SO₂ group. The expression heteroalkyl furthermore refers to a carboxylic acid or to a group derived from a carboxylic acid, such as, for example, acyl, acylalkyl, alkoxycarbonyl, acyloxy, acyloxyalkyl, carboxyalkylamide or alkoxycarbonyloxy. Furthermore, the term heteroalkyl refers to groups in which one or more hydrogen atoms have been replaced by a halogen atom (preferably F or Cl).

Preferably, a heteroalkyl group contains from 1 to 12 carbon atoms and from 1 to 8 heteroatoms selected from oxygen, nitrogen and sulfur (especially oxygen and nitrogen). Especially preferably, a heteroalkyl group contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen). The term C₁-C₆ heteroalkyl refers to a heteroalkyl group containing from 1 to 6 carbon atoms and 1, 2, 3 or 4 heteroatoms selected from O, S and/or N (especially O and/or N). The term C₂-C₆ heteroalkyl refers to a heteroalkyl group containing from 2 to 6 carbon atoms and 1, 2, 3 or 4 heteroatoms selected from O, S and/or N (especially O and/or N). The term C₁-C₄ heteroalkyl refers to a heteroalkyl group containing from 1 to 4 carbon atoms and 1, 2 or 3 heteroatoms selected from O, S and/or N (especially O and/or N).

Further preferably, the expression heteroalkyl refers to an alkyl group as defined above (straight-chain or branched) in which one or more (preferably 1 to 6; especially preferably 1, 2, 3 or 4) carbon atoms have been replaced by an oxygen, sulfur or nitrogen atom or a CO group; this group preferably contains from 1 to 6 (e.g. 1, 2, 3 or 4) carbon atoms and 1, 2, 3 or 4 (especially 1, 2 or 3) heteroatoms selected from oxygen, nitrogen and sulfur (especially oxygen and nitrogen); this group may preferably be substituted by one or more (preferably 1 to 6; especially preferably 1, 2, 3 or 4) fluorine, chlorine, bromine or iodine atoms or OH, =O, SH, =S, NH₂, =NH, N₃, CN or NO₂ groups.

Examples of heteroalkyl groups are groups of formulae: R^{a}-O-Y^{a}-, R^{a}-S-Y^{a}-, R^{a}-SO-Y^{a}-, R^{a}-SO₂-Y^{a}-, R^{a}-N(R^{b})-SO₂-Y^{a}-, R^{a}-SO₂-N(R^{b})-Y^{a}-, R^{a}-N(R^{b})-Y^{a}-, R^{a}-CO-Y^{a}-, R^{a-}C(=NR^{d})-Y^{a}-, R^{a}-O-CO-Y^{a}-, R^{a}-CO-O-Y^{a}-, R^{a}-CO-N(R^{b})-Y^{a}-, R^{a}-N(R^{b})-CO-Y^{a}-, R^{a}-N(R^{p})-C(=NR^{d})-Y^{a}-, R^{a}-O-CO-N(R^{b})-Y^{a}-, R^{a}-N(R^{b})-CO-O-Y^{a}-, R^{a}-N(R^{b})-CO-N(R^{c})-Y^{a}-, R^{a}-O-CO-O-Y^{a}-, R^{a}-N(R^{b})-C(=NR^{d})-N(R^{c})-Y^{a}-, R^{a}-CS-Y^{a}-, R^{a}-O-CS-Y^{a}-, R^{a}-CS-O-Y^{a}-, R^{a}-CS-N(R^{b})-Y^{a}-, R^{a}-N(R^{b})-CS-Y^{a}-, R^{a}-O-CS-N(R^{b})-Y^{a}-, R^{a}-N(R^{b})-CS-O-Y^{a}-, R^{a}-N(R^{b})-CS-N(R^{c})-Y^{a}-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}-S-CO-O-Y^{a}-, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wherein R^{a} being a hydrogen atom, a C₁-C₆ alkyl, a C₂-C₆ alkenyl or a C₂-C₆ alkynyl group; R^{b} being a hydrogen atom, a C₁-C₆ alkyl, a C₂-C₆ alkenyl or a C₂-C₆ alkynyl group; R^{c} being a hydrogen atom, a C₁-C₆ alkyl, a C₂-C₆ alkenyl or a C₂-C₆ alkynyl group; R^{d} being a hydrogen atom, a C₁-C₆ alkyl, a C₂-C₆ alkenyl or a C₂-C₆ alkynyl group and Y^{a} being a bond, a C₁-C₆ alkylene, a C₂-C₆ alkenylene or a C₂-C₆ alkynylene group, wherein each heteroalkyl group contains at least one carbon atom. Further, one or more hydrogen atoms of the above groups may be replaced by fluorine or chlorine atoms.

Specific examples of heteroalkyl groups are methoxy, trifluoromethoxy, -OCD₃, ethoxy, n-propyloxy, isopropyloxy, butoxy, *tert*-butyloxy, methoxymethyl, ethoxymethyl, -CH₂CH₂OH, -CH₂OH, -SO₂Me, -NHAc, -CONH₂, methoxyethyl, 1-methoxyethyl, 1-ethoxyethyl, 2-methoxyethyl or 2-ethoxyethyl, methylamino, ethylamino, propylamino, isopropylamino, dimethylamino, diethylamino, isopropylethylamino, methylamino methyl, ethylamino methyl, diisopropylamino ethyl, methylthio, ethylthio, isopropylthio, enol ether, dimethylamino methyl, dimethylamino ethyl, acetyl, propionyl, butyryloxy, acetyloxy, methoxycarbonyl, ethoxycarbonyl, propionyloxy, acetylamino or propionylamino, carboxymethyl, carboxyethyl or carboxypropyl, N-ethyl-N-methylcarbamoyl or N-methylcarbamoyl. Further examples of heteroalkyl groups are nitrile (-CN), isonitrile, cyanate, thiocyanate, isocyanate, isothiocyanate and alkylnitrile groups.

The expression cycloalkyl refers to a saturated or partially unsaturated (for example, a cycloalkenyl group) cyclic group that contains one or more rings (preferably 1 or 2), and contains from 3 to 14 ring carbon atoms, preferably from 3 to 10 (especially 3, 4, 5, 6 or 7) ring carbon atoms. The expression cycloalkyl refers furthermore to groups in which one or more hydrogen atoms have been replaced by fluorine, chlorine, bromine or iodine atoms or by OH, =O, SH, =S, NH₂, =NH, N₃ or NO₂ groups, thus, for example, cyclic ketones such as, for example, cyclohexanone, 2-cyclohexenone or cyclopentanone. Further specific examples of cycloalkyl groups are a cyclopropyl, cyclobutyl, cyclopentyl, spiro[4,5]decanyl, norbornyl, cyclohexyl, cyclopentenyl, cyclohexadienyl, decalinyl, bicyclo[4.3.0]nonyl, tetraline, cyclopentylcyclohexyl, fluorocyclohexyl or cyclohex-2-enyl group.

The expression heterocycloalkyl refers to a cycloalkyl group as defined above in which one or more (preferably 1, 2 or 3) ring carbon atoms have been replaced by an oxygen, nitrogen, silicon, boron, selenium, phosphorus or sulfur atom (preferably by an oxygen, sulfur or nitrogen atom) or a SO group or a SO₂ group. A heterocycloalkyl group has preferably 1 or 2 ring(s) containing from 3 to 10 (especially 3, 4, 5, 6 or 7) ring atoms (preferably selected from C, O, N and S). The expression heterocycloalkyl refers furthermore to groups that are substituted by fluorine, chlorine, bromine or iodine atoms or by OH, =O, SH, =S, NH₂, =NH, N₃ or NO₂ groups. Examples are a piperidyl, prolinyl, imidazolidinyl, piperazinyl, morpholinyl (e.g. -N(CH₂CH₂)₂O), urotropinyl, pyrrolidinyl, tetrahydrothiophenyl, tetrahydropyranyl, tetrahydrofuryl or 2-pyrazolinyl group and also lactames, lactones, cyclic imides and cyclic anhydrides.

The expression alkylcycloalkyl refers to groups that contain both cycloalkyl and also alkyl, alkenyl or alkynyl groups in accordance with the above definitions, for example alkylcycloalkyl, cycloalkylalkyl, alkylcycloalkenyl, alkenylcycloalkyl and alkynylcycloalkyl groups. An alkylcycloalkyl group preferably contains a cycloalkyl group that contains one or two rings having from 3 to 10 (especially 3, 4, 5, 6 or 7) ring carbon atoms, and one or two alkyl, alkenyl or alkynyl groups (especially alkyl groups) having 1 or 2 to 6 carbon atoms.

The expression heteroalkylcycloalkyl refers to alkylcycloalkyl groups as defined above in which one or more (preferably 1, 2 or 3) carbon atoms have been replaced by an oxygen, nitrogen, silicon, boron, selenium, phosphorus or sulfur atom (preferably by an oxygen, sulfur or nitrogen atom) or a SO group or a SO₂ group. A heteroalkylcycloalkyl group preferably contains 1 or 2 rings having from 3 to 10 (especially 3, 4, 5, 6 or 7) ring atoms, and one or two alkyl, alkenyl, alkynyl or heteroalkyl groups (especially alkyl or heteroalkyl groups) having from 1 or 2 to 6 carbon atoms. Examples of such groups are alkylheterocycloalkyl, alkylheterocycloalkenyl, alkenylheterocycloalkyl, alkynylheterocycloalkyl, heteroalkylcycloalkyl, heteroalkylheterocycloalkyl and heteroalkylheterocycloalkenyl, the cyclic groups being saturated or mono-, di- or tri-unsaturated.

The expression aryl refers to an aromatic group that contains one or more rings containing from 6 to 14 ring carbon atoms, preferably from 6 to 10 (especially 6) ring carbon atoms. The expression aryl refers furthermore to groups that are substituted by fluorine, chlorine, bromine or iodine atoms or by OH, SH, NH₂, N₃ or NO₂ groups. Examples are the phenyl, naphthyl, biphenyl, 2-fluorophenyl, anilinyl, 3-nitrophenyl or 4-hydroxyphenyl group.

The expression heteroaryl refers to an aromatic group that contains one or more rings containing from 5 to 14 ring atoms, preferably from 5 to 10 (especially 5 or 6 or 9 or 10) ring atoms, comprising one or more (preferably 1, 2, 3 or 4) oxygen, nitrogen, phosphorus or sulfur ring atoms (preferably O, S or N). The expression heteroaryl refers furthermore to groups that are substituted by fluorine, chlorine, bromine or iodine atoms or by OH, SH, N₃, NH₂ or NO₂ groups. Examples are pyridyl (e.g. 4-pyridyl), imidazolyl (e.g. 2-imidazolyl), phenylpyrrolyl (e.g. 3-phenylpyrrolyl), thiazolyl, isothiazolyl, 1,2,3-triazolyl, 1,2,4-triazolyl, oxadiazolyl, thiadiazolyl, indolyl, indazolyl, tetrazolyl, pyrazinyl, pyrimidinyl, pyridazinyl, 4-hydroxypyridyl (4-pyridonyl), 3,4-hydroxypyridyl (3,4-pyridonyl), oxazolyl, isoxazolyl, triazolyl, tetrazolyl, isoxazolyl, indazolyl, indolyl, benzimidazolyl, benzoxazolyl, benzisoxazolyl, benzthiazolyl, pyridazinyl, quinolinyl, isoquinolinyl, pyrrolyl, purinyl, carbazolyl, acridinyl, pyrimidyl, 2,3'-bifuryl, pyrazolyl (e.g. 3-pyrazolyl) and isoquinolinyl groups.

The expression aralkyl refers to groups containing both aryl and also alkyl, alkenyl, alkynyl and/or cycloalkyl groups in accordance with the above definitions, such as, for example, arylalkyl, arylalkenyl, arylalkynyl, arylcycloalkyl, arylcycloalkenyl, alkylaryl-cycloalkyl and alkylarylcycloalkenyl groups. Specific examples of aralkyls are toluene, xylene, mesitylene, styrene, benzyl chloride, o-fluorotoluene, 1H-indene, tetraline, dihydronaphthalene, indanone, phenylcyclopentyl, cumene, cyclohexylphenyl, fluorene and indane. An aralkyl group preferably contains one or two aromatic ring systems (especially 1 or 2 rings), each containing from 6 to 10 carbon atoms and one or two alkyl, alkenyl and/or alkynyl groups containing from 1 or 2 to 6 carbon atoms and/or a cycloalkyl group containing 5 or 6 ring carbon atoms.

The expression heteroaralkyl refers to groups containing both aryl and/or heteroaryl groups and also alkyl, alkenyl, alkynyl and/or heteroalkyl and/or cycloalkyl and/or heterocycloalkyl groups in accordance with the above definitions. A heteroaralkyl group preferably contains one or two aromatic ring systems (especially 1 or 2 rings), each containing from 5 or 6 to 9 or 10 ring atoms (preferably selected from C, N, O and S) and one or two alkyl, alkenyl and/or alkynyl groups containing 1 or 2 to 6 carbon atoms and/or one or two heteroalkyl groups containing 1 to 6 carbon atoms and 1, 2 or 3 heteroatoms selected from O, S and N and/or one or two cycloalkyl groups each containing 5 or 6 ring carbon atoms and/or one or two heterocycloalkyl groups, each containing 5 or 6 ring atoms comprising 1, 2, 3 or 4 oxygen, sulfur or nitrogen atoms.

Examples are arylheteroalkyl, arylheterocycloalkyl, arylheterocycloalkenyl, arylalkylheterocycloalkyl, arylalkenylheterocycloalkyl, arylalkynylheterocycloalkyl, arylalkylheterocycloalkenyl, heteroarylalkyl, heteroarylalkenyl, heteroarylalkynyl, heteroarylheteroalkyl, heteroarylcycloalkyl, heteroarylcycloalkenyl, heteroaryl-heterocycloalkyl, heteroaryl heterocycloalkenyl, heteroarylalkylcycloalkyl, heteroaryl-alkylheterocycloalkenyl, heteroarylheteroalkylcycloalkyl, heteroaryl heteroalkyl-cycloalkenyl and heteroarylheteroalkylheterocycloalkyl groups, the cyclic groups being saturated or mono-, di- or tri-unsaturated. Specific examples are a tetrahydroisoquinolinyl, benzoyl, phthalidyl, 2- or 3-ethylindolyl, 4-methylpyridino, 2-, 3- or 4-methoxyphenyl, 4-ethoxyphenyl, 2-, 3- or 4-carboxyphenylalkyl group.

As already stated above, the expressions cycloalkyl, heterocycloalkyl, alkylcycloalkyl, heteroalkylcycloalkyl, aryl, heteroaryl, aralkyl and heteroaralkyl also refer to groups that are substituted by fluorine, chlorine, bromine or iodine atoms or by OH, =O, SH, =S, NH₂, =NH, N₃ or NO₂ groups.

The term halogen refers to F, Cl, Br or I.

The term "optionally substituted" refers to a group which is unsubstituted or substituted by one or more (especially by one, two or three; preferably by one or two; especially preferably by one) substituents. If a group comprises more than one substituent, these substituents are independently selected, i.e., they may be the same or different.

Examples for substituents are fluorine, chlorine, bromine and iodine and OH, SH, NH₂, =O, -SO₃H, -SO₂NH₂, -COOH, -COOMe, -COOEt, CH₂OH, -COMe (Ac), -NHSO₂Me, -SO₂NMe₂, -CH₂NH₂, -NHAc, -SO₂Me, -CONH₂, -CN, -NHCONH₂, -NHC(NH)NH₂, - NOHCH₃, -N₃ and -NO₂ groups. Further examples of substituents are C₁-C₁₀ alkyl, C₂-C₁₀ alkenyl, C₂-C₁₀ alkynyl, C₁-C₁₀ heteroalkyl, C₃-C₁₈ cycloalkyl, C₁-C₁₇ heterocycloalkyl, C₄-C₂₀ alkylcycloalkyl, C₁-C₁₉ heteroalkylcycloalkyl, C₆-C₁₈ aryl, C₁-C₁₇ heteroaryl, C₇-C₂₀ aralkyl and C₁-C₁₉ heteroaralkyl groups; especially C₁-C₆ alkyl, C₂-C₆ alkenyl, C₂-C₆ alkynyl, C₁-C₆ heteroalkyl, C₃-C₁₀ cycloalkyl, C₁-C₉ heterocycloalkyl, C₄-C₁₂ alkylcycloalkyl, C₁-C₁₁ heteroalkylcycloalkyl, C₆-C₁₀ aryl, C₁-C₉ heteroaryl, C₇-C₁₂ aralkyl and C₁-C₁₁ heteroaralkyl groups, further preferably C₁-C₆ alkyl and C₁-C₆ heteroalkyl groups.

Preferred substituents are halogen atoms (e.g. F, Cl, Br) and groups of formula -OH, =O, -O-C₁₋₆ alkyl (e.g. -OMe, -OCD₃, -OEt, -O-nPr, -O-iPr, -O-nBu, -O-iBu and -O-tBu), -NH₂, -NHC₁₋₆ alkyl, -N(C₁₋₆ alkyl)₂, -COOH, -COOMe, -COOEt, -CH₂OH, -CH₂NH₂, - CH₂CH₂-O-CH₃, -COMe, -NHSO₂Me, -PO(CH₃)₂, -SO₂NMe₂, -SO₃H, -SO₂NH₂, - CONH₂, -CH₂NH₂, -CN, -C₁₋₆ alkyl (e.g. -Me, -Et, -nPr, -iPr, -nBu, -iBu, -tBu and -CF₃), -SH, -S-CO-C₁₋₆ alkyl, -S-C₁₋₆ alkyl, -NHAc, -NO₂, -C≡CH, -CH=C(CH₃)₂, - CH=CHCH₂OCH₂CH₃, -NHCONH₂, -SO₂NMe₂, -SO₂Me, phenyl, cyclopropyl, -O-cyclopropyl, and heterocycloalkyl groups containing from 3 to 6 ring atoms selected from O, N and C (especially one nitrogen atom and from 3 to 6 ring atoms).

Further preferred substituents are F, Cl, Br, =O, a C₁₋₄ alkyl group (such as Me, Et, CF₃, iPr, tBu), a O-C₁₋₄ alkyl group (such as OMe, OCD₃, OCHF₂, OCH₂F, OiPr, OCF₃), NH₂, OH, a NHC₁₋₄ alkyl group, a N(C₁₋₄ alkyl)₂ group (such as NMe₂), -CH₂OH, - COOEt, -COOMe, -SO₂Me, -CH₂NH₂, -CH₂OH, -SO₂NMe₂, -NHCOCH₃, -SCF₃, - OCH₂CH₂NMe₂, -CH₂CH₂OCH₃, -NHCONMe₂, -PO(CH₃)₂, -COMe, -CONH₂, -COOH, -CN, -C≡CH, -CH=C(CH₃)₂, -CH=CHCH₂OCH₂CH₃, a pyrrolidinyl group, a-N(CH₂CH₂)₂O group, and an azetidinyl group.

When an aryl, heteroaryl, cycloalkyl, alkylcycloalkyl, heteroalkylcycloalkyl, heterocycloalkyl, aralkyl or heteroaralkyl group contains more than one ring, these rings may be bonded to each other via a single or double bond or these rings may be annulated.

The rings of any cycloalkyl aryl group, heterocycloalkyl aryl group, cycloalkyl heteroaryl group and heterocycloalkyl heteroaryl group may be bonded to each other via a single or double bond or these rings may be annulated.

It should be appreciated that certain compounds of formula (I) may have tautomeric forms from which only one might be specifically mentioned or depicted in the following description, different geometrical isomers (which are usually denoted as cis/trans isomers or more generally as (E) and (Z) isomers) or different optical isomers as a result of one or more chiral carbon atoms (which are usually nomenclatured under the Cahn-Ingold-Prelog or R/S system). All these tautomeric forms, geometrical or optical isomers (as well as racemates and diastereomers) and polymorphous forms are included in the invention. Since the compounds of formula (I) may contain asymmetric C-atoms, they may be present either as achiral compounds, mixtures of diastereomers, mixtures of enantiomers or as optically pure compounds. The present invention comprises both all pure enantiomers and all pure diastereomers, and also the mixtures thereof in any mixing ratio.

According to a further embodiment of the present invention, one or more hydrogen atoms of the compounds of the present invention may be replaced by deuterium. Deuterium modification improves the metabolic properties of a drug with little or no change in its intrinsic pharmacology. Deuterium substitution at specific molecular positions improves metabolic stability, reduces formation of toxic metabolites and/or increases the formation of desired active metabolites. Accordingly, the present invention also encompasses the partially and fully deuterated compounds of formula (I). The term hydrogen also encompasses deuterium.

The therapeutic use of compounds according to formula (I), their salts (especially their pharmacologically acceptable salts), solvates and hydrates, respectively, as well as formulations and pharmaceutical compositions also lie within the scope of the present invention.

The present invention further provides pharmaceutical compositions comprising one or more compounds described herein or a salt (especially a pharmaceutically acceptable salt), solvate or hydrate thereof, optionally in combination with one or more carrier substances and/or one or more adjuvants.

The present invention further provides a compound or a pharmaceutical composition as described herein for use in the prophylaxis, decolonization and treatment of a *Staphylococcus aureus* infection; especially for use in the prophylaxis and treatment of pneumonia caused by *Staphylococcus aureus.*

The present invention moreover provides a compound or a pharmaceutical composition as described herein for the preparation of a medicament, especially for use in the prophylaxis, decolonization and treatment of a *Staphylococcus aureus* infection; especially for use in the prophylaxis and treatment of pneumonia caused by *Staphylococcus aureus.*

Examples of pharmacologically acceptable salts of sufficiently basic compounds are salts of physiologically acceptable mineral acids like hydrochloric, hydrobromic, sulfuric and phosphoric acid; or salts of organic acids like methanesulfonic, p-toluenesulfonic, lactic, acetic, trifluoroacetic, citric, succinic, fumaric, maleic and salicylic acid. Further, a sufficiently acidic compound may form alkali or earth alkali metal salts, for example sodium, potassium, lithium, calcium or magnesium salts; ammonium salts; or organic base salts, for example methylamine, dimethylamine, trimethylamine, triethylamine, ethylenediamine, ethanolamine, choline hydroxide, meglumin, piperidine, morpholine, tris-(2-hydroxyethyl)amine, lysine or arginine salts; all of which are also further examples of salts of the compounds described herein.

The compounds described herein may be solvated, especially hydrated. The hydratization/hydration may occur during the process of production or as a consequence of the hygroscopic nature of the initially water-free compounds. The solvates and/or hydrates may e.g. be present in solid or liquid form.

In general, the compounds and pharmaceutical compositions described herein will be administered by using the known and acceptable modes known in the art.

For oral administration such therapeutically useful agents can be administered by one of the following routes: oral, e.g. as tablets, dragees, coated tablets, pills, semisolids, soft or hard capsules, for example soft and hard gelatine capsules, aqueous or oily solutions, emulsions, suspensions or syrups, parenteral including intravenous, intramuscular and subcutaneous injection, e.g. as an injectable solution or suspension, rectal as suppositories, by inhalation or insufflation, e.g. as a powder formulation, as microcrystals or as a spray (e.g. liquid aerosol), transdermal, for example via an transdermal delivery system (TDS) such as a plaster containing the active ingredient or intranasal. For the production of such tablets, pills, semisolids, coated tablets, dragees and hard, e.g. gelatine, capsules the therapeutically useful product may be mixed with pharmaceutically inert, inorganic or organic excipients as are e.g. lactose, sucrose, glucose, gelatine, malt, silica gel, starch or derivatives thereof, talc, stearinic acid or their salts, dried skim milk, and the like. For the production of soft capsules one may use excipients as are e.g. vegetable, petroleum, animal or synthetic oils, wax, fat, and polyols. For the production of liquid solutions, emulsions or suspensions or syrups one may use as excipients e.g. water, alcohols, aqueous saline, aqueous dextrose, polyols, glycerin, lipids, phospholipids, cyclodextrins, vegetable, petroleum, animal or synthetic oils. Especially preferred are lipids and more preferred are phospholipids (preferred of natural origin; especially preferred with a particle size between 300 to 350 nm) preferred in phosphate buffered saline (pH = 7 to 8, preferred 7.4). For suppositories one may use excipients as are e.g. vegetable, petroleum, animal or synthetic oils, wax, fat and polyols. For aerosol formulations one may use compressed gases suitable for this purpose, as are e.g. oxygen, nitrogen and carbon dioxide. The pharmaceutically useful agents may also contain additives for conservation, stabilization, e.g. UV stabilizers, emulsifiers, sweetener, aromatizers, salts to change the osmotic pressure, buffers, coating additives and antioxidants.

In general, in the case of oral or parenteral administration to adult humans weighing approximately 80 kg, a daily dosage of about 1 mg to about 10,000 mg, preferably from about 5 mg to about 1,000 mg, should be appropriate, although the upper limit may be exceeded when indicated. The daily dosage can be administered as a single dose or in divided doses, or for parenteral administration, it may be given as continuous infusion or subcutaneous injection.

### EXAMPLES

### Abbreviations and Acronyms

Abbreviations and Acronyms used in the description of the chemistry and in the Examples that follow are:
- aq.: aqueous
- Ar: argon
- Boc: tert-Butyloxycarbonyl
- br.: broad
- CDCl₃: deuterated chloroform
- CD₃OD: deuterated methanol
- CHCl₃: chloroform
- cHex: cyclohexane
- conc.: Concentrated
- cpd.: compound
- CuCl: cuprous chloride
- Copper(I) iodide: cuprous iodide
- d: doublet
- D₂O: deuterated water
- DCM: dichloromethane
- de-Boc: Boc-deprotection
- deprot.: deprotection
- DIPEA: Diisopropylethylamine
- DME: dimethoxyethane
- DMSO: dimethylsulfoxide
- DMSO-d₆: deuterated dimethylsulfoxide
- ES: electrospray
- Et₂NH: diethylamin
- Et₂O: diethylether
- EtOAc: ethyl acetate
- EtOH: ethanol
- FA: formic acid
- FCS: fetal calf serum
- h: hour
- HBBS: Hanks's Balanced Salt Solution
- HCl: hydrochloric acid
- HEPES: 4-(2-hydroxyethyl)-1-piperazineethanesulfonic acid
- HPLC: high performance liquid chromatography
- H₂O: water
- H₂SO₄: sulfuric acid
- hydrog.: hydrogenation
- K₂CO₃: potassium carbonate
- KOH: potassium hydroxide
- K₃PO₄: potassium triphosphate
- LDH: lactate dehydrogenase
- m: multiplet
- MeCN: acetonitrile
- MeOH: methanol
- MgSO₄: magnesium sulfate
- min: minutes
- MS: mass spectrometry
- NaH: sodium hydride
- NaHCO₃: sodium hydrogencarbonate
- NaCl: sodium chloride
- NaOD: deuterated sodium hydroxide
- NaOH: sodium hydroxide
- Na₂SO₄: sodium sulfate
- Na₂S₂O4: sodium dithionite
- NBS: N-bromosuccinimide
- NH₃: ammonia
- NH₄HCO₃: ammonium bicarbonate
- NIS: N-lodsuccinimide
- NMR: nuclear magnetic resonance
- PBS: Phosphate Buffered Saline
- Pd₂(dba)₃: Tris-(dibenzylidenaceton)-dipalladium(0)
- Pd(OAc)₂: palladium diacetate
- Pd(PPh₃)₂Cl₂: Bis(triphenylphosphin)palladium(II)-dichloride
- Pd(PPh₃)₄: Tetrakis(triphenylphosphine)palladium(0)
- pet-ether: petroleum ether
- PPh₃: triphenylphosphine
- q: quartet
- quint: quintet
- rpm: rounds per minute
- r. t.: room temperature
- s: singlet
- sat.: saturated
- SnCl₂*2H₂O: stannous chloride dihydrate
- SOCl₂: thionyl chloride
- t: triplet
- TBS: tert-butyldimethylsilyl
- TFA: trifluoroacetic acid
- TIPS: triisopropylsilyl
- UPLC: Ultra Performance Liquid Chromatography
- wt: weight
- Xantphos: (9,9-Dimethyl-9H-xanthene-4,5-diyl)bis(diphenylphosphane)

### 1. Methods of making the compounds of formula (I) of the present invention

In general, the compounds of formula (I) used of the invention might be prepared by standard techniques known in the art, by known processes analogous thereto, and/or by the processes described herein, using starting materials which are either commercially available or producible according to conventional chemical methods. The particular processes to be utilised in the preparation of the compounds of formula (I) of this invention depends upon the specific compound desired. Such factors as the type of substitution at various locations of the molecule and the commercial availability of the starting materials play a role in the path to be followed and in the chosen reaction conditions for the preparation of the specific compounds of formula (I) of this invention. Those factors are readily recognised by one of ordinary skill in the art.

The following preparative methods are presented to aid the reader in the synthesis of the compounds of the present invention.

### 2. Experimental procedures

### LC-MS method

HPLC - electrospray mass spectra (HPLC ES-MS) were obtained using a Waters Acquity Ultra Performance Liquid Chromatography (UPLC) equipped with a SQ 3100 Mass detector spectrometer.

| | |
|---|---|
| Column: | Acquity UPLC BEH C18 1.7 µm, 2.1 x 50 mm |
| Flow: | 0.500 mL/min |
| Eluents: | A: H₂O with 0.05% formic acid and B: MeCN with 0.05% formic acid. |
| Gradient: | elution from 5% to 100% B over 3.5 min with an initial hold of 0.5 min and |

a final hold at 100% B of 0.5 min. Total run time: 5 min.

The gradient described could be altered in function of the physico-chemical properties of the compound analysed and is in no way restrictive.

### Preparative HPLC method

Preparative HPLC was performed using a Waters System consisting of a Waters 2767 Sample Manager, a Waters 2545 Binary Gradient Module, a Waters SFO (System Fluidics Organizer), a Waters 3100 Mass Detector, and a Waters 2498 UV/Visible Detector.

| | |
|---|---|
| Column: | XBridge^{®} Prep C18 5 µm OBD^{™}, 19 x 150 mm |
| Flow: | 20 mL/min |
| Eluents: | A: H₂O with 0.1% TFA and B: MeCN with 0.1% TFA. |

Alternatively, preparative HPLC was performed using a Waters System consisting of 2707 Autosampler and waters 2998 PDA detector supported by Empower Software. LC-MS - electrospray mass spectra (UPLC ES-MS) were obtained using a Waters Acquity Ultra Performance Liquid Chromatography (UPLC) equipped with a SQ detector-2 supported by Masslynx Software.

| | |
|---|---|
| Column: | KROMOSIL-C18 (150*25MM), 7u |
| Flow: | 25.0 mL/min |
| Eluents: | H₂O with 10mM NH₄HCO₃ and B: MeCN |

Alternatively, preparative HPLC was performed using an Agilent System consisting of an Agilent Infinity 1260 Autosampler, an Agilent Infinity 1260 Binary Gradient Module, an Agilent 6120 Quadrupole Mass Detector and an Agilent Infinity 1260 DAD VL UV/Visible Detector.

| | |
|---|---|
| Column: | XBridge^{®} BEH Prep C18 5 µm, 19 mm X 150 mm |
| Flow: | 32 mL/min |
| Eluents: | A: H₂O with 0.1% TFA and B: MeCN with 0.1% TFA. |

General Gradient: elution from X% to Y% B over 20 min with an initial hold of 2 min and a final increase to 100% B over 2 min and hold at 100% B of 2 min followed by a 1 min gradient back to the initial composition. Total run time: 26 min. X = Y - 30% where Y = concentration of elution for the above described LC-MS method.

The gradient described could be altered in function of the physico-chemical properties of the compound analyzed and is in no way restrictive.

### Accurate Mass method

High resolution masses were obtained using Maxis II TM HD mass spectrometer (Bruker).

### NMR methods

Proton (¹H) nuclear magnetic resonance (NMR) spectra were measured with an Oxford Varian 400/54 (400MHz) spectrometer or a Bruker Avance II (300MHz) spectrometer, or with a Bruker Avance III (500MHz) spectrometer with residual protonated solvent (CHCl3 δ 7.26; MeOH δ 3.30; DMSO δ 2.49) as standard. The NMR data of the synthesized examples are in agreement with their corresponding structural assignments.

### 2.1 Experimental Examples of the Invention

### 2.1.1. Synthetic methods

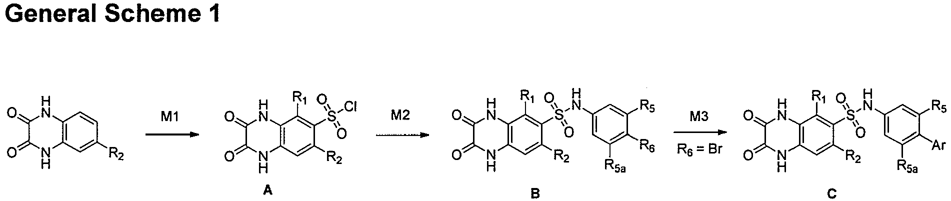

The majority of the compounds of the invention were synthesised according to general scheme 1 described above, where **M1** is a chlorosulfonylation reaction of a commercially available 6-substituted quinoxaline-2,3(1H,4H)-dione to give a sulfonyl chloride of formula **A.** The sulfonamide formation is the coupling step **M2** between sulfonyl chloride **A** and a commercially available aniline derivative to give compounds of formula **B.** When substituent R⁶ is an amine, the non-commercially available anilines **E** were synthesised from a fluoro- or chloro-nitrobenzene derivative as described in general scheme 2. When substituent R⁶ is a halogen, a further Suzuki coupling could be performed to yield biaryls of formula **C,** where R⁶ is an aryl or hereroaryl group, identified by -Ar in general scheme 1. Similarly, a Buchwald reaction could be performed to obtain tertiary anilines, as an alternative to what highlighted in general scheme 2. It should be apparent to a person skilled in the art that the sequence of the synthetic steps is dependent on starting materials availability and functional group compatibility and could vary from compound to compound. In particular, steps **M2** and **M3** could easily be reversed to obtain in a first instance a biarylaniline or a para-substituted dianiline intermediate, which could then be reacted with sulphonyl chlorides A to obtain the final compounds of formula C. Similar conditions as for described methods **M2** and **M3** can be applied.

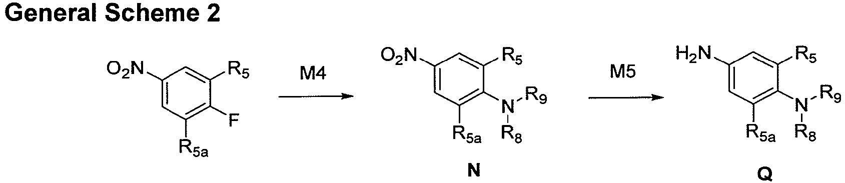

The following specific examples are presented to illustrate the invention, but they should not be construed as limiting the scope of the invention in any way. In the tables listing the intermediates, the compounds might have characterization such as (M+H)⁺ mass spectrometry data, HPLC purity and / or NMR. When the route to final compounds C encompasses different reactions steps as those described in General Scheme 1, the synthetic procedure is also exemplified below.

### 2.1.2. Preparation of intermediate compounds of formula (A)

### Intermediate 1A - synthesis according to Method 1 (M1)

### 7-methyl-2,3-dioxo-1,2,3,4-tetrahydroquinoxaline-6-sulfonyl chloride (1A)

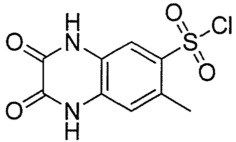

Chlorosulfonic acid (953 µl, 14.2 mmol) was added to 1,4-dihydro-6-methylquinoxaline-2,3-dione (500 mg, 2.8 mmol) and stirred at 100 °C for 1.5 h. The solution was cooled down to r. t. and poured onto ice. The suspension was filtered and then washed with ice-H₂O. The product was dried over night to yield the desired product **1A** (509 mg, 95%) as a yellow solid.

¹H NMR (400 MHz, DMSO-*d*₆) δ 11.82 (s, 1H), 11.77 (s, 1H), 7.59 (s, 1H), 6.83 (s, 1H), 2.44 (s, 3H).

MS (ES) C₉H₇ClN₂O₄S requires: 274, found: 275 (M+H)⁺, 95%.

The following sulphonyl chloride intermediates were synthesised in a similar manner as described in Method **M1:**

**Table 1 - Sulfonyl chloride intermediates of Formula A**

| 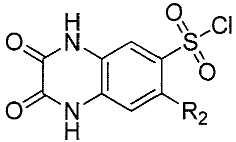 | | |
|---|---|---|
| Intermediate | R₂ | (M+H)⁺, purity |
| 2A | -F | MS (ES) C₈H₄ClFN₂O₄S requires: 278, found 279 (M+H)⁺, 96% |
| 3A | -Cl | MS (ES) C₈H₄Cl₂N₂O₄S requires: 294, found 295 (M+H)⁺, 100% |
| 4A | -Br | MS (ES) C₈H₄ClBrN₂O₄S requires: 340, found 339 (M-H)⁻, 95% |
| 5A | -Et | MS (ES) C₁₀H₉ClN₂O₄S requires: 288, found 289 (M+H)⁺, 94% |
| 6A | -*i*Pr | MS (ES) C₁₁H₁₁ClN₂O₄S requires: 302, found 303 (M+H)⁺, 95% |

| Intermediate | R² | (M+H)⁺, purity |
|---|---|---|
| 7A | -OMe | CAS: 959-01-3 |
| 8A | -OCF₃ | Crude: ~85% purity by ¹H NMR |
| 12A | -CD₃ | Crude: ~80% purity by ¹H NMR |

The following sulphonyl chlorides intermediates were synthesised with different methods:

### Intermediate 9A

### 7-nitro-2,3-dioxo-1,2,3,4-tetrahydroquinoxaline-6-sulfonyl chloride (9A)

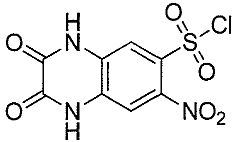

A stirred mixture of conc. HNO₃ (10 mL, 65%) and conc. H₂SO₄ (20 mL, 96%) was cooled in ice bath below 5 °C. 2,3-dioxo-1,2,3,4-tetrahydroquinoxaline-6-sulfonyl chloride (1 g, 3.8 mmol) was carefully added keeping the temperature below 5 °C. The reaction mixture was stirred for 1 h at 0 - 5 °C and then for 2 h at r. t. The reaction mixture was poured onto ice and extracted with EtOAc. The combined organic phases were washed with H₂O, sat. NaHCO₃ solution and again H₂O, dried over Na₂SO₄, filtered, and concentrated *in vacuo* to yield the desired product **9A** (750 mg, 65%), which was used in the following step without further purification.

¹H NMR (500 MHz, DMSO-*d*₆) δ 12.14 (s, 1 H), 12.08 (s, 1 H), 7.65 (s, 1 H), 7.26 (s, 1 H).

### Intermediate 10A

### 5-fluoro-7-methylquinoxaline-2,3(1H,4H)-dione (10D)

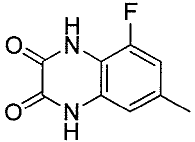

3-fluoro-5-methyl-benzene-1,2-diamine (200 mg, 1.40 mmol) was added to an oven-dried microwave vial, followed by diethyl oxalate (2 mL) and the mixture was heated to 185 °C for 4 h. The reaction was allowed to cool down to r. t., diluted with of Et₂O, the obtained solids were filtered, washed with Et₂O and dried on air to afford the desired product **(D)** (169 mg, 61%) as a brown solid.

¹H NMR (300 MHz, DMSO-*d*₆) δ 11.94 (s, 2H), 6.84 (d, *J* = 11.6 Hz, 1H), 6.72 (s, 1H), 2.26 (s, 3H).

MS (ES) C₉H₇FN₂O₂ requires: 194, found: 195 (M+H)⁺, 95%.

### 5-fluoro-7-methyl-2,3-dioxo-1,2,3,4-tetrahydroquinoxaline-6-sulfonyl chloride (10A)

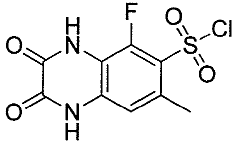

Intermediate **10D** (100 mg, 0.515 mmol) was added to an oven-dried microwave vial followed by chlorosulfonic acid (0.35 mL), and the mixture was heated to 65 °C for 16 h. Thionyl chloride (123 mg, 1.03 mmol) was then added to the reaction mixture and further stirred at 65 °C for 5 h. The reaction mixture was poured onto to an ice-H₂O and the precipitated solids were collected by filtration, washed with H₂O and then dried on air to give the corresponding product as a mixture of isomers: ~26% of desired compound **(10A)** (minor isomer) and ~73% of the undesired product **(11A)** (major isomer). The mixture was used in the following step without attempt at separating the regioisomers.

### 5-fluoro-2,3-dihydroxy-7-methyl-quinoxaline-6-sulfonyl chloride (minor isomer - desired product):

¹H NMR (300 MHz, DMSO-*d*₆) δ 12.73 (s, 2H), 6.64 (s, 1H), 2.47 (s, 3H).

MS (ES) C₁₁H₁₂FN₃O₄S requires: 301, found: 302 (M+H)⁺, ~30% (derivatization was used for LC/MS measurement to avoid hydrolysis. The compound was converted into dimethyl sulfonamide (M+H⁺=301)).

### 8-Fluoro-2,3-dihydroxy-6-methyl-quinoxaline-5-sulfonyl chloride (major isomer - undesired product):

¹H NMR (300 MHz, DMSO-*d*₆) δ 11.93 (s, 2H), 6.92 (d, J = 11.8 Hz, 1H), 2.54 (s, 3H). MS (ES) C11H12FN3O4S requires: 301, found: 302 (M+H)⁺, ~68% (derivatization was used for LC/MS measurement to avoid hydrolysis. The compound was converted into dimethyl sulfonamide (M+H⁺=301)).

### Synthesis of deuterated intermediate 12D

Deuterated building block **12D** was synthesised according to scheme 3:

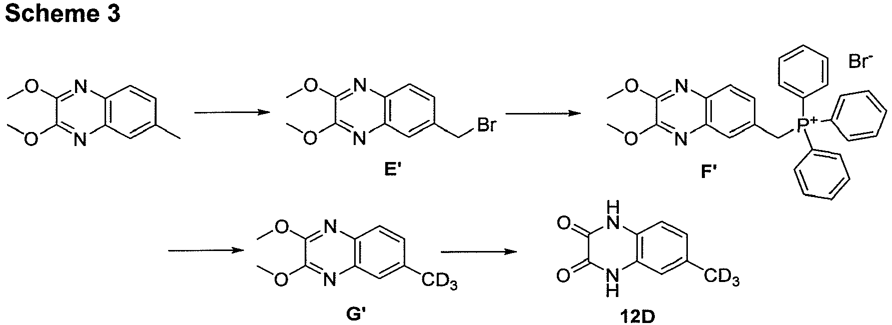

### 6-(Bromomethyl)-2,3-dimethoxyquinoxaline (E')

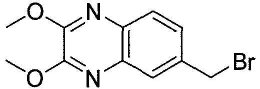

A mixture of 2,3-dimethoxy-6-methylquinoxaline (2.8 g, 13.7 mmol), NBS (2.7 g, 15.1 mmol) and benzoyl peroxide (350 mg, 25% H₂O) in absolute CHCl₃ without stabilizer (90 mL) was heated under reflux for 16h. The reaction was allowed to cool down to r. t. and the solvents were reduced *in vacuo.* The residue was purified by column chromatography on silica gel using a gradient of EtOAc in pet-ether to yield the desired product **(E')** (2.78 g, 72%) as a white solid.

MS (ES) C₁₁H₁₁BrN₂O₂ requires: 282/284, found: 283/285 (M+H)⁺, 90%.

### ((2,3-Dimethoxyquinoxalin-6-yl)methyl)triphenylphosphonium bromide (F')

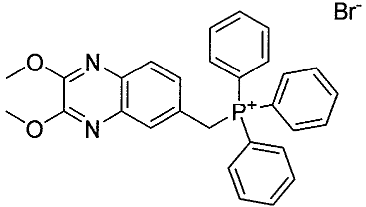

A mixture of 6-(bromomethyl)-2,3-dimethoxyquinoxaline **(E')** (2.8 g, 9.8 mmol) and PPh₃ (2.6 g, 9.8 mmol) in toluene (20 mL) was heated at reflux for 4 h The reaction was allowed to cool down to r. t. and the solid was collected by filtration, washed with toluene and dried under vacuum at 50°C to yield the desired compound **(F')** (4.1 g, 77%) as a white solid.

¹H NMR (700 MHz, DMSO-ds): 7.93 - 7.89 (m, 3H), 7.77 - 7.68 (m, 12H), 7.58 (d, *J =* 8.4 Hz, 1H), 7.43 (t, *J =* 2.0 Hz, 1H), 7.05 (dt, *J* = 8.4, 2.0 Hz, 1H), 5.35 (d, J_{P-H} = 15.6 Hz, 2H), 4.01 (s, 3H), 3.96 (s, 3H). Purity: 90%.

### 2,3-Dimethoxy-6-(methyl-d³)quinoxaline (G')

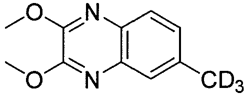

To a solution of ((2,3-dimethoxyquinoxalin-6-yl)methyl)triphenylphosphonium bromide **(F')** (4.1 g, 7.5 mmol) in THF (20 mL) was added a solution of NaOD in D₂O (10 mL, 40 w/w %). The reaction mixture was stirred at 25°C for 18 h. EtOAc and H2O were added to the reaction mixture. The organic layer was separated, dried over Na₂SO₄ and reduced *in vacuo.* The residue was purified by column chromatography in pet-ether to yield the desired product **(G')** (1.08 g, 69%) as a white solid.

MS (ES) C₁₁H₉D₃N₂O₂ requires: 207, found: 208 (M+H)⁺, 95%.

### 6-(Methyl-d³)-1,4-dihydroquinoxaline-2,3-dione (12D)

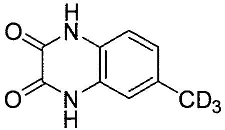

2 M HCl (15 mL) was added to a solution of 2,3-dimethoxy-6-(methyl-d³)quinoxaline **(G')** in dioxane, and the reaction was heated at 80 °C for 16 h. The mixture was allowed to cool down to r. t. and dioxane was reduced *in vacuo.* The resulting precipitate was filtered, washed with water and dried *in vacuo* to yield the desired product **12D** (847 mg, 90%) as a white solid.

MS (ES) C₉H₅D₃N₂O₂ requires: 179, found: 180 (M+H)⁺, 99%.

### Intermediate 1B - synthesis according to Method 2a (M2a)

### N-(4-bromo-3-chlorophenyl)-7-methyl-2,3-dioxo-1,2,3,4-tetrahydroquinoxaline-6-sulfonamide (75 - 1B)

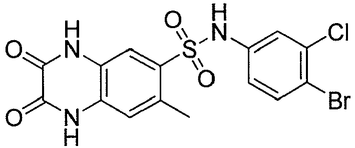

To a mixture of sulfonyl chloride **1A** (327 mg, 1.19 mmol) in dry pyridine (1.2 mL) 4-bromo-3-chloroanilin (270 mg, 1.31 mmol) was added and stirred at r. t. After 1.5 h the mixture was diluted with a 1M aq. HCl solution and extracted with DCM. The combined organic phases were dried on MgSO₄, filtered and evaporated *in vacuo.* The crude product was purified by flash chromatography on silica gel using a gradient of EtOAc in cHex to yield the desired product **(75** - **1B)** (272 mg, 100%) as a light brown solid.

¹H NMR (400 MHz, DMSO-*d*₆) δ 12.05 (s, 1H), 11.90 (s, 1H), 10.80 (s, 1H), 7.70 (s, 1H), 7.58 (d, J = 8.7 Hz, 1H), 7.23 (d, J = 2.5 Hz, 1H), 6.97 (s, 1H), 6.91 (dd, J = 8.8, 2.6 Hz, 1H), 2.48 (s, 3H).

MS (ES) C₁₅H₁₁BrClN₃O₄S requires: 445, found: 446 (M+H)⁺, 100%

### Compound 222 - synthesis according to Method 2 (M2b)

### 7-methyl-2,3-dioxo-N-(5-(trifluoromethoxy)pyridin-2-yl)-1,2,3,4-tetrahydroquinoxaline-6-sulfonamide (222)

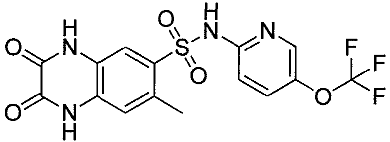

Sulfonyl chloride **1A** (30 mg, 0.109 mmol) and 5-(trifluoromethoxy)pyridin-2-amine (23.3 mg, 0.131 mmol) were dissolved in dry THF (1.6 mL). NaH (60% in mineral oil, 21.8 mg, 0.546 mmol) was added at once and the mixture was and stirred at r. t. for 1 h. The mixture was diluted with a sat. NH₄Cl aq. solution, extracted with EtOAc and washed with H₂O. The combined organic phases were dried on MgSO₄, filtered and evaporated *in vacuo.* The crude product was purified by preparative HPLC using a gradient of MeCN in H₂O with 0.1% TFA to yield the desired product (222) (16.2 mg, 37%) as a white powder.

¹H NMR (400 MHz, DMSO-*d*₆) δ 12.09 (s, 1H), 11.95 (s, 1H), 11.63 (s, 1H), 8.36 (s, 1H), 7.84 (s, 1H), 7.15 (s, 1H), 6.99 (s, 1H), 2.52 (s, 3H).

MS (ES) C₁₅H₁₁F₃N₄O₅S requires: 416, found: 417 (M+H)⁺, 100%

### Compound 1C - synthesis according to Method 3 (M3)

### N-(2-chloro-4'-fluoro-[1,1'-biphenyl]-4-yl)-7-methyl-2,3-dioxo-1,2,3,4-tetrahydroquinoxaline-6-sulfonamide (3 - 1C)

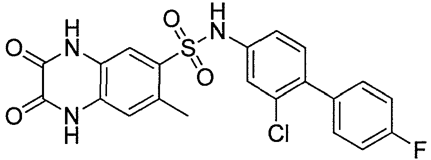

Intermediate **1B** (30.0 mg, 0.067 mmol), 4-fluorophenylboronic acid (18.9 mg, 0.135 mmol), K₂CO₃ (18.6 mg; 0.135 mmol) and Pd(PPh₃)₄ (1.56 mg, 0.0013 mmol) were suspended in DME/H₂O (2:1, 2mL) and heated at 120 °C for 1.5 h in a microwave. After cooling to r. t., the mixture was filtered and evaporated *in vacuo.* The crude product was purified by reverse phase flash chromatography on C18 using a gradient of MeCN in H₂O to yield the desired product **(3 - 1C)** (8.8mg, 28%) as a white solid.

¹H NMR (300 MHz, DMSO-*d*₆) δ 12.13 (s, 1 H), 11.99 (s, 1H), 10.86 (s, 1H), 7.81 (s, 1 H), 7.38 (ddd, *J* = 8.6, 5.5, 2.6 Hz, 2H), 7.34 - 7.17 (m, 4H), 7.09 (dd, *J* = 8.4, 2.3 Hz, 1H), 7.03 (s, 1H), 2.55 (s, 3H).

MS (ES) C₂₁H₁₅ClFN₃O₄S requires: 459, found: 458 M-H⁺, 100%.

### Synthesis of 2,3-Dioxo-N-(4-(trifluoromethoxy)phenyl)-7-trifluoromethyl-1,2,3,4-tetrahydroquinoxaline-6-sulfonamide (190)

### 4-Amino-5-nitro-2-(trifluoromethyl)benzenesulfonyl chloride (1E)

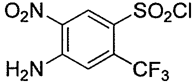

2-Nitro-5-(trifluoromethyl)aniline (1.0 g, 4.9 mmol) was added to chlorosulfonic acid (10 mL) at r. t. The reaction mixture was stirred for 5 h at 115 °C, upon which it was allowed to cool down to r. t. and was poured onto ice. The aq. layer was extracted with EtOAc. The combined organic phases were washed with H₂O, dried over Na₂SO₄, filtered and concentrated *in vacuo* to yield the crude product **(1E)**, which was used in the following step without further purification.

### 4-Amino-5-nitro-N-(4-(trifluoromethoxy)phenyl)-2-(trifluoromethyl)benzenesulfonamide (1F)

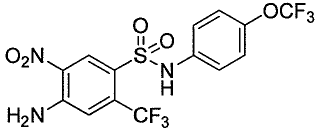

4-(trifluoromethoxy)aniline (1.8 g, 10.2 mmol) was reacted with crude sulfonyl chloride **D** according to method **M2** to yield the desired product **(1F)** (724 mg, 33% over 2 steps).

¹H NMR (500 MHz, DMSO-*d*₆) δ 10.66 (s, 1 H), 8.64 (s, 1 H), 8.32 (br. s, 2 H), 7.61 (s, 1 H), 7.28 (d, *J* = 8.6 Hz, 2 H), 7.18 (d, *J =* 8.6 Hz, 2 H).

MS (ES) C₁₄H₁₀F₆N₃O₅S requires: 445, found: 446 (M+H)⁺.

### 4,5-Diamino-N-(4-(trifluoromethoxy)phenyl)-2-(trifluoromethyl)benzenesulfonamide (1G)

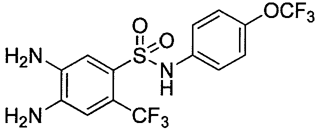

To a solution of intermediate **1F** (700 mg, 1.57 mmol) in EtOH (10 mL) was added SnCl₂*2H₂O (1.1 g, 5 mmol) and conc. HCl (17 mL). The reaction mixture was stirred for 30 min at 75 °C and cooled to r. t. pH-value was adjusted to 13-14 using 40% aq. KOH. The mixture was extracted with EtOAc, and the combined organic phases were dried over Na₂SO₄, filtered and concentrated *in vacuo* to yield the desired product **1G** (660 mg, ~100%), which was used in the following step without further purification.

MS (ES) C₁₄H₁₂F₆N₃O₃S requires: 415, found: 416 (M+H)⁺.

### 2,3-Dioxo-N-(4-(trifluoromethoxy)phenyl)-7-trifluoromethyl-1,2,3,4-tetrahydroquinoxaline-6-sulfonamide (190)

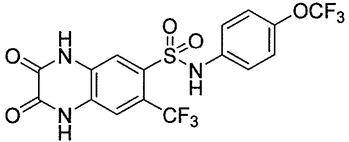

To a suspension of the crude dianiline **1G** (660 mg, ca. 1.57mmol) in HCl (4 N, 10 mL) was added oxalic acid (180 mg, 2 mmol) and HCl (4 N, 5 mL). The mixture was stirred at 130 ⁰C for 2.5 h, upon which it was allowed to cool down to r. t. The mixture was extracted with EtOAc, and the combined organic phases were dried over Na₂SO₄, filtered, and concentrated *in vacuo.* The residue was purified by two subsequent column chromatographies: the first column on silica gel using a gradient of MeOH in DCM, the second on reverse phase C18 silica using a gradient of MeCN in H₂O to yield the desired product **(190)** (100 mg, 13% over 2 steps).

¹H NMR (500 MHz, DMSO-*d*₆) δ 12.27 (br. s, 2 H), 10.87 (s, 1 H), 7.86 (s, 1 H), 7.59 (s, 1 H), 7.29 (d, *J* = 8.9 Hz, 2 H), 7.18 (d, *J = 8.9* Hz, 2 H).

HRMS (ESI) calcd. for C₁₆H₁₀F₆N₃O₅S (M+H)⁺ 470.0245, found 470.0241.

### Synthesis of 7-Cyclopropyl-2,3-dioxo-N-(4-(trifluoromethoxy)phenyl)-1,2,3,4-tetrahydroquinoxaline-6-sulfonamide (194)

### 2-Bromo-4-fluoro-5-nitroaniline (H)

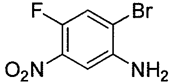

2-Bromo-4-fluoroaniline (3.0 g, 15.8 mmol) was carefully added to conc. H₂SO₄ (30 mL), and the mixture was stirred at 30 °C for 1 h. The reaction mixture was cooled to - 5 - -10 °C (ice/NaCl bath) and KNO₃ (1.7 g, 16.6 mmol) was added in batches. The reaction mixture was stirred at 0 °C for 3 h, poured into ice-H₂O and extracted with EtOAc. The combined organic phases were washed with aq. NaHCO₃ and H₂O, dried over Na₂SO₄, filtered and concentrated *in vacuo.* The crude product was purified by flash chromatography on silica gel using a gradient of EtOAc in petroleum ether to yield the desired product **(H)** (2.2 g, 59%).

MS (ES) C₆H₅BrFN₂O₂ requires: 234/236, found: 235/237 (M+H)⁺.

### 2-Cyclopropyl-4-fluoro-5-nitroaniline (J)

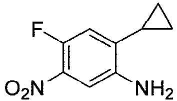

A mixture of bromo derivative **H** (1.2 g, 5 mmol), cyclopropyl boronic acid (560 mg, 6.5 mmol), Pd(OAc)₂ (113 mg, 0.5 mmol, 10 %), tricyclohexylphosphine (210 mg, 1 mmol) and K₃PO₄ (3.7 g, 17.5 mmol) was evacuated and backfilled with Ar three times, then H₂O (2 mL) and toluene (24 mL) were added. The mixture was further degassed with Ar and stirred at 100 °C for 12 h under Ar atmosphere, upon which it was allowed to cool down to r. t. EtOAc was added, and the organic layer was washed with brine, dried over Na₂SO₄, filtered and concentrated *in vacuo.* The residue was purified by flash chromatography on silica gel using a gradient of EtOAc in petroleum ether to yield the desired product **(J)** (687 mg, 70%).

¹H NMR (500 MHz, DMSO-*d*₆): 7.30 (d, *J*_{H-F} = 6.8 Hz, 1 H), 6.89 (d, *J*_{H-F} = 12.8 Hz, 1 H), 5.53 (s, 2 H), 1.85 - 1.76 (m, 1 H), 1.00 - 0.93 (m, 2 H), 0.71 - 0.63 (m, 2 H).

¹⁹F NMR (470 MHz, DMSO-*d*₆) δ -135.29.

### 2-Cyclopropyl-4-fluoro-5-nitrobenzenesulfonyl chloride (K)

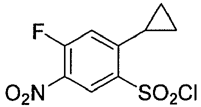

In a first flask, intermediate J (687 mg, 3.5 mmol) was dissolved in conc. HCl (8 mL), and the resulting solution was cooled to -5 °C, using an ice/NaCl bath. A solution of sodium nitrite (276 mg, 4 mmol) in distilled H₂O (5 mL) was added in portions with stirring, while maintaining the temperature below 0 °C. The mixture was then kept at this temperature. In a second flask, SOCl₂ (1.8 mL, 3 g, 25 mmol) was added dropwise to distilled H₂O (12 mL), which had been pre-cooled to -5 °C using an ice/NaCl bath. The resulting solution was allowed to warm to r. t., CuCl (50 mg, 0.5 mmol) was added, and the reaction mixture was re-cooled to -5 °C. With continued cooling and stirring, the contents of the first flask were added in small portions to the contents of the second flask, and the mixture was stirred for 1 h at -5 °C. The mixture was then extracted with EtOAc, and the combined organic phases were dried over Na₂SO₄, filtered, and concentrated *in vacuo,* to yield the desired product **(K)** (860mg, 88%), which was used in the following step without further purification or characterization.

### 2-Cyclopropyl-4-fluoro-5-nitro-N-(4-(trifluoromethoxy)phenyl)benzenesulfonamide (L)

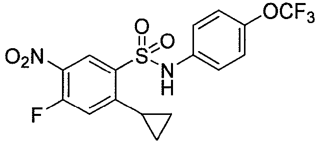

4-(trifluoromethoxy)aniline (549 mg, 3.1 mmol) was reacted with crude sulfonyl chloride **J** (860 mg, 3.1 mmol) according to method **M2** to yield the desired product (L) (697 mg, 54%).

¹H NMR (500 MHz, DMSO-ds): 11.04 - 10.88 (br. s, 1 H), 8.56 (d, *J*_{H-F} = 7.7 Hz, 1 H), 7.26 (d, *J =* 9.2 Hz, 2 H), 7.24 (d, *J*_{H-F} = 13.0 Hz, 1 H), 7.18 (d, *J* = 9.2 Hz, 2 H), 2.79 - 2.69 (m, 1 H), 1.25 - 1.20 (m, 2 H), 1.01 - 0.96 (m, 2 H).

¹⁹F NMR (470 MHz, DMSO-*d*₆) δ -57.10.

### 4-Amino-2-cyclopropyl-5-nitro-N-(4-(trifluoromethoxy)phenyl)benzenesulfonamide (2F)

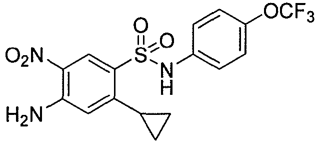

Sat. aq. NH₃ (10 mL) was added to the solution of fluoro derivative **L** (697 mg, 1.66 mmol) in EtOH (5 mL) at r. t. The mixture was stirred at r. t. overnight. The solvent was removed under reduced pressure and the mixture was extracted with EtOAc, dried over Na₂SO₄, filtered and concentrated *in vacuo.* The residue was purified by column chromatography on silica gel using a gradient of EtOAc in pet-ether to yield the desired product **(2F)** (390 mg, 56%).

¹H NMR (500 MHz, DMSO-*d*₆) δ 10.57 (s, 1 H), 8.53 (s, 1 H), 7.76 (s, 2 H), 7.26 (d, *J* = 9.2 Hz, 2 H), 7.15 (d, *J* = 9.2 Hz, 2 H), 6.57 (s, 1 H), 2.57 - 2.51 (m, 1 H), 1.15 - 1.09 (m, 2 H), 0.68 - 0.63 (m, 2 H).

¹⁹F NMR (470 MHz, DMSO-*d*₆): -57.09.

MS (ES) C₁₆H₁₅F₃N₃O₅S requires: 417, found: 418 (M+H)⁺.

### 4,5-Diamino-2-cyclopropyl-N-(4-(trifluoromethoxy)phenyl)benzenesulfonamide (2G)

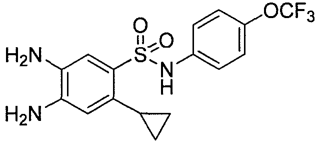

To a solution of nitro derivative **2F** (390 mg, 0.94 mmol) in dioxane (12 mL) at 10 °C was added a suspension of SnCl₂*2H₂O (1.05 g, 4.68 mmol) in conc. HCl (2 mL). The mixture was stirred for 6.5 h at r. t., neutralized with 40% NaOH and extracted with EtOAc. The combined organic phases were dried over Na₂SO₄, filtered and concentrated *in vacuo* to yield the desired product **(2G)** (464 mg, 27% excess wt), which was used in the following step without further purification.

MS (ES) C₁₆H₁₇F₃N₃O₅S requires: 387, found: 388 (M+H)⁺.

### 7-Cyclopropyl-2,3-dioxo-N-(4-(trifluoromethoxy)phenyl)-1,2,3,4-tetrahydroquinoxaline-6-sulfonamide (194)

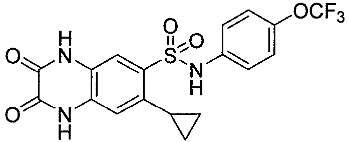

The crude product dianiline **2G** (232 mg, ca. 0.47 mmol) was dissolved in diethyl oxalate (2 mL). The mixture was heated to 120 °C and stirred for 2 h. The reaction mixture was allowed to cool to r. t. and separated by two subsequent column chromatographies: the first column on silica gel using a gradient of MeOH in DCM, the second on reverse phase C18 silica using a gradient of MeCN in H₂O to yield the desired product **(194)** (43 mg, 21%).

¹H NMR (500 MHz, DMSO-*d*₆) δ 11.93 (s, 1 H), 11.90 (s, 1 H), 10.64 (s, 1 H), 7.76 (s, 1 H), 7.26 (d, *J* = 9.1 Hz, 2 H), 7.14 (d, *J* = 9.1 Hz, 2 H), 6.71 (s, 1 H), 2.63 - 2.55 (m, 1 H), 1.10 - 1.04 (m, 2 H), 0.60 - 0.55 (m, 2 H).

¹⁹F NMR (470 MHz, DMSO-*d*₆) δ -57.08.

MS (ES) C₁₈H₁₅F₃N₃O₅S requires: 441, found: 442 (M+H)⁺.

### Intermediate 1N - synthesis according to Method 4 (M4) 4-(2-chloro-4-nitrophenyl)morpholine (1N)

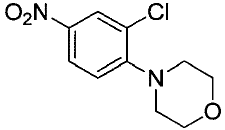

Morpholine (100 µL, 1.14 mmol) and 2-chloro-1-fluoro-4-nitrobenzene (100mg, 0.570 mmol) were dissolved in DMSO (1 mL), and K₂CO₃ (157 mg, 1.14 mmol) was added. The mixture was shaken at 105 °C for 5.5 h, followed by cooling down to r. t. The mixture was diluted with H₂O and extracted with EtOAc. The combined organic phases were dried on MgSO₄, filtered and evaporated *in vacuo.* The crude product was purified by flash chromatography on silica gel using a gradient of EtOAc in cHex to yield the desired product **(1N)** (114 mg, 82%) as a yellow solid.

MS (ES) C₁₀H₁₁ClN₂O₃ requires: 242, found: 243 (M+H)⁺, 100%.

### Intermediate 1Q - synthesis according to Method 5 (M5) 3-chloro-4-morpholinoaniline (1Q)

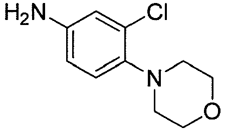

Nitro-derivative **1N** (96 mg, 0.396 mmol) was dissolved in EtOH (6 mL), and Fe (110 mg, 1.978 mmol) was added, followed by a 2 M HCl solution (1 mL). The mixture was stirred at 100 °C for 1 h, and allowed to cool down to r. t. The mixture was diluted with EtOAc and washed with a sat. NaHCO₃ solution. The aqueous phase was extracted once more with EtOAc, and the combined organic phases were dried on MgSO₄, filtered over celite and evaporated *in vacuo* to yield the desired product **(1Q)** (83 mg, 99%) as a brown powder.

¹H NMR (400 MHz, DMSO-*d*₆) δ 6.89 (dd, J = 8.6, 0.8 Hz, 1H), 6.63 (dd, J = 2.5, 0.8 Hz, 1H), 6.49 (ddd, J = 8.6, 2.6, 0.9 Hz, 1H), 5.04 (s, 2H), 3.73 - 3.64 (m, 4H), 2.83 - 2.75 (m, 4H).

MS (ES) C₁₀H₁₃ClN₂O requires: 212, found: 213 (M+H)⁺, 96%.

The following anilines intermediates were synthesised in a similar manner as described in Method **M4** and **M5:**

**Table 1a - Anilines intermediates of Formula Q**

| 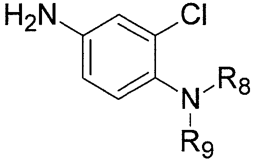 | | |
|---|---|---|
| Intermediate | R₉-N-R₈ | (M+H)⁺ |
| 2Q | 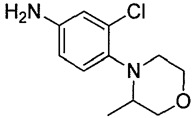 | MS (ES): C₁₁H₁₅ClN₂O requires: 226, found: 227 (M+H)⁺. |
| 3Q | 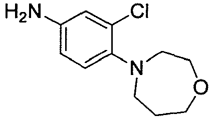 | MS (ES): C₁₁H₁₅ClN₂O requires: 226, found: 227 (M+H)⁺. |
| 4Q | 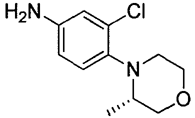 | MS (ES): C₁₁H₁₅ClN₂O requires: 226, found: 227 (M+H)⁺. |
| 5Q | 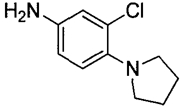 | MS (ES): C₁₀H₁₃ClN₂ requires: 196, found: 197 (M+H)⁺. |
| 6Q | 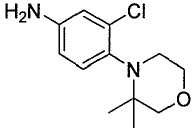 | MS (ES): C₁₂H₁₇ClN₂O requires: 240, found: 241 (M+H)⁺. |

### 4-amino-2-fluoro-5-nitrobenzenesulfonyl chloride (3E)

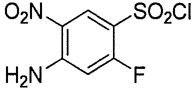

5-fluoro-2-nitroaniline (2.00 g, 12.8 mmol) was added portionwise to chlorosulfonic acid (10 mL). After stirring for 4 h at 120 °C the solution was cooled down to 0 °C and poured onto ice-H₂O. The mixture was extracted with EtOAc. The combined organic phases were dried over Na₂SO₄, filtered and concentrated *in vacuo* to yield the desired product **(3E)** (2.95 g, 90%) as a brown oil.

¹H-NMR (500 MHz, DMSO-*d*₆) δ 8.28 (d, ⁴J_{H-F} = 7.5 Hz, 1H), 6.68 (d, ³J_{H-F} = 11.8 Hz, 1H).

### 4-amino-2-fluoro-5-nitro-N-(4-(trifluoromethoxy)phenyl)benzenesulfonamide (3F)

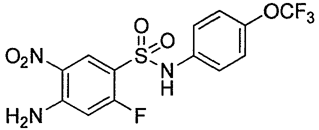

Dry pyridine (1.4 mL, 17.3 mmol) was added to a solution of 4-(trifluoromethoxy)-aniline (1.55 mL, 11.6 mmol) in dry DCM (10 mL) under Ar atmosphere. A solution of intermediate **3E** (2.95 g, 11.6 mmol) in dry DCM (40 mL) was added over a period of 20 min at 0 °C using a metal cannula. The reaction mixture was allowed to warm to r. t. and stirred for 13 h. The solvents were reduced *in vacuo* and H₂O was added to the residue and the mixture was extracted with EtOAc. The combined organic phases were dried over Na₂SO₄, filtered and concentrated *in vacuo.* The crude was purified by flash chromatography on silica gel using a gradient of acetone in DCM, followed by recrystallization using a mixture of acetone and DCM to yield the desired product **(3F)** (1.66 g, 36 %) as yellow needles.

¹H-NMR (500 MHz, DMSO-*d*₆) δ 10.75 (s, 1H), 8.41 (d, ⁴J_{H-F} = 7.6 Hz, 2H), 8.15 (br. s, 2H), 7.29 (m, 2H), 7.19 (m, 2H), 6.82 (d, ³J_{H-F} = 12.4 Hz, 1H).

MS (ES) C₁₃H₉F₄N₃O₅S requires: 395, found 396 (M+H)⁺.

### 7-amino-8-nitro-2-(4-(trifluoromethoxy)phenyl)-3,4-dihydro-2H-benzo[b][1,4,5]oxathiazepine 1,1-dioxide (R)

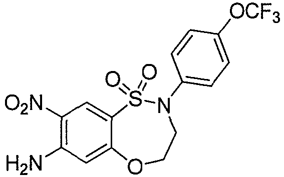

Intermediate **3F** (400 mg, 1.01 mmol) was dissolved in dry DMF (12 mL) and added to previously *in vacuo* flame dried K₂CO₃ (442 mg, 3.20 mmol) under Ar atmosphere. 2-lodoethanol (0.7 mL, 8.96 mmol) was added and the reaction mixture was stirred for at 50 °C for 24 h. The solvents were reduced *in vacuo.* After addition of H₂O the pH of the mixture was adjusted to 4 with 1 M HCl. The precipitated solid was dissolved in EtOAc, and the mixture was washed with a half-saturated NaCl solution. The organic phase was dried, filtered, and reduced *in vacuo* to yield the alkylated intermediate. The latter was dissolved in dry DMF (5 mL) and Cs₂CO₃ (658 mg, 2.02 mmol) was added.

The reaction mixture was stirred at 80 °C for 6 h, upon which the solvent was reduced *in vacuo.* H₂O was added and the pH of the mixture was adjusted to 6 with 1 M HCl. The precipitated solid was dissolved in EtOAc and the organic phase washed with half-saturated NaCl solution. The organic phase was dried over Na₂SO₄, filtered, and the solvent reduced *in vacuo.* The crude was purified by flash chromatography on silica gel using a gradient of EtOAc in petroleum ether to yield the desired product **(R)** (326 mg, 77 %) as a yellow solid.

¹H-NMR (700 MHz, DMSO-*d*₆) δ 8.24 (s, 1H), 8.05 (br. s, 2H), 7.37 (s, 4H), 6.81 (s, 1H), 4.37 (m, 2H), 4.06 (m, 2H).

MS (ES) C₁₅H₁₂F₃N₃O₆S requires: 419, found 420 (M+H)⁺.

### 7,8-diamino-2-(4-(trifluoromethoxy)phenyl)-3,4-dihydro-2H-benzo[b][1,4,5]oxathiazepine 1,1-dioxide (S)

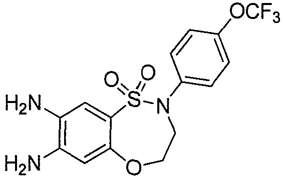

Intermediate R (150 mg, 0.357 mmol) was dissolved in dioxane (5 mL) and conc. NH₃ solution (0.6 mL) was added. Na₂S₂O₄ (790 mg, 4.54 mmol) was dissolved in H₂O (8 mL) and added dropwise to the reaction solution. After stirring for 4.5 h at r. t., the organic solvents were reduced *in vacuo* and H₂O was added. The pH of the aq. phase was adjusted to 6 with 1 M HCl and the latter was extracted with EtOAc. The combined organic phases were dried over Na₂SO₄, filtered, and reduced *in vacuo* to yield the desired product **(S)** (111 mg, 80%) as a beige solid.

¹H-NMR (500 MHz, DMSO-*d*₆) δ 7.35 (m, 2H), 7.27 (m, 2H), 6.79 (s, 1H), 6.36 (s, 1H), 5.48 (br. s, 2H), 4.68 (br. s, 2H), 4.08 (m, 2H), 4.00 (m, 2H).

MS (ESI) C₁₃H₁₄F₃N₃O₄S requires: 389, found 390 (M+H)⁺.

### 2-(4-(trifluoromethoxy)phenyl)-3,4,7,10-tetrahydro-2H-[1,4,5]oxathiazepino[2,3-g]quinoxaline-8,9-dione 1,1-dioxide (237)

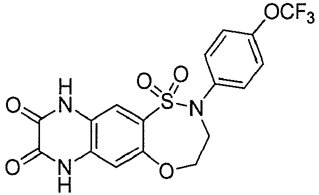

A mixture of dianiline **S** (110 mg, 0.283 mmol) and dimethyl oxalate (1.57 g, 13.3 mmol) was stirred at 120 °C for 4 h, upon which the it was allowed to cool down to r. t. and dissolved in MeOH. The solvents were reduced *in vacuo* and the residue was purified by preparative HPLC using a gradient of MeCN in H₂O to yield the desired product **(237)** (104 mg, 83 %) as a white solid.

¹H-NMR (500 MHz, DMSO-*d*₆) δ 12.07 (br. s, 1H), 11.97 (br. s, 1H), 7.42 (s, 1H), 7.35 (m, 2H), 7.29 (m, 2H), 6.99 (s, 1H), 4.29 (m, 2H), 4.09 (m_{c}, 2H).

MS (ESI) C₁₇H₁₂F₃N₃O₆S requires: 443, found 444 (M+H)⁺.

Dimethylamine derivative **221** was also synthesized from intermediate **3F** using the procedure described below.

### 4-amino-2-(dimethylamino)-5-nitro-N-(4-(trifluoromethoxy)phenyl)benzenesulfonamide (4F)

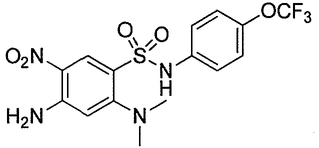

Intermediate **3F** (182 mg, 0.460 mmol) was dissolved in dry DMF (5 mL) and DIPEA (280 µL, 1.65 mmol) was added, followed by a 2 M N,N-dimethylamine solution in THF (500 µL, 1 mmol). The solution was stirred at 100 °C for 4 h and the solvent was removed *in vacuo.* The residue was dissolved in EtOAc and washed with H₂O. The organic layer was dried over Na₂SO₄, filtered, and reduced *in vacuo.* The residue was purified by flash chromatography on silica gel using a gradient of EtOAc in petroleum ether to yield the desired product **4F** (188 mg, 97 %) as a yellow solid.

¹H-NMR (500 MHz, DMSO-*d*₆) δ 10.26 (s, 1H), 8.50 (s, 1H), 7.73 (br. s, 2H), 7.22 (m_{c}, 2H), 7.11 (m_{c}, 2H), 6.51 (s, 1H), 2.74 (s, 6H).

MS (ESI) C₁₅H₁₅F₃N₄O₅S requires: 420, found 421 (M+H)⁺.

### 4,5-diamino-2-(dimethylamino)-N-(4-(trifluoromethoxy)phenyl)benzenesulfonamide (4G)

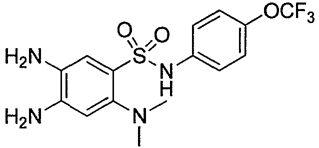

Intermediate **4F** (93.3 mg, 0.222 mmol) was dissolved in 2.5 mL 1,4-dioxane and conc. aq. NH₃ solution (0.3 mL, ca. 33%) was added. Na₂S₂O₄ (445 mg, 2.56 mmol) was dissolved in deionized H₂O (4 mL) and added dropwise to the starting material. The mixture was stirred for 3 h at r. t., upon which the organic solvents were reduced *in vacuo.* The pH value was adjusted to 6 with 1 M HCl. The precipitated solid was extracted with EtOAc, and the combined organic layers were dried over Na₂SO₄, filtered and reduced *in vacuo* to yield the desired product **4G** (77.2 mg, 89%) as a pale pink solid, which was used in the following step without further purification.

¹H-NMR (500 MHz, DMSO-d₆) δ 9.59 (br. s, 1H), 7.17 (m_{c}, 4H), 7.01 (s, 1H), 6.49 (s, 1H), 5.24 (s, 2H), 4.64 (s, 2H), 2.47 (s, 6H).

MS (ESI) C₁₅H₁₇F₃N₄O₃S requires: 390, found 391 (M+H)⁺.

The following dianilines intermediates were synthesised in a similar manner as described for intermediate **4G.** Reaction conditions and bases employed were dependent on functional group compatibility and operator, and could vary from compound to compound, as should be apparent to a person skilled in the art.

**Table 1b - dianilines intermediates of Formula G**

| 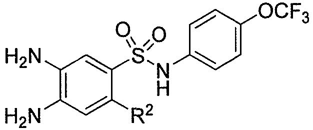 | | |
|---|---|---|
| Intermediate | R² | (M+H)⁺ |
| 5G | 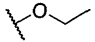 | MS (ES): C₁₅H₁₅F₃N₃O₄S requires: 391, found: 392 (M+H)⁺. |
| 6G | 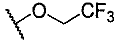 | MS (ES): C₁₅H₁₃F₆N₃O₄S requires: 445, found: 446 (M+H)⁺. |
| 7G | 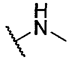 | MS (ES): C₁₄H₁₅F₃N₄O₃S requires: 376, found: 377 (M+H)⁺. |
| 8G | 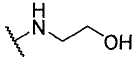 | MS (ES): C₁₃H₁₇F₃N₄O₄S requires: 406, found: 407 (M+H)⁺. |
| 9G | 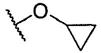 | MS (ES): C₁₆H₁₆F₃N₃O₄S requires: 403, found: 404 (M+H)⁺. |

### 7-(dimethylamino)-2,3-dioxo-N-(4-(trifluoromethoxy)phenyl)-1,2,3,4-tetrahydroquinoxaline-6-sulfonamide (221)

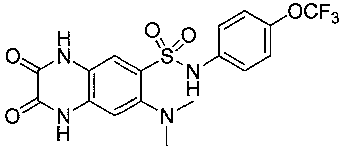

Dianiline **4G** (77.2 mg, 0.198 mmol) and 1,1'-oxalyldiimidazole (49.8 mg, 0.262 mmol), were dissolved in dry THF (5 mL) under Ar atmosphere. After stirring for 2 h at 50 °C, additional 1,1'-oxalyldiimidazole (19.4 mg, 102 µmol) was added to the reaction. The solution was stirred for 1 h at 50 °C, upon which the volatiles were removed *in vacuo* and the residue purified by reverse phase chromatography on C18 using a gradient of MeCN in H₂O, to yield the desired product **221** (49.6 mg, 56 %) as a white solid.

¹H-NMR (500 MHz, DMSO-*d*₆) δ 11.98 (s, 1H), 11.93 (s, 1H), 10.13 (s, 1H), 7.68 (s, 1H), 7.24-7.20 (m, 2H), 7.20-7.17 (m, 2H), 7.08 (s, 1H), 2.56 (s, 6H).

MS (ESI) C₁₇H₁₅F₃N₄O₅S requires: 444, found 445 (M+H)⁺.

An exemplified hydrogenation procedure to obtain compound **306** from compound **299** is described below. A similar procedure was employed to reduce compound **274** to **284** and compound **297** to compound **304.**

### N-(3-bromo-4-cyclohexylphenyl)-7-methyl-2,3-dioxo-1,2,3,4-tetrahydroquinoxaline-6-sulfonamide (306)

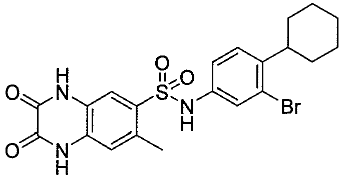

To a stirred solution of **299** (150 mg, 0.30 mmol) in MeOH was added 10% Pt-C (300 mg) and the mixture was stirred at RT for 16 h under H₂ balloon atmosphere. The suspension was filtered through a celite pad and the pad was washed with MeOH. The filtrate was concentrated under reduced pressure and the crude product was purified by prep HPLC by using NH₄HCO₃ in H₂O: acetonitrile as an eluent. The compound containing fractions were concentrated and dried to yield the desired product **306** (22 mg, 14%) as an off-white solid.

¹H NMR (400 MHz, DMSO) δ: 12.08 (s, 1H), 11.95 (s, 1H), 10.56 (s, 1H), 7.74 (s, 1H), 7.24 (d, *J* =2.0 Hz, 1H), 7.20-7.19 (m, 1H), 7.05-7.0 (m, 2H), 2.80-2.65 (m, 1H), 2.50 (s, 3H), 1.88-1.55 (m, 5H), 1.45-1.10 (m, 5H).

MS (ESI) C₂₁H₂₂BrN₃O₄S requires: 493, found 490 (M-H)⁻.

Occasionally, Boc-protected boronic acids or boronic esters were employed in the Suzuki coupling described in **M3.** An exemplified Boc-deprotection procedure to obtain final compound **311** is described below. A similar procedure was employed to yield compounds **342** and **343.**

### N-(3'-amino-2-chloro-[1,1'-biphenyl]-4-yl)-7-methyl-2,3-dioxo-1,2,3,4-tetrahydroquinoxaline-6-sulfonamide (311)

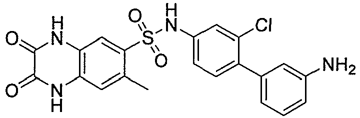

To a stirred solution of tert-butyl (2'-chloro-4'-((7-methyl-2,3-dioxo-1,2,3,4-tetrahydroquinoxaline)-6-sulfonamido)-[1,1'-biphenyl]-3-yl)carbamate (40 mg, 0.07 mmol) in DCM (0.50 mL) was added 4M HCl in 1, 4-dioxane (0.20 mL) at 0 °C. The reaction was stirred at RT for 16 h. The mixture was concentrated *in vacuo* and the residue was washed with Et₂O (2 mL), pentane (2 mL). The solvents were removed *in vacuo* to yield the desired product **311** (16 mg, 8.7%) as an off-white solid, HCl salt.

¹H NMR (400 MHz, DMSO) δ: 12.12 (s, 1H), 11.97 (s, 1H), 10.84 (s, 1H), 7.80 (s, 1H), 7.37-7.30 (m, 1H), 7.27-7.23 (m, 2H), 7.11-7.08 (m, 1H), 7.04-6.95 (m, 4H), 2.55 (s, 3H).

MS (ESI) C₂₁H₁₇ClN₄O₄S requires: 456, found 455 (M-H)⁻.

To obtain the anilines required for the synthesis of compounds **250** and **255,** 2-Bromo-4'-fluoro-[1,1'-biphenyl]-4-amine obtained from standard method **M3** was Boc-protected, following which a Buchwald reaction was performed. Boc-deprotection yielded the required aniline.

### tert-Butyl (2-bromo-4'-fluoro-[1,1'-biphenyl]-4-yl)carbamate (1S):

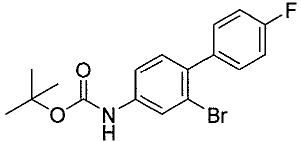

To a stirred solution of 2-bromo-4'-fluoro-[1,1'-biphenyl]-4-amine (1.0 g, 3.76 mmol) in THF (10 mL) at RT were added DIPEA (0.5 mL) and Boc₂O (0.9 mL, 3.76 mmol). The reaction was stirred for 16 h at 80 °C. The mixture was concentrated under reduced pressure and the residue was purified by flash chromatography on silica gel using a gradient of EtOAc in petroleum, followed by by prep-HPLC using a gradient of 0.1% HCOOH in H₂O in MeOH to yield the desired product **1S** (600 mg, 43%) as a brown solid.MS (ESI)

MS (ESI) C₁₇H₁₇BrFNO₂ requires: 365, found 310 [M-*t*Bu+H]⁺.

### tert-Butyl (4'-fluoro-2-morpholino-[1,1'-biphenyl]-4-yl)carbamate (1T)

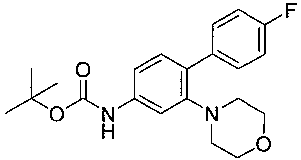

To a degassed solution of intermediate **1S** (200 mg, 0.546 mmol), morpholine (95 mg, 1.09 mmol) and t-BuONa (105 mg, 1.09 mmol) in toluene (5 mL) were added Pd₂(dba)₃ (50 mg, 0.054 mmol) and Xantphos (63 mg, 0.10 mmol) at rt. The reaction was stirred for 16 h at 100 °C, upon which the mixture was cooled to rt, quenched with water and extracted with EtOAc. The combined organic layer was dried over anhydrous Na₂SO₄, filtered and the filtrate was concentrated *in vacuo.* The residue was purified by flash chromatography on silica gel, using a gradient of EtOAc in pet-ether as an eluent to afford the desired compound **1T** (70 mg, 34%) as a brown solid.

MS (ESI) C₂₁H₂₅FN₂O₃ requires: 372, found 373 [M+H]⁺.

### 4'-fluoro-2-morpholino-[1,1'-biphenyl]-4-amine (1U):

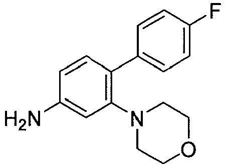

4N HCl in 1, 4-dioxane (0.5 mL) were added to a stirred solution of intermediate **1T** (70 mg, 1.05 mmol) in DCM (3 mL) at 0 °C, and the reaction was stirred for 16 h at rt. The mixture was concentrated *in vacuo* and the residue was washed with Et₂O and dried to yield the desired product **1U** (80 mg, excess weight). The crude compound was used in the following step without further purification.

MS (ESI) C₁₆H₁₇FN₂O requires: 272, found 273 [M+H]⁺.

**Table 1c -anilines intermediates of Formula T**

| Intermediate | Formula | (M+H)⁺ |
|---|---|---|
| 2U | 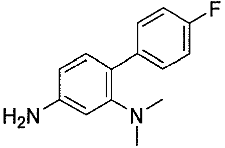 | MS (ESI) C₁₄H₁₅FN₂ requires: 230, found 231 [M+H]⁺. |

When the final compound contained one or more acetylene groups, silyl-group protections and deprotections were introduced to improve conversion and facilitate isolation of the intermediates. All or parts of the below-described route, leading to compound **251** were employed. It should be apparent to a person skilled in the art that the sequence of the synthetic steps, as well as reaction conditions and the protecting groups employed, are dependent on starting materials availability, functional group compatibility and operator, and could vary from compound to compound.

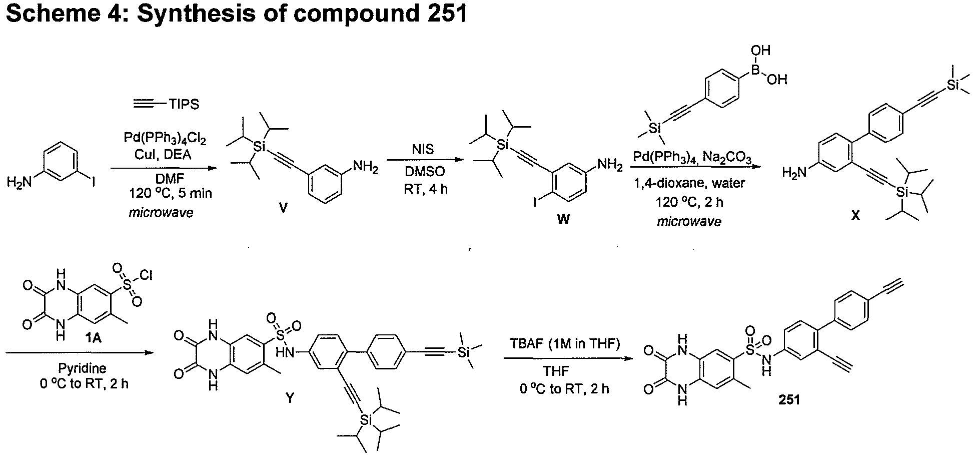

### 3-((triisopropylsilyl)ethynyl)aniline (1V):

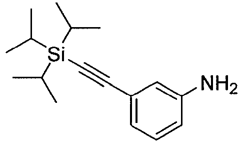

To a degassed solution of 3-iodoaniline (2.0 g, 9.13 mmol) and (triisopropylsilyl)-acetylene (1.8 g, 10.04 mmol) in DMF (10 mL) at r. t. were added Et₂NH (14 mL, 137.0 mmol), Cul (70 mg, 0.365) and Pd(PPh₃)₂Cl₂ (128 mg, 0.183 mmol). The resulting reaction mixture was degassed with Ar for 15 min and stirred for 5 min at 150 °C in microwave. The reaction was allowed to cool to rt, and quenched with H₂O, and extracted with Et₂O. The combined organic layers were dried over anhydrous Na₂SO₄, filtered and reduced *in vacuo.* The residue was purified by column chromatography on silica gel using a gradient of EtOAc in pet-ether to yield the desired product **(1V)** (1.8 g, 72%) as a brown gum.

MS (ESI) C₁₇H₂₇NSi requires: 273, found 274 [M+H]⁺.

### 4-lodo-3-((triisopropylsilyl)ethynyl)aniline (1W):

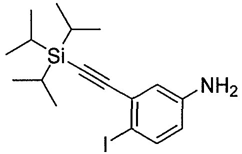

NIS (2.1 g, 3.66 mmol) was added to a stirred solution of intermediate **1V** (2.5 g, 9.15 mmol) in DMSO (25 mL) and the reaction was stirred at r. t. under Ar atmosphere for 4 h. The mixture was poured into cold water and extracted with EtOAc. The combined organic layers were washed with brine, dried over anhydrous Na₂SO₄, filtered, and concentrated *in vacuo.* The residue was purified by column chromatography on silica gel using a gradient of EtOAc in pet-ether to yield the desired product **1W** (1.6 g, 44%) as a brown gum.

MS (ESI) C₁₇H₂₆INSi requires: 399, found 400 [M+H]⁺.

### 2-((triisopropylsilyl)ethynyl)-4'-((trimethylsilyl)ethynyl)-[1,1'-biphenyl]-4-amine (1X):

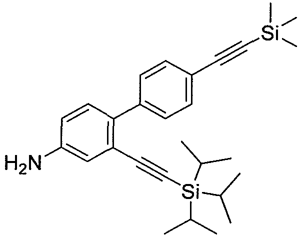

Compound **1W** (300 mg, 0.88 mmol) and (4-((trimethylsilyl)ethynyl)phenyl)boronic acid (174 mg, 0.79 mmol) were dissolved in dioxane:H₂O (5:1, 6 mL) and degassed Na₂CO₃ (188 mg, 1.76 mmol) and Pd(PPh₃)₄ (31 mg, 0.02 mmol) were added at rt. The reaction was degassed with Ar for 15 min and stirred at 120 °C for 2 h in the microwave. The mixture was allowed to coolto rt, quenched with H₂O, and extracted with EtOAc. The combined organic layers were dried over anhydrous Na₂SO₄, filtered and reduced *in vacuo.* The residue was purified by column chromatography on silica gel using a gradient of EtOAc in pet-ether to yield the desired product **1X** (210 mg, 63%) as a pale yellow gum.

MS (ESI) C₂₈H₃₉NSi₂ requires: 445, found 446 [M+H]⁺.

### 7-methyl-2,3-dioxo-N-(2-((triisopropylsilyl)ethynyl)-4'-((trimethylsilyl)ethynyl)-[1,1'-biphenyl]-4-yl)-1,2,3,4-tetrahydroquinoxaline-6-sulfonamide (1Y)

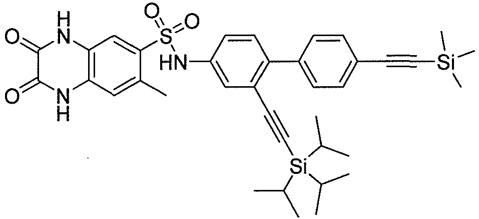

To a stirred solution of compound **1X** (170 mg, 0.38 mmol) in pyridine (3 mL) at 0 °C was added intermediate **1A** (118 mg, 0.45 mmol) and the reaction was stirred at r. t. for 2 h. The mixture was quenched with cold water and extracted with EtOAc. The combined organic layers were washed with brine, dried over anhydrous Na₂SO₄, filtered and reduced *in vacuo.* The crude product was purified by prep-HPLC using a gradient of NH₄HCO₃ in H₂O in ACN to yield the desired product **1Y** (15 mg, 5%) as an off-white solid.

MS (ESI) C₃₇H₄₅N₃O₄SSi₂ requires: 683, not seen

### N-(2,4'-diethynyl-[1,1'-biphenyl]-4-yl)-7-methyl-2,3-dioxo-1,2,3,4-tetrahydroquinoxaline-6-sulfonamide (251)

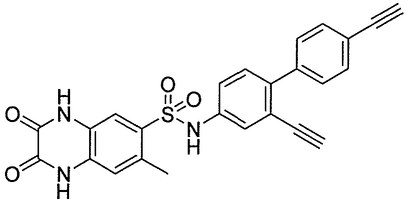

TBAF (1M in THF, 0.05 mL, 0.05 mmol) was added to a stirred solution of compound **1X** (15 mg, 0.022 mmol) in THF (2 mL) at 0 °C. The reaction for 2 h at rt. The mixture was quenched with cold water and extracted with EtOAc. The combined organic layers were washed with brine, dried over anhydrous Na₂SO₄, filtered and reduced *in vacuo.* The residue was purified by prep-HPLC using a gradient of NH₄HCO₃ in H₂O in ACN to yield the desired product **251** (3 mg, 33%) as an off-white solid.

MS (ESI) C₂₅H₁₇N₃O₄S requires: 455, found 454 [M-H]⁺.

A similar deprotection step was also necessary when the boronic acid building block contained a TBS-protected phenol, as is the case for compound **386.**

Further compounds exemplifying the invention are described in **Table 2.**

When not otherwise specified, it should be assumed that **M1, M2,** sometimes followed by **M3** were used to yield the target compounds. This is highlighted in the 'Synthetic Sequence' column. Occasionally, as specified in the table, a further deprotection or hydrolysis step was required to obtain the final product, as would be recognized by a person skilled in the art. It should also be apparent to a person skilled in the art that reaction conditions such as temperature, dilution, reaction time or work-up procedures, including pH adjustment, are dependent on reaction partners and functional group compatibility and could vary from compound to compound. For commercially available compounds, the CAS number is given.

**Table 2 - Compounds of formula (I) of the invention**

| | Structure | Synthetic Sequence | ¹H-NMR or CAS Number | (M+H)⁺ | (M-H)⁻ | LC purity |
|---|---|---|---|---|---|---|
| 1 | 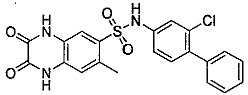 | M1, M2, M3 | ¹H NMR (400 MHz, DMSO-*d*₆) δ 12.08 (s, 1H), 11.95 (s, 1H), 10.79 (s, 1H), 7.78 (s, 1H), 7.43 - 7.29 (m, 5H), 7.26 (dd, *J* = 8.4, 0.8 Hz, 1H), 7.21 (dd, *J* = 2.2, 0.9 Hz, 1H), 7.07 (ddd, *J* = 8.5, 2.3, 0.9 Hz, 1H), 7.01 (s, 1H), 2.53 (s, 3H). | 442 | | 98% |
| 2 | 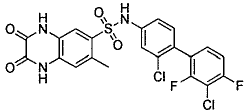 | M1, M2, M3 | ¹H NMR (400 MHz, Methanol-*d*₄) δ 7.86 (s, 1H), 7.28 (d, *J* = 2.2 Hz, 1H), 7.23 - 7.07 (m, 5H), 2.64 (s, 3H). | 512 | | 100% |
| 3 | 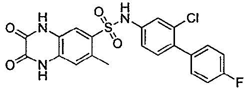 | M1, M2, M3 | ¹H NMR (300 MHz, DMSO-*d*₆) δ 12.13 (s, 1H), 11.99 (s, 1H), 10.86 (s, 1H), 7.81 (s, 1H), 7.38 (ddd, *J* = 8.6, 5.5, 2.6 Hz, 2H), 7.32 - 7.20 (m, 4H), 7.09 (dd, *J* = 8.4, 2.3 Hz, 1H), 7.03 (s, 1H), 2.55 (s, 3H). | 460 | | 100% |
| 4 | 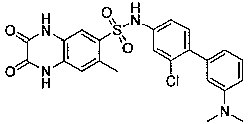 | M1, M2, M3 | ¹H NMR (300 MHz, DMSO-*d*₆) δ 12.12 (s, 1H), 11.98 (s, 1H), 10.80 (s, 1H), 7.81 (s, 1H), 7.34 - 7.15 (m, 3H), 7.13 - 7.00 (m, 2H), 6.79 (d, *J* = 8.6 Hz, 1H), 6.68 (t, *J* = 5.8 Hz, 2H), 2.91 (s, 6H), 2.56 (s, 3H). | 485 | | 97% |
| 5 | 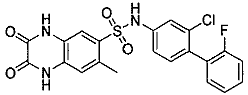 | M1, M2, M3 | ¹H NMR (400 MHz, DMSO-*d*₆) δ 12.09 (s, 1H), 11.95 (s, 1H), 10.87 (s, 1H), 7.80 (s, 1H), 7.46 - 7.38 (m, 1H), 7.30 - 7.20 (m, 5H), 7.11 - 7.06 (m, 1H), 7.02 (s, 1H), 2.54 (s, 3H). | 460 | | 97% |
| 6 | 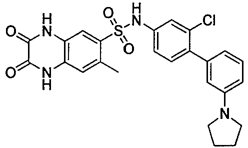 | M1, M2, M3 | ¹H NMR (300 MHz, Methanol-*d*₄) δ 7.83 (s, 1H), 7.21 (d, *J* = 2.2 Hz, 1H), 7.18 - 7.00 (m, 4H), 6.49 (dd, *J* = 7.9, 2.0 Hz, 2H), 6.41 (t, *J* = 2.0 Hz, 1H), 3.24 - 3.13 (m, 4H), 2.63 (s, 3H), 2.04 - 1.91 (m, 4H). | 511 | | 96% |
| 7 | 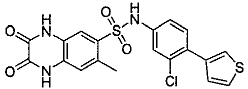 | M1, M2, M3 | ¹H NMR (300 MHz, DMSO-*d*₆) δ 12.11 (s, 1H), 11.97 (s, 1H), 10.80 (s, 1H), 7.80 (s, 1H), 7.61 (s, 2H), 7.39 (d, *J* = 8.4 Hz, 1H), 7.29 - 7.19 (m, 2H), 7.11 - 6.99 (m, 2H), 2.55 (s, 3H). | 448 | | 91% |
| 8 | 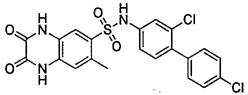 | M1, M2, M3 | ¹H NMR (300 MHz, Methanol-*d*₄) δ 8.57 (s, 1H), 8.23 - 8.15 (m, 2H), 8.15 - 8.05 (m, 2H), 8.04 - 7.96 (m, 2H), 7.85 (dd, *J* = 8.4, 2.3 Hz, 1H), 7.79 (s, 1H), 2.78 (s, 3H). | 476 | | 98% |
| 9 | 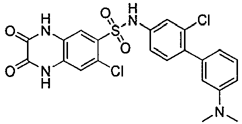 | M1, M2, M3 | ¹H NMR (400 MHz, Methanol-*d*₄) δ 7.97 (s, 1H), 7.53 - 7.47 (m, 1H), 7.37 (d, *J* = 2.2 Hz, 1H), 7.36 - 7.32 (m, 2H), 7.31 (s, 1H), 7.28 (d, *J* = 8.4 Hz, 1H), 7.25 - 7.19 (m, 2H), 3.19 (s, 6H). | 505 | | 94% |
| 10 | 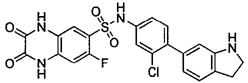 | M1, M2, M3 | ¹H NMR (400 MHz, DMSO-*d*₆) δ 12.18 (s, 1H), 11.97 (s, 1H), 10.95 (s, 1H), 7.62 (d, *J* = 6.6 Hz, 1H), 7.24 (ddd, *J* = 5.2, 3.1, 0.8 Hz, 2H), 7.17 (d, *J\* = 7.8 Hz, 1H), 7.10 (ddd, *J* = 8.5, 2.3, 0.9 Hz, 1H), 7.00 (d, *J* = 10.6 Hz, 1H), 6.73 (s, 2H), 3.52 (t, *J* = 8.2 Hz, 2H), 3.00 (t, *J* = 8.2 Hz, 2H). | 487 | | 99% |
| 11 | 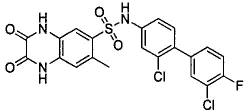 | M1, M2, M3 | ¹H NMR (400 MHz, DMSO-*d*₆) δ 12.12 (s, 1H), 11.98 (s, 1H), 10.88 (s, 1H), 7.82 (s, 1H), 7.56 (dd, *J* = 7.2, 2.2 Hz, 1H), 7.43 (d, *J* = 8.6 Hz, 1H), 7.38 - 7.30 (m, 2H), 7.24 (d, *J* = 2.2 Hz, 1H), 7.07 (s, 1H), 7.03 (d, *J* = 0.8 Hz, 1H), 2.54 (s, 3H). | | 493 | 91% |
| 12 | 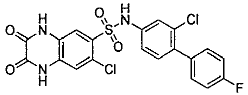 | M1, M2, M3 | ¹H NMR (400 MHz, Methanol-*d*₄) δ 7.91 (s, 1H), 7.32 - 7.23 (m, 4H), 7.20 - 7.13 (m, 2H), 7.08 - 7.01 (m, 2H). | | 480 | 90% |
| 13 | 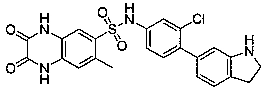 | M1, M2, M3 | ¹H NMR (300 MHz, Methanol-*d*₄) δ 7.86 (s, 1H), 7.53 - 7.47 (m, 1H), 7.41 (d, *J* = 1.2 Hz, 1H), 7.38 (dd, *J* = 7.8, 1.6 Hz, 1H), 7.28 (d, *J* = 2.2 Hz, 1H), 7.23 (d, *J* = 8.4 Hz, 1H), 7.15 - 7.10 (m, 1H), 7.10 (t, *J* = 1.2 Hz, 1H), 3.87 (t, *J* = 7.8 Hz, 2H), 3.36 (d, *J* = 7.8 Hz, 2H), 2.65 - 2.63 (m, 3H). | 483 . | | 92% |
| 14 | 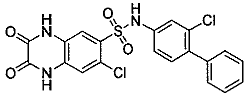 | M1, M2, M3 | ¹H NMR (400 MHz, Methanol-*d*₄) δ 7.92 (s, 1H), 7.37 - 7.27 (m, 7H), 7.19 - 7.13 (m, 2H). | 463 | | 91% |
| 15 | 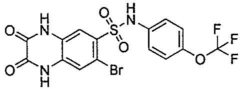 | M1, M2 | ¹H NMR (400 MHz, DMSO-*d*₆) δ 12.06 (s, 1H), 11.97 (s, 1H), 10.76 (s, 1H), 7.81 (d, *J* = 0.7 Hz, 1H), 7.32 (s, 1H), 7.20 (d, *J* = 8.6 Hz, 2H), 7.10 (d, *J* = 9.0 Hz, 2H). | | 478/480 | 99% |
| 16 | 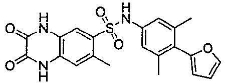 | M1, M2, M3 | ¹H NMR (400 MHz, DMSO-*d*₆) δ 12.05 (s, 1H), 11.92 (s, 1H), 10.45 (s, 1H), 7.78 (d, *J* = 0.8 Hz, 1H), 7.67 (dt, *J* = 1.8, 0.9 Hz, 1H), 6.98 (s, 1H), 6.82 (s, 2H), 6.52 (ddd, *J* = 3.0, 1.9, 0.8 Hz, 1H), 6.33 (dq, *J* = 3.3, 0.7 Hz, 1H), 2.52 (s, 3H), 1.99 (s, 6H). | 426 | | 97% |
| 17 | 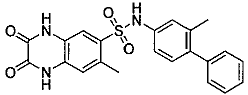 | M1, M2, M3 | ¹H NMR (400 MHz, DMSO-*d*₆) δ 12.06 (s, 1H), 11.92 (s, 1H), 10.42 (s, 1H), 7.77 (s, 1H), 7.37 - 7.26 (m, 3H), 7.25 - 7.19 (m, 2H), 7.05 - 6.97 (m, 3H), 6.93 (dd, *J* = 8.2, 2.4 Hz, 1H), 2.53 (s, 3H), 2.10 (s, 3H). | 422 | | 98% |
| 18 | 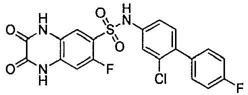 | M1, M2, M3 | ¹H NMR (400 MHz, DMSO-*d*₆) δ 12.17 (s, 1H), 11.97 (s, 1H), 10.99 (s, 1H), 7.62 (d, *J* = 6.6 Hz, 1H), 7.41 - 7.34 (m, 2H), 7.30 (d, *J* = 8.4 Hz, 1H), 7.27 - 7.18 (m, 3H), 7.12 (dd, *J* = 8.4, 2.3 Hz, 1H), 7.00 (d, *J* = 10.6 Hz, 1H). | | 462 | 99% |
| 19 | 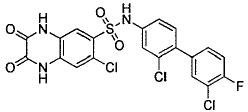 | M1, M2, M3 | ¹H NMR (400 MHz, DMSO-*d*₆) δ 12.15 (s, 1H), 12.05 (s, 1H), 11.04 (s, 1H), 7.88 (s, 1H), 7.56 (dd, *J* = 7.2, 2.2 Hz, 1H), 7.44 (dd, *J* = 9.3, 8.6 Hz, 1H), 7.37 - 7.30 (m, 2H), 7.25 (d, *J* = 2.2 Hz, 1H), 7.20 (s, 1H), 7.11 (dd, *J* = 8.4, 2.3 Hz, 1H). | | 514 | 99% |
| 20 | 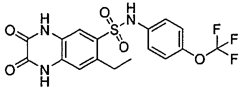 | M1, M2 | ¹H NMR (400 MHz, Methanol-*d*₄) δ 7.77 (s, 1H), 7.16 - 7.08 (m, 5H), 3.03 (q, *J* = 7.5 Hz, 2H), 1.26 (t, *J* = 7.5 Hz, 3H). | | 428 | 96% |
| 21 | 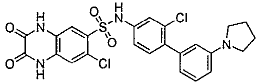 | M1, M2, M3 | ¹H NMR (400 MHz, DMSO-*d*₆) δ 12.15 (s, 1H), 12.04 (s, 1H), 10.93 (s, 1H), 7.87 (s, 1H), 7.26 (d, *J* = 8.4 Hz, 1H), 7.24 - 7.20 (m, 2H), 7.14 (t, *J* = 7.9 Hz, 1H), 7.09 (dd, *J* = 8.4, 2.3 Hz, 1H), 6.50 (dt, *J* = 8.2, 1.8 Hz, 2H), 6.40 (t, *J* = 2.0 Hz, 1H), 3.18 (s, 4H), 1.96 - 1.87 (m, 4H). | | 529 | 93% |
| 22 | 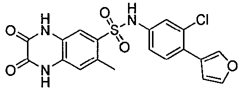 | M1, M2, M3 | ¹H NMR (400 MHz, DMSO-*d*₆) δ 12.07 (s, 1H), 11.94 (s, 1H), 10.73 (s, 1H), 7.99 (t, *J* = 1.2 Hz, 1H), 7.76 (s, 1H), 7.71 (t, *J* = 1.6 Hz, 1H), 7.43 (d, *J* = 8.5 Hz, 1H), 7.19 (d, *J* = 2.2 Hz, 1H), 7.03 (dt, *J* = 8.5, 1.6 Hz, 1H), 6.99 (s, 1H), 6.78 (dt, *J* = 1.9, 1.0 Hz, 1H), 2.52 (s, 3H). | 432 | | 100% |
| 23 | 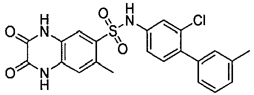 | M1, M2, M3 | ¹H NMR (400 MHz, DMSO-*d*₆) δ 12.08 (s, 1H), 11.95 (s, 1H), 10.77 (s, 1H), 7.78 (s, 1H), 7.30 - 7.23 (m, 2H), 7.21 (d, *J* = 2.2 Hz, 1H), 7.17 - 7.08 (m, 3H), 7.06 (ddd, *J* = 8.4, 2.2, 0.6 Hz, 1H), 7.01 (s, 1H), 2.53 (s, 3H), 2.29 (s, 3H). | 456 | | 98% |
| 24 | 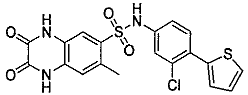 | M1, M2, M3 | ¹H NMR (400 MHz, DMSO-*d*₆) δ 12.07 (s, 1H), 11.94 (s, 1H), 10.84 (s, 1H), 7.77 (s, 1H), 7.60 (ddd, *J* = 5.1, 1.2, 0.5 Hz, 1H), 7.48 (d, *J* = 8.5 Hz, 1H), 7.31 - 7.28 (m, 1H), 7.22 (d, *J* = 2.3 Hz, 1H), 7.10 (ddd, *J* = 5.1, 3.6, 0.5 Hz, 1H), 7.06 (dd, *J* = 8.6, 2.3 Hz, 1H), 7.00 (s, 1H), 2.52 (s, 3H). | 446 | | 98% |
| 25 | 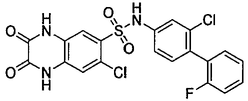 | M1, M2, M3 | ¹H NMR (400 MHz, Methanol-*d*₄) δ 7.93 (s, 1H), 7.41 - 7.33 (m, 1H), 7.32 - 7.28 (m, 2H), 7.21 - 7.08 (m, 5H). | 480 | | 91% |
| 26 | 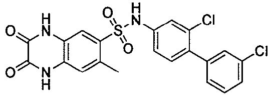 | M1, M2, M3 | ¹H NMR (400 MHz, DMSO-*d*₆) δ 12.09 (s, 1H), 11.95 (s, 1H), 10.85 (s, 1H), 7.79 (s, 1H), 7.45 - 7.39 (m, 2H), 7.39 - 7.36 (m, 1H), 7.33 - 7.26 (m, 2H), 7.22 (d, *J* = 2.2 Hz, 1H), 7.11 - 7.05 (m, 1H), 7.01 (s, 1H), 2.54 (s, 3H). | | 474 | 99% |
| 27 | 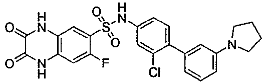 | M1, M2, M3 | ¹H NMR (400 MHz, DMSO-*d*₆) δ 12.17 (s, 1H), 11.97 (s, 1H), 10.93 (s, 1H), 7.62 (d, *J* = 6.5 Hz, 1H), 7.28 (dd, *J* = 8.4, 0.9 Hz, 1H), 7.24 (dd, *J* = 2.2, 0.9 Hz, 1H), 7.18 - 7.13 (m, 1H), 7.10 (ddd, *J* = 8.4, 2.3, 1.0 Hz, 1H), 7.01 (d, *J* = 10.5 Hz, 1H), 6.50 (dd, *J* = 8.5, 2.5 Hz, 2H), 6.40 (d, *J* = 2.4 Hz, 1H), 3.22 - 3.15 (m, 4H), 1.94 - 1.88 (m, 4H). | 515 | | 96% |
| 28 | 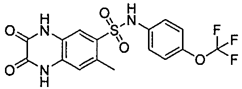 | M1, M2 | ¹H NMR (400 MHz, DMSO-*d*₆) δ 12.02 (s, 1H), 11.89 (s, 1H), 10.68 (s, 1H), 7.71 (s, 1H), 7.26 - 7.17 (m, 2H), 7.13 - 7.06 (m, 2H), 6.97 (d, *J* = 0.9 Hz, 1H), 2.49 (s, 3H). | 416 | | 99% |
| 29 | 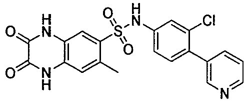 | M1, M2, M3 | ¹H NMR (300 MHz, DMSO-*d*₆) δ 12.14 (s, 1H), 12.00 (s, 1H), 10.98 (s, 1H), 8.70 - 8.60 (m, 2H), 8.01 (dt, *J* = 8.1, 1.9 Hz, 1H), 7.83 (s, 1H), 7.62 (dd, *J* = 8.0, 5.0 Hz, 1H), 7.40 (d, *J* = 8.4 Hz, 1H), 7.28 (d, *J* = 2.2 Hz, 1H), 7.14 (dd, *J* = 8.4, 2.2 Hz, 1H), 7.04 (s, 1H), 2.54 (s, 3H). | 443 | | 99% |
| 30 | 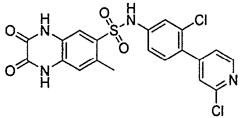 | M1, M2, M3 | ¹H NMR (400 MHz, DMSO-*d*₆) δ 12.07 (s, 1H), 10.97 (s, 1H), 8.43 (dd, *J* = 5.2, 0.7 Hz, 1H), 7.80 (s, 1H), 7.51 (dt, *J* = 1.5, 0.7 Hz, 1H), 7.44 - 7.35 (m, 2H), 7.24 (d, *J* = 2.2 Hz, 1H), 7.10 (dd, *J* = 8.5, 2.2 Hz, 1H), 7.01 (s, 1H), 2.53 (s, 3H). | 477 | | 91% |
| 31 | 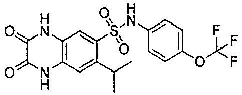 | M1, M2 | ¹H NMR (400 MHz, DMSO-*d*₆) δ 11.93 (d, *J* = 14.2 Hz, 2H), 10.67 (s, 1H), 7.72 (s, 1H), 7.29 - 7.21 (m, 2H), 7.17 (s, 1H), 7.14 - 7.08 (m, 2H), 3.77 (p, *J* = 6.7 Hz, 1H), 1.10 (d, *J* = 6.7 Hz, 6H). | | 442 | 99% |
| 32 | 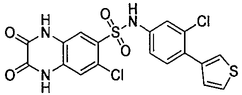 | M1, M2, M3 | ¹H NMR (400 MHz, DMSO-*d*₆) δ 12.13 (s, 1H), 12.03 (s, 1H), 10.94 (s, 1H), 7.86 (s, 1H), 7.61 - 7.55 (m, 2H), 7.38 (d, *J* = 8.5 Hz, 1H), 7.27 - 7.21 (m, 2H), 7.20 (s, 1H), 7.08 (dd, *J* = 8.5, 2.3 Hz, 1H). | 468 | | 98% |
| 33 | 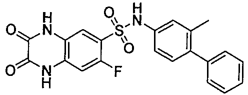 | M1, M2, M3 | ¹H NMR (400 MHz, Methanol-*d*₄) δ 7.63 (d, *J* = 6.4 Hz, 1H), 7.38 - 7.31 (m, 2H), 7.30 - 7.25 (m, 1H), 7.21 - 7.16 (m, 2H), 7.06 (q, *J* = 1.0 Hz, 1H), 7.04 - 6.98 (m, 3H), 2.13 (s, 3H). | 425 | | 95% |
| 34 | 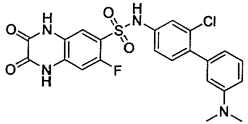 | M1, M2, M3 | ¹H NMR (400 MHz, DMSO-*d*₆) δ 12.18 (s, 1H), 11.98 (s, 1H), 10.96 (s, 1H), 7.62 (d, *J* = 6.6 Hz, 1H), 7.29 (d, *J* = 8.4 Hz, 1H), 7.26 - 7.19 (m, 2H), 7.11 (dd, *J* = 8.4, 2.3 Hz, 1H), 7.01 (d, *J* = 10.7 Hz, 1H), 6.78 (dd, *J* = 8.4, 2.5 Hz, 1H), 6.73 - 6.64 (m, 2H), 2.90 (s, 6H). | 488 | | 96% |
| 35 | 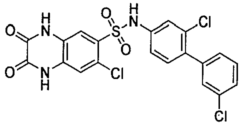 | M1, M2, M3 | ¹H NMR (400 MHz, DMSO-*d*₆) δ 12.15 (s, 1H), 12.05 (s, 1H), 11.03 (s, 1H), 7.88 (s, 1H), 7.46 - 7.41 (m, 2H), 7.38 (q, *J* = 1.3 Hz, 1H), 7.34 - 7.28 (m, 2H), 7.25 (d, *J* = 2.2 Hz, 1H), 7.21 (s, 1H), 7.11 (dd, *J* = 8.4, 2.3 Hz, 1H). | | 494 | 100 |
| 36 | 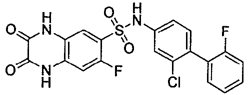 | M1, M2, M3 | ¹H NMR (400 MHz, DMSO-*d*₆) δ 12.18 (s, 1H), 11.97 (s, 1H), 11.05 (s, 1H), 7.64 (d, *J* = 6.6 Hz, 1H), 7.47 - 7.39 (m, 1H), 7.32 - 7.20 (m, 5H), 7.13 (dd, *J* = 8.4, 2.2 Hz, 1H), 7.01 (d, *J* = 10.6 Hz, 1H). | | 462 | 98% |
| 37 | 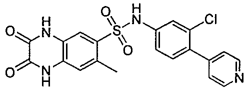 | M1, M2, M3 | ¹H NMR (400 MHz, DMSO-*d*₆) δ 12.10 (s, 1H), 11.96 (s, 1H), 11.00 (s, 1H), 8.75 - 8.67 (m, 2H), 7.81 (s, 1H), 7.64 - 7.56 (m, 2H), 7.40 (d, *J* = 8.5 Hz, 1H), 7.26 (d, *J* = 2.2 Hz, 1H), 7.14 (dd, *J* = 8.5, 2.2 Hz, 1H), 7.02 (s, 1H), 2.52 (s, 3H). | 443 | | 99% |
| 38 | 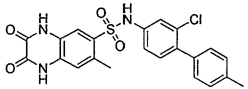 | M1, M2, M3 | ¹H NMR (300 MHz, DMSO-*d*₆) δ 12.13 (s, 1H), 11.99 (s, 1H), 10.82 (s, 1H), 7.80 (s, 1H), 7.23 (d, *J* = 8.2 Hz, 6H), 7.12 - 6.97 (m, 2H), 2.55 (s, 3H), 2.32 (s, 3H). | 456 | | 97% |
| 39 | 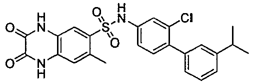 | M1, M2, M3 | ¹H NMR (300 MHz, DMSO-*d*₆) δ 12.13 (s, 1H), 11.99 (s, 1H), 10.83 (s, 1H), 7.81 (s, 1H), 7.37 - 7.26 (m, 2H), 7.24 (d, *J* = 2.0 Hz, 2H), 7.19 (s, 1H), 7.17 - 7.12 (m, 1H), 7.09 (dd, *J* = 8.4, 2.1 Hz, 1H), 7.04 (s, 1H), 2.95 - 2.83 (m, 1H), 2.56 (s, 3H), 1.19 (d, *J* = 6.9 Hz, 6H). | 484 | | 92% |
| 40 | 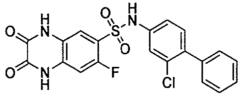 | M1, M2, M3 | ¹H NMR (400 MHz, DMSO-*d*₆) δ 12.17 (s, 1H), 11.97 (s, 1H), 10.98 (s, 1H), 7.63 (d, *J* = 6.6 Hz, 1H), 7.43 - 7.38 (m, 2H), 7.37 - 7.31 (m, 3H), 7.30 (d, *J* = 8.3 Hz, 1H), 7.26 (d, *J* = 2.2 Hz, 1H), 7.15 - 7.10 (m, 1H), 7.01 (d, *J* = 10.7 Hz, 1H). | 445 | | 99% |
| 41 | 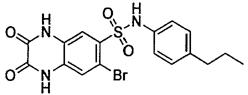 | M1, M2 | ¹H NMR (400 MHz, DMSO-*d*₆) δ 11.99 (br. s, 1H), 10.40 (br. s, 1H) 7.80 (s, 1H), 7.34 (s, 1H), 7.01 (d, *J* = 8.6 Hz, 2H), 6.99 - 6.93 (m, 2H), 2.44 - 2.35 (m, 2H), 1.52 - 1.39 (m, 2H), 0.80 (t, *J* = 7.3 Hz, 3H). | 440 | | 99% |
| 42 | 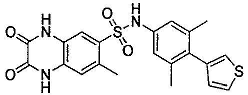 | M1, M2, M3 | ¹H NMR (400 MHz, DMSO-*d*₆) δ 12.05 (s, 1H), 11.92 (s, 1H), 10.31 (s, 1H), 7.77 (s, 1H), 7.56 (ddd, *J* = 4.9, 2.9, 0.7 Hz, 1H), 7.20 (ddd, *J* = 3.0, 1.3, 0.7 Hz, 1H), 6.98 (s, 1H), 6.87 (ddd, *J* = 4.9, 1.3, 0.7 Hz, 1H), 6.79 (d, *J* = 0.8 Hz, 2H), 2.52 (s, 3H), 1.89 (s, 6H). | 442 | | 98% |
| 43 | 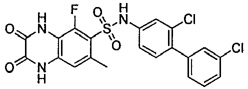 | M1, M2, M3 | ¹H NMR (400 MHz, DMSO-*d*₆) δ 12.14 (s, 1H), 12.05 (s, 1H), 11.05 (s, 1H), 7.44 - 7.41 (m, 2H), 7.38 (q, *J =* 1.4 Hz, 1H), 7.35 - 7.27 (m, 2H), 7.23 (d, *J* = 2.2 Hz, 1H), 7.10 (dd, *J* = 8.4, 2.3 Hz, 1H), 6.81 (s, 1H), 2.56 (s, 3H). | 494 | | 94% |
| 44 | 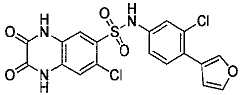 | M1, M2, M3 | ¹H NMR (400 MHz, DMSO-*d*₆) δ 12.13 (s, 1H), 12.03 (s, 1H), 10.91 (s, 1H), 8.00 (t, *J* = 1.2 Hz, 1H), 7.85 (s, 1H), 7.71 (t,*J* = 1.7 Hz, 1H), 7.45 (d, *J* = 8.5 Hz, 1H), 7.22 (d, *J* = 2.3 Hz, 1H), 7.19 (s, 1H), 7.07 (dd, *J* = 8.5, 2.3 Hz, 1H), 6.79 (dd, *J* = 1.9, 0.9 Hz, 1H). | 452 | | 98% |
| 45 | 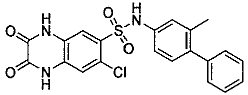 | M1, M2, M3 | ¹H NMR (400 MHz, DMSO-*d*₆) δ 12.13 (s, 1H), 12.02 (s, 1H), 10.60 (s, 1H), 7.86 (s, 1H), 7.41 - 7.33 (m, 2H), 7.32 - 7.26 (m, 1H), 7.25 - 7.17 (m, 3H), 7.07 - 7.00 (m, 2H), 7.00 - 6.94 (m, 1H), 2.11 (s, 3H). | | 440 | 95% |
| 46 | 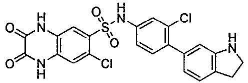 | M1, M2, M3 | ¹H NMR (400 MHz, DMSO-*d*₆) δ 12.15 (s, 1H), 12.04 (s, 1H), 10.94 (s, 1H), 7.87 (s, 1H), 7.25 - 7.20 (m, 3H), 7.16 (d, *J* = 7.6 Hz, 1H), 7.08 (dd, *J* = 8.5, 2.2 Hz, 1H), 6.72 (s, 2H), 3.55 - 3.47 (m, 2H), 2.99 (t, *J* = 8.2 Hz, 2H). | 503 | | 91% |
| 47 | 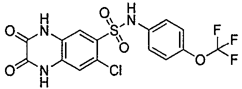 | M1, M2 | ¹H NMR (400 MHz, Methanol-*d*₄) δ 7.84 (s, 1H), 7.26 (s, 1H), 7.23 - 7.20 (m, 2H), 7.12 (dtt, *J* = 6.8, 1.9, 1.0 Hz, 2H). | 436 | | 95% |
| 48 | 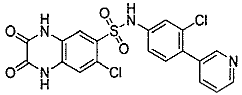 | M1, M2, M3 | ¹H NMR (400 MHz, DMSO-*d*₆) δ 12.18 (s, 1H), 12.08 (s, 1H), 11.12 (s, 1H), 8.64 (dd, *J* = 4.7, 1.8 Hz, 2H), 7.96 (dt, *J* = 8.1, 2.0 Hz, 1H), 7.92 (s, 1H), 7.58 (dd, *J* = 8.0, 4.9 Hz, 1H), 7.42 (d, *J* = 8.4 Hz, 1H), 7.32 (d, *J* = 2.2 Hz, 1H), 7.24 (s, 1H), 7.18 (dd, *J* = 8.4, 2.2 Hz, 1H). | 463 | | 99% |
| 49 | 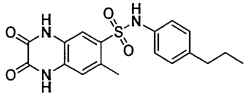 | M1, M2 | ¹H NMR (400 MHz, DMSO-*d*₆) δ 11.51 (br. s, 1H), 7.70 (s, 1H), 7.04 - 6.99 (m, 2H), 6.99 - 6.91 (m, 3H), 2.52 (s, 3H), 2.41 (dd, *J* = 8.5, 6.7 Hz, 2H), 1.54 - 1.42 (m, 2H), 0.82 (t, *J* = 7.3 Hz, 3H). | 374 | | 97% |
| 50 | 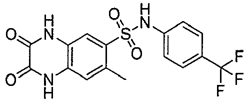 | M1, M2 | ¹H NMR (400 MHz, DMSO-*d*₆) δ 12.07 (s, 1H), 11.92 (s, 1H), 10.98 (s, 1H), 7.77 (s, 1H), 7.58 (d, *J* = 8.5 Hz, 2H), 7.20 (d, *J* = 8.4 Hz, 2H), 6.98 (s, 1H), 2.50 (d, *J* = 0.9 Hz, 3H). | 440 | | 96% |
| 51 | 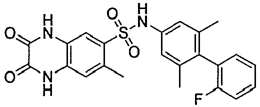 | M1, M2, M3 | ¹H NMR (400 MHz, Methanol-*d*₄) δ 7.82 (s, 1H), 7.35 (tdd, *J* = 7.5, 5.2, 1.8 Hz, 1H), 7.24 - 6.99 (m, 4H), 6.85 (s, 2H), 2.63 (s, 3H), 1.89 (s, 6H). | 454 | | 98% |
| 52 | 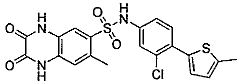 | M1, M2, M3 | ¹H NMR (400 MHz, Methanol-*d*₄) δ 7.82 (s, 1H), 7.34 (d, *J* = 8.5 Hz, 1H), 7.22 (d, *J* = 2.3 Hz, 1H), 7.07 (s, 1H), 7.04 - 7.00 (m, 2H), 6.71 (d, *J* = 3.1 Hz, 1H), 2.62 (s, 3H), 2.46 (d, *J* = 1.0 Hz, 3H). | 462 | | 98% |
| 53 | 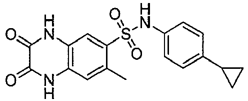 | M1, M2 | ¹H NMR (400 MHz, DMSO-*d*₆) δ 12.03 (s, 1H), 11.88 (s, 1H), 10.16 (s, 1H), 7.66 (s, 1H), 7.03 - 6.81 (m, 5H), 1.75 (td, *J* = 8.4, 4.3 Hz, 1H), 0.88 - 0.75 (m, 2H), 0.57 - 0.47 (m, 2H). | 372 | | 100% |
| 54 | 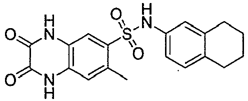 | M1, M2 | ¹H NMR (400 MHz, DMSO-*d*₆) δ 12.18 - 11.67 (br. m, 2H), 10.12 (br. s, 1H), 7.68 (s, 1H), 6.95 (d,*J* = 0.9 Hz, 1H), 6.84 (d, *J* = 8.1 Hz, 1H), 6.78 - 6.70 (m, 2H), 2.54 (d, *J* = 5.4 Hz, 4H), 2.49 (s, 3H), 1.62 (dq, *J* = 6.6, 2.8 Hz, 4H). | 386 | | 98% |
| 55 | 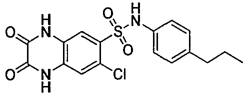 | M1, M2 | ¹H NMR (400 MHz, DMSO-*d*₆) δ 12.13 - 11.80 (br. m, 2H), 10.34 (br. s, 1H), 7.76 (s, 1H), 7.16 (s, 1H), 7.06 - 6.93 (m, 4H), 2.39 (dd, *J* = 8.5, 6.7 Hz, 2H), 1.54 - 1.39 (m, 2H), 0.80 (t, *J* = 7.3 Hz, 3H). | 392 | | 100% |
| 56 | 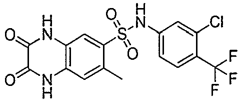 | M1, M2 | ¹H NMR (400 MHz, DMSO-*d*₆) δ 12.10 (s, 1H), 11.96 (s, 1H), 11.29 (br. s, 1H), 7.80 (s, 1H), 7.71 (d ,*J* = 8.7 Hz, 1H), 7.28 (s, 1H), 7.13 (d, *J* = 8.8 Hz, 1H), 7.02 (s, 1H), 2.53 (s, 3H). | 434 | | 96% |
| 57 | 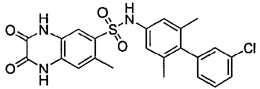 | M1, M2, M3 | ¹H NMR (400 MHz, DMSO-*d*₆) δ 12.07 (s, 1H), 11.94 (s, 1H), 10.38 (s, 1H), 7.79 (s, 1H), 7.45 - 7.34 (m, 2H), 7.11 (t, *J* = 1.9 Hz, 1H), 7.04 - 6.97 (m, 2H), 6.82 (s, 2H), 2.53 (s, 3H), 1.84 (s, 6H). | 470 | | 94% |
| 58 | 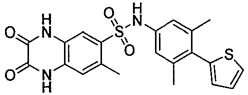 | M1, M2, M3 | ¹H NMR (400 MHz, Methanol-*d*₄) δ 7.82 (s, 1H), 7.42 (dd, *J* = 5.2, 1.2 Hz, 1H), 7.08 - 7.05 (m, 2H), 6.84 (p, *J* = 0.6 Hz, 2H), 6.72 (dd, *J* = 3.5, 1.2 Hz, 1H), 2.63 (s, 3H), 1.99 (s, 6H). | 442 | | 97% |
| 59 | 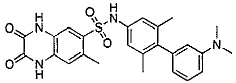 | M1, M2, M3 | ¹H NMR (400 MHz, DMSO-*d*₆) δ 12.25 - 11.73 (br. m, 2H), 10.29 (br. s, 1H), 7.78 (d, *J* = 0.9 Hz, 1H), 7.22 - 7.12 (m, 1H), 6.99 (s, 1H), 6.78 (s, 2H), 6.65 - 6.60 (m, 1H), 6.32 - 6.24 (m, 2H), 2.83 (s, 6H), 2.54 (s, 3H), 1.86 (s, 6H). | 479 | | 94% |
| 60 | 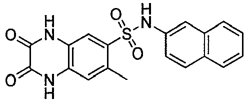 | M1, M2 | ¹H NMR (400 MHz, Methanol-*d*₄) δ 7.81 (s, 1H), 7.75 - 7.61 (m, 3H), 7.51 (d, *J* = 2.2 Hz, 1H), 7.39 (ddd, *J* = 8.3, 6.9, 1.4 Hz, 1H), 7.33 (ddd,*J* = 8.2, 6.9, 1.3 Hz, 1H),, 7.24 (dd, *J* = 8.8, 2.2 Hz, 1H), 7.02 (s, 1H), 2.63 (s, 3H). | 382 | | 99% |
| 61 | 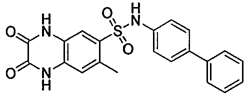 | M1, M2 | ¹H NMR (400 MHz, DMSO-*d*₆) δ 12.21 - 11.61 (br. m, 2H), 10.56 (br. s, 1H), 7.76 (s, 1H), 7.59 - 7.45 (m, 4H), 7.38 (t, *J* = 7.7 Hz, 2H), 7.32 - 7.23 (m, 1H), 7.16 - 7.05 (m, 2H), 6.97 (s, 1H), 2.52 (s, 3H). | 408 | | 97% |
| 62 | 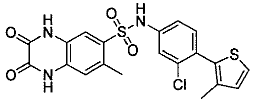 | M1, M2, M3 | ¹H NMR (400 MHz, DMSO-*d*₆) δ 12.09 (s, 1H), 11.94 (s, 1H), 10.86 (s, 1H), 7.78 (s, 1H), 7.47 (d, *J* = 5.1 Hz, 1H), 7.31 - 7.20 (m, 2H), 7.08 - 7.00 (m, 2H), 6.93 (d, *J* = 5.1 Hz, 1H), 2.53 (s, 3H), 1.95 (s, 3H). | 462 | | 98% |
| 63 | 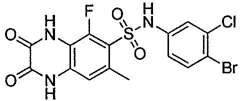 | M1, M2 | ¹H NMR (400 MHz, Methanol-*d*₄) δ 7.51 (d, *J* = 8.7 Hz, 1H), 7.31 (d, *J* = 2.5 Hz, 1H), 6.98 (dd, *J =* 8.7, 2.6 Hz, 1H), 6.85 - 6.83 (m, 1H), 2.60 (s, 3H). | | 462 | 82% |
| 64 | 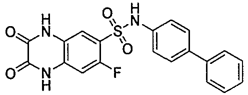 | M1, M2 | ¹H NMR (400 MHz, DMSO-*d*₆) δ 12.13 (s, 1H), 11.95 (s, 1H), 10.69 (s, 1H), 7.59 (d, *J* = 6.6 Hz, 1H), 7.58 - 7.52 (m, 4H), 7.39 (t, *J* = 7.7 Hz, 2H), 7.29 (t, *J* = 7.3 Hz, 1H), 7.19 - 7.15 (m, 2H), 6.97 (d, *J* = 10.6 Hz, 1H). | | 410 | 99% |
| 65 | 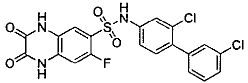 | M1, M2, M3 | ¹H NMR (400 MHz, DMSO-*d*₆) δ 12.18 (s, 1H), 11.98 (s, 1H), 11.04 (s, 1H), 7.63 (d, *J* = 6.5 Hz, 1H), 7.41 - 7.37 (m, 1H), 7.27 (d, *J* = 2.2 Hz, 1H), 7.13 (ddd, *J* = 8.5, 2.3, 0.8 Hz, 1H), 7.01 (d, *J* = 10.6 Hz, 1H). | | 478 | 99% |
| 66 | 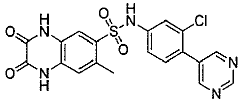 | M1, M2, M3 | ¹H NMR (300 MHz, Methanol-*d*₄) δ 9.02 (s, 1H), 8.71 (s, 2H), 7.79 (s, 1H), 7.25 (dd, *J* = 5.3, 3.1 Hz, 2H), 7.09 (dd, *J* = 8.4, 2.2 Hz, 1H), 6.99 (s, 1H), 2.55 (s, 3H). | | 442 | 97% |
| 67 | 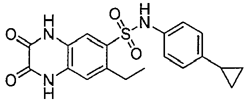 | M1, M2 | ¹H NMR (400 MHz, Methanol-*d*₄) δ 7.71 (s, 1H), 7.11 (s, 1H), 6.96 - 6.86 (m, 4H), 3.01 (q, *J* = 7.5 Hz, 2H), 1.78 (tt, *J* = 8.4, 5.1 Hz, 1H), 1.26 (t, *J* = 7.5 Hz, 3H), 0.92 - 0.81 (m, 2H), 0.61 - 0.48 (m, 2H). | | 384 | 91% |
| 68 | 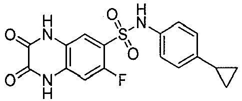 | M1, M2 | ¹H NMR (400 MHz, DMSO-*d*₆) δ 12.15 - 11.68 (br. m, 2H), 10.29 (br. s, 1H), 7.45 (d, *J* = 6.5 Hz, 1H), 6.92 - 6.85 (m, 5H), 1.73 (tt, *J* = 8.3, 5.1 Hz, 1H), 0.83 - 0.75 (m, 2H), 0.53 - 0.44 (m, 2H). | 376 | | 90% |
| 69 | 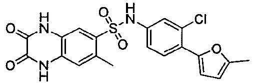 | M1, M2, M3 | ¹H NMR (400 MHz, DMSO-*d*₆) δ 12.06 (s, 1H), 11.92 (s, 1H), 10.75 (s, 1H), 7.75 (s, 1H), 7.61 (d, *J* = 8.7 Hz, 1H), 7.17 (d, *J* = 2.3 Hz, 1H), 7.07 (dd, *J* = 8.7, 2.3 Hz, 1H), 6.99 (s, 1H), 6.83 (d, *J* = 3.3 Hz, 1H), 6.19 (dd, *J* = 3.4, 1.2 Hz, 1H), 2.51 (s, 3H), 2.28 (s, 3H). | 446 | | 97% |
| 70 | 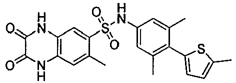 | M1, M2, M3 | ¹H NMR (400 MHz, DMSO-*d*₆) δ 12.06 (s, 1H), 11.92 (s, 1H), 10.41 (s, 1H), 7.78 (s, 1H), 6.99 (s, 1H), 6.81 (s, 2H), 6.76 (dt, *J* = 3.3, 1.2 Hz, 1H), 6.59 - 6.49 (m, 1H), 2.52 (s, 3H), 2.42 (s, 3H), 1.96 (s, 6H). | | 454 | 94% |
| 71 | 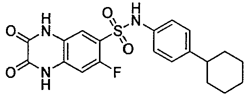 | M1, M2, M3 | ¹H NMR (400 MHz, DMSO-*d*₆) δ 12.13 (br. s, 1H), 11.92 (br. s, 1H), 10.41 (br. s, 1H), 7.53 (d, *J* = 6.6 Hz, 1H), 7.10 - 7.03 (m, 2H), 7.01 - 6.93 (m, 3H), 2.41 - 2.25 (m, 1H), 1.78 - 1.61 (m, 5H), 1.34 - 1.10 (m, 5H). | | 416 | 97% |
| 72 | 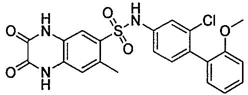 | M1, M2, M3 | ¹H NMR (400 MHz, DMSO-*d*₆) δ 12.09 (s, 1H), 11.95 (s, 1H), 10.74 (s, 1H), 7.80 (s, 1H), 7.37 - 7.29 (m, 1H), 7.20 - 7.11 (m, 2H), 7.07 - 7.00 (m, 4H), 6.98 - 6.90 (m, 1H), 3.65 (s, 3H), 2.54 (s, 3H). | 472 | | 98% |
| 73 | 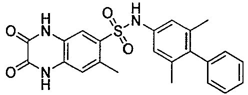 | M1, M2, M3 | ¹H NMR (400 MHz, DMSO-*d*₆) δ 12.06 (s, 1H), 11.93 (s, 1H), 10.32 (s, 1H), 7.79 (s, 1H), 7.38 (tt, *J* = 6.8, 0.9 Hz, 2H), 7.33 - 7.25 (m, 1H), 7.06 - 6.96 (m, 3H), 6.81 (s, 2H), 2.53 (s, 3H), 1.83 (s, 6H). | 436 | | 100% |
| 74 | 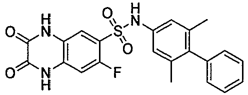 | M1, M2, M3 | ¹H NMR (400 MHz, DMSO-*d*₆) δ 12.16 (s, 1H), 11.95 (s, 1H), 10.52 (s, 1H), 7.61 (d, *J* = 6.5 Hz, 1H), 7.39 (t, *J* = 7.5 Hz, 2H), 7.30 (dd, *J* = 8.4, 6.3 Hz, 1H), 7.07 - 6.97 (m, 3H), 6.85 (s, 2H), 1.84 (s, 6H). | | 438 | 99% |
| 75 | 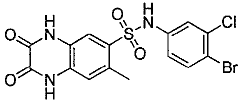 | M1, M2 | ¹H NMR (400 MHz, DMSO-*d*₆) δ 12.05 (s, 1H), 11.90 (s, 1H), 10.80 (s, 1H), 7.70 (s, 1H), 7.58 (d, *J* = 8.7 Hz, 1H), 7.23 (d, *J* = 2.5 Hz, 1H), 6.97 (s, 1H), 6.91 (dd, *J* = 8.8, 2.6 Hz, 1H), 2.48 (s, 3H). | 446 | | 100% |
| 76 | 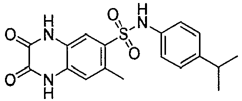 | commerci al | 894919-20-1 | | | |
| 77 | 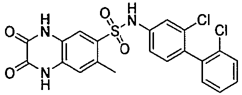 | M1, M2, M3 | ¹H NMR (400 MHz, DMSO-*d*₆) δ 12.10 (s, 1H), 11.96 (s, 1H), 10.8.3 (s, 1H), 7.80 (s, 1H), 7.55 - 7.47 (m, 1H), 7.37 (pd, *J* = 7.4, 1.8 Hz, 2H), 7.27 - 7.17 (m, 3H), 7.10 - 7.00 (m, 2H), 2.54 (s, 3H). | 476 | | 99% |
| 78 | 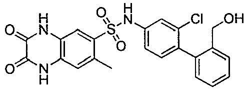 | M1, M2, M3 | ¹H NMR (400 MHz, DMSO-*d*₆) δ 12.09 (s, 1H), 11.95 (s, 1H), 10.77 (s, 1H), 7.79 (s, 1H), 7.54 - 7.49 (m, 1H), 7.36 (td, *J* = 7.5, 1.5 Hz, 1H), 7.24 (td,*J* = 7.6, 1.4 Hz, 1H), 7.19 (d, *J* = 2.2 Hz, 1H), 7.15 (d, *J* = 8.3 Hz, 1H), 7.06 - 6.96 (m, 3H), 4.21 - 4.03 (m, 2H), 2.53 (s, 3H). | | 469 | 94% |
| 79 | 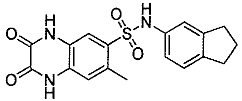 | M1, M2 | ¹H NMR (400 MHz, DMSO-*d*₆) δ 12.02 (s, 1H), 11.88 (s, 1H), 10.13 (s, 1H), 7.67 (s, 1H), 7.01 (d, *J* = 8.1 Hz, 1H), 6.95 (d, *J* = 0.9 Hz, 1H), 6.91 (d, *J* = 2.0 Hz, 1H), 6.79 (dd, *J* = 8.0, 2.1 Hz, 1H), 2.70 (q, *J* = 7.8 Hz, 4H), 2.49 (s, 3H), 1.91 (p, *J* = 7.4 Hz, 2H). | 372 | | 98% |
| 80 | 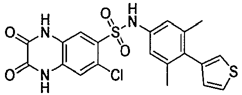 | M1, M2, M3 | ¹H NMR (400 MHz, DMSO-*d*₆) δ 12.12 (s, 1H), 12.01 (s, 1H), 10.49 (s, 1H), 7.85 (s, 1H), 7.58 (ddd, *J* = 5.0, 2.9, 0.8 Hz, 1H), 7.22 (dt, *J* = 2.9, 1.0 Hz, 1H), 7.19 (s, 1H), 6.88 (dt, *J* = 4.9, 1.0 Hz, 1H), 6.83 (s, 2H), 1.90 (s, 6H). | | 460 | 99% |
| 81 | 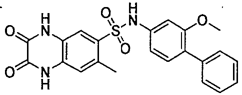 | M1, M2, M3 | ¹H NMR (400 MHz, DMSO-*d*₆) δ 12.05 (s, 1H), 11.96 (s, 1H), 10.54 (s, 1H), 7.82 (s, 1H), 7.38 - 7.27 (m, 4H), 7.27 - 7.19 (m, 1H), 7.10 (dd, *J* = 8.2, 0.8 Hz, 1H), 6.99 (s, 1H), 6.82 (d, *J* = 2.1 Hz, 1H), 6.71 - 6.63 (m, 1H), 3.64 (s, 3H), 2.54 (s, 3H). | 438 | | 98% |
| 82 | 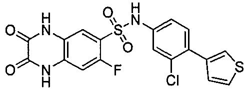 | M1, M2, M3 | ¹H NMR (400 MHz, Methanol-*d*₄) δ 7.66 (d, *J* = 6.4 Hz, 1H), 7.45 - 7.39 (m, 2H), 7.33 - 7.28 (m, 2H), 7.21 (dd, *J* = 4.9, 1.4 Hz, 1H), 7.11 (dd, *J* = 8.4, 2.3 Hz, 1H), 7.01 (d, *J* = 10.4 Hz, 1H). | | 450 | 96% |
| 83 | 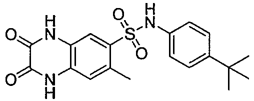 | M1, M2 | ¹H NMR (400 MHz, DMSO-*d*₆) δ 12.04 (s, 1H), 11.89 (s, 1H), 10.24 (s, 1H), 7.71 (s, 1H), 7.26 - 7.14 (m, 2H), 7.01 - 6.89 (m, 3H), 2.48 (s, 3H), 1.16 (s, 9H). | 388 | | 99% |
| 84 | 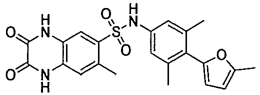 | M1, M2, M3 | ¹H NMR (400 MHz, DMSO-*d*₆) δ 12.05 (s, 1H), 11.92 (s, 1H), 10.42 (s, 1H), 7.77 (s, 1H), 6.98 (s, 1H), 6.80 (s, 2H), 6.17 (dd, *J* = 3.1, 0.7 Hz, 1H), 6.10 (dq, *J* = 3.0, 0.8 Hz, 1H), 2.52 (s, 3H), 2.23 (s, 3H), 2.02 (s, 6H). | 440 | | 98% |
| 85 | 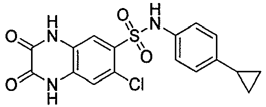 | M1, M2 | ¹H NMR (400 MHz, Methanol-*d*₄) δ 7.77 (s, 1H), 7.24 (s, 1H), 7.03 - 6.98 (m, 2H), 6.91 - 6.86 (m, 2H), 1.76 (tt, *J* = 8.4, 5.1 Hz, 1H), 0.90 - 0.81 (m, 2H), 0.57 - 0.49 (m, 2H). | 390 | | 97% |
| 86 | 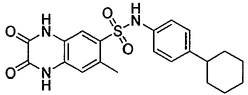 | M1, M2 | ¹H NMR (400 MHz, DMSO-*d*₆) δ 12.04 (s, 1H), 11.89 (s, 1H), 10.21 (s, 1H), 7.70 (s, 1H), 7.07 - 7.00 (m, 2H), 6.98 - 6.90 (m, 3H), 2.49 (d, *J* = 0.7 Hz, 3H), 2.34 (s, 1H), 1.75 - 1.57 (m, 5H), 1.37 - 1.07 (m, 5H). | 414 | | 98% |
| 87 | 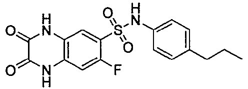 | M1, M2 | ¹H NMR (400 MHz, Methanol-*d*₄) δ 7.45 (d, *J* = 6.4 Hz, 1H), 7.00 - 6.85 (m, 5H), 2.41 - 2.32 (m, 2H), 1.50 - 1.38 (m, 2H), 0.76 (t, *J* = 7.3 Hz, 3H). | | 376 | 90% |
| 88 | 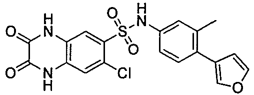 | M1, M2, M3 | ¹H NMR (400 MHz, DMSO-*d*₆) δ 12.10 (br. s, 1H), 12.00 (br. s, 1H), 10.54 (s, 1H), 7.82 (s, 1H), 7.76 (t, *J* = 1.1 Hz, 1H), 7.67 (t, *J* = 1.7 Hz, 1H), 7.23 - 7.16 (m, 2H), 6.99 (d, *J* = 2.3 Hz, 1H), 6.93 (dd, *J* = 8.3, 2.4 Hz, 1H), 6.67 (dd, *J* = 1.9, 0.9 Hz, 1H), 2.23 (s, 3H). | | 430 | 95% |
| 89 | 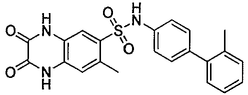 | M1, M2, M3 | ¹H NMR (400 MHz, DMSO-*d*₆) δ 12.06 (s, 1H), 11.91 (s, 1H), 10.45 (s, 1H), 7.75 (s, 1H), 7.25 - 7.13 (m, 5H), 7.10 - 7.05 (m, 3H), 6.99 (s, 1H), 2.51 (d, *J* = 0.8 Hz, 3H), 2.12 (s, 3H). | 422 | | 95% |
| 90 | 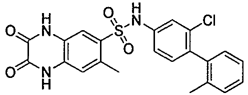 | M1, M2, M3 | ¹H NMR (400 MHz, Methanol-*d*₄) δ 7.83 (s, 1H), 7.26 - 7.18 (m, 3H), 7.18 - 7.12 (m, 1H), 7.09 (d, *J* = 0.9 Hz, 1H), 7.07 - 7.02 (m, 2H), 6.97 (dd, *J* = 7.4, 1.2 Hz, 1H), 2.64 (d, *J* = 0.7 Hz, 3H), 1.97 (s, 3H). | 456 | | 96% |
| 91 | 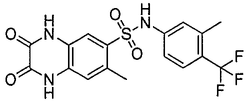 | M1, M2 | ¹H NMR (400 MHz, DMSO-*d*₆) δ 12.07 (s, 1H), 11.92 (s, 1H), 10.90 (s, 1H), 7.78 (s, 1H), 7.49 (d, J = 8.6 Hz, 1H), 7.04 (s, 1H), 7.01 - 6.97 (m, 2H), 2.50 (s, 3H), 2.32 - 2.29 (m, 3H). | | 412 | 99% |
| 92 | 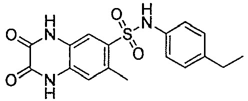 | commerci al | 894915-45-8 | | | |
| 93 | 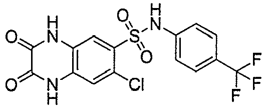 | M1, M2 | ¹H NMR (400 MHz, Methanol-*d*₄) δ 7.92 (s, 1H), 7.53 - 7.46 (m, 2H), 7.32 - 7.26 (m, 2H), 7.24 (s, 1H). | 420 | | 97% |
| 94 | 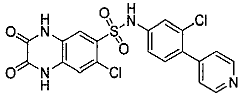 | M1, M2, M3 | ¹H NMR (400 MHz, Methanol-*d*₄) δ 8.86 - 8.78 (m, 2H), 8.04 - 7.97 (m, 3H), 7.51 - 7.42 (m, 2H), 7.35 - 7.28 (m, 2H). | 463 | | 96% |
| 95 | 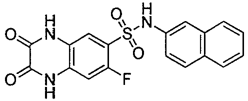 | M1, M2 | ¹H NMR (400 MHz, DMSO-*d*₆) δ 12.09 (s, 1H), 11.90 (s, 1H), 10.79 (s, 1H), 7.79 (dd, *J* = 8.6, 5.0 Hz, 2H), 7.73 (d, J = 8.2 Hz, 1H), 7.60 (d, *J* = 6.6 Hz, 1H), 7.54 (d, J = 2.1 Hz, 1H), 7.47 - 7.34 (m, 2H), 7.30 (dd, *J* = 8.8, 2.2 Hz, 1H), 6.93 (d, *J* = 10.6 Hz, 1H). | | 384 | 98% |
| 96 | 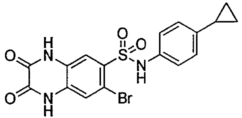 | M1, M2 | ¹H NMR δ (300 MHz, DMSO-*d*₆) δ 12.13 (s, 1H), 12.03 (s, 1H), 10.41 (s, 1H), 7.82 (s, 1H), 7.36 (s, 1H), 7.00 - 6.89 (m, 4H), 1.78 (tt, *J* = 8.4, 5.1 Hz, 1H), 0.90 - 0.81 (m, 2H), 0.59 - 0.50 (m, 2H). | 436 | | 95% |
| 97 | 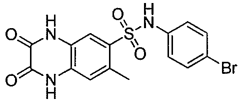 | M1, M2 | ¹H NMR (400 MHz, DMSO-d6) δ 12.05 (s, 1H), 11.89 (s, 1H), 10.54 (s, 1H), 7.69 (s, 1H), 7.44 - 7.37 (m, 2H), 7.01 - 6.96 (m, 3H), 2.50 (s, 3H). | 410 | | 98% |
| 98 | 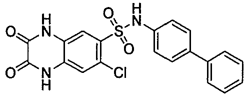 | M1, M2 | ¹H NMR (400 MHz, DMSO-*d*₆) δ 12.16 - 11.93 (br. m, 2H), 10.67 (br. s, 1H), 7.84 (s, 1H), 7.58 - 7.48 (m, 4H), 7.38 (dd, *J* = 8.3, 6.9 Hz, 2H), 7.33 - 7.24 (m, 1H), 7.20 - 7.12 (m, 3H). | 426 | | 99% |
| 99 | 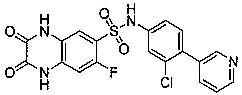 | M1, M2, M3 | ¹H NMR (400 MHz, DMSO-*d*₆) δ 12.18 (s, 1H), 11.98 (s, 1H), 11.09 (s, 1H), 8.62 - 8.57 (m, 2H), 7.91 - 7.84 (m, 1H), 7.64 (d, *J* = 6.6 Hz, 1H), 7.55 - 7.48 (m, 1H), 7.39 (d, *J* = 8.5 Hz, 1H), 7.30 (d, *J* = 2.3 Hz, 1H), 7.17 (dd, *J* = 8.4, 2.2 Hz, 1H), 7.01 (d, *J* = 10.5 Hz, 1H). | | 445 | 99% |
| 100 | 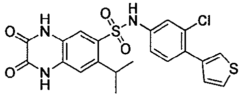 | M1, M2, M3 | ¹H NMR (400 MHz, DMSO-*d*₆) δ 11.96 - 11.93 (m, 2H), 10.82 (s, 1H), 7.76 (s, 1H), 7.59 (dd, *J =* 3.0, 1.4 Hz, 1H), 7.56 (dd, *J* = 4.9, 3.0 Hz, 1H), 7.36 (d, J = 8.4 Hz, 1H), 7.23 (dd, *J* = 5.0, 1.4 Hz, 1H), 7.20 - 7.17 (m, 2H), 7.03 (dd, *J* = 8.5, 2.3 Hz, 1H), 3.80 (p, *J* = 6.7 Hz, 1H), 1.13 (d, *J* = 6.7 Hz, 6H). | | 474 | 99% |
| 101 | 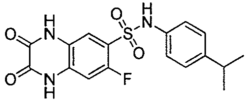 | M1, M2 | ¹H NMR (400 MHz, DMSO-*d*₆) δ 12.13 (br. s, 1H), 11.92 (br. s, 1H), 10.41 (br. s, 1H), 7.53 (d, *J* = 6.5 Hz, 1H), 7.09 (d, *J* = 8.4 Hz, 2H), 7.03 - 6.92 (m, 3H), 2.75 (p, *J* = 7.0 Hz, 1H), 1.09 (d, *J* = 6.9 Hz, 6H). | | 376 | 99% |
| 102 | 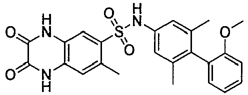 | M1, M2, M3 | ¹H NMR (400 MHz, DMSO-*d*₆) δ 12.05 (br. s, 1H), 11.93 (br. s, 1H), 10.28 (br. s, 1H), 7.79 (s, 1H), 7.29 (ddd, *J* = 8.7, 7.3, 1.8 Hz, 1H), 7.06 - 6.98 (m, 2H), 6.94 (td, *J* = 7.3, 1.0 Hz, 1H), 6.86 (dd, *J* = 7.4, 1.8 Hz, 1H), 6.77 (d, *J* = 0.9 Hz, 2H), 3.61 (s, 3H), 2.53 (s, 3H), 1.77 (s, 6H). | 466 | | 97% |
| 103 | 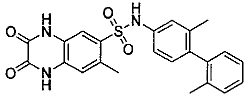 | M1, M2, M3 | ¹H NMR (400 MHz, DMSO-*d*₆) δ 12.07 (s, 1H), 11.93 (s, 1H), 10.35 (s, 1H), 7.76 (s, 1H), 7.26 - 7.13 (m, 3H), 7.02 - 6.92 (m, 3H), 6.91 - 6.85 (m, 2H), 2.55 - 2.50 (m, 3H), 1.90 - 1.84 (m, 6H). | 436 | | 98% |
| 104 | 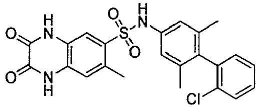 | M1, M2, M3 | ¹H NMR (400 MHz, DMSO-*d*₆) δ 12.04 (s, 1H), 11.91 (s, 1H), 10.35 (s, 1H), 7.79 (s, 1H), 7.56 - 7.50 (m, 1H), 7.40 - 7.33 (m, 2H), 7.14 - 7.08 (m, 1H), 7.00 (s, 1H), 6.81 (s, 2H), 2.53 (s, 3H), 1.78 (s, 6H). | 470 | | 95% |
| 105 | 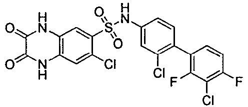 | M1, M2, M3 | ¹H NMR (400 MHz, Methanol-*d*₄) δ 7.94 (s, 1H), 7.33 (dd, *J* = 1.9, 0.7 Hz, 1H), 7.28 (s, 1H), 7.23 - 7.17 (m, 3H), 7.15 (dd, *J* = 8.6, 1.5 Hz, 1H). | | 530 | 93% |
| 106 | 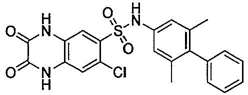 | M1, M2, M3 | ¹H NMR (400 MHz, Methanol-*d*₄) δ 7.88 (s, 1H), 7.39 - 7.31 (m, 2H), 7.28 (d, *J* = 7.3 Hz, 2H), 7.01 - 6.95 (m, 2H), 6.93 - 6.87 (m, 2H), 1.86 (s, 6H). | | 454 | 92% |
| 107 | 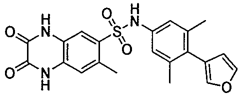 | M1, M2, M3 | ¹H NMR (400 MHz, DMSO-*d*₆) δ 12.05 (s, 1H), 11.92 (s, 1H), 10.30 (s, 1H), 7.76 (s, 1H), 7.73 - 7.67 (m, 1H), 7.51 (dt, *J* = 1.4, 0.7 Hz, 1H), 6.97 (s, 1H), 6.79 (d, *J* = 0.8 Hz, 2H), 6.36 (dt, *J* = 1.6, 0.7 Hz, 1H), 2.52 (s, 3H), 1.97 (s, 6H). | 426 | | 98% |
| 108 | 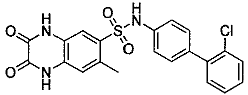 | M1, M2, M3 | ¹H NMR (400 MHz, DMSO-*d*₆) δ 11.92 (br. s, 2H), 10.66 (br. s, 1H), 7.78 (s, 1H), 7.50 - 7.47 (m, 1H), 7.37 - 7.23 (m, 5H), 7.11 - 7.06 (m, 2H), 6.99 (s, 1H), 2.53 (s, 3H). | 442 | | 97% |
| 109 | 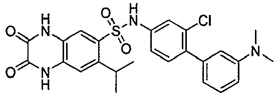 | M1, M2, M3 | ¹H NMR (400 MHz, DMSO-*d*₆) δ 11.96 (br. s, 2H), 11.95 (br. s, 1H), 10.80 (s, 1H), 7.77 (s, 1H), 7.25 (d, *J* = 8.4 Hz, 1H), 7.22 - 7.16 (m, 3H), 7.04 (dd, *J* = 8.4, 2.2 Hz, 1H), 6.73 (d, *J* = 8.4 Hz, 1H), 6.61 (d, *J* = 9.4 Hz, 2H), 3.81 (p, *J* = 6.7 Hz, 2H), 2.85 (s, 6H), 1.14 (d, *J* = 6.7 Hz, 6H). | | 511 | 97% |
| 110 | 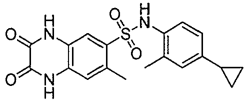 | M1, M2 | ¹H NMR (400 MHz, DMSO-d₆) δ 11.86 (br. s, 2H), 9.46 (br. s, 1H), 7.47 (s, 1H), 7.00 (d, *J* = 0.8 Hz, 1H), 6.82 (d, *J* = 1.8 Hz, 1H), 6.77 - 6.69 (m, 2H), 2.46 (s, 3H), 2.00 (s, 3H), 1.77 (tt, *J* = 8.4, 5.1 Hz, 1H), 0.93 - 0.79 (m, 2H), 0.63 - 0.50 (m, 2H). | 386 | | 98% |
| 111 | 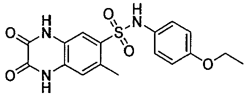 | M1, M2 | ¹H NMR (400 MHz, DMSO-*d*₆) δ 12.04 (s, 1H), 11.87 (s, 1H), 9.92 (s, 1H), 7.59 (s, 1H), 6.96 (s, 1H), 6.95 - 6.89 (m, 2H), 6.79 - 6.71 (m, 2H), 3.88 (q, *J* = 7.0 Hz, 2H), 2.47 (s, 3H), 1.27 - 1.20 (m, 3H). | 376 | | 100% |
| 112 | 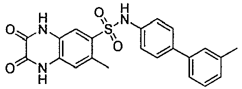 | M1, M2, M3 | ¹H NMR (400 MHz, DMSO-*d*₆) δ 11.82 (br. s, 2H), 10.54 (br. s, 1H), 7.75 (s, 1H), 7.35 - 7.19 (m, 5H), 7.04 - 6.86 (m, 4H), 2.51 (s, 3H), 2.29 (s, 3H). | | 420 | 98% |
| 113 | 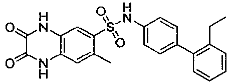 | M1, M2, M3 | ¹H NMR (400 MHz, DMSO-*d*₆) δ 11.92 (br. s, 2H), 10.48 (br. s, 1H), 7.75 (s, 1H), 7.26 - 7.21 (m, 2H), 7.19 - 7.10 (m, 3H), 7.09 - 7.01 (m, 3H), 6.98 (s, 1H), 2.52 (s, 3H), 2.42 (q, *J* = 7.5 Hz, 2H), 0.93 (t, *J* = 7.5 Hz, 3H). | 436 | | 91% |
| 114 | 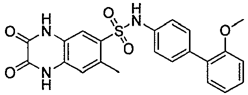 | M1, M2, M3 | ¹H NMR (400 MHz, DMSO-*d*₆) δ 11.91 (br. s, 1H), 10.47 (br. s, 1H), 7.77 (s, 1H), 7.32 - 7.22 (m, 3H), 7.17 (dd, *J* = 7.6, 1.8 Hz, 1H), 7.07 - 7.00 (m, 3H), 7.00 - 6.92 (m, 2H), 3.69 (d, *J* = 0.6 Hz, 3H), 2.53 (s, 3H). | 438 | | 98% |
| 115 | 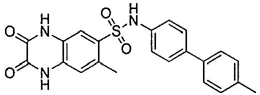 | M1, M2, M3 | ¹H NMR (400 MHz, DMSO-*d*₆) δ 12.04 (s, 1H), 11.91 (s, 1H), 10.46 (s, 1H), 7.75 (s, 1H), 7.48 (d, *J* = 8.1 Hz, 1H), 7.43 (d, J = 7.9 Hz, 1H), 7.18 (d, *J* = 7.9 Hz, 2H), 7.10 (d, *J* = 8.3 Hz, 2H), 6.97 (s, 1H), 2.52 (s, 3H), 2.28 (s, 3H). | 422 | | 91% |
| 116 | 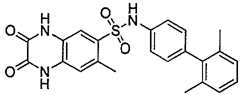 | M1, M2, M3 | ¹H NMR (400 MHz, DMSO-*d*₆) δ 12.07 (s, 1H), 11.92 (s, 1H), 10.34 (s, 1H), 7.71 (s, 1H), 7.11 - 7.01 (m, 5H), 7.00 - 6.93 (m, 3H), 2.50 (s, 3H), 1.84 (s, 6H). | | 434 | 98% |
| 117 | 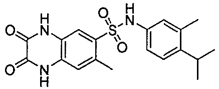 | M1, M2 | ¹H NMR (400 MHz, DMSO-*d*₆) δ 12.04 (s, 1H), 11.90 (s, 1H), 10.15 (s, 1H), 7.71 (s, 1H), 7.05 - 7.01 (m, 1H), 6.97 (d, *J* = 0.8 Hz, 1H), 6.82 (d, *J* = 7.6 Hz, 2H), 2.94 (p, *J* = 6.9 Hz, 1H), 2.50 (s, 3H), 2.15 (s, 3H), 1.06 (d, *J* = 6.8 Hz, 6H). | 388 | | 100% |
| 118 | 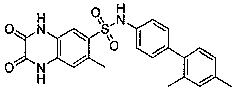 | M1, M2, M3 | ¹H NMR (400 MHz, DMSO-*d*₆) δ 7.73 (s, 1H), 7.09 - 7.01 (m, 4H), 6.99 (d, *J* = 0.8 Hz, 1H), 6.96 (s, 1H), 6.91 (t, *J* = 1.0 Hz, 2H), 2.51 (s, 3H), 2.21 (s, 3H), 2.04 (s, 3H). | | 434 | 95% |
| 119 | 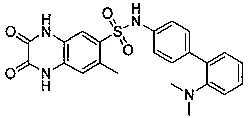 | M1, M2, M3 | ¹H NMR (400 MHz, DMSO-*d*₆) δ 12.06 (s, 1H), 11.91 (s, 1H), 10.39 (s, 1H), 7.74 (s, 1H), 7.34 (d, *J* = 8.3 Hz, 2H), 7.22 (s, 1H), 7.15 - 6.90 (m, 6H), 2.52 (s, 6H), 2.40 (s, 3H). | 451 | | 98% |
| 120 | 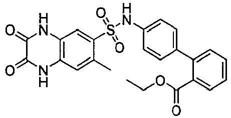 | M1, M2, M3 | ¹H NMR (400 MHz, DMSO-*d*₆) δ 12.07 (s, 1H), 11.92 (s, 1H), 10.49 (s, 1H), 7.76 (s, 1H), 7.63 (dd, *J* = 7.6, 1.4 Hz, 1H), 7.53 (td,*J* = 7.5, 1.3 Hz, 1H), 7.40 (tt, *J* = 7.5, 0.9 Hz, 1H), 7.33 - 7.27 (m, 1H), 7.14 - 7.04 (m, 4H), 6.97 (s, 1H), 3.94 - 3.80 (m, 2H), 2.52 (s, 3H), 0.72 (td, *J* = 7.1, 0.6 Hz, 3H). | 480 | | 98% |
| 121 | 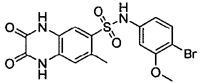 | M1, M2 | ¹H NMR (400 MHz, DMSO-*d*₆) δ 12.05 (s, 1H), 11.93 (s, 1H), 10.58 (s, 1H), 7.76 (s, 1H), 7.36 (dd, *J* = 8.5, 0.6 Hz, 1H), 6.97 (s, 1H), 6.80 (d, *J* = 2.4 Hz, 1H), 6.58 - 6.48 (m, 1H), 3.73 (s, 3H), 2.50 (s, 3H). | 440 | | 100% |
| 122 | 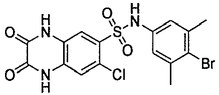 | M1, M2 | ¹H NMR (400 MHz, DMSO-*d*₆) δ 12.03 (s, 1H), 11.89 (s, 1H), 10.40 (s, 1H), 7.71 (s, 1H), 6.96 (s, 1H), 6.85 (s, 2H), 2.49 (s, 3H), 2.21 (s, 6H). | 438 | | 100% |
| 123 | 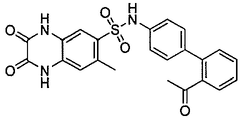 | M1, M2, M3 | ¹H NMR (400 MHz, DMSO-*d*₆) δ 12.06 (s, 1H), 11.91 (s, 1H), 10.50 (s, 1H), 7.73 (s, 1H), 7.53 - 7.24 (m, 4H), 7.15 - 7.05 (m, 4H), 6.97 (s, 1H), 2.51 (s, 3H), 1.96 (s, 3H). | 450 | | 100% |
| 124 | 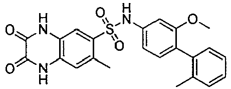 | M1, M2, M3 | ¹H NMR (400 MHz, DMSO-*d*₆) δ 12.07 (s, 1H), 11.96 (s, 1H), 10.47 (s, 1H), 7.80 (s, 1H), 7.20 - 7.14 (m, 2H), 7.14 - 7.08 (m, 1H), 6.99 (s, 1H), 6.95 (dt, *J* = 7.2, 1.1 Hz, 1H), 6.88 (d, *J* = 8.1 Hz, 1H), 6.78 (d, *J* = 2.0 Hz, 1H), 6.63 (dd, *J* = 8.1, 2.0 Hz, 1H), 3.58 (s, 3H), 2.53 (d, *J* = 0.7 Hz, 3H), 1.92 (s, 3H). | 452 | | 100% |
| 125 | 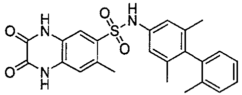 | M1, M2, M3 | ¹H NMR (400 MHz, DMSO-*d*₆) δ 11.92 (br. s, 2H), 10.23 (br. s, 1H), 7.76 (s, 1H), 7.30 - 7.14 (m, 3H), 6.99 (s, 1H), 6.90 - 6.82 (m, 1H), 6.80 (s, 2H), 2.52 (s, 3H), 1.78 (s, 3H), 1.73 (s, 6H). | 450 | | 100% |
| 126 | 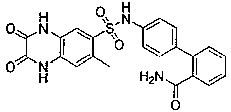 | M1, M2 | ¹H NMR (400 MHz, DMSO-*d*₆) δ 11.90 (br. s, 2H), 10.48 (br. s, 1H), 7.76 (s, 1H), 7.53 (d, *J* = 2.1 Hz, 1H), 7.41 - 7.26 (m, 3H), 7.26 - 7.14 (m, 4H), 7.06 - 6.93 (m, 3H), 2.50 (s, 3H). | 451 | | 98% |
| 127 | 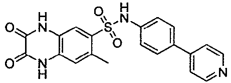 | M1, M2 | ¹H NMR (400 MHz, DMSO-*d*₆) δ 12.04 (s, 1H), 11.92 (s, 1H), 10.76 (s, 1H), 8.57 - 8.49 (m, 2H), 7.78 (s, 1H), 7.67 (d, *J* = 8.5 Hz, 2H), 7.60 - 7.55 (m, 2H), 7.20 - 7.10 (m, 2H), 6.97 (s, 1H), 2.52 (s, 3H). | 409 | | 90% |
| 128 | 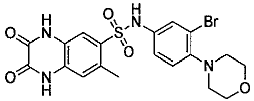 | M1, M2 | ¹H NMR (400 MHz, DMSO-*d*₆) δ 12.06 (s, 1H), 11.92 (s, 1H), 10.39 (s, 1H), 7.69 (s, 1H), 7.28 (d, *J* = 2.2 Hz, 1H), 7.06 - 6.96 (m, 3H), 3.71 - 3.60 (m, 4H), 2.88 - 2.73 (m, 4H), 2.49 (s, 3H). | 495/497 | | 97% |
| 129 | 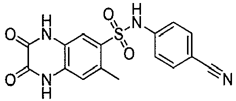 | M1, M2 | ¹H NMR (400 MHz, DMSO-*d*₆) δ 12.07 (s, 1H), 11.92 (s, 1H), 11.14 (s, 1H), 7.78 (s, 1H), 7.71 - 7.61 (m, 2H), 7.20 - 7.11 (m, 2H), 6.98 (s, 1H), 2.49 (s, 3H). | 357 | | 100% |
| 130 | 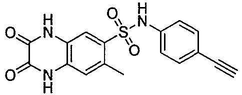 | M1, M2 | ¹H NMR (400 MHz, DMSO-ds) δ 11.90 (br. s, 2H), 10.65 (br. s, 1H), 7.73 (s, 1H), 7.34 - 7.28 (m, 2H), 7.05 - 6.99 (m, 2H), 6.96 (d, *J* = 0.8 Hz, 1H), 2.49 (s, 3H). | 356 | | 97% |
| 131 | 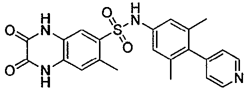 | M1, M2, M3 | ¹H NMR (400 MHz, DMSO-*d*₆) δ 12.08 (s, 1H), 11.95 (s, 1H), 10.51 (s, 1H), 8.75 (d, *J* = 5.9 Hz, 2H), 7.81 (d, *J* = 1.1 Hz, 1H), 7.48 (d, *J* = 5.9 Hz, 2H), 7.00 (d, *J* = 1.0 Hz, 1H), 6.89 - 6.84 (m, 2H), 2.54 (d, *J* = 0.9 Hz, 3H), 1.87 (d, *J* = 1.1 Hz, 6H). | 437 | | 100% |
| 132 | 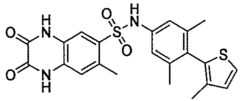 | M1, M2, M3 | ¹H NMR (400 MHz, DMSO-*d*₆) δ 12.06 (s, 1H), 11.93 (s, 1H), 10.40 (s, 1H), 7.78 (s, 1H), 7.44 (d, *J* = 5.1 Hz, 1H), 6.99 (d, *J* = 0.9 Hz, 1H), 6.96 (d, *J* = 5.1 Hz, 1H), 6.82 (s, 2H), 2.52 (s, 3H), 1.87 (s, 6H), 1.78 (s, 3H). | 456 | | 100% |
| 133 | 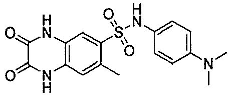 | M1, M2 | ¹H NMR (400 MHz, DMSO-*d*₆) δ 12.04 (s, 1H), 11.87 (s, 1H), 9.81 (s, 1H), 7.59 (s, 1H), 6.97 (s, 1H), 6.87 (d, *J* = 8.5 Hz, 2H), 6.68 (s, 2H), 2.82 (s, 6H), 2.49 (s, 3H). | 375 | | 100% |
| 134 | 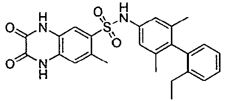 | M1, M2, M3 | ¹H NMR (400 MHz, DMSO-*d*₆) δ 12.06 (br. s, 1H), 11.94 (br. s, 1H), 10.23 (br. s, 1H), 7.75 (s, 1H), 7.32 - 7.15 (m, 3H), 6.98 (d, *J* = 0.8 Hz, 1H), 6.87 - 6.77 (m, 3H), 2.51 (s, 3H), 2.07 (q, *J* = 7.5 Hz, 2H), 1.74 (s, 6H), 0.91 - 0.82 (m, 3H). | 464 | | 100% |
| 135 | 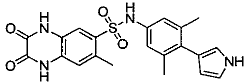 | M1, M2, M3 | ¹H NMR (400 MHz, DMSO-*d*₆) δ 12.04 (s, 1H), 11.91 (s, 1H), 10.74 (s, 1H), 10.15 (s, 1H), 7.75 (s, 1H), 6.98 (s, 1H), 6.81 - 6.71 (m, 3H), 6.51 (dq, *J* = 2.9, 1.4 Hz, 1H), 5.81 (dp, *J* = 2.6, 1.2 Hz, 1H), 2.52 (s, 3H), 1.95 (s, 6H). | 425 | | 100% |
| 136 | 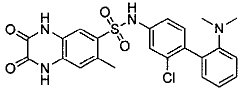 | M1, M2, M3 | ¹H NMR (300 MHz, Methanol-*d*₄) δ 7.93 (s, 1H), 7.77 (d, *J* = 8.2 Hz, 1H), 7.63 (dd, *J* = 8.6, 7.1 Hz, 1H), 7.48 (t, *J* = 7.5 Hz, 1H), 7.37 (d, *J* = 2.2 Hz, 1H), 7.31 (d, *J* = 8.4 Hz, 1H), 7.20 (ddd,*J* = 8.4, 4.4, 1.9 Hz, 2H), 7.11 (s, 1H), 3.08 (s, 6H), 2.67 (s, 3H). | 485 | | 94% |
| 137 | 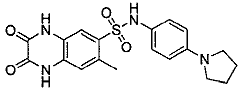 | M1, M2 | ¹H NMR (400 MHz, DMSO-*d*₆) δ 12.03 (s, 1H), 11.85 (s, 1H), 9.56 (s, 1H), 7.53 (s, 1H), 6.97 (s, 1H), 6.84 - 6.76 (m, 2H), 6.36 (d, *J* = 8.5 Hz, 2H), 3.14 - 3.04 (m, 4H), 2.49 (d, *J* = 1.9 Hz, 3H), 1.92 - 1.83 (m, 4H). | 401 | | 100% |
| 138 | 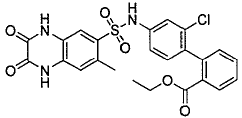 | M1, M2, M3 | ¹H NMR (400 MHz, DMSO-*d*₆) δ 12.10 (s, 1H), 11.97 (s, 1H), 10.74 (s, 1H), 7.87 - 7.82 (m, 1H), 7.80 (s, 1H), 7.63 - 7.56 (m, 1H), 7.49 (td, *J* = 7.7, 1.3 Hz, 1H), 7.23 - 7.18 (m, 1H), 7.17 - 7.11 (m, 2H), 7.05 (dd, *J* = 8.2, 2.0 Hz, 1H), 7.01 (s, 1H), 3.92 - 3.77 (m, 2H), 2.54 (s, 3H), 0.69 (td, *J* = 7.1, 0.8 Hz, 3H). | 514 | | 98% |
| 139 | 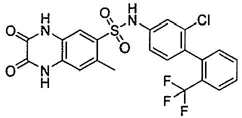 | M1, M2, M3 | ¹H NMR (400 MHz, DMSO-*d*₆) δ 12.10 (s, 1H), 11.96 (s, 1H), 10.79 (s, 1H), 7.78 (d, *J* = 5.4 Hz, 2H), 7.67 (t, *J* = 7.5 Hz, 1H), 7.59 (t, J = 7.6 Hz, 1H), 7.26 (d, *J* = 7.5 Hz, 1H), 7.21 - 7.14 (m, 2H), 7.05 - 6.99 (m, 2H), 2.52 (s, 3H). | 510 | | 98% |
| 140 | 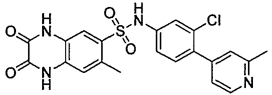 | M1, M2, M3 | ¹H NMR (300 MHz, DMSO-*d*₆) δ 12.12 (s, 1H), 11.99 (s, 1H), 10.94 (s, 1H), 8.46 (d, *J* = 5.2 Hz, 1H), 7.81 (s, 1H), 7.32 (d, *J* = 8.4 Hz, 1H), 7.24 (t, *J* = 2.4 Hz, 2H), 7.18 (d, *J* = 5.3 Hz, 1H), 7.14 - 7.06 (m, 1H), 7.02 (s, 1H), 2.55 (s, 3H), 1.35 (s, 3H). | 457 | | 100% |
| 141 | 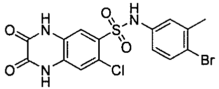 | M1, M2 | ¹H NMR (400 MHz, DMSO-*d*₆) δ 12.11 (s, 1H), 11.99 (s, 1H), 10.66 (s, 1H), 7.79 (s, 1H), 7.41 (d, *J* = 8.6 Hz, 1H), 7.16 (s, 1H), 7.07 - 7.01 (m, 1H), 6.84 (dd, *J* = 8.6, 2.8 Hz, 1H), 2.21 (s, 3H). | 446 | | 100% |
| 142 | 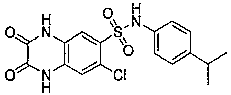 | M1, M2 | ¹H NMR (400 MHz, Methanol-*d*₄) δ 7.79 (d, *J* = 0.3 Hz, 1H), 7.25 (d, *J* = 0.3 Hz, 1H), 7.05 (s, 4H), 2.77 (p, *J* = 6.9 Hz, 1H), 1.13 (d, *J* = 6.9 Hz, 6H). | 394 | | 94% |
| 143 | 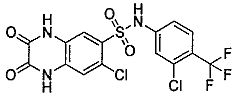 | M1, M2 | ¹H NMR (400 MHz, Methanol-*d*₄) δ 7.95 (s, 1H), 7.58 (d, *J* = 8.7 Hz, 1H), 7.36 - 7.31 (m, 1H), 7.26 (s, 1H), 7.19 (ddt, *J* = 8.7, 1.7, 0.9 Hz, 1H). | 454 | | 95% |
| 144 | 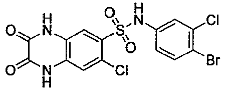 | M1, M2 | ¹H NMR (400 MHz, DMSO-*d*₆) δ 12.12 (s, 1H), 12.01 (s, 1H), 11.00 (s, 1H), 7.81 (s, 1H), 7.61 (d, *J* = 8.8 Hz, 1H), 7.26 (d, *J* = 2.5 Hz, 1H), 7.17 (s, 1H), 6.96 (dd, *J* = 8.7, 2.6 Hz, 1H). | | 464 | 100% |
| 145 | 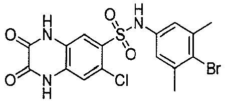 | M1, M2 | ¹H NMR (400 MHz, Methanol-*d*₄) δ 7.83 (s, 1H), 7.24 (s, 1H), 6.92 (p, *J* = 0.6 Hz, 2H), 2.26 (t, *J* = 0.6 Hz, 6H). | 460 | | 100% |
| 146 | 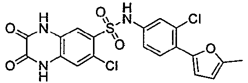 | M1, M2, M3 | ¹H NMR (400 MHz, Methanol-*d*₄) δ 7.88 (s, 1H), 7.62 (d, *J* = 8.7 Hz, 1H), 7.24 (d, *J* = 2.5 Hz, 2H), 7.11 (dd, *J* = 8.7, 2.3 Hz, 1H), 6.83 (d, *J* = 3.3 Hz, 1H), 6.08 (dp, *J* = 2.1, 1.4, 1.0 Hz, 1H), 2.31 - 2.27 (m, 3H). | 466 | | 100% |
| 147 | 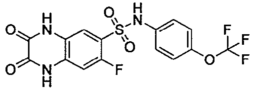 | M1, M2 | ¹H NMR (400 MHz, Methanol-*d*₄) δ 7.52 (d, *J* = 6.3 Hz, 1H), 7.16 - 7.10 (m, 2H), 7.08 - 7.01 (m, 2H), 6.94 (d, *J* = 10.4 Hz, 1H). | 420 | | 98% |
| 148 | 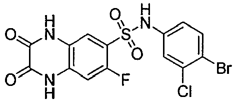 | M1, M2 | ¹H NMR (400 MHz, Methanol-*d*₄) δ 7.62 (d, *J* = 6.4 Hz, 1H), 7.48 (d, *J* = 8.7 Hz, 1H), 7.31 (d, *J* = 2.5 Hz, 1H), 7.05 - 6.93 (m, 2H). | | 448 | 99% |
| 149 | 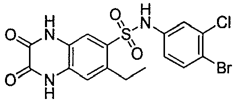 | M1, M2 | ¹H NMR (400 MHz, DMSO-*d*₆) δ 12.04 (s, 1H), 11.93 (s, 1H), 10.88 (s, 1H), 7.72 (d, *J* = 1.5 Hz, 1H), 7.60 (dd, *J* = 8.8, 1.5 Hz, 1H), 7.23 (t, *J* = 2.1 Hz, 1H), 7.06 (d, J = 1.5 Hz, 1H), 6.92 *(dt, J* = 8.8, 2.0 Hz, 1H), 2.99 - 2.88 (m, 2H), 1.14 (td, *J* = 7.4, 1.5 Hz, 3H). | | 458 | 100% |
| 150 | 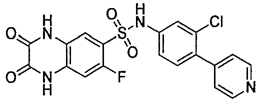 | M1, M2, M3 | ¹H NMR (400 MHz, DMSO-*d*₆) δ 12.20 (s, 1H), 11.99 (s, 1H), 11.19 (s, 1H), 8.77 - 8.69 (m, 2H), 7.68 - 7.60 (m, 3H), 7.43 (d, *J* = 8.4 Hz, 1H), 7.32 (d, *J* = 2.2 Hz, 1H), 7.19 (dd, *J* = 8.4, 2.2 Hz, 1H), 7.01 (d, *J* = 10.6 Hz, 1H). | 447 | | 98% |
| 151 | 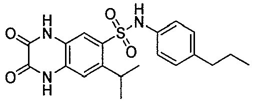 | M1, M2 | ¹H NMR (400 MHz, Methanol-*d*₄) δ 7.76 (s, 1H), 7.21 (s, 1H), 6.98 (q, *J* = 8.5 Hz, 4H), 3.87 (p, *J* = 6.8 Hz, 1H), 2.50 - 2.40 (m, 2H), 1.54 (h, *J* = 7.4 Hz, 2H), 1.21 (d, *J* = 6.8 Hz, 6H), 0.86 (t, *J* = 7.3 Hz, 3H). | | 400 | 98% |
| 152 | 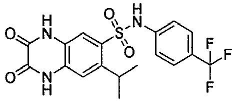 | M1, M2 | ¹H NMR (400 MHz, Methanol-*d*₄) δ 7.87 (s, 1H), 7.50 (s, 1H), 7.48 (d, *J* = 2.2 Hz, 1H), 7.23 (s, 2H), 7.21 (s, 1H), 3.89 (p,*J* = 6.8 Hz, 1H), 1.22 (d, *J* = 6.8 Hz, 6H). | | 426 | 100% |
| 153 | 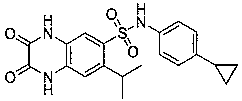 | M1, M2 | ¹H NMR (400 MHz, DMSO-*d*₆) δ 11.92 (s, 1H), 11.88 (s, 1H), 10.24 (s, 1H), 7.68 (s, 1H), 7.15 (s, 1H), 6.90 (d, *J* = 1.1 Hz, 4H), 3.79 (p, *J* = 6.6 Hz, 1H), 1.76 (tt, *J* = 8.3, 5.0 Hz, 1H), 1.10 (d, *J* = 6.7 Hz, 6H), 0.87 - 0.78 (m, 2H), 0.55 - 0.48 (m, 2H). | 400 | | 95% |
| 154 | 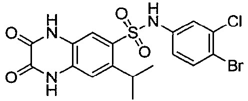 | M1, M2 | ¹H NMR (400 MHz, DMSO-*d*₆) δ 11.95 (s, 1H), 11.92 (s, 1H), 10.91 (s, 1H), 7.72 (s, 1H), 7.61 (d, *J* = 8.7 Hz, 1H), 7.24 (d, *J* = 2.5 Hz, 1H), 7.18 (s, 1H), 6.92 (dd, *J =* 8.8, 2.6 Hz, 1H), 3.76 (p, *J* = 6.7 Hz, 1H), 1.11 (d, *J* = 6.7 Hz, 6H). | | 472 | 100% |
| 155 | 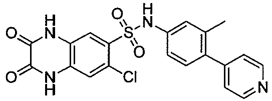 | M1, M2, M3 | ¹H NMR (400 MHz, DMSO-*d*₆) δ 12.14 (s, 1H), 12.04 (s, 1H), 10.86 (s, 1H), 8.76 - 8.71 (m, 2H), 7.89 (s, 1H), 7.67 (d, *J* = 5.6 Hz, 2H), 7.25 - 7.17 (m, 2H), 7.09 - 7.01 (m, 2H), 2.20 (s, 3H). | 443 | | 100% |
| 156 | 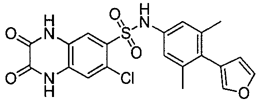 | M1, M2, M3 | ¹H NMR (400 MHz, Methanol-*d*₄) δ 7.87 (s, 1H), 7.55 (t,*J* = 1.7 Hz, 1H), 7.28 (dd, *J* = 1.6, 0.9 Hz, 1H), 7.25 (s, 1H), 6.89 (p, *J* = 0.6 Hz, 2H), 6.25 (dd, *J* = 1.8, 0.9 Hz, 1H), 2.01 (s, 6H). | | 444 | 100% |
| 157 | 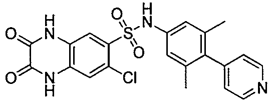 | M1, M2, M3 | ¹H NMR (400 MHz, Methanol-*d*₄) δ 8.85 - 8.81 (m, 2H), 7.92 (s, 1H), 7.82 - 7.78 (m, 2H), 7.27 (s, 1H), 7.00 (q, *J* = 0.6 Hz, 2H), 1.98 (s, 6H). | | 455 | 100% |
| 158 | 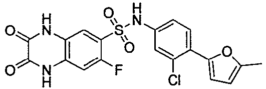 | M1, M2, M3 | ¹H NMR (400 MHz, DMSO-*d*₆) δ 12.15 (s, 1H), 11.95 (s, 1H), 10.93 (s, 1H), 7.68 - 7.56 (m, 2H), 7.22 (d, *J* = 2.2 Hz, 1H), 7.12 (dd, *J* = 8.7, 2.3 Hz, 1H), 6.97 (d, *J* = 10.6 Hz, 1H), 6.86 (d, *J* = 3.4 Hz, 1H), 6.23 - 6.17 (m, 1H), 2.29 (s, 3H). | | 448 | 91% |
| 159 | 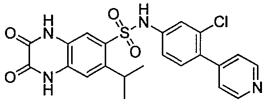 | M1, M2, M3 | ¹H NMR (400 MHz, DMSO-*d*₆) δ 11.97 (s, 1H), 11.95 (s, 1H), 11.04 (s, 1H), 8.70 - 8.65 (m, 2H), 7.79 (s, 1H), 7.55 (d, *J* = 5.2 Hz, 2H), 7.39 (dd, *J* = 8.5, 1.3 Hz, 1H), 7.25 (d, *J* = 2.0 Hz, 1H), 7.20 (s, 1H), 7.12 (ddd, *J* = 8.5, 2.2, 0.7 Hz, 1H), 3.87 - 3.75 (m, 1H), 1.14 (d, *J* = 6.7 Hz, 6H). | 471 | | 100% |
| 160 | 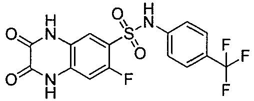 | M1, M2 | ¹H NMR (400 MHz, Methanol-*d*₄) δ 7.67 (d, *J* = 6.4 Hz, 1H), 7.50 (d, *J* = 8.5 Hz, 2H), 7.29 (d, *J* = 8.5 Hz, 2H), 6.97 (d, *J* = 10.4 Hz, 1H). | | 402 | 98% |
| 161 | 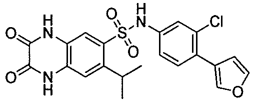 | M1, M2, M3 | ¹H NMR (400 MHz, DMSO-*d*₆) δ 11.97 - 11.90 (m, 2H), 10.80 (s, 1H), 7.98 (s, 1H), 7.75 (s, 1H), 7.70 (t, *J* = 1.8 Hz, 1H), 7.42 (d, *J* = 8.4 Hz, 1H), 7.17 (s, 2H), 7.01 (d, *J* = 8.6 Hz, 1H), 6.77 (dt, *J* = 1.6, 0.7 Hz, 1H), 3.86 - 3.74 (m, 1H), 1.13 (d, *J* = 6.7 Hz, 6H). | 460 | | 100% |
| 162 | 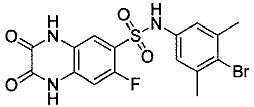 | M1, M2 | ¹H NMR (400 MHz, DMSO-*d*₆) δ 12.13 (s, 1H), 11.91 (s, 1H), 10.59 (s, 1H), 7.55 (d, *J* = 6.5 Hz, 1H), 6.95 (d, *J* = 10.6 Hz, 1H), 6.90 (s, 2H), 2.21 (s, 6H). | | 442 | 97% |
| 163 | 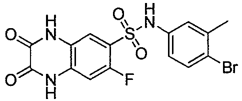 | M1, M2 | ¹H NMR (400 MHz, DMSO-*d*₆) δ 12.13 (s, 1H), 11.92 (s, 1H), 10.65 (s, 1H), 7.54 (d, *J* = 6.6 Hz, 1H), 7.41 (d, *J* = 8.6 Hz, 1H), 7.05 (d, *J* = 2.7 Hz, 1H), 6.95 (d, *J* = 10.6 Hz, 1H), 6.85 (dd, *J* = 8.6, 2.7 Hz, 1H), 2.21 (s, 3H). | | 428/426 | 96% |
| 164 | 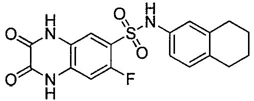 | M1, M2 | ¹H NMR (400 MHz, DMSO-*d*₆) δ 12.08 (br. s, 1H), 11.91 (br. s, 1H), 10.31 (br. s, 1H), 7.52 (d, *J* = 6.6 Hz, 1H), 6.95 (d, *J* = 10.5 Hz, 1H), 6.87 (d, *J* = 8.1 Hz, 1H), 6.82 - 6.75 (m, 2H), 2.56 (d, *J* = 4.0 Hz, 4H), 1.63 (p, *J* = 3.3 Hz, 4H). | | 388 | 100% |
| 165 | 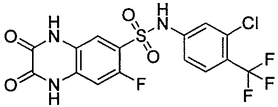 | M1, M2 | ¹H NMR (400 MHz, DMSO-*d*₆) δ 12.19 (s, 1H), 11.99 (s, 1H), 11.45 (br. s, 1H), 7.73 (d, *J* = 8.7 Hz, 1H), 7.64 (d, *J* = 6.6 Hz, 1H), 7.33 (d, *J* = 2.2 Hz, 1H), 7.19 (dd, *J* = 8.8, 2.1 Hz, 1H), 7.00 (d, *J* = 10.7 Hz, 1H). | | 436 | 100% |
| 166 | 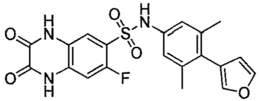 | M1, M2, M3 | ¹H NMR (400 MHz, DMSO-*d*₆) δ 12.15 (s, 1H), 11.94 (s, 1H), 10.50 (s, 1H), 7.70 (d, *J* = 1.7 Hz, 1H), 7.61 (d, *J* = 7.6 Hz, 1H), 7.59 - 7.49 (m, 1H), 6.98 (d, *J* = 10.4 Hz, 1H), 6.84 (s, 2H), 6.38 (d, *J* = 1.8 Hz, 1H), 1.98 (s, 6H). | | 428 | 95% |
| 167 | 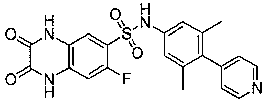 | M1, M2, M3 | ¹H NMR (400 MHz, DMSO-*d*₆) δ 12.17 (s, 1H), 11.96 (s, 1H), 10.69 (s, 1H), 8.73 (d, *J* = 5.3 Hz, 2H), 7.63 (d, *J* = 6.5 Hz, 1H), 7.44 (d, *J* = 5.3 Hz, 2H), 7.00 (d, *J* = 10.6 Hz, 1H), 6.90 (s, 2H), 1.88 (s, 6H). | | 439 | 100% |
| 168 | 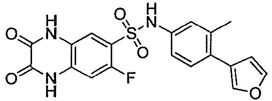 | M1, M2, M3 | ¹H NMR (400 MHz, DMSO-*d*₆) δ 12.13 (s, 1H), 11.93 (s, 1H), 10.56 (s, 1H), 7.77 (t, *J* = 1.2 Hz, 1H), 7.68 (t, *J* = 1.7 Hz, 1H), 7.57 (d, *J* = 6.5 Hz, 1H), 7.22 (d, *J* = 8.3 Hz, 1H), 7.02 - 6.92 (m, 3H), 6.69 (dd, *J* = 1.8, 0.9 Hz, 1H), 2.24 (s, 3H). | | 414 | 96% |
| 169 | 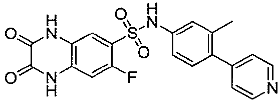 | M1, M2, M3 | ¹H NMR (400 MHz, Methanol-*d*₄) δ 8.75 (d, *J* = 6.4 Hz, 2H), 7.93 - 7.86 (m, 2H), 7.69 (d, *J* = 6.4 Hz, 1H), 7.27 (d, *J* = 9.0 Hz, 1H), 7.19 (dd, *J =* 6.1, 2.4 Hz, 2H), 7.00 (d, *J* = 10.4 Hz, 1H), 2.29 (s, 3H). | | 425 | 100% |
| 170 | 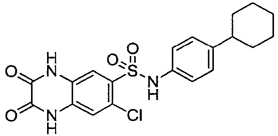 | M1, M2, M3 | ¹H NMR (400 MHz, DMSO-*d*₆) δ 12.10 (s, 1H), 11.99 (s, 1H), 10.39 (s, 1H), 7.78 (d, *J* = 0.8 Hz, 1H), 7.17 (d, *J* = 0.8 Hz, 1H), 7.05 (d, *J* = 8.4 Hz, 2H), 6.98 (d, *J* = 8.5 Hz, 2H), 2.39 - 2.27 (m, 1H), 1.73 - 1.63 (m, 5H), 1.35 - 1.18 (m, 5H). | 434 | | 98% |
| 171 | 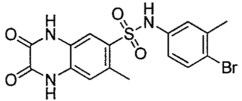 | commerci al | 894918-88-8 | | | |
| 172 | 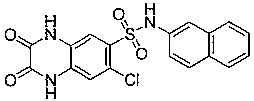 | M1, M2 | ¹H NMR (400 MHz, DMSO-*d*₆) δ 12.06 (br. s, 1H), 11.96 (br. s, 1H), 10.78 (br. s, 1H), 7.86 (s, 1H), 7.82 - 7.73 (m, 2H), 7.70 (d, *J* = 8.2 Hz, 1H), 7.51 (d, *J* = 2.2 Hz, 1H), 7.47 - 7.32 (m, 2H), 7.30 (dd, *J* = 8.8, 2.2 Hz, 1H), 7.14 (s, 1H). | 402 | | 100% |
| 173 | 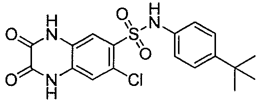 | M1, M2 | ¹H NMR (400 MHz, DMSO-d₆) δ 12.12 (s, 1H), 12.00 (s, 1H), 10.42 (s, 1H), 7.79 (s, 1H), 7.27 - 7.16 (m, 3H), 7.03 - 6.96 (m, 2H), 1.17 (s, 9H). | 408 | | 100% |
| 174 | 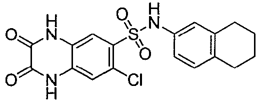 | M1, M2 | ¹H NMR (400 MHz, DMSO-*d*₆) δ 12.09 (s, 1H), 11.97 (s, 1H), 10.29 (s, 1H), 7.76 (s, 1H), 7.16 (s, 1H), 6.86 (d, *J* = 8.2 Hz, 1H), 6.83 - 6.74 (m, 2H), 2.59 - 2.52 (m, 4H), 1.62 (h, *J* = 3.1 Hz, 4H). | 406 | | 97% |
| 175 | 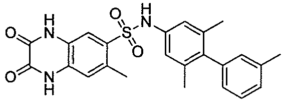 | M1, M2, M3 | ¹H NMR (400 MHz, DMSO-*d*₆) δ 12.07 (br. s, 1H), 11.93 (br. s, 1H), 10.30 (br. s, 1H), 7.78 (s, 1H), 7.26 (t, *J* = 7.6 Hz, 1H), 7.10 (d, *J* = 7.5 Hz, 1H), 7.00 (s, 1H), 6.81 (d, *J* = 12.5 Hz, 4H), 2.53 (s, 3H), 2.28 (s, 3H), 1.83 (s, 6H). | 450 | | 100% |
| 176 | 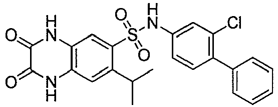 | M1, M2, M3 | ¹H NMR (400 MHz, DMSO-*d*₆) δ 11.96 (s, 1H), 10.84 (s, 1H), 7.78 (s, 1H), 7.42 - 7.30 (m, 5H), 7.27 (d, *J* = 8.4 Hz, 1H), 7.21 (d, *J* = 3.6 Hz, 2H), 7.07 (d, *J* = 8.0 Hz, 1H), 3.89-3.73 (m, 1H), 1.14 (d, *J* = 6.7 Hz, 6H). | 470 | | 92% |
| 177 | 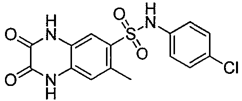 | commerci al | 931964-86-2 | | | |
| 178 | 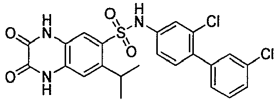 | M1, M2, M3 | ¹H NMR (400 MHz, DMSO-*d*₆) δ 11.97 (s, 1H), 11.96 (s, 1H), 10.91 (s, 1H), 7.79 (s, 1H), 7.44 - 7.41 (m, 2H), 7.38 (dd, *J* = 2.0, 1.0 Hz, 1H), 7.34 - 7.26 (m, 2H), 7.24 - 7.19 (m, 2H), 7.08 (dd, *J* = 8.4, 2.3 Hz, 1H), 3.82 (p, *J* = 6.9 Hz, 1H), 1.14 (d, *J* = 6.7 Hz, 6H). | 504 | | 100% |
| 179 | 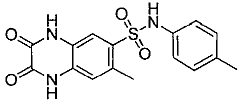 | commerci al | 894921-04-1 | | | |
| 180 | 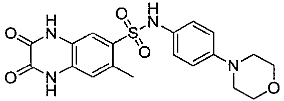 | M1, M2 | ¹H NMR (400 MHz, DMSO-*d*₆) δ 7.58 (s, 1H), 6.99 (d, *J* = 0.9 Hz, 1H), 6.91 - 6.82 (m, 2H), 6.79 - 6.70 (m, 2H), 3.66 - 3.59 (m, 5H), 2.97 - 2.90 (m, 4H), 2.48 - 2.43 (m, 3H). | 417 | | 99% |
| 181 | 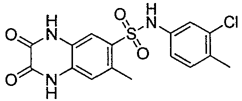 | commerci al | 894917-28-3 | | | |
| 182 | 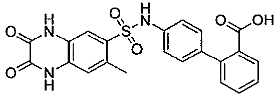 | M1, M2, M3 + hydrolysis | ¹H NMR (400 MHz, DMSO-*d*₆) δ 12.12 - 11.79 (m2H), 10.48 (br. s, 1H), 7.78 (d,*J* = 1.3 Hz, 1H), 7.62 (dt,*J* = 7.7, 1.4 Hz, 1H), 7.47 (tt, *J* = 7.6, 1.3 Hz, 1H), 7.36 (tt, *J* = 7.6, 1.3 Hz, 1H), 7.25 (dd, *J* = 7.8, 1.2 Hz, 1H), 7.17 (dd, *J* = 8.3, 1.1 Hz, 2H), 7.08 - 7.01 (m, 2H), 6.99 (s, 1H), 2.53 - 2.50 (m, 3H). | 452 | | 93% |
| 183 | 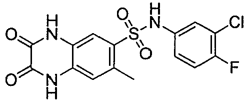 | commerci al | 894919-92-7 | | | |
| 184 | 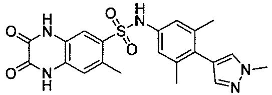 | M1, M2, M3 | ¹H NMR (400 MHz, DMSO-*d*₆) δ 12.05 (s, 1H), 11.93 (s, 1H), 10.27 (s, 1H), 7.76 (s, 1H), 7.55 (s, 1H), 7.25 (d, *J* = 0.7 Hz, 1H), 6.98 (s, 1H), 6.79 (s, 2H), 3.82 (s, 3H), 2.52 (s, 3H), 1.96 (s, 6H). | 440 | | 100% |
| 185 | 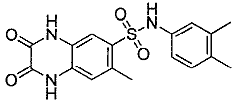 | commerci al | 894918-00-4 | | | |
| 186 | 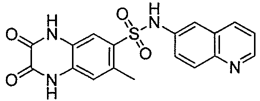 | M1, M2 | ¹H NMR (400 MHz, DMSO-*d*₆) δ 12.00 (br. s, 1H), 11.87 (s, 1H), 8.73 (dd, *J* = 4.3, 1.6 Hz, 1H), 8.18 - 8.09 (m, 2H), 7.88 (d, *J* = 8.8 Hz, 1H), 7.78 (s, 1H), 7.50 (d, *J* = 8.6 Hz, 2H), 7.43 (dd, *J* = 8.3, 4.2 Hz, 1H), 6.95 (s, 1H), 2.53 (s, 3H). | 383 | | 100% |
| 187 | 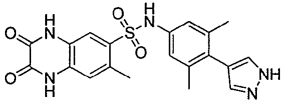 | M1, M2, M3 | ¹H NMR (400 MHz, DMSO-*d*₆) δ 12.06 (s, 1H), 11.92 (s, 1H), 10.41 (s, 1H), 7.78 (s, 1H), 6.99 (s, 1H), 6.81 (s, 2H), 6.76 (dt, *J* = 3.3, 1.2 Hz, 1H), 6.59 - 6.49 (m, 1H), 2.52 (s, 3H), 2.42 (d, *J* = 1.1 Hz, 3H), 1.96 (s, 6H). | 426 | | 100% |
| 188 | 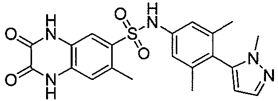 | M1, M2, M3 | ¹H NMR (400 MHz, DMSO-*d*₆) δ 12.07 (s, 1H), 11.94 (s, 1H), 10.48 (s, 1H), 7.79 (s, 1H), 7.44 (d, *J* = 1.8 Hz, 1H), 6.99 (s, 1H), 6.85 (s, 2H), 6.04 (d, *J* = 1.9 Hz, 1H), 2.52 (s, 3H), 1.84 (s, 6H). | 440 | | 94% |
| 189 | 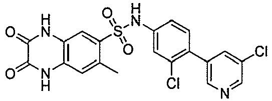 | M1, M2, M3 | ¹H NMR (400 MHz, DMSO-*d*₆) δ 12.10 (s, 1H), 11.96 (s, 1H), 10.94 (s, 1H), 8.62 (d, *J* = 2.3 Hz, 1H), 8.50 (d, *J* = 1.9 Hz, 1H), 7.96 (t, *J* = 2.1 Hz, 1H), 7.81 (s, 1H), 7.39 (d, *J* = 8.4 Hz, 1H), 7.26 (d, *J* = 2.2 Hz, 1H), 7.11 (dd, *J* = 8.5, 2.2 Hz, 1H), 7.02 (s, 1H), 2.54 (s, 3H). | 477 | | 100% |
| 190 | 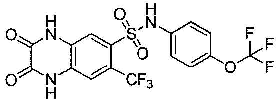 | described | ¹H NMR (500 MHz, DMSO-*d*₆) δ 12.27 (br. s, 2 H), 10.87 (s, 1 H), 7.86 (s, 1 H), 7.59 (s, 1 H), 7.29 (d, *J* = 8.9 Hz, 2 H), 7.18 (d, *J* = 8.9 Hz, 2 H). | 470 | | 98% |
| 191 | 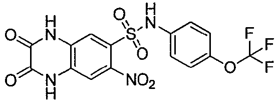 | Nitration, M2 | ¹H NMR (700 MHz, DMSO-*d*₆) δ 12.34 (s, 1 H), 12.30 (s, 1 H), 10.84 (s, 1 H), 7.69 (s, 1 H), 7.62 (s, 1 H), 7.32 (d, *J* = 9.1 Hz, 2 H), 7.23 (d, *J* = 9.1 Hz, 2 H). | 447 | | 95% |
| 192 | 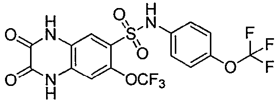 | M1, M2 | ¹H NMR (500 MHz, DMSO-*d*₆) δ 12.20 - 11-99 (m, 2 H), 10.74 (s, 1 H), 7.73 (s, 1 H), 7.28 (d, *J* = 9.0 Hz, 2 H), 7.21 (s, 1 H), 7.17 (d, *J* = 9.0 Hz, 2 H). | 486 | | 100% |
| 193 | 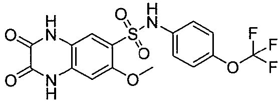 | M1, M2 | ¹H NMR (500 MHz, DMSO-*d*₆) δ 12.01 (s, 1 H), 11.83 (s, 1 H), 10.24 (s, 1 H), 7.60 (s, 1 H), 7.24 (d, *J* = 9.1 Hz, 2 H), 7.17 (d, *J* = 9.1 Hz, 2 H), 6.78 (s, 1 H), 3.80 (s, 3 H). | 432 | | 99% |
| 194 | 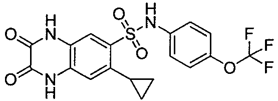 | described | ¹H NMR (500 MHz, DMSO-*d*₆) δ 11.93 (s, 1 H), 11.90 (s, 1 H), 10.64 (s, 1 H), 7.76 (s, 1H), 7.26 (d, *J* = 9.1 Hz, 2 H), 7.14 (d, *J* = 9.1 Hz, 2 H), 6.71 (s, 1 H), 2.63 - 2.55 (m, 1 H), 1.10 - 1.04 (m, 2 H), 0.60 - 0.55 (m, 2 H). | 442 | | 100% |
| 195 | 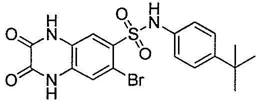 | M1, M2 | ¹H NMR (500 MHz, DMSO-*d*₆) δ 7.87 (s, 1 H), 7.38 (s, 1 H), 7.24 (d, *J* = 8.8 Hz, 2 H), 7.01 (d, *J* = 8.8 Hz, 2 H), 1.18 (s, 9 H). | 452 | | 100% |
| 196 | 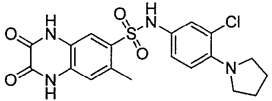 | M1, (M4, M5), M2 | ¹H NMR (400 MHz, DMSO-*d*₆) δ 12.07 (s, 1H), 11.91 (s, 1H), 10.10 (s, 1H), 7.65 (s, 1H), 7.06 - 6.96 (m, 2H), 6.93 - 6.76 (m, 2H), 3.22 - 3.12 (m, 4H), 2.50 (s, 3H), 1.87 - 1.75 (m, 4H). | 435 | | 100% |
| 197 | 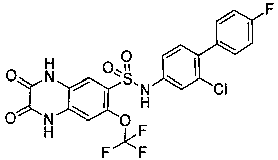 | M1, M2 | ¹H NMR (500 MHz, DMSO-*d*₆) δ 12.14 (s, 1H), 12.12 (s, 1H), 10.96 (s, 1H), 7.77 (s, 1H), 7.41 - 7.36 (m, 2H), 7.32 (d, *J* = 8.4 Hz, 1H), 7.29 - 7.22 (m, 4H), 7.11 (dd, *J =* 8.4, 2.2 Hz, 1H). | 530 | | 100% |
| 198 | 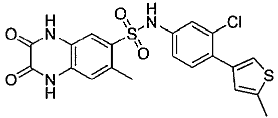 | M1 / M3, M2 | | 462 | | 98% |
| 199 | 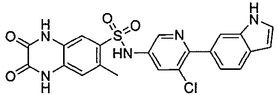 | M1 / M3, M2 | | 482 | | 99% |
| 200 | 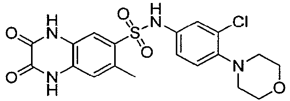 | M1, (M4, M5), M2 | ¹H NMR (400 MHz, DMSO-*d*₆) δ 12.06 (s, 1H), 11.92 (s, 1H), 10.39 (s, 1H), 7.69 (s, 1H), 7.10 (d, *J* = 2.5 Hz, 1H), 7.04 - 6.93 (m, 3H), 3.68 - 3.63 (m, 4H), 2.86 - 2.78 (m, 4H). | 451 | | 100% |
| 201 | 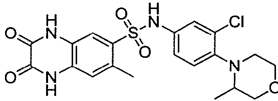 | M1, (M4, M5), M2 | ¹H NMR (400 MHz, DMSO-*d*₆) δ 12.09 (s, 1H), 11.95 (s, 1H), 10.48 (s, 1H), 7.72 (s, 1H), 7.14 (d, *J=* 8.8 Hz, 1H), 7.10 (d, *J=* 2.4 Hz, 1H), 7.00 (s, 1H), 6.98-6.95 (m, 1H), 3.76-3.59 (m, 3H), 3.26-3.19 (m, 2H), 2.90 (d, *J* = 12.0 Hz, 1H), 2.57-2.54 (m, 4H), 0.66 (d, *J* = 6.0 Hz, 3H). | 465 | | 97% |
| 202 | 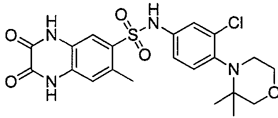 | Different route to **6Q** | | 479 | | 99% |
| 203 | 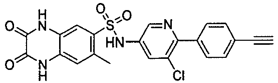 | M1, M2, M3 + TIPS deprot. | | 467 | | 98% |
| 204 | 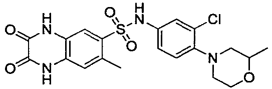 | M1, (M4, M5), M2 | ¹H NMR (400 MHz, DMSO-*d*₆) 12.08 (s, 1H), 11.93 (s, 1H), 10.39 (s, 1H), 7.70 (s, 1H), 7.11 (d, *J* = 2.5 Hz, 1H), 7.06 - 6.93 (m, 3H), 3.81 (ddd, *J* = 11.1, 3.1, 1.7 Hz, 1H), 3.74 - 3.44 (m, 5H), 2.99 (ddt, *J* = 16.4, 11.5, 2.1 Hz, 2H), 2.62 (td, *J* = 11.4, 3.0 Hz, 1H), 2.34 (dd, *J =* 11.4, 9.7 Hz, 1H), 1.06 (d, *J* = 6.2 Hz, 3H). | 465 | | 98% |
| 205 | 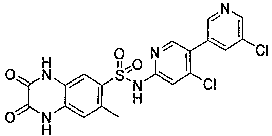 | M1 / M3, M2 | | 478 | | 99% |
| 206 | 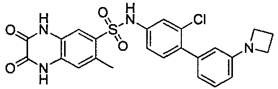 | M1 / M3, M2 | | 497 | | 99% |
| 207 | 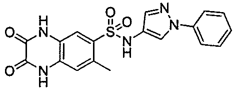 | M1, M2 | ¹H NMR (400 MHz, DMSO-*d*₆) δ 12.05 (s, 1H), 11.85 (s, 1H), 9.98 (s, 1H), 8.20 (d, J = 0.7 Hz, 1H), 7.75 - 7.68 (m, 2H), 7.60 (s, 1H), 7.48 - 7.37 (m, 3H), 7.27 (t, J = 7.4 Hz, 1H), 7.01 (s, 1H), 2.53 (s, 3H). | 398 | | 100% |
| 208 | 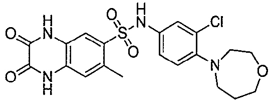 | M1, (M4, M5), M2 | ¹H NMR (400 MHz, DMSO-*d*₆) δ 12.09 (s, 1H), 11.94 (s, 1H), 10.35 (s, 1H), 7.70 (s, 1H), 7.12 - 7.04 (m, 2H), 7.00 (s, 1H), 6.92 (dd, *J* = 8.8, 2.6 Hz, 1H), 3.79 - 3.66 (m, 4H), 3.13 - 3.05 (m, 4H), 2.56 (s, 3H), 1.90 (p, *J* = 5.9 Hz, 2H). | 465 | | 98% |
| 209 | 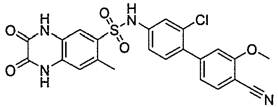 | M1 / M3, M2 | | | 495 | 98% |
| 210 | 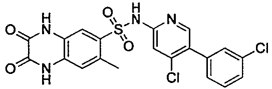 | M1/ M3, M2 | | | 475 | 100% |
| 211 | 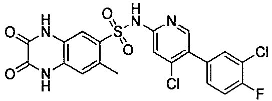 | M1/ M3, M2 | | | 493 | 100% |
| 212 | 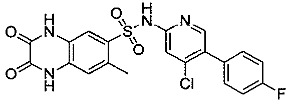 | M1, M2b, M3 | ¹H NMR (400 MHz, DMSO-*d*₆) δ 12.09 (s, 1H), 11.97 (s, 1H), 8.09 (s, 1H), 7.89 (s, 1H), 7.51 - 7.40 (m, 2H), 7.33 - 7.24 (m, 2H), 7.20 - 7.12 (m, 1H), 7.01 (s, 1H), 2.56 (s, 3H). | 461 | | 91% |
| 213 | 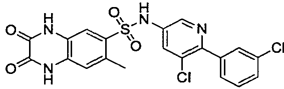 | M1, M2, M3 | ¹H NMR (400 MHz, DMSO-*d*₆) δ 12.12 (s, 1H), 11.96 (s, 1H), 11.20 (s, 1H), 8.37 (d, *J* = 2.3 Hz, 1H), 7.82 (s, 1H), 7.68 - 7.44 (m, 5H), 7.05 (s, 1H), 2.57 (s, 3H). | | 475 | 100% |
| 214 | 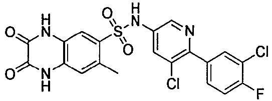 | M1, M2, M3 | ¹H NMR (400 MHz, DMSO-*d*₆) δ 12.12 (s, 1H), 11.96 (s, 1H), 11.20 (s, 1H), 8.36 (d, *J* = 2.2 Hz, 1H), 7.85 - 7.78 (m, 2H), 7.67 - 7.60 (m, 2H), 7.49 (t, *J* = 9.0 Hz, 1H), 7.05 (s, 1H), 2.56 (s, 3H). | 495 | | 100% |
| 215 | 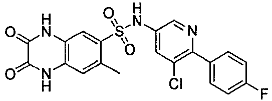 | M1, M2, M3 | ¹H NMR (400 MHz, DMSO-*d*₆) δ 12.12 (s, 1H), 11.96 (s, 1H), 11.14 (s, 1H), 8.35 (d, J = 2.3 Hz, 1H), 7.81 (s, 1H), 7.64 (ddd, J = 12.7, 6.4, 2.2 Hz, 3H), 7.34 - 7.23 (m, 2H), 7.05 (s, 1H), 2.56 (s, 3H). | 461 | | 100% |
| 216 | 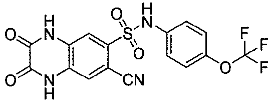 | M2, different route | | 427 | | 100% |
| 217 | 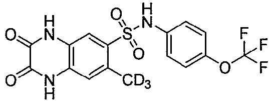 | **12D,** M1, M2 | | 419 | | 100% |
| 218 | 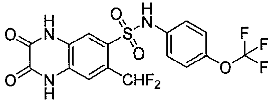 | M2, different route | | 452 | | 100% |
| 219 | 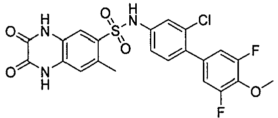 | M1, M2, M3 | | 508 | | 99% |
| 220 | 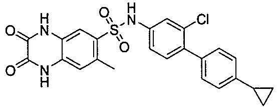 | M1, M2, M3 | | 482 | | 98% |
| 221 | 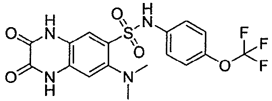 | described | ¹H-NMR (500 MHz, DMSO-*d*₆) δ 11.98 (s, 1H), 11.93 (s, 1H), 10.13 (s, 1H), 7.68 (s, 1H), 7.24-7.20 (m, 2H), 7.20-7.17 (m, 2H), 7.08 (s, 1H), 2.56 (s, 6H). | 445 | | 98% |
| 222 | 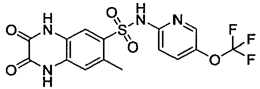 | M1, M2b | ¹H NMR (400 MHz, DMSO-*d*₆) δ 12.09 (s, 1H), 11.96 (s, 1H), 11.49 (s, 1H), 8.20 (d, *J* = 2.7 Hz, 1H), 7.86 (d, *J* = 1.3 Hz, 1H), 7.80 (d, *J* = 9.1 Hz, 7.05 (d, *J* = 9.1 Hz, 1H), 6.99 (s, 1H), 2.53 (s, 3H). | 417 | | 99% |
| 223 | 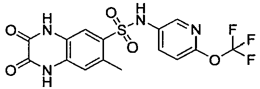 | M1, M2 | ¹H NMR (400 MHz, DMSO-*d*₆) δ 12.10 (s, 1H), 11.91 (s, 1H), 10.85 (s, 1H), 8.04 (d, *J* = 2.8 Hz, 1H), 7.71 (s, 1H), 7.64 (dd, *J* = 8.8, 2.8 Hz, 1H), 7.24 (d, *J* = 8.8 Hz, 1H), 7.03 (s, 1H), 2.52 (s, 3H). | 417 | | 100% |
| 224 | 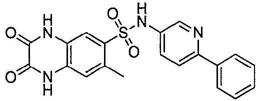 | M1, M2 | ¹H NMR (400 MHz, DMSO-*d*₆) δ 12.09 (s, 1H), 11.93 (s, 1H), 10.78 (s, 1H), 8.40 (d, *J* = 2.6 Hz, 1H), 8.02 - 7.93 (m, 2H), 7.90 - 7.85 (m, 1H), 7.77 (d, *J* = 2.2 Hz, 1H), 7.53 (dt, *J* = 8.7, 2.5 Hz, 1H), 7.48 - 7.41 (m, 2H), 7.41 - 7.35 (m, 1H), 7.02 (s, 1H), 2.56 (d, *J* = 2.2 Hz, 3H). | 409 | | 100% |
| 225 | 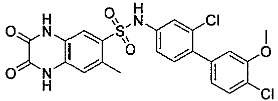 | M1 / M3, M2 | | | 504 | 97% |
| 226 | 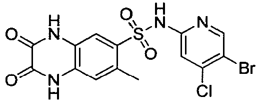 | M1, M2b | ¹H NMR (400 MHz, DMSO-*d*₆) δ 12.09 (s, 1H), 11.95 (s, 1H), 11.63 (s, 1H), 8.36 (s, 1H), 7.84 (s, 1H), 7.15 (s, 1H), 6.99 (s, 1H), 2.52 (s, 3H). | | 445 | 91% |
| 227 | 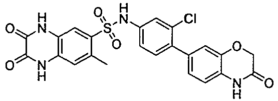 | M1, M2, M3 | | | 511 | 94% |
| 228 | 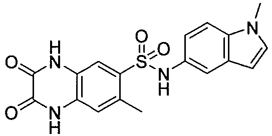 | M1, M2 | ¹H NMR (500 MHz, DMSO-*d*₆) δ 12.03 (s, 1H), 11.86 (s, 1H), 9.89 (s, 1H), 7.60 (s, 1H), 7.28 - 7.25 (m, 2H), 7.22 (d, *J* = 2.0 Hz, 1H), 6.97 (s, 1H), 6.87 (dd, *J* = 8.7, 2.0 Hz, 1H), 6.30 (dd, *J* = 3.0, 0.8 Hz, 1H), 3.70 (s, 3 H), 2.52 (s, 3H). | 385 | | 100% |
| 229 | 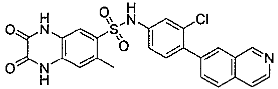 | M1, M2, M3 | | 493 | | 95% |
| 230 | 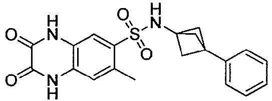 | M1, M2 | ¹H NMR (400 MHz, DMSO-*d*₆) δ 12.05 (s, 1H), 11.91 (s, 1H), 8.66 (s, 1H), 7.73 (s, 1H), 7.27 - 7.11 (m, 3H), 7.16 - 7.05 (m, 2H), 7.01 (d, *J* = 0.8 Hz, 1H), 2.51 (s, 3H), 1.95 (d, *J* = 0.6 Hz, 6H). | 398 | | 96% |
| 231 | 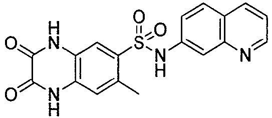 | M1, M2 | ¹H NMR (400 MHz, DMSO-*d*₆) δ 12.05 (s, 1H), 11.96 (s, 1H), 11.17 (s, 1H), 8.87 (dd, *J* = 4.6, 1.8 Hz, 1H), 8.45 (d, *J* = 8.2 Hz, 1H), 7.97 (d, *J* = 8.9 Hz, 1H), 7.85 (s, 1H), 7.64 (d, *J* = 2.1 Hz, 1H), 7.52 (dd, *J* = 8.3, 4.6 Hz, 1H), 7.44 (dt, *J* = 9.0, 2.1 Hz, 1H), 6.97 (s, 1H), 2.54 (d, *J* = 1.9 Hz, 3H). | 383 | | 100% |
| 232 | 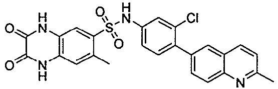 | M1, M2, M3 | | 507 | | 99% |
| 233 | 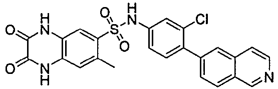 | M1, M2, M3 | | 493 | | 96% |
| 234 | 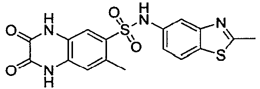 | M1, M2 | ¹H NMR (400 MHz, DMSO-*d*₆) δ 12.04 (s, 1H), 11.92 (s, 1H), 10.56 (s, 1H), 7.86 (d, *J* = 8.5 Hz, 1H), 7.77 (d, *J* = 4.4 Hz, 1H), 7.64 - 7.56 (m, 1H), 7.12 (dt, *J* = 8.6, 2.2 Hz, 1H), 6.98 (s, 1H), 2.73 (d, *J* = 4.2 Hz, 3H), 2.54 (s, 3H). | 403 | | 97% |
| 235 | 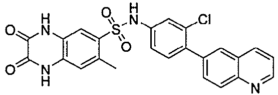 | M1, M2, M3 | | 493 | | 90% |
| 236 | 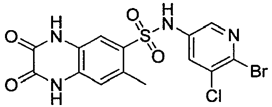 | M1, M2 | ¹H NMR (400 MHz, DMSO-*d*₆) δ 12.09 (s, 1H), 11.95 (s, 1H), 11.64 (s, 1H), 8.36 (s, 1H), 7.84 (s, 1H), 7.14 (s, 1H), 6.99 (s, 1H), 2.52 (s, 3H). | | 445 | 100% |
| 237 | 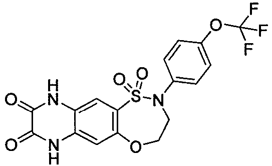 | described | ¹H-NMR (500 MHz, DMSO-*d*₆) δ 12.07 (br. s, 1H), 11.97 (br. s, 1H), 7.42 (s, 1H), 7.35 (m_{c}, 2H), 7.29 (m_{c}, 2H), 6.99 (s, 1H), 4.29 (m_{c}, 2H), 4.09 (m_{c}, 2H). | 444 | | 95% |
| 238 | 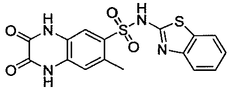 | M1, M2 | ¹H NMR (400 MHz, DMSO-*d*₆) δ 13.17 (s, 1H), 12.06 (s, 1H), 11.96 (d, *J* = 4.2 Hz, 1H), 7.84 - 7.75 (m, 2H), 7.39 (t, *J* = 7.4 Hz, 1H), 7.31 (d, *J* = 7.8 Hz, 1H), 7.25 (t, *J* = 7.5 Hz, 1H), 7.02 (s, 1H), 2.54 (d, *J* = 2.6 Hz, 3H). | 389 | | 99% |
| 239 | 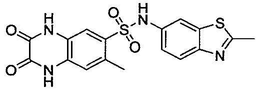 | M1, M2 | ¹H NMR (400 MHz, DMSO-*d*₆) δ 12.05 (s, 1H), 11.88 (s, 1H), 10.52 (s, 1H), 7.75 (d, *J* = 8.7 Hz, 1H), 7.73 - 7.64 (m, 2H), 7.16 (dd, *J* = 8.8, 2.3 Hz, 1H), 6.98 (s, 1H), 2.72 (s, 3H), 2.52 (s, 3H). | 403 | | 99% |
| 240 | 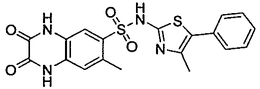 | M1, M2 | ¹H NMR (400 MHz, DMSO-*d*₆) δ 12.76 (s, 1H), 12.03 (s, 1H), 11.90 (s, 1H), 7.78 (s, 1H), 7.50 - 7.34 (m, 5H), 7.01 (s, 1H), 2.55 (s, 3H), 2.20 (s, 3H). | 429 | | 100% |
| 241 | 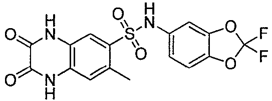 | M1, M2 | ¹H NMR (500 MHz, DMSO-*d*₆) δ 12.09 (s, 1H), 11.90 (s, 1H), 10.56 (s, 1H), 7.67 (s, 1H), 7.28 (d, *J* = 8.7 Hz, 1H), 7.08 (d, *J* = 2.1 Hz, 1H), 7.00 (s, 1H), 6.82 (dd, *J* = 8.7, 2.1 Hz, 1H), 2.51 (s, 3H). | 412 | | 100% |
| 242 | 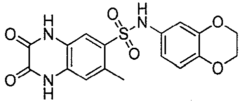 | M1, M2 | ¹H NMR (500 MHz, DMSO-*d*₆) δ 12.07 (s, 1H), 11.92 (s, 1H), 10.06 (s, 1H), 7.66 (s, 1H), 6.99 (s, 1 H), 6.70 (d, *J* = 8.7 Hz, 1H), 6.56 (d, *J* = 2.5 Hz, 1H), 6.50 (dd, *J* = 8.7, 2.5 Hz, 1H), 4.19-4.10 (m, 4H), 2.50 (s, 3 H). | 390 | | 100% |
| 243 | 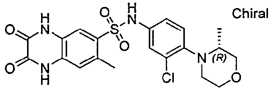 | M1, (M4, M5), M2 | ¹H NMR (400 MHz) δ 12.07 (s, 1H) 11.95 (s, 1H), 10.48 (s, 1H), 7.72 (s, 1H), 7.19 - 7.07 (m, 2H), 7.02 - 6.92 (m, 2H), 3.79 - 3.56 (m, 3H), 3.29 - 3.18 (m, 2H), 2.95 - 2.87 (m, 1H), 2.59 - 2.50 (m, 4H), 0.66 (d, *J* = 5.8 Hz, 3H). | 465 | | 99% |
| 244 | 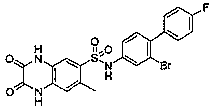 | M1 / M3, M2 | | | 502 | 97% |
| 245 | 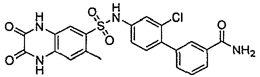 | M1, M2, M3 | | 485 | | 99% |
| 246 | 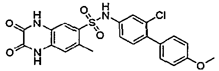 | M1, M2, M3 | | | 470 | 99% |
| 247 | 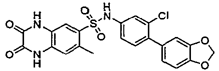 | M1, M2, M3 | | | 484 | 98% |
| 248 | 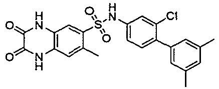 | M1, M2, M3 | | | 468 | 98% |
| 249 | 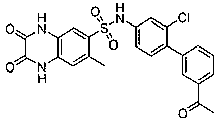 | M1, M2, M3 | | | 482 | 98% |
| 250 | 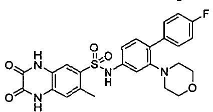 | M1, M2 with aniline **1T** | | 511 | | 99% |
| 251 | 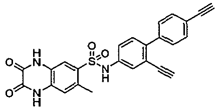 | described | | | 454 | 99% |
| 252 | 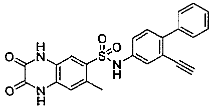 | M1 /**1V** + M3, M2 + TIPS deprot. | | | 430 | 99% |
| 253 | 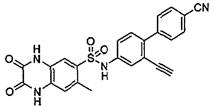 | M1 / **1V** + M3, M2 + TIPS deprot. | | | 455 | 99% |
| 254 | 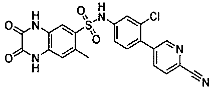 | M1, M2, M3 | | | 466 | 99% |
| 255 | 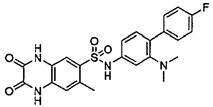 | M1, M2 with aniline **2T** | | 469 | | 99% |
| 256 | 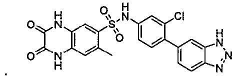 | M1, M2, M3 | | | 481 | 91% |
| 257 | 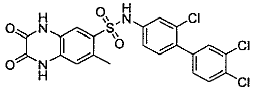 | M1, M2, M3 | | | 508 | 97% |
| 258 | 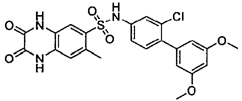 | M1, M2, M3 | | | 500 | 96% |
| 259 | 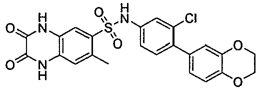 | M1, M2, M3 | | | 498 | 98% |
| 260 | 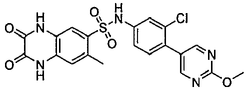 | M1, M2, M3 | | 474 | | 97% |
| 261 | 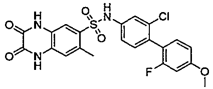 | M1, M2, M3 | | | 488 | 98% |
| 262 | 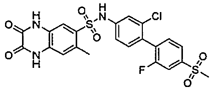 | M1, M2, M3 | | | 536 | 97% |
| 263 | 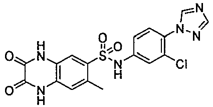 | M1, M4, M5, M2 | | | 431 | 98% |
| 264 | 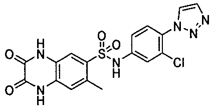 | M1, M4, M5, M2 | | 433 | | 98% |
| 265 | 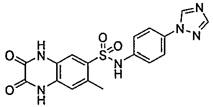 | M1, M4, M5, M2 | | | 397 | 97% |
| 266 | 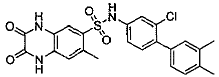 | M1, M2, M3 | | | 468 | 98% |
| 267 | 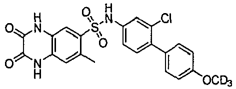 | M1, M2, M3 | | | 473 | 99% |
| 268 | 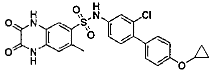 | M1, M2, M3 | | | 496 | 99% |
| 269 | 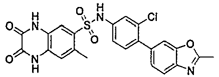 | M1, M2, M3 | | | 495 | 99% |
| 270 | 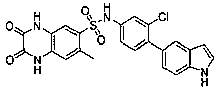 | M1, M2, M3 | | | 479 | 98% |
| 271 | 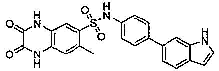 | M1, M2, M3 | | | 479 | 99% |
| 272 | 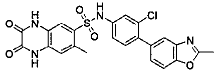 | M1, M2, M3 | | | 495 | 99% |
| 273 | 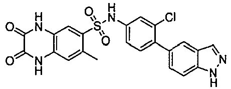 | M1, M2, M3 | | | 480 | 98% |
| 274 | 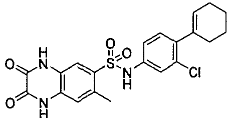 | M1 / M3, M2 | | 446 | 446 | 99% |
| 275 | 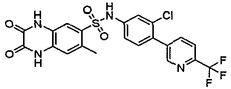 | M1, M2, M3 | | | 509 | 97% |
| 276 | 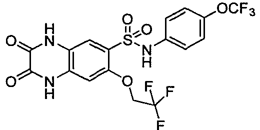 | As for cpd. 221 | | | 498 | 99% |
| 277 | 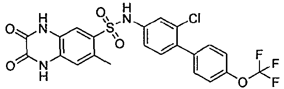 | M1, M2, M3 | | | 524 | 98% |
| 278 | 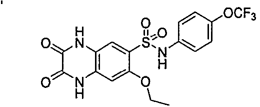 | As for cpd. 221 | | | 444 | 97% |
| 279 | 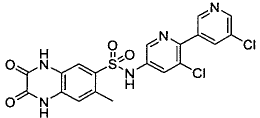 | M1 / M3, M2 | | 478 | | 98% |
| 280 | 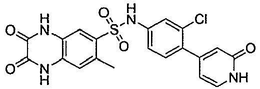 | M1, M2, M3 | | | 457 | 98% |
| 281 | 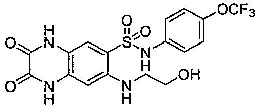 | As for cpd. 221 | | | 459 | 99% |
| 282 | 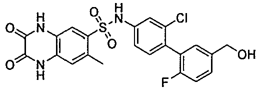 | M1, M2, M3 | | | 488 | 98% |
| 283 | 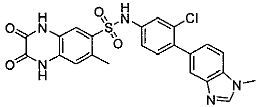 | M1 / M3, M2 | | 496 | | 98% |
| 284 | 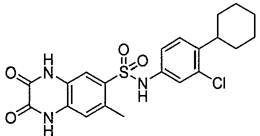 | 274 + hydrog. | | | 446 | 96% |
| 285 | 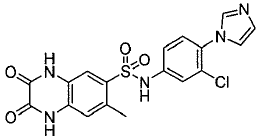 | M1, M4, M5, M2 | | 432 | | 99% |
| 286 | 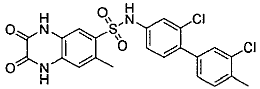 | M1, M2, M3 | | | 488 | 98% |
| 287 | 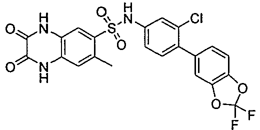 | M1, M2, M3 | | | 520 | 98% |
| 288 | 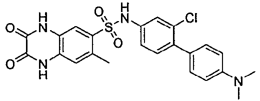 | M1, M2, M3 | | 485 | | 97% |
| 289 | 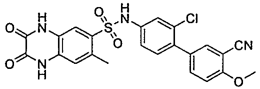 | M1, M2, M3 | | | 495 | 98% |
| 290 | 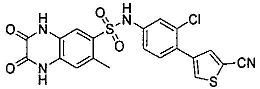 | M1, M2, M3 | | | 471 | 96% |
| 291 | 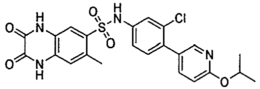 | M1, M2, M3 | | 501 | | 99% |
| 292 | 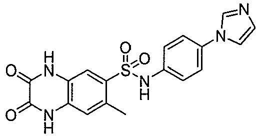 | M1, M4, M5, M2 | | | 396 | 98% |
| 293 | 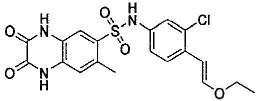 | M1, M2, M3 | | | 434 | 98% |
| 294 | 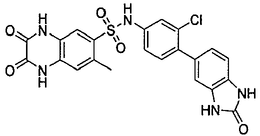 | M1 / M3, M2 | | | 496 | 99% |
| 295 | 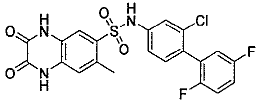 | M1, M2, M3 | | 478 | | 96% |
| 296 | 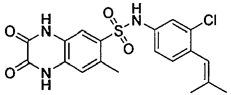 | M1, M2, M3 | | | 418 | 99% |
| 297 | 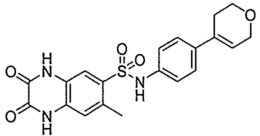 | M1, M2, M3 | | | 412 | 98% |
| 298 | 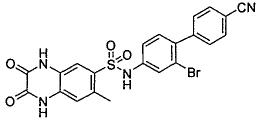 | M1, M2, M3 | | 511 | | 91% |
| 299 | 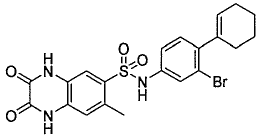 | M1 / M3, M2 | | | 488 | 96% |
| 300 | 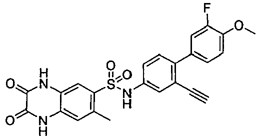 | M1 /**1V** + M3, M2 + TIPS deprot. | | | 478 | 96% |
| 301 | 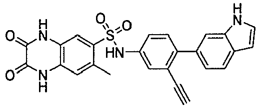 | M1 /**1V** + M3, M2 + TIPS deprot. | | 469 | | 99% |
| 302 | 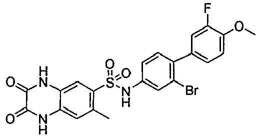 | M1, M2, M3 | | | 532 | 99% |
| 303 | 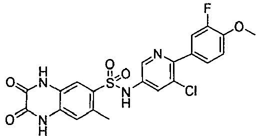 | M1, M2, M3 | | 491 | | 96% |
| 304 | 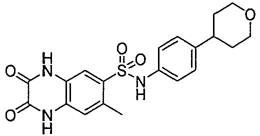 | 297 + hydrog. | | 416 | | 99% |
| 305 | 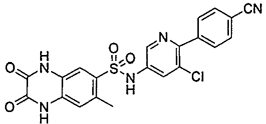 | M1, M2, M3 | | 468 | | 99% |
| 306 | 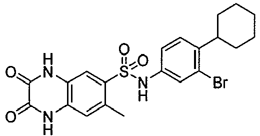 | 299 + hydrog. | | | 490 | 98% |
| 307 | 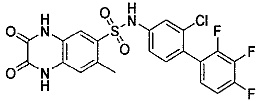 | M1, M2, M3 | | 496 | | 99% |
| 308 | 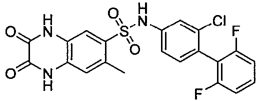 | M1, M2, M3 | | 478 | | 98% |
| 309 | 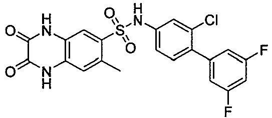 | M1, M2, M3 | | | 476 | 99% |
| 310 | 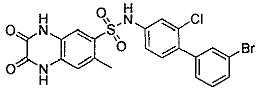 | M1 / M3, M2 | | 542 (M+Na)⁺ | | 100% |
| 311 | 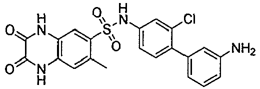 | M1, M2, M3 + de-boc | | | 455 | 99% |
| 312 | 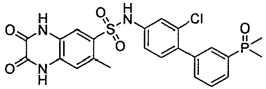 | M1 / M3 + POMe₂, M2 | | 518 | | 100% |
| 313 | 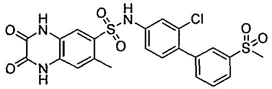 | M1 / M3, M2 | | 520 | | 96% |
| 314 | 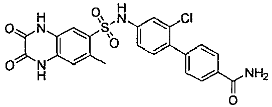 | M1, M2, M3 | | | 484 | 100% |
| 315 | 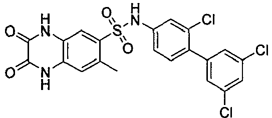 | M1, M2, M3 | | | 510 | 97% |
| 316 | 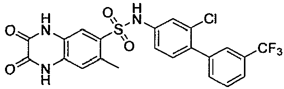 | M1, M2, M3 | | | 508 | 98% |
| 317 | 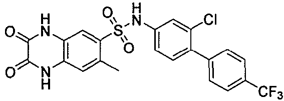 | M1, M2, M3 | | | 508 | 100% |
| 318 | 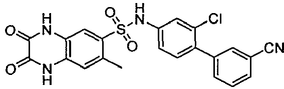 | M1, M2, M3 | | | 465 | 95% |
| 319 | 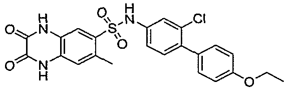 | M1, M2, M3 | | | 484 | 97% |
| 320 | 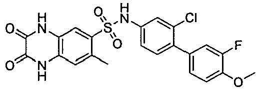 | M1, M2, M3 | | | 488 | 98% |
| 321 | 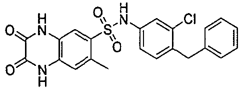 | M1, M2, M3 | | | 454 | 90% |
| 322 | 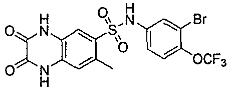 | M1, M2 | | | 492 | 100% |
| 323 | 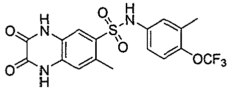 | M1, M2 | | | 428 | 100% |
| 324 | 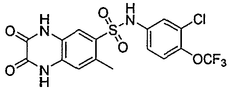 | M1, M2 | | | 448 | 100% |
| 325 | 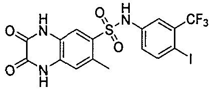 | M1, M2 | | | 524 | 100% |
| 326 | 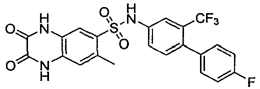 | M1, M2, M3 | | | 492 | 100% |
| 327 | 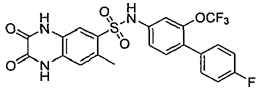 | M1, M2, M3 | | | 508 | 97% |
| 328 | 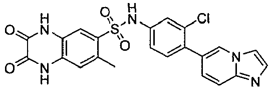 | M1, M2, M3 | | 482 | | 100% |
| 329 | 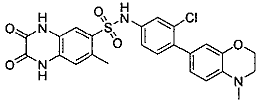 | M1, M2, M3 | | 513 | | 92% |
| 330 | 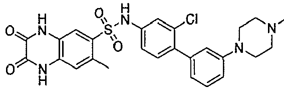 | M1, M2, M3 | | 540 | | 99% |
| 331 | 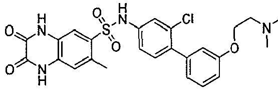 | M1, M2, M3 | | 529 | | 95% |
| 332 | 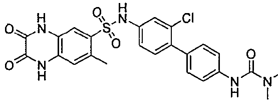 | M1, M2, M3 | | | 526 | 99% |
| 333 | 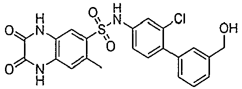 | M1, M2, M3 | | | 470 | 99% |
| 334 | 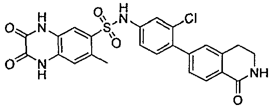 | M1, M2, M3 | | | 509 | 89% |
| 335 | 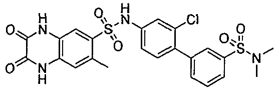 | M1, M2, M3 | | | 547 | 93% |
| 336 | 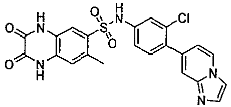 | M1, M2, M3 | | 482 | | 100% |
| 337 | 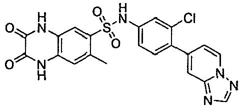 | M1, M2, M3 | | 483 | | 98% |
| 338 | 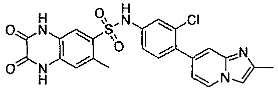 | M1, M2, M3 | | 496 | | 100% |
| 339 | 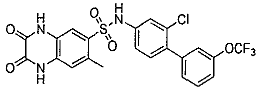 | M1, M2, M3 | | | 524 | 94% |
| 340 | 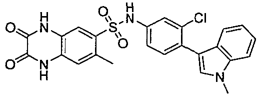 | M1, M2, M3 | | | 493 | 98% |
| 341 | 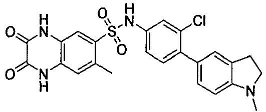 | M1, M2, M3 | | 497 | | 100% |
| 342 | 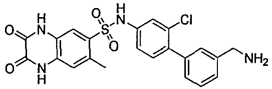 | M1, M2, M3 + de-boc | | 471 | | 92% |
| 343 | 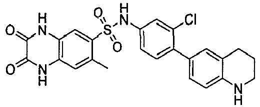 | M1, M2, M3 + de-boc | | 497 | | 96% |
| 344 | 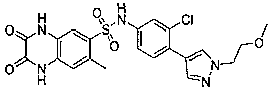 | M1, M2, M3 | | 490 | | 90% |
| 345 | 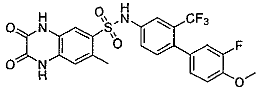 | M1, M2, M3 | | | 522 | 100% |
| 346 | 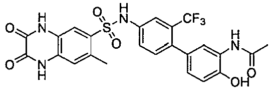 | M1, M2, M3 + benzimid azole opening | | | 547 | 93% |
| 347 | 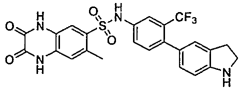 | M1, M2, M3 | | 517 | | 100% |
| 348 | 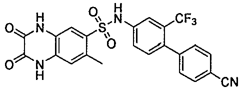 | M1, M2, M3 | | | 499 | 100% |
| 349 | 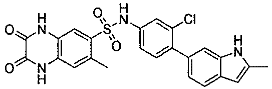 | M1, M2, M3 | | | 493 | 100% |
| 350 | 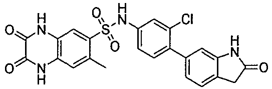 | M1, M2, M3 | | | 495 | 100% |
| 351 | 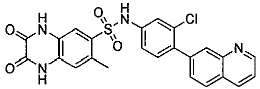 | M1, M2, M3 | | | 491 | 96% |
| 352 | 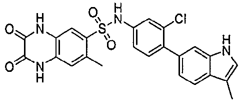 | M1, M2, M3 | | | 493 | 100% |
| 353 | 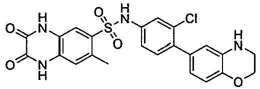 | M1, M2, M3 | | | 497 | 95% |
| 354 | 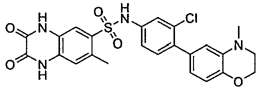 | M1, M2, M3 | | | 511 | 97% |
| 355 | 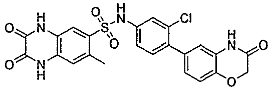 | M1, M2, M3 | | | 511 | 96% |
| 356 | 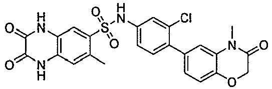 | M1, M2, M3 | | | 525 | 97% |
| 357 | 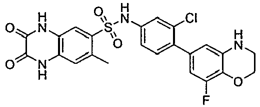 | M1, M2, M3 | | 517 | | 97% |
| 358 | 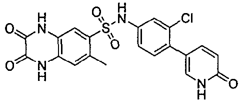 | M1, M2, M3 | | | 459 | 100% |
| 359 | 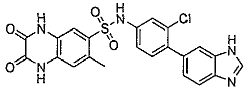 | M1, M2, M3 | | 482 | | 96% |
| 360 | 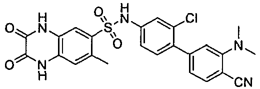 | M1, M2, boronic ester synthesis, M3 | | 510 | | 95% |
| 361 | 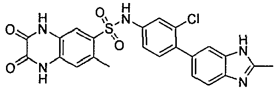 | M1, M2, M3 | | 496 | | 100% |
| 362 | 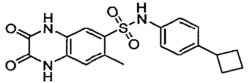 | M1, M2 | | | 384 | 100% |
| 363 | 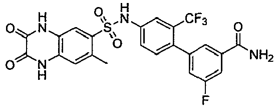 | M1, M2, M3 | | 537 | | 99% |
| 364 | 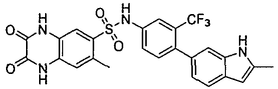 | M1, M2, M3 | | | 527 | 94% |
| 365 | 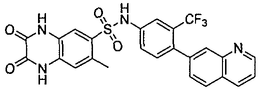 | M1, M2, M3 | | | 525 | 100% |
| 366 | 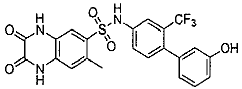 | M1, M2, M3 | | | 490 | 100% |
| 367 | 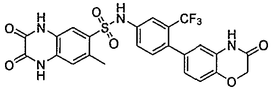 | M1, M2, M3 | | | 545 | 98% |
| 368 | 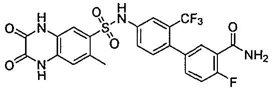 | M1, M2, M3 | | | 535 | 100% |
| 369 | 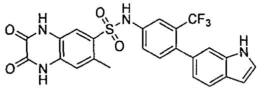 | M1, M2, M3 | | | 513 | 100% |
| 370 | 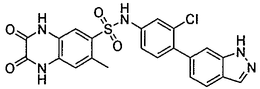 | M1, M2, M3 | | | 480 | 100% |
| 371 | 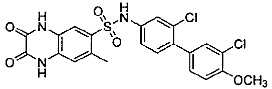 | M1, M2, M3 | | | 504 | 97% |
| 372 | 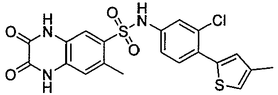 | M1, M2, M3 | | 462 | | 97% |
| 373 | 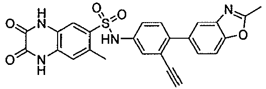 | M1 / **1V** + M3, M2 + TIPS deprot. | | 487 | | 97% |
| 374 | 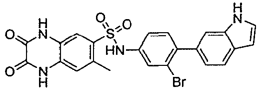 | M1, M2, M3 | | 525 | | 98% |
| 375 | 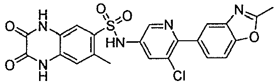 | M1/ M3, M2 | | 498 | | 97% |
| 376 | 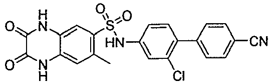 | M1 / M3, M2 | | 467 | | 99% |
| 377 | 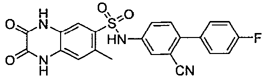 | M1 / M3, M2 | | 451 | | 100% |
| 378 | 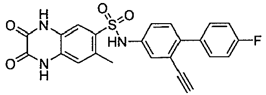 | M1 / M3, M2 | | 450 | | 98% |
| 379 | 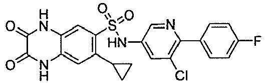 | As for cpd. 194 | | 487 | | 90% |
| 380 | 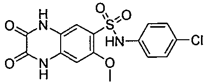 | M1, M2 | | 382 | | 90% |
| 381 | 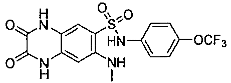 | As for cpd. 221 | | 431 | | 96% |
| 382 | 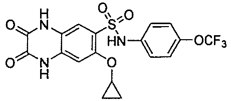 | As for cpd. 221 | | 458 | | 96% |
| 383 | 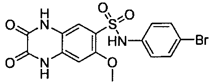 | M1, M2 | | 426 | | 99% |
| 384 | 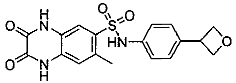 | M1, M2 | | 388 | | 99% |
| 385 | 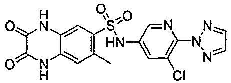 | M1, (M4, M5), M2 | | 434 | | 100% |
| 386 | 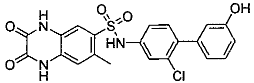 | M1 / M3, M2 + TBS deprot. | | 480 (M+Na)⁺ | | 95% |
| 387 | 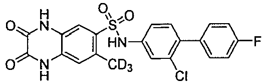 | **12D,** M1 / M3, M2 | | 463 | | 100% |
| 388 | 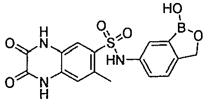 | M1, M2 | | 405 (M+H₂O)⁺ | | 98% |
| 389 | 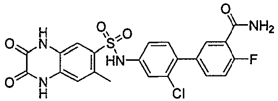 | M1 / M3, M2 | | 525 (M+Na)⁺ | | 97% |
| 390 | 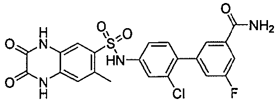 | M1/ M3, M2 | | 525 (M+Na)⁺ | | 98% |
| 391 | 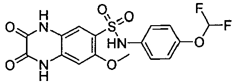 | M1, M2 | | 414 | | 97% |
| 392 | 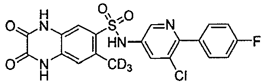 | **12D,** M1 / M3, M2 | | 464 | | 100% |
| 393 | 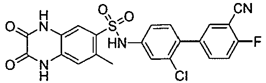 | M1 / M3, M2 | | 507 (M+Na)⁺ | | 91% |
| 394 | 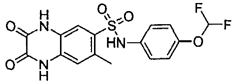 | M1, M2 | | 398 | | 97% |
| 395 | 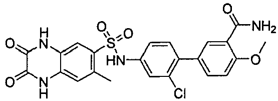 | M1 / M3, M2 | | 515 | | 98% |
| 396 | 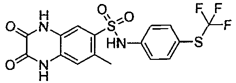 | M1, M2 | | 432 | | 98% |
| 397 | 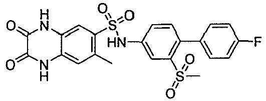 | M1 / M3, M2 | | 503 | | 97% |
| 398 | 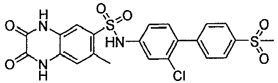 | M1 / M3, M2 | | 520 | | 98% |
| 399 | 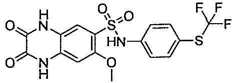 | M1, M2 | | 448 | | 100% |
| 400 | 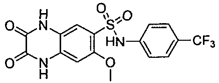 | M1, M2 | | 416 | | 99% |
| 401 | 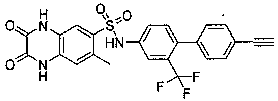 | M1, M2, M3 + TIPS deprot. | | 498 | | 92% |

### 3. Hemolysin alpha-based Ca²⁺-Influx Assay

The assay is based on the Fluo-4 NW Calcium Assay Kit (#F36205) from Thermo Scientific. Here the effect of hemolysin alpha from staphylococcus aureus was monitored by loading non adherent U397 cells with the Ca²⁺-sensitive dye Fluo-4 hemolysin alpha addition leads to the formation of Ca²⁺ permissive pores in the membrane of the U397 cells which results in a dose dependent increase of fluorescence.

Briefly, the protocol described here was applied for screening and activity determination in a low-volume 384-well microtiter plate with cell culture treated surface. For high-throughput application in the 1536-well microtiter plate format, volumes of the reagent mixes were adjusted, maintaining the volumetric ratio.
a. Hemolysin alpha was diluted with PBS from its stock to a working concentration of 35 nM. U937 cells were diluted in assay buffer (HBBS, 20mM HEPES) to 4000 cells/µl. 1x Fluo-4 NW dye loading solution was prepared according to manufacturer's specifications including 5 mM probenecid to reduce background fluorescence.
b. Chemical compounds were applied into empty assay plates using contact-free acoustic droplet-dispensing (Echo520^{®} Labcyte Inc., Sunnyvale CA) from 10 mM compound stocks in 100 % DMSO, to a final concentration of 10 µM or in serial dilution series of the required concentration range. Equal amounts of DMSO without any compound were added to control samples.
c. 5 µL of the U937 cells + 5µl of 1x Fluo-4 NW dye load were dispensed using a Multidrop^{®} dispenser (Thermo Fisher Scientific, Waltham MA) into the wells of a microtiter plate. The plate was centrifuged at 1000 rpm and incubated at 37 °C for 30 min followed by an incubation step at r. t. for another 30 min.
d. The reaction was started by addition of 4µl of hemolysin alpha to a final concentration of 10nM followed by a centrifugation step for 1 min at 1000 rpm. No hemolysin alpha was added to negative control samples.
e. After incubation at room temperature for 4 h the generated signal was measured with an EnVision plate reader (Perkin Elmer, Waltham MA), using excitation at 485 nm and an emission at 535 nm.

### 4. LDH-Glo Cytotoxicity Assay

The LDH-Glo-Cytotoxicity Assay is a bioluminescent plate-based assay to quantify the release of cellular Lactate Dehydogenase (LDH) into the assay medium upon plasma membrane damage by hemolysin treatment of the cells. LDH in the supernatant reduces an added substrate to generate luciferin which is converted into a bioluminescent signal by the Ultra Glo Luciferase (Promega).

A549-Cells (DSMZ, #ACC107), that are used for the assay, are maintained in RPMI 1640 cell culture medium + glutamine (PAN Biotech GmbH, Aidenbach, Germany; #P04-22100; P04-05500) supplemented with 10% fetal calf serum (Capricorn, #FBS-11A) and are grown at 37°C, 5% CO₂.

For the LDH assay compounds or DMSO are prediluted at different concentrations in 15 µl cell culture medium RPMI 1640 + 5% FCS + 10 mM HEPES in black µclear 384-well-plates (Greiner BioOne). Shortly afterwards 10 µl of 70 nM *S*. *aureus* Alpha hemolysin (IBT BIOSERVICES, #1401-002) was added to get a final assay concentration of 20 nM. After adding 10 µl of A549 cells (20.000 cells/well diluted in assay medium) the assay plates (total assay volume: 35 µl) were incubated for 5h at 37 °C / 5% CO₂ in humidified chambers in order to allow hemolysis.

As a positive internal control, we use the hemolysin antibody (IBT Bioservices, #0210-001) at a concentration range from 0.005 - 10 µg / ml and determine the IC₅₀ concentration. The standard IC₅₀ concentration for the antibody is approximately 50 ng / ml.

The determination of the LDH concentration was done after the 5 h incubation time according to the instructions of the One Glo Luminescent assay Kit (Promega, cat no. G7891). Shortly, 20 µl of cell culture supernatant were incubated in a separated black µclear 384-well plate at 25 °C for 20 min, mixed with 20 µl of the LDH reagent using an orbital shaker (1 min, 300 rpm) and further incubated for 5 min at 2°C. The reaction was stopped by addition of 10 µl stop-reagent, provided with the assay kit. Shortly afterwards the fluorescent signal was measured by Victor X5 plate reader (Perkin Elmer) using the filters 531 nm (extinction) and 590 nm (emission). EC₅₀ values were calculated with the software Excel Fit (IDBS, Guildford, UK) from 3-fold dilution series comprising at least 8 concentrations in duplicates.

### 5. Biological activities of compounds

Activities of compounds are listed in **Table 3** together with compound number and IUPAC names. Biological activities are determined by two main assays with HIα-induced cell damage: hemolysin-α Ca2+-influx on U937 cells according to Example 3 and LDH-Glo Cytotoxicity Assay on A549 cells according to Example 4, and were grouped according to the following scheme:

| | | | | |
|---|---|---|---|---|
| Ca²⁺-infux Assay (IC-50) | < 30nM | 30nM ≤ x < 100nM | 100nM ≤ x < 1µM | 1µM ≤ x < 3.5µM |
| | +++ | ++ | + | (+) |
| A549-LDH Assay (IC-50) | < 30nM | 30nM ≤ x < 100nM | 100nM ≤ x < 1µM | 1µM ≤ x < 6µM |
| | +++ | ++ | + | (+) |

**Table 3 - IUPAC chemical names and biological activities**

| *Ex* | *IUPAC Name* | *Ca²⁺-infux Assay activity* | *A549-LDH Assay activity* |
|---|---|---|---|
| 1 | N-(2-chloro-[1,1'-biphenyl]-4-yl)-7-methyl-2,3-dioxo-1,2,3,4-tetrahydroquinoxaline-6-sulfonamide | +++ | ++ |
| 2 | N-(2,3'-dichloro-2',4'-difluoro-[1,1'-biphenyl]-4-yl)-7-methyl-2,3-dioxo-1,2,3,4-tetrahydroquinoxaline-6-sulfonamide | +++ | + |
| 3 | N-(2-chloro-4'-fluoro-[1,1'-biphenyl]-4-yl)-7-methyl-2,3-dioxo-1,2,3,4-tetrahydroquinoxaline-6-sulfonamide | +++ | ++ |
| 4 | N-(2-chloro-3'-(dimethylamino)-[1,1'-biphenyl]-4-yl)-7-methyl-2,3-dioxo-1,2,3,4-tetrahydroquinoxaline-6-sulfonamide | +++ | ++ |
| 5 | N-(2-chloro-2'-fluoro-[1,1'-biphenyl]-4-yl)-7-methyl-2,3-dioxo-1,2,3,4-tetrahydroquinoxaline-6-sulfonamide | +++ | ++ |
| 6 | N-(2-chloro-3'-(pyrrolidin-1-yl)-[1,1'-biphenyl]-4-yl)-7-methyl-2,3-dioxo-1,2,3,4-tetrahydroquinoxaline-6-sulfonamide | +++ | + |
| 7 | N-(3-chloro-4-(thiophen-3-yl)phenyl)-7-methyl-2,3-dioxo-1,2,3,4-tetrahydroquinoxaline-6-sulfonamide | +++ | + |
| 8 | N-(2,4'-dichloro-[1,1'-biphenyl]-4-yl)-7-methyl-2,3-dioxo-1,2,3,4-tetrahydroquinoxaline-6-sulfonamide | +++ | + |
| 9 | 7-chloro-N-(2-chloro-3'-(dimethylamino)-[1,1'-biphenyl]-4-yl)-2,3-dioxo-1,2,3,4-tetrahydroquinoxaline-6-sulfonamide | +++ | + |
| 10 | N-(3-chloro-4-(indolin-6-yl)phenyl)-7-fluoro-2,3-dioxo-1,2,3,4-tetrahydroquinoxaline-6-sulfonamide | +++ | + |
| 11 | N-(2,3'-dichloro-4'-fluoro-[1,1'-biphenyl]-4-yl)-7-methyl-2,3-dioxo-1,2,3,4-tetrahydroquinoxaline-6-sulfonamide | +++ | + |
| 12 | 7-chloro-N-(2-chloro-4'-fluoro-[1,1'-biphenyl]-4-yl)-2,3-dioxo-1,2,3,4-tetrahydroquinoxaline-6-sulfonamide | +++ | + |
| 13 | N-(3-chloro-4-(indolin-6-yl)phenyl)-7-methyl-2,3-dioxo-1,2,3,4-tetrahydroquinoxaline-6-sulfonamide | +++ | + |
| 14 | 7-chloro-N-(2-chloro-[1,1'-biphenyl]-4-yl)-2,3-dioxo-1,2,3,4-tetrahydroquinoxaline-6-sulfonamide | +++ | + |
| 15 | 7-bromo-2,3-dioxo-N-(4-(trifluoromethoxy)phenyl)-1,2,3,4-tetrahydroquinoxaline-6-sulfonamide | +++ | ++ |
| 16 | N-(4-(furan-2-yl)-3,5-dimethylphenyl)-7-methyl-2,3-dioxo-1,2,3,4-tetrahydroquinoxaline-6-sulfonamide | +++ | +++ |
| 17 | 7-methyl-N-(2-methyl-[1,1'-biphenyl]-4-yl)-2,3-dioxo-1,2,3,4-tetrahydroquinoxaline-6-sulfonamide | +++ | +++ |
| 18 | N-(2-chloro-4'-fluoro-[1,1'-biphenyl]-4-yl)-7-fluoro-2,3-dioxo-1,2,3,4-tetrahydroquinoxaline-6-sulfonamide | +++ | ++ |
| 19 | 7-chloro-N-(2,3'-dichloro-4'-fluoro-[1,1'-biphenyl]-4-yl)-2,3-dioxo-1,2,3,4-tetrahydroquinoxaline-6-sulfonamide | +++ | + |
| 20 | 7-ethyl-2,3-dioxo-N-(4-(trifluoromethoxy)phenyl)-1,2,3,4-tetrahydroquinoxaline-6-sulfonamide | +++ | ++ |
| 21 | 7-chloro-N-(2-chloro-3'-(pyrrolidin-1-yl)-[1,1'-biphenyl]-4-yl)-2,3-dioxo-1,2,3,4-tetrahydroquinoxaline-6-sulfonamide | +++ | + |
| 22 | N-(3-chloro-4-(furan-3-yl)phenyl)-7-methyl-2,3-dioxo-1,2,3,4-tetrahydroquinoxaline-6-sulfonamide | +++ | + |
| 23 | N-(2-chloro-3'-methyl-[1,1'-biphenyl]-4-yl)-7-methyl-2,3-dioxo-1,2,3,4-tetrahydroquinoxaline-6-sulfonamide | +++ | ++ |
| 24 | N-(3-chloro-4-(thiophen-2-yl)phenyl)-7-methyl-2,3-dioxo-1,2,3,4-tetrahydroquinoxaline-6-sulfonamide | +++ | ++ |
| 25 | 7-chloro-N-(2-chloro-2'-fluoro-[1,1'-biphenyl]-4-yl)-2,3-dioxo-1,2,3,4-tetrahydroquinoxaline-6-sulfonamide | +++ | + |
| 26 | N-(2,3'-dichloro-[1,1'-biphenyl]-4-yl)-7-methyl-2,3-dioxo-1,2,3,4-tetrahydroquinoxaline-6-sulfonamide | +++ | ++ |
| 27 | N-(2-chloro-3'-(pyrrolidin-1-yl)-[1,1'-biphenyl]-4-yl)-7-fluoro-2,3-dioxo-1,2,3,4-tetrahydroquinoxaline-6-sulfonamide | +++ | (+) |
| 28 | 7-methyl-2,3-dioxo-N-(4-(trifluoromethoxy)phenyl)-1,2,3,4-tetrahydroquinoxaline-6-sulfonamide | +++ | +++ |
| 29 | N-(3-chloro-4-(pyridin-3-yl)phenyl)-7-methyl-2,3-dioxo-1,2,3,4-tetrahydroquinoxaline-6-sulfonamide | +++ | + |
| 30 | N-(3-chloro-4-(2-chloropyridin-4-yl)phenyl)-7-methyl-2,3-dioxo-1,2,3,4-tetrahydroquinoxaline-6-sulfonamide | +++ | ++ |
| 31 | 7-isopropyl-2,3-dioxo-N-(4-(trifluoromethoxy)phenyl)-1,2,3,4-tetrahydroquinoxaline-6-sulfonamide | +++ | +++ |
| 32 | 7-chloro-N-(3-chloro-4-(thiophen-3-yl)phenyl)-2,3-dioxo-1,2,3,4-tetrahydroquinoxaline-6-sulfonamide | +++ | + |
| 33 | 7-fluoro-N-(2-methyl-[1,1'-biphenyl]-4-yl)-2,3-dioxo-1,2,3,4-tetrahydroquinoxaline-6-sulfonamide | +++ | ++ |
| 34 | N-(2-chloro-3'-(dimethylamino)-[1,1'-biphenyl]-4-yl)-7-fluoro-2,3-dioxo-1,2,3,4-tetrahydroquinoxaline-6-sulfonamide | +++ | + |
| 35 | 7-chloro-N-(2,3'-dichloro-[1,1'-biphenyl]-4-yl)-2,3-dioxo-1,2,3,4-tetrahydroquinoxaline-6-sulfonamide | +++ | + |
| 36 | N-(2-chloro-2'-fluoro-[1,1'-biphenyl]-4-yl)-7-fluoro-2,3-dioxo-1,2,3,4-tetrahydroquinoxaline-6-sulfonamide | +++ | + |
| 37 | N-(3-chloro-4-(pyridin-4-yl)phenyl)-7-methyl-2,3-dioxo-1,2,3,4-tetrahydroquinoxaline-6-sulfonamide | +++ | ++ |
| 38 | N-(2-chloro-4'-methyl-[1,1'-biphenyl]-4-yl)-7-methyl-2,3-dioxo-1,2,3,4-tetrahydroquinoxaline-6-sulfonamide | ++ | + |
| 39 | N-(2-chloro-3'-isopropyl-[1,1'-biphenyl]-4-yl)-7-methyl-2,3-dioxo-1,2,3,4-tetrahydroquinoxaline-6-sulfonamide | ++ | (+) |
| 40 | N-(2-chloro-[1,1'-biphenyl]-4-yl)-7-fluoro-2,3-dioxo-1,2,3,4-tetrahydroquinoxaline-6-sulfonamide | ++ | + |
| 41 | 7-bromo-2,3-dioxo-N-(4-propylphenyl)-1,2,3,4-tetrahydroquinoxaline-6-sulfonamide | ++ | ++ |
| 42 | N-(3,5-dimethyl-4-(thiophen-3-yl)phenyl)-7-methyl-2,3-dioxo-1,2,3,4-tetrahydroquinoxaline-6-sulfonamide | ++ | + |
| 43 | N-(2,3'-dichloro-[1,1'-biphenyl]-4-yl)-5-fluoro-7-methyl-2,3-dioxo-1,2,3,4-tetrahydroquinoxaline-6-sulfonamide | ++ | (+) |
| 44 | 7-chloro-N-(3-chloro-4-(furan-3-yl)phenyl)-2,3-dioxo-1,2,3,4-tetrahydroquinoxaline-6-sulfonamide | ++ | + |
| 45 | 7-chloro-N-(2-methyl-[1,1'-biphenyl]-4-yl)-2,3-dioxo-1,2,3,4-tetrahydroquinoxaline-6-sulfonamide | ++ | ++ |
| 46 | 7-chloro-N-(3-chloro-4-(indolin-6-yl)phenyl)-2,3-dioxo-1,2,3,4-tetrahydroquinoxaline-6-sulfonamide | ++ | + |
| 47 | 7-chloro-2,3-dioxo-N-(4-(trifluoromethoxy)phenyl)-1,2,3,4-tetrahydroquinoxaline-6-sulfonamide | ++ | ++ |
| 48 | 7-chloro-N-(3-chloro-4-(pyridin-3-yl)phenyl)-2,3-dioxo-1,2,3,4-tetrahydroquinoxaline-6-sulfonamide | ++ | (+) |
| 49 | 7-methyl-2,3-dioxo-N-(4-propylphenyl)-1,2,3,4-tetrahydroquinoxaline-6-sulfonamide | +++ | ++ |
| 50 | 7-methyl-2,3-dioxo-N-(4-(trifluoromethyl)phenyl)-1,2,3,4-tetrahydroquinoxaline-6-sulfonamide | ++ | ++ |
| 51 | N-(2'-fluoro-2,6-dimethyl-[1,1'-biphenyl]-4-yl)-7-methyl-2,3-dioxo-1,2,3,4-tetrahydroquinoxaline-6-sulfonamide | ++ | ++ |
| 52 | N-(3-chloro-4-(5-methylthiophen-2-yl)phenyl)-7-methyl-2,3-dioxo-1,2,3,4-tetrahydroquinoxaline-6-sulfonamide | ++ | + |
| 53 | N-(4-cyclopropylphenyl)-7-methyl-2,3-dioxo-1,2,3,4-tetrahydroquinoxaline-6-sulfonamide | ++ | +++ |
| 54 | 7-methyl-2,3-dioxo-N-(5,6,7,8-tetrahydronaphthalen-2-yl)-1,2,3,4-tetrahydroquinoxaline-6-sulfonamide | ++ | + |
| 55 | 7-chloro-2,3-dioxo-N-(4-propylphenyl)-1,2,3,4-tetrahydroquinoxaline-6-sulfonamide | ++ | ++ |
| 56 | N-(3-chloro-4-(trifluoromethyl)phenyl)-7-methyl-2,3-dioxo-1,2,3,4-tetrahydroquinoxaline-6-sulfonamide | ++ | (+) |
| 57 | N-(3'-chloro-2,6-dimethyl-[1,1'-biphenyl]-4-yl)-7-methyl-2,3-dioxo-1,2,3,4-tetrahydroquinoxaline-6-sulfonamide | ++ | ++ |
| 58 | N-(3,5-dimethyl-4-(thiophen-2-yl)phenyl)-7-methyl-2,3-dioxo-1,2,3,4-tetrahydroquinoxaline-6-sulfonamide | ++ | (+) |
| 59 | N-(3'-(dimethylamino)-2,6-dimethyl-[1,1'-biphenyl]-4-yl)-7-methyl-2,3-dioxo-1,2,3,4-tetrahydroquinoxaline-6-sulfonamide | ++ | + |
| 60 | 7-methyl-N-(naphthalen-2-yl)-2,3-dioxo-1,2,3,4-tetrahydroquinoxaline-6-sulfonamide | ++ | ++ |
| 61 | N-([1,1'-biphenyl]-4-yl)-7-methyl-2,3-dioxo-1,2,3,4-tetrahydroquinoxaline-6-sulfonamide | +++ | ++ |
| 62 | N-(3-chloro-4-(3-methylthiophen-2-yl)phenyl)-7-methyl-2,3-dioxo-1,2,3,4-tetrahydroquinoxaline-6-sulfonamide | ++ | + |
| 63 | N-(4-bromo-3-chlorophenyl)-5-fluoro-7-methyl-2,3-dioxo-1,2,3,4-tetrahydroquinoxaline-6-sulfonamide | ++ | (+) |
| 64 | N-([1,1'-biphenyl]-4-yl)-7-fluoro-2,3-dioxo-1,2,3,4-tetrahydroquinoxaline-6-sulfonamide | ++ | + |
| 65 | N-(2,3'-dichloro-[1,1'-biphenyl]-4-yl)-7-fluoro-2,3-dioxo-1,2,3,4-tetrahydroquinoxaline-6-sulfonamide | ++ | (+) |
| 66 | N-(3-chloro-4-(pyrimidin-5-yl)phenyl)-7-methyl-2,3-dioxo-1,2,3,4-tetrahydroquinoxaline-6-sulfonamide | ++ | + |
| 67 | N-(4-cyclopropylphenyl)-7-ethyl-2,3-dioxo-1,2,3,4-tetrahydroquinoxaline-6-sulfonamide | ++ | ++ |
| 68 | N-(4-cyclopropylphenyl)-7-fluoro-2,3-dioxo-1,2,3,4-tetrahydroquinoxaline-6-sulfonamide | ++ | + |
| 69 | N-(3-chloro-4-(5-methylfuran-2-yl)phenyl)-7-methyl-2,3-dioxo-1,2,3,4-tetrahydroquinoxaline-6-sulfonamide | ++ | (+) |
| 70 | N-(3,5-dimethyl-4-(5-methylthiophen-2-yl)phenyl)-7-methyl-2,3-dioxo-1,2,3,4-tetrahydroquinoxaline-6-sulfonamide | ++ | + |
| 71 | N-(4-cyclohexylphenyl)-7-fluoro-2,3-dioxo-1,2,3,4-tetrahydroquinoxaline-6-sulfonamide | + | + |
| 72 | N-(2-chloro-2'-methoxy-[1,1'-biphenyl]-4-yl)-7-methyl-2,3-dioxo-1,2,3,4-tetrahydroquinoxaline-6-sulfonamide | + | + |
| 73 | N-(2,6-dimethyl-[1,1'-biphenyl]-4-yl)-7-methyl-2,3-dioxo-1,2,3,4-tetrahydroquinoxaline-6-sulfonamide | + | + |
| 74 | N-(2,6-dimethyl-[1,1'-biphenyl]-4-yl)-7-fluoro-2,3-dioxo-1,2,3,4-tetrahydroquinoxaline-6-sulfonamide | + | + |
| 75 | N-(4-bromo-3-chlorophenyl)-7-methyl-2,3-dioxo-1,2,3,4-tetrahydroquinoxaline-6-sulfonamide | + | + |
| 76 | N-(4-isopropylphenyl)-7-methyl-2,3-dioxo-1,2,3,4-tetrahydroquinoxaline-6-sulfonamide | + | + |
| 77 | N-(2,2'-dichloro-[1,1'-biphenyl]-4-yl)-7-methyl-2,3-dioxo-1,2,3,4-tetrahydroquinoxaline-6-sulfonamide | + | + |
| 78 | N-(2-chloro-2'-(hydroxymethyl)-[1,1'-biphenyl]-4-yl)-7-methyl-2,3-dioxo-1,2,3,4-tetrahydroquinoxaline-6-sulfonamide | + | + |
| 79 | N-(2,3-dihydro-1H-inden-5-yl)-7-methyl-2,3-dioxo-1,2,3,4-tetrahydroquinoxaline-6-sulfonamide | + | ++ |
| 80 | 7-chloro-N-(3,5-dimethyl-4-(thiophen-3-yl)phenyl)-2,3-dioxo-1,2,3,4-tetrahydroquinoxaline-6-sulfonamide | + | |
| 81 | N-(2-methoxy-[1,1'-biphenyl]-4-yl)-7-methyl-2,3-dioxo-1,2,3,4-tetrahydroquinoxaline-6-sulfonamide | + | + |
| 82 | N-(3-chloro-4-(thiophen-3-yl)phenyl)-7-fluoro-2,3-dioxo-1,2,3,4-tetrahydroquinoxaline-6-sulfonamide | + | (+) |
| 83 | N-(4-(tert-butyl)phenyl)-7-methyl-2,3-dioxo-1,2,3,4-tetrahydroquinoxaline-6-sulfonamide | + | ++ |
| 84 | N-(3,5-dimethyl-4-(5-methylfuran-2-yl)phenyl)-7-methyl-2,3-dioxo-1,2,3,4-tetrahydroquinoxaline-6-sulfonamide | + | + |
| 85 | 7-chloro-N-(4-cyclopropylphenyl)-2,3-dioxo-1,2,3,4-tetrahydroquinoxaline-6-sulfonamide | + | ++ |
| 86 | N-(4-cyclohexylphenyl)-7-methyl-2,3-dioxo-1,2,3,4-tetrahydroquinoxaline-6-sulfonamide | + | + |
| 87 | 7-fluoro-2,3-dioxo-N-(4-propylphenyl)-1,2,3,4-tetrahydroquinoxaline-6-sulfonamide | + | ++ |
| 88 | 7-chloro-N-(4-(furan-3-yl)-3-methylphenyl)-2,3-dioxo-1,2,3,4-tetrahydroquinoxaline-6-sulfonamide | ++ | + |
| 89 | 7-methyl-N-(2'-methyl-[1,1'-biphenyl]-4-yl)-2,3-dioxo-1,2,3,4-tetrahydroquinoxaline-6-sulfonamide | + | + |
| 90 | N-(2-chloro-2'-methyl-[1,1'-biphenyl]-4-yl)-7-methyl-2,3-dioxo-1,2,3,4-tetrahydroquinoxaline-6-sulfonamide | + | + |
| 91 | 7-methyl-N-(3-methyl-4-(trifluoromethyl)phenyl)-2,3-dioxo-1,2,3,4-tetrahydroquinoxaline-6-sulfonamide | + | + |
| 92 | N-(4-ethylphenyl)-7-methyl-2,3-dioxo-1,2,3,4-tetrahydroquinoxaline-6-sulfonamide | +++ | +++ |
| 93 | 7-chloro-2,3-dioxo-N-(4-(trifluoromethyl)phenyl)-1,2,3,4-tetrahydroquinoxaline-6-sulfonamide | + | + |
| 94 | 7-chloro-N-(3-chloro-4-(pyridin-4-yl)phenyl)-2,3-dioxo-1,2,3,4-tetrahydroquinoxaline-6-sulfonamide | + | + |
| 95 | 7-fluoro-N-(naphthalen-2-yl)-2,3-dioxo-1,2,3,4-tetrahydroquinoxaline-6-sulfonamide | + | |
| 96 | 7-bromo-N-(4-cyclopropylphenyl)-2,3-dioxo-1,2,3,4-tetrahydroquinoxaline-6-sulfonamide | + | |
| 97 | N-(4-bromophenyl)-7-methyl-2,3-dioxo-1,2,3,4-tetrahydroquinoxaline-6-sulfonamide | + | ++ |
| 98 | N-([1,1'-biphenyl]-4-yl)-7-chloro-2,3-dioxo-1,2,3,4-tetrahydroquinoxaline-6-sulfonamide | + | + |
| 99 | N-(3-chloro-4-(pyridin-3-yl)phenyl)-7-fluoro-2,3-dioxo-1,2,3,4-tetrahydroquinoxaline-6-sulfonamide | + | (+) |
| 100 | N-(3-chloro-4-(thiophen-3-yl)phenyl)-7-isopropyl-2,3-dioxo-1,2,3,4-tetrahydroquinoxaline-6-sulfonamide | + | (+) |
| 101 | 7-fluoro-N-(4-isopropylphenyl)-2,3-dioxo-1,2,3,4-tetrahydroquinoxaline-6-sulfonamide | + | + |
| 102 | N-(2'-methoxy-2,6-dimethyl-[1,1'-biphenyl]-4-yl)-7-methyl-2,3-dioxo-1,2,3,4-tetrahydroquinoxaline-6-sulfonamide | + | ++ |
| 103 | N-(2,2'-dimethyl-[1,1'-biphenyl]-4-yl)-7-methyl-2,3-dioxo-1,2,3,4-tetrahydroquinoxaline-6-sulfonamide | + | + |
| 104 | N-(2'-chloro-2,6-dimethyl-[1,1'-biphenyl]-4-yl)-7-methyl-2,3-dioxo-1,2,3,4-tetrahydroquinoxaline-6-sulfonamide | + | + |
| 105 | 7-chloro-N-(2,3'-dichloro-2',4'-difluoro-[1,1'-biphenyl]-4-yl)-2,3-dioxo-1,2,3,4-tetrahydroquinoxaline-6-sulfonamide | + | + |
| 106 | 7-chloro-N-(2,6-dimethyl-[1,1'-biphenyl]-4-yl)-2,3-dioxo-1,2,3,4-tetrahydroquinoxaline-6-sulfonamide | + | ++ |
| 107 | N-(4-(furan-3-yl)-3,5-dimethylphenyl)-7-methyl-2,3-dioxo-1,2,3,4-tetrahydroquinoxaline-6-sulfonamide | + | ++ |
| 108 | N-(2'-chloro-[1,1'-biphenyl]-4-yl)-7-methyl-2,3-dioxo-1,2,3,4-tetrahydroquinoxaline-6-sulfonamide | + | + |
| 109 | N-(2-chloro-3'-(dimethylamino)-[1,1'-biphenyl]-4-yl)-7-isopropyl-2,3-dioxo-1,2,3,4-tetrahydroquinoxaline-6-sulfonamide | + | + |
| 110 | N-(4-cyclopropyl-2-methylphenyl)-7-methyl-2,3-dioxo-1,2,3,4-tetrahydroquinoxaline-6-sulfonamide | + | ++ |
| 111 | N-(4-ethoxyphenyl)-7-methyl-2,3-dioxo-1,2,3,4-tetrahydroquinoxaline-6-sulfonamide | + | |
| 112 | 7-methyl-N-(3'-methyl-[1,1'-biphenyl]-4-yl)-2,3-dioxo-1,2,3,4-tetrahydroquinoxaline-6-sulfonamide | + | + |
| 113 | N-(2'-ethyl-[1,1'-biphenyl]-4-yl)-7-methyl-2,3-dioxo-1,2,3,4-tetrahydroquinoxaline-6-sulfonamide | + | + |
| 114 | N-(2'-methoxy-[1,1'-biphenyl]-4-yl)-7-methyl-2,3-dioxo-1,2,3,4-tetrahydroquinoxaline-6-sulfonamide | + | + |
| 115 | 7-methyl-N-(4'-methyl-[1,1'-biphenyl]-4-yl)-2,3-dioxo-1,2,3,4-tetrahydroquinoxaline-6-sulfonamide | + | |
| 116 | N-(2',6'-dimethyl-[1,1'-biphenyl]-4-yl)-7-methyl-2,3-dioxo-1,2,3,4-tetrahydroquinoxaline-6-sulfonamide | + | |
| 117 | N-(4-isopropyl-3-methylphenyl)-7-methyl-2,3-dioxo-1,2,3,4-tetrahydroquinoxaline-6-sulfonamide | + | + |
| 118 | N-(2',4'-dimethyl-[1,1'-biphenyl]-4-yl)-7-methyl-2,3-dioxo-1,2,3,4-tetrahydroquinoxaline-6-sulfonamide | + | |
| 119 | N-(2'-(dimethylamino)-[1,1'-biphenyl]-4-yl)-7-methyl-2,3-dioxo-1,2,3,4-tetrahydroquinoxaline-6-sulfonamide | + | + |
| 120 | ethyl 4'-((7-methyl-2,3-dioxo-1,2,3,4-tetrahydroquinoxaline)-6-sulfonamido)-[1,1'-biphenyl]-2-carboxylate | + | (+) |
| 121 | N-(4-bromo-3-methoxyphenyl)-7-methyl-2,3-dioxo-1,2,3,4-tetrahydroquinoxaline-6-sulfonamide | + | (+) |
| 122 | N-(4-bromo-3,5-dimethylphenyl)-7-chloro-2,3-dioxo-1,2,3,4-tetrahydroquinoxaline-6-sulfonamide | + | + |
| 123 | N-(2'-acetyl-[1,1'-biphenyl]-4-yl)-7-methyl-2,3-dioxo-1,2,3,4-tetrahydroquinoxaline-6-sulfonamide | + | (+) |
| 124 | N-(2-methoxy-2'-methyl-[1,1'-biphenyl]-4-yl)-7-methyl-2,3-dioxo-1,2,3,4-tetrahydroquinoxaline-6-sulfonamide | + | + |
| 125 | 7-methyl-2,3-dioxo-N-(2,2',6-trimethyl-[1,1'-biphenyl]-4-yl)-1,2,3,4-tetrahydroquinoxaline-6-sulfonamide | + | (+) |
| 126 | 4'-((7-methyl-2,3-dioxo-1,2,3,4-tetrahydroquinoxaline)-6-sulfonamido)-[1,1'-biphenyl]-2-carboxamide | (+) | + |
| 127 | 7-methyl-2,3-dioxo-N-(4-(pyridin-4-yl)phenyl)-1,2,3,4-tetrahydroquinoxaline-6-sulfonamide | + | + |
| 128 | N-(3-bromo-4-morpholinophenyl)-7-methyl-2,3-dioxo-1,2,3,4-tetrahydroquinoxaline-6-sulfonamide | + | + |
| 129 | N-(4-cyanophenyl)-7-methyl-2,3-dioxo-1,2,3,4-tetrahydroquinoxaline-6-sulfonamide | (+) | |
| 130 | N-(4-ethynylphenyl)-7-methyl-2,3-dioxo-1,2,3,4-tetrahydroquinoxaline-6-sulfonamide | + | + |
| 131 | N-(3,5-dimethyl-4-(pyridin-4-yl)phenyl)-7-methyl-2,3-dioxo-1,2,3,4-tetrahydroquinoxaline-6-sulfonamide | + | |
| 132 | N-(3,5-dimethyl-4-(3-methylthiophen-2-yl)phenyl)-7-methyl-2,3-dioxo-1,2,3,4-tetrahydroquinoxaline-6-sulfonamide | + | + |
| 133 | N-(4-(dimethylamino)phenyl)-7-methyl-2,3-dioxo-1,2,3,4-tetrahydroquinoxaline-6-sulfonamide | + | |
| 134 | N-(2'-ethyl-2,6-dimethyl-[1,1'-biphenyl]-4-yl)-7-methyl-2,3-dioxo-1,2,3,4-tetrahydroquinoxaline-6-sulfonamide | + | ++ |
| 135 | N-(3,5-dimethyl-4-(1H-pyrrol-3-yl)phenyl)-7-methyl-2,3-dioxo-1,2,3,4-tetrahydroquinoxaline-6-sulfonamide | + | + |
| 136 | N-(2-chloro-2'-(dimethylamino)-[1,1'-biphenyl]-4-yl)-7-methyl-2,3-dioxo-1,2,3,4-tetrahydroquinoxaline-6-sulfonamide | + | + |
| 137 | 7-methyl-2,3-dioxo-N-(4-(pyrrolidin-1-yl)phenyl)-1,2,3,4-tetrahydroquinoxaline-6-sulfonamide | + | ++ |
| 138 | ethyl 2'-chloro-4'-((7-methyl-2,3-dioxo-1,2,3,4-tetrahydroquinoxaline)-6-sulfonamido)-[1,1'-biphenyl]-2-carboxylate | (+) | (+) |
| 139 | N-(2-chloro-2'-(trifluoromethyl)-[1,1'-biphenyl]-4-yl)-7-methyl-2,3-dioxo-1,2,3,4-tetrahydroquinoxaline-6-sulfonamide | + | (+) |
| 140 | N-(3-chloro-4-(2-methylpyridin-4-yl)phenyl)-7-methyl-2,3-dioxo-1,2,3,4-tetrahydroquinoxaline-6-sulfonamide | + | + |
| 141 | N-(4-bromo-3-methylphenyl)-7-chloro-2,3-dioxo-1,2,3,4-tetrahydroquinoxaline-6-sulfonamide | + | + |
| 142 | 7-chloro-N-(4-isopropylphenyl)-2,3-dioxo-1,2,3,4-tetrahydroquinoxaline-6-sulfonamide | + | + |
| 143 | 7-chloro-N-(3-chloro-4-(trifluoromethyl)phenyl)-2,3-dioxo-1,2,3,4-tetrahydroquinoxaline-6-sulfonamide | + | (+) |
| 144 | N-(4-bromo-3-chlorophenyl)-7-chloro-2,3-dioxo-1,2,3,4-tetrahydroquinoxaline-6-sulfonamide | + | (+) |
| 145 | N-(4-bromo-3,5-dimethylphenyl)-7-chloro-2,3-dioxo-1,2,3,4-tetrahydroquinoxaline-6-sulfonamide | + | |
| 146 | 7-chloro-N-(3-chloro-4-(5-methylfuran-2-yl)phenyl)-2,3-dioxo-1,2,3,4-tetrahydroquinoxaline-6-sulfonamide | + | (+) |
| 147 | 7-fluoro-2,3-dioxo-N-(4-(trifluoromethoxy)phenyl)-1,2,3,4-tetrahydroquinoxaline-6-sulfonamide | + | + |
| 148 | N-(4-bromo-3-chlorophenyl)-7-fluoro-2,3-dioxo-1,2,3,4-tetrahydroquinoxaline-6-sulfonamide | + | |
| 149 | N-(4-bromo-3-chlorophenyl)-7-ethyl-2,3-dioxo-1,2,3,4-tetrahydroquinoxaline-6-sulfonamide | + | + |
| 150 | N-(3-chloro-4-(pyridin-4-yl)phenyl)-7-fluoro-2,3-dioxo-1,2,3,4-tetrahydroquinoxaline-6-sulfonamide | + | + |
| 151 | 7-isopropyl-2,3-dioxo-N-(4-propylphenyl)-1,2,3,4-tetrahydroquinoxaline-6-sulfonamide | + | |
| 152 | 7-isopropyl-2,3-dioxo-N-(4-(trifluoromethyl)phenyl)-1,2,3,4-tetrahydroquinoxaline-6-sulfonamide | + | ++ |
| 153 | N-(4-cyclopropylphenyl)-7-isopropyl-2,3-dioxo-1,2,3,4-tetrahydroquinoxaline-6-sulfonamide | + | |
| 154 | N-(4-bromo-3-chlorophenyl)-7-isopropyl-2,3-dioxo-1,2,3,4-tetrahydroquinoxaline-6-sulfonamide | + | |
| 155 | 7-chloro-N-(3-methyl-4-(pyridin-4-yl)phenyl)-2,3-dioxo-1,2,3,4-tetrahydroquinoxaline-6-sulfonamide | + | |
| 156 | 7-chloro-N-(4-(furan-3-yl)-3,5-dimethylphenyl)-2,3-dioxo-1,2,3,4-tetrahydroquinoxaline-6-sulfonamide | + | |
| 157 | 7-chloro-N-(3,5-dimethyl-4-(pyridin-4-yl)phenyl)-2,3-dioxo-1,2,3,4-tetrahydroquinoxaline-6-sulfonamide | (+) | |
| 158 | N-(3-chloro-4-(5-methylfuran-2-yl)phenyl)-7-fluoro-2,3-dioxo-1,2,3,4-tetrahydroquinoxaline-6-sulfonamide | + | |
| 159 | N-(3-chloro-4-(pyridin-4-yl)phenyl)-7-isopropyl-2,3-dioxo-1,2,3,4-tetrahydroquinoxaline-6-sulfonamide | (+) | |
| 160 | 7-fluoro-2,3-dioxo-N-(4-(trifluoromethyl)phenyl)-1,2,3,4-tetrahydroquinoxaline-6-sulfonamide | (+) | |
| 161 | N-(3-chloro-4-(furan-3-yl)phenyl)-7-isopropyl-2,3-dioxo-1,2,3,4-tetrahydroquinoxaline-6-sulfonamide | + | |
| 162 | N-(4-bromo-3,5-dimethylphenyl)-7-fluoro-2,3-dioxo-1,2,3,4-tetrahydroquinoxaline-6-sulfonamide | + | |
| 163 | N-(4-bromo-3-methylphenyl)-7-fluoro-2,3-dioxo-1,2,3,4-tetrahydroquinoxaline-6-sulfonamide | + | |
| 164 | 7-fluoro-2,3-dioxo-N-(5,6,7,8-tetrahydronaphthalen-2-yl)-1,2,3,4-tetrahydroquinoxaline-6-sulfonamide | + | |
| 165 | N-(3-chloro-4-(trifluoromethyl)phenyl)-7-fluoro-2,3-dioxo-1,2,3,4-tetrahydroquinoxaline-6-sulfonamide | (+) | |
| 166 | 7-fluoro-N-(4-(furan-3-yl)-3,5-dimethylphenyl)-2,3-dioxo-1,2,3,4-tetrahydroquinoxaline-6-sulfonamide | + | |
| 167 | N-(3,5-dimethyl-4-(pyridin-4-yl)phenyl)-7-fluoro-2,3-dioxo-1,2,3,4-tetrahydroquinoxaline-6-sulfonamide | (+) | |
| 168 | 7-fluoro-N-(4-(furan-3-yl)-3-methylphenyl)-2,3-dioxo-1,2,3,4-tetrahydroquinoxaline-6-sulfonamide | + | (+) |
| 169 | 7-fluoro-N-(3-methyl-4-(pyridin-4-yl)phenyl)-2,3-dioxo-1,2,3,4-tetrahydroquinoxaline-6-sulfonamide | + | |
| 170 | 7-chloro-N-(4-cyclohexylphenyl)-2,3-dioxo-1,2,3,4-tetrahydroquinoxaline-6-sulfonamide | + | + |
| 171 | N-(4-bromo-3-methylphenyl)-7-methyl-2,3-dioxo-1,2,3,4-tetrahydroquinoxaline-6-sulfonamide | + | ++ |
| 172 | 7-chloro-N-(naphthalen-2-yl)-2,3-dioxo-1,2,3,4-tetrahydroquinoxaline-6-sulfonamide | + | + |
| 173 | N-(4-(tert-butyl)phenyl)-7-chloro-2,3-dioxo-1,2,3,4-tetrahydroquinoxaline-6-sulfonamide | + | ++ |
| 174 | 7-chloro-2,3-dioxo-N-(5,6,7,8-tetrahydronaphthalen-2-yl)-1,2,3,4-tetrahydroquinoxaline-6-sulfonamide | + | ++ |
| 175 | 7-methyl-2,3-dioxo-N-(2,3',6-trimethyl-[1,1'-biphenyl]-4-yl)-1,2,3,4-tetrahydroquinoxaline-6-sulfonamide | + | + |
| 176 | N-(2-chloro-[1,1'-biphenyl]-4-yl)-7-isopropyl-2,3-dioxo-1,2,3,4-tetrahydroquinoxaline-6-sulfonamide | + | |
| 177 | N-(4-chlorophenyl)-7-methyl-2,3-dioxo-1,2,3,4-tetrahydroquinoxaline-6-sulfonamide | + | + |
| 178 | N-(2,3'-dichloro-[1,1'-biphenyl]-4-yl)-7-isopropyl-2,3-dioxo-1,2,3,4-tetrahydroquinoxaline-6-sulfonamide | + | |
| 179 | 7-methyl-2,3-dioxo-N-(p-tolyl)-1,2,3,4-tetrahydroquinoxaline-6-sulfonamide | + | |
| 180 | 7-methyl-N-(4-morpholinophenyl)-2,3-dioxo-1,2,3,4-tetrahydroquinoxaline-6-sulfonamide | (+) | |
| 181 | N-(3-chloro-4-methylphenyl)-7-methyl-2,3-dioxo-1,2,3,4-tetrahydroquinoxaline-6-sulfonamide | (+) | |
| 182 | 4'-((7-methyl-2,3-dioxo-1,2,3,4-tetrahydroquinoxaline)-6-sulfonamido)-[1,1'-biphenyl]-2-carboxylic acid | (+) | (+) |
| 183 | N-(3-chloro-4-fluorophenyl)-7-methyl-2,3-dioxo-1,2,3,4-tetrahydroquinoxaline-6-sulfonamide | (+) | |
| 184 | N-(3,5-dimethyl-4-(1-methyl-1H-pyrazol-4-yl)phenyl)-7-methyl-2,3-dioxo-1,2,3,4-tetrahydroquinoxaline-6-sulfonamide | (+) | |
| 185 | N-(3,4-dimethylphenyl)-7-methyl-2,3-dioxo-1,2,3,4-tetrahydroquinoxaline-6-sulfonamide | (+) | |
| 186 | 7-methyl-2,3-dioxo-N-(quinolin-6-yl)-1,2,3,4-tetrahydroquinoxaline-6-sulfonamide | (+) | |
| 187 | N-(3,5-dimethyl-4-(1H-pyrazol-4-yl)phenyl)-7-methyl-2,3-dioxo-1,2,3,4-tetrahydroquinoxaline-6-sulfonamide | (+) | |
| 188 | N-(3,5-dimethyl-4-(1-methyl-1H-pyrazol-5-yl)phenyl)-7-methyl-2,3-dioxo-1,2,3,4-tetrahydroquinoxaline-6-sulfonamide | (+) | |
| 189 | N-(3-chloro-4-(5-chloropyridin-3-yl)phenyl)-7-methyl-2,3-dioxo-1,2,3,4-tetrahydroquinoxaline-6-sulfonamide | ++ | |
| 190 | 2,3-dioxo-N-(4-(trifluoromethoxy)phenyl)-7-(trifluoromethyl)-1,2,3,4-tetrahydroquinoxaline-6-sulfonamide | + | |
| 191 | 7-nitro-2,3-dioxo-N-(4-(trifluoromethoxy)phenyl)-1,2,3,4-tetrahydroquinoxaline-6-sulfonamide | + | |
| 192 | 2,3-dioxo-7-(trifluoromethoxy)-N-(4-(trifluoromethoxy)phenyl)-1,2,3,4-tetrahydroquinoxaline-6-sulfonamide | + | |
| 193 | 7-methoxy-2,3-dioxo-N-(4-(trifluoromethoxy)phenyl)-1,2,3,4-tetrahydroquinoxaline-6-sulfonamide | + | |
| 195 | 7-bromo-N-(4-(tert-butyl)phenyl)-2,3-dioxo-1,2,3,4-tetrahydroquinoxaline-6-sulfonamide | (+) | |
| 196 | N-(3-chloro-4-(pyrrolidin-1-yl)phenyl)-7-methyl-2,3-dioxo-1,2,3,4-tetrahydroquinoxaline-6-sulfonamide | ++ | |
| 197 | N-(2-chloro-4'-fluoro-[1,1'-biphenyl]-4-yl)-2,3-dioxo-7-(trifluoromethoxy)-1,2,3,4-tetrahydroquinoxaline-6-sulfonamide | (+) | (+) |
| 198 | N-(3-chloro-4-(5-methylthiophen-3-yl)phenyl)-7-methyl-2,3-dioxo-1,2,3,4-tetrahydroquinoxaline-6-sulfonamide | | |
| 199 | N-(5-chloro-6-(1H-indol-6-yl)pyridin-3-yl)-7-methyl-2,3-dioxo-1,2,3,4-tetrahydroquinoxaline-6-sulfonamide | | |
| 200 | N-(3-chloro-4-morpholinophenyl)-7-methyl-2,3-dioxo-1,2,3,4-tetrahydroquinoxaline-6-sulfonamide | (+) | |
| 201 | N-(3-chloro-4-(3-methylmorpholino)phenyl)-7-methyl-2,3-dioxo-1,2,3,4-tetrahydroquinoxaline-6-sulfonamide | + | |
| 202 | N-(3-chloro-4-(3,3-dimethylmorpholino)phenyl)-7-methyl-2,3-dioxo-1,2,3,4-tetrahydroquinoxaline-6-sulfonamide | + | + |
| 203 | N-(5-chloro-6-(4-ethynylphenyl)pyridin-3-yl)-7-methyl-2,3-dioxo-1,2,3,4-tetrahydroquinoxaline-6-sulfonamide | | |
| 204 | N-(3-chloro-4-(2-methylmorpholino)phenyl)-7-methyl-2,3-dioxo-1,2,3,4-tetrahydroquinoxaline-6-sulfonamide | (+) | |
| 205 | N-(4,5'-dichloro-[3,3'-bipyridin]-6-yl)-7-methyl-2,3-dioxo-1,2,3,4-tetrahydroquinoxaline-6-sulfonamide | | |
| 206 | N-(3'-(azetidin-1-yl)-2-chloro-[1,1'-biphenyl]-4-yl)-7-methyl-2,3-dioxo-1,2,3,4-tetrahydroquinoxaline-6-sulfonamide | +++ | ++ |
| 207 | 7-methyl-2,3-dioxo-N-(1-phenyl-1H-pyrazol-4-yl)-1,2,3,4-tetrahydroquinoxaline-6-sulfonamide | (+) | |
| 208 | N-(3-chloro-4-(1,4-oxazepan-4-yl)phenyl)-7-methyl-2,3-dioxo-1,2,3,4-tetrahydroquinoxaline-6-sulfonamide | (+) | |
| 209 | N-(2-chloro-4'-cyano-3'-methoxy-[1,1'-biphenyl]-4-yl)-7-methyl-2,3-dioxo-1,2,3,4-tetrahydroquinoxaline-6-sulfonamide | | |
| 210 | N-(4-chloro-5-(3-chlorophenyl)pyridin-2-yl)-7-methyl-2,3-dioxo-1,2,3,4-tetrahydroquinoxaline-6-sulfonamide | ++ | + |
| 211 | N-(4-chloro-5-(3-chloro-4-fluorophenyl)pyridin-2-yl)-7-methyl-2,3-dioxo-1,2,3,4-tetrahydroquinoxaline-6-sulfonamide | + | + |
| 212 | N-(4-chloro-5-(4-fluorophenyl)pyridin-2-yl)-7-methyl-2,3-dioxo-1,2,3,4-tetrahydroquinoxaline-6-sulfonamide | ++ | |
| 213 | N-(5-chloro-6-(3-chlorophenyl)pyridin-3-yl)-7-methyl-2,3-dioxo-1,2,3,4-tetrahydroquinoxaline-6-sulfonamide | + | |
| 214 | N-(5-chloro-6-(3-chloro-4-fluorophenyl)pyridin-3-yl)-7-methyl-2,3-dioxo-1,2,3,4-tetrahydroquinoxaline-6-sulfonamide | + | |
| 215 | N-(5-chloro-6-(4-fluorophenyl)pyridin-3-yl)-7-methyl-2,3-dioxo-1,2,3,4-tetrahydroquinoxaline-6-sulfonamide | ++ | |
| 216 | 7⁻cyano-2,3-dioxo-N-(4-(trifluoromethoxy)phenyl)-1,2,3,4-tetrahydroquinoxaline-6-sulfonamide | + | + |
| 217 | 7-(methyl-d3)-2,3-dioxo-N-(4-(trifluoromethoxy)phenyl)-1,2,3,4-tetrahydroquinoxaline-6-sulfonamide | +++ | +++ |
| 218 | 7-(difluoromethyl)-2,3-dioxo-N-(4-(trifluoromethoxy)phenyl)-1,2,3,4-tetrahydroquinoxaline-6-sulfonamide | + | + |
| 219 | N-(2-chloro-3',5'-difluoro-4'-methoxy-[1,1'-biphenyl]-4-yl)-7-methyl-2,3-dioxo-1,2,3,4-tetrahydroquinoxaline-6-sulfonamide | | |
| 220 | N-(2-chloro-4'-cyclopropyl-[1,1'-biphenyl]-4-yl)-7-methyl-2,3-dioxo-1,2,3,4-tetrahydroquinoxaline-6-sulfonamide | | |
| 221 | 7-(dimethylamino)-2,3-dioxo-N-(4-(trifluoromethoxy)phenyl)-1,2,3,4-tetrahydroquinoxaline-6-sulfonamide | ++ | |
| 222 | 7-methyl-2,3-dioxo-N-(5-(trifluoromethoxy)pyridin-2-yl)-1,2,3,4-tetrahydroquinoxaline-6-sulfonamide | + | |
| 223 | 7-methyl-2,3-dioxo-N-(6-(trifluoromethoxy)pyridin-3-yl)-1,2,3,4-tetrahydroquinoxaline-6-sulfonamide | + | |
| 224 | 7-methyl-2,3-dioxo-N-(6-phenylpyridin-3-yl)-1,2,3,4-tetrahydroquinoxaline-6-sulfonamide | + | |
| 225 | N-(2,4'-dichloro-3'-methoxy-[1,1'-biphenyl]-4-yl)-7-methyl-2,3-dioxo-1,2,3,4-tetrahydroquinoxaline-6-sulfonamide | | |
| 226 | N-(5-bromo-4-chloropyridin-2-yl)-7-methyl-2,3-dioxo-1,2,3,4-tetrahydroquinoxaline-6-sulfonamide | (+) | |
| 227 | N-(3-chloro-4-(3-oxo-3,4-dihydro-2H-benzo[b][1,4]oxazin-7-yl)phenyl)-7-methyl-2,3-dioxo-1,2,3,4-tetrahydroquinoxaline-6-sulfonamide | | |
| 228 | 7-methyl-N-(1-methyl-1H-indol-5-yl)-2,3-dioxo-1,2,3,4-tetra hydroquinoxaline-6-sulfonamide | (+) | |
| 229 | N-(3-chloro-4-(isoquinolin-7-yl)phenyl)-7-methyl-2,3-dioxo-1,2,3,4-tetrahydroquinoxaline-6-sulfonamide | | |
| 230 | 7-methyl-2,3-dioxo-N-(3-phenylbicyclo[1.1.1]pentan-1-yl)-1,2,3,4-tetrahydroquinoxaline-6-sulfonamide | + | + |
| 231 | 7-methyl-2,3-dioxo-N-(quinolin-7-yl)-1,2,3,4-tetrahydroquinoxaline-6-sulfonamide | + | |
| 232 | N-(3-chloro-4-(2-methylquinolin-6-yl)phenyl)-7-methyl-2,3-dioxo-1,2,3,4-tetrahydroquinoxaline-6-sulfonamide | | |
| 233 | N-(3-chloro-4-(isoquinolin-6-yl)phenyl)-7-methyl-2,3-dioxo-1,2,3,4-tetrahydroquinoxaline-6-sulfonamide | | |
| 234 | 7-methyl-N-(2-methylbenzo[d]thiazol-5-yl)-2,3-dioxo-1,2,3,4-tetrahydroquinoxaline-6-sulfonamide | (+) | |
| 235 | N-(3-chloro-4-(quinolin-6-yl)phenyl)-7-methyl-2,3-dioxo-1,2,3,4-tetrahydroqu¡noxaline-6-sulfonamide | | |
| 236 | N-(6-bromo-5-chloropyridin-3-yl)-7-methyl-2,3-dioxo-1,2,3,4-tetrahydroquinoxaline-6-sulfonamide | (+) | |
| 237 | 2-(4-(trifluoromethoxy)phenyl)-3,4-dihydro-2H-[1,4,5]oxathiazepino[2,3-g]quinoxaline-8,9(7H,10H)-dione 1,1-dioxide | + | |
| 238 | N-(benzo[d]thiazol-2-yl)-7-methyl-2,3-dioxo-1,2,3,4-tetrahydroquinoxaline-6-sulfonamide | + | |
| 239 | 7-methyl-N-(2-methylbenzo[d]thiazol-6-yl)-2,3-dioxo-1,2,3,4-tetrahydroquinoxaline-6-sulfonamide | (+) | |
| 240 | 7-methyl-N-(4-methyl-5-phenylthiazol-2-yl)-2,3-dioxo-1,2,3,4-tetrahydroquinoxaline-6-sulfonamide | (+) | |
| 241 | N-(2,2-difluorobenzo[d][1,3]dioxol-5-yl)-7-methyl-2,3-dioxo-1,2,3,4-tetrahydroquinoxaline-6-sulfonamide | + | |
| 242 | N-(2,3-dihydrobenzo[b] [1,4]dioxin-6-yl)-7-methyl-2,3-dioxo-1,2,3,4-tetrahydroquinoxaline-6-sulfonamide | + | |
| 243 | (R)-N-(3-chloro-4-(3-methylmorpholino)phenyl)-7-methyl-2,3-dioxo-1,2,3,4-tetrahydroquinoxaline-6-sulfonamide | (+) | + |
| 244 | N-[3-bromo-4-(4-fluorophenyl)phenyl]-7-methyl-2,3-dioxo-1,4-dihydroquinoxaline-6-sulfonamide | +++ | ++ |
| 245 | 3-[2-chloro-4-[(7-methyl-2,3-dioxo-1,4-dihydroquinoxalin-6-yl)sulfonylamino]phenyl]benzamide | +++ | ++ |
| 246 | N-[3-chloro-4-(4-methoxyphenyl)phenyl]-7-methyl-2,3-dioxo-1,4-dihydroquinoxaline-6-sulfonamide | +++ | ++ |
| 247 | N-[4-(1,3-benzodioxol-5-yl)-3-chloro-phenyl]-7-methyl-2,3-dioxo-1,4-dihydroquinoxaline-6-sulfonamide | +++ | ++ |
| 248 | N-[3-chloro-4-(3,5-dimethylphenyl)phenyl]-7-methyl-2,3-dioxo-1,4-dihydroquinoxaline-6-sulfonamide | + | + |
| 249 | N-[4-(3-acetylphenyl)-3-chloro-phenyl]-7-methyl-2,3-dioxo-1,4-dihydroquinoxaline-6-sulfonamide | +++ | + |
| 250 | N-[4-(4-fluorophenyl)-3-morpholino-phenyl]-7-methyl-2,3-dioxo-1,4-dihydroquinoxaline-6-sulfonamide | + | (+) |
| 251 | N-[3-ethynyl-4-(4-ethynylphenyl)phenyl]-7-methyl-2,3-dioxo-1,4-dihydroquinoxaline-6-sulfonamide | +++ | +++ |
| 252 | N-(3-ethynyl-4-phenyl-phenyl)-7-methyl-2,3-dioxo-1,4-dihydroquinoxaline-6-sulfonamide | +++ | ++ |
| 253 | N-[4-(4-cyanophenyl)-3-ethynyl-phenyl]-7-methyl-2,3-dioxo-1,4-dihydroquinoxaline-6-sulfonamide | +++ | ++ |
| 254 | N-[3-chloro-4-(6-cyano-3-pyridyl)phenyl]-7-methyl-2,3-dioxo-1,4-dihydroquinoxaline-6-sulfonamide | + | + |
| 255 | N-[3-(dimethylamino)-4-(4-fluorophenyl)phenyl]-7-methyl-2,3-dioxo-1,4-dihydroquinoxaline-6-sulfonamide | + | + |
| 256 | N-[4-(1H-benzotriazol-5-yl)-3-chloro-phenyl]-7-methyl-2,3-dioxo-1,4-dihydroquinoxaline-6-sulfonamide | +++ | ++ |
| 257 | N-[3-chloro-4-(3,4-dichlorophenyl)phenyl]-7-methyl-2,3-dioxo-1,4-dihydroquinoxaline-6-sulfonamide | +++ | ++ |
| 258 | N-[3-chloro-4-(3,5-dimethoxyphenyl)phenyl]-7-methyl-2,3-dioxo-1,4-dihydroquinoxaline-6-sulfonamide | + | + |
| 259 | N-[3-chloro-4-(2,3-dihydro-1,4-benzodioxin-6-yl)phenyl]-7-methyl-2,3-dioxo-1,4-dihydroquinoxaline-6-sulfonamide | +++ | + |
| 260 | N-[3-chloro-4-(2-methoxypyrimidin-5-yl)phenyl]-7-methyl-2,3-dioxo-1,4-dihydroquinoxaline-6-sulfonamide | + | + |
| 261 | N-[3-chloro-4-(2-fluoro-4-methoxy-phenyl)phenyl]-7-methyl-2,3-dioxo-1,4-dihydroquinoxaline-6-sulfonamide | +++ | ++ |
| 262 | N-[3-chloro-4-(2-fluoro-4-methylsulfonyl-phenyl)phenyl]-7-methyl-2,3-dioxo-1,4-dihydroquinoxaline-6-sulfonamide | (+) | (+) |
| 263 | N-[3-chloro-4-(1,2,4-triazol-1-yl)phenyl]-7-methyl-2,3-dioxo-1,4-dihydroquinoxaline-6-sulfonamide | (+) | + |
| 264 | N-[3-chloro-4-(triazol-1-yl)phenyl]-7-methyl-2,3-dioxo-1,4-dihydroquinoxaline-6-sulfonamide | + | + |
| 265 | 7-methyl-2,3-dioxo-N-[4-(1,2,4-triazol-1-yl)phenyl]-1,4-dihydroquinoxaline-6-sulfonamide | (+) | (+) |
| 266 | N-[3-chloro-4-(3,4-dimethylphenyl)phenyl]-7-methyl-2,3-dioxo-1,4-dihydroquinoxaline-6-sulfonamide | + | + |
| 267 | N-[3-chloro-4-[4-(trideuteriomethoxy)phenyl]phenyl]-7-methyl-2,3-dioxo-1,4-dihydroquinoxaline-6-sulfonamide | +++ | ++ |
| 268 | N-[3-chloro-4-[4-(cyclopropoxy)phenyl]phenyl]-7-methyl-2,3-dioxo-1,4-dihydroquinoxaline-6-sulfonamide | ++ | + |
| 269 | N-[3-chloro-4-(2-methyl-1,3-benzoxazol-6-yl)phenyl]-7-methyl-2,3-dioxo-1,4-dihydroquinoxaline-6-sulfonamide | +++ | +++ |
| 270 | N-[3-chloro-4-(1H-indol-5-yl)phenyl]-7-methyl-2,3-dioxo-1,4-dihydroquinoxaline-6-sulfonamide | + | ++ |
| 271 | N-[3-chloro-4-(1H-indol-6-yl)phenyl]-7-methyl-2,3-dioxo-1,4-dihydroquinoxaline-6-sulfonamide | +++ | +++ |
| 272 | N-[3-chloro-4-(2-methyl-1,3-benzoxazol-5-yl)phenyl]-7-methyl-2,3-dioxo-1,4-dihydroquinoxaline-6-sulfonamide | +++ | +++ |
| 273 | N-[3-chloro-4-(1H-indazol-5-yl)phenyl]-7-methyl-2,3-dioxo-1,4-dihydroquinoxaline-6-sulfonamide | ++ | + |
| 274 | N-[3-chloro-4-(cyclohexen-1-yl)phenyl]-7-methyl-2,3-dioxo-1,4-dihydroquinoxaline-6-sulfonamide | ++ | ++ |
| 275 | N-[3-chloro-4-[6-(trifluoromethyl)-3-pyridyl]phenyl]-7-methyl-2,3-dioxo-1,4-dihydroquinoxaline-6-sulfonamide | + | (+) |
| 276 | 2,3-dioxo-7-(2,2,2-trifluoroethoxy)-N-[4-(trifluoromethoxy)phenyl]-1,4-dihydroquinoxaline-6-sulfonamide | ++ | +++ |
| 277 | N-[3-chloro-4-[4-(trifluoromethoxy)phenyl]phenyl]-7-methyl-2,3-dioxo-1,4-dihydroquinoxaline-6-sulfonamide | + | + |
| 278 | 7-ethoxy-2,3-dioxo-N-[4-(trifluoromethoxy)phenyl]-1,4-dihydroquinoxaline-6-sulfonamide | + | +++ |
| 279 | N-[5-chloro-6-(5-chloro-3-pyridyl)-3-pyridyl]-7-methyl-2,3-dioxo-1,4-dihydroquinoxaline-6-sulfonamide | + | + |
| 280 | N-[3-chloro-4-(2-oxo-1H-pyridin-4-yl)phenyl]-7-methyl-2,3-dioxo-1,4-dihydroquinoxaline-6-sulfonamide | (+) | + |
| 281 | 7-(2-hydroxyethylamino)-2,3-dioxo-N-[4-(trifluoromethoxy)phenyl]-1,4-dihydroquinoxaline-6-sulfonamide | +++ | +++ |
| 282 | N-[3-chloro-4-[2-fluoro-5-(hydroxymethyl)phenyl]phenyl]-7-methyl-2,3-dioxo-1,4-dihydroquinoxaline-6-sulfonamide | ++ | + |
| 283 | N-[3-chloro-4-(1-methylbenzimidazol-5-yl)phenyl]-7-methyl-2,3-dioxo-1,4-dihydroquinoxaline-6-sulfonamide | + | + |
| 284 | N-(3-chloro-4-cyclohexyl-phenyl)-7-methyl-2,3-dioxo-1,4-dihydroquinoxaline-6-sulfonamide | ++ | + |
| 285 | N-(3-chloro-4-imidazol-1-yl-phenyl)-7-methyl-2,3-dioxo-1,4-dihydroquinoxaline-6-sulfonamide | + | + |
| 286 | N-[3-chloro-4-(3-chloro-4-methyl-phenyl)phenyl]-7-methyl-2,3-dioxo-1,4-dihydroquinoxaline-6-sulfonamide | ++ | + |
| 287 | N-[3-chloro-4-(2,2-difluoro-1,3-benzodioxol-5-yl)phenyl]-7-methyl-2,3-dioxo-1,4-dihydroquinoxaline-6-sulfonamide | ++ | ++ |
| 288 | N-[3-chloro-4-[4-(dimethylamino)phenyl]phenyl]-7-methyl-2,3-dioxo-1,4-dihydroquinoxaline-6-sulfonamide | + | (+) |
| 289 | N-[3-chloro-4-(3-cyano-4-methoxy-phenyl)phenyl]-7-methyl-2,3-dioxo-1,4-dihydroquinoxaline-6-sulfonamide | ++ | + |
| 290 | N-[3-chloro-4-(5-cyano-3-thienyl)phenyl]-7-methyl-2,3-dioxo-1,4-dihydroquinoxaline-6-sulfonamide | +++ | ++ |
| 291 | N-[3-chloro-4-(6-isopropoxy-3-pyridyl)phenyl]-7-methyl-2,3-dioxo-1,4-dihydroquinoxaline-6-sulfonamide | + | + |
| 292 | N-(4-imidazol-1-ylphenyl)-7-methyl-2,3-dioxo-1,4-dihydroquinoxaline-6-sulfonamide | (+) | (+) |
| 293 | N-[3-chloro-4-[(E)-2-ethoxyvinyl]phenyl]-7-methyl-2,3-dioxo-1,4-dihydroquinoxaline-6-sulfonamide | + | (+) |
| 294 | N-[3-chloro-4-(2-oxo-1,3-dihydrobenzimidazol-5-yl)phenyl]-7-methyl-2,3-dioxo-1,4-dihydroquinoxaline-6-sulfonamide | + | + |
| 295 | N-[3-chloro-4-(2,5-difluorophenyl)phenyl]-7-methyl-2,3-dioxo-1,4-dihydroquinoxaline-6-sulfonamide | ++ | + |
| 296 | N-[3-chloro-4-(2-methylprop-1-enyl)phenyl]-7-methyl-2,3-dioxo-1,4-dihydroquinoxaline-6-sulfonamide | ++ | ++ |
| 297 | N-[4-(3,6-dihydro-2H-pyran-4-yl)phenyl]-7-methyl-2,3-dioxo-1,4-dihydroquinoxaline-6-sulfonamide | + | + |
| 298 | N-[3-bromo-4-(4-cyanophenyl)phenyl]-7-methyl-2,3-dioxo-1,4-dihydroquinoxaline-6-sulfonamide | +++ | ++ |
| 299 | N-[3-bromo-4-(cyclohexen-1-yl)phenyl]-7-methyl-2,3-dioxo-1,4-dihydroquinoxaline-6-sulfonamide | ++ | ++ |
| 300 | N-[3-ethynyl-4-(3-fluoro-4-methoxy-phenyl)phenyl]-7-methyl-2,3-dioxo-1,4-dihydroquinoxaline-6-sulfonamide | +++ | +++ |
| 301 | N-[3-ethynyl-4-(1H-indol-6-yl)phenyl]-7-methyl-2,3-dioxo-1,4-dihydroquinoxaline-6-sulfonamide | +++ | +++ |
| 302 | N-[3-bromo-4-(3-fluoro-4-methoxy-phenyl)phenyl]-7-methyl-2,3-dioxo-1,4-dihydroquinoxaline-6-sulfonamide | +++ | +++ |
| 303 | N-[5-chloro-6-(3-fluoro-4-methoxy-phenyl)-3-pyridyl]-7-methyl-2,3-dioxo-1,4-dihydroquinoxaline-6-sulfonamide | + | + |
| 304 | 7-methyl-2,3-dioxo-N-(4-tetrahydropyran-4-ylphenyl)-1,4-dihydroquinoxaline-6-sulfonamide | (+) | + |
| 305 | N-[5-chloro-6-(4-cyanophenyl)-3-pyridyl]-7-methyl-2,3-dioxo-1,4-dihydroquinoxaline-6-sulfonamide | + | ++ |
| 306 | N-(3-bromo-4-cyclohexyl-phenyl)-7-methyl-2,3-dioxo-1,4-dihydroquinoxaline-6-sulfonamide | ++ | + |
| 307 | N-[3-chloro-4-(2,3,4-trifluorophenyl)phenyl]-7-methyl-2,3-dioxo-1,4-dihydroquinoxaline-6-sulfonamide | ++ | ++ |
| 308 | N-[3-chloro-4-(2,6-difluorophenyl)phenyl]-7-methyl-2,3-dioxo-1,4-dihydroquinoxaline-6-sulfonamide | ++ | ++ |
| 309 | N-[3-chloro-4-(3,5-difluorophenyl)phenyl]-7-methyl-2,3-dioxo-1,4-dihydroquinoxaline-6-sulfonamide | +++ | + |
| 310 | N-[4-(3-bromophenyl)-3-chloro-phenyl]-7-methyl-2,3-dioxo-1,4-dihydroquinoxaline-6-sulfonamide | +++ | + |
| 311 | N-[4-(3-aminophenyl)-3-chloro-phenyl]-7-methyl-2,3-dioxo-1,4-dihydroquinoxaline-6-sulfonamide | ++ | +++ |
| 312 | N-[3-chloro-4-(3-dimethylphosphorylphenyl)phenyl]-7-methyl-2,3-dioxo-1,4-dihydroquinoxaline-6-sulfonamide | + | + |
| 313 | N-[3-chloro-4-(3-methylsulfonylphenyl)phenyl]-7-methyl-2,3-dioxo-1,4-dihydroquinoxaline-6-sulfonamide | + | + |
| 314 | 4-[2-chloro-4-[(7-methyl-2,3-dioxo-1,4-dihydroquinoxalin-6-yl)sulfonylamino]phenyl]benzamide | + | + |
| 315 | N-[3-chloro-4-(3,5-dichlorophenyl)phenyl]-7-methyl-2,3-dioxo-1,4-dihydroquinoxaline-6-sulfonamide | + | (+) |
| 316 | N-[3-chloro-4-[3-(trifluoromethyl)phenyl]phenyl]-7-methyl-2,3-dioxo-1,4-dihydroquinoxaline-6-sulfonamide | ++ | (+) |
| 317 | N-[3-chloro-4-[4-(trifluoromethyl)phenyl]phenyl]-7-methyl-2,3-dioxo-1,4-dihydroquinoxaline-6-sulfonamide | + | (+) |
| 318 | N-[3-chloro-4-(3-cyanophenyl)phenyl]-7-methyl-2,3-dioxo-1,4-dihydroquinoxaline-6-sulfonamide | ++ | + |
| 319 | N-[3-chloro-4-(4-ethoxyphenyl)phenyl]-7-methyl-2,3-dioxo-1,4-dihydroquinoxaline-6-sulfonamide | ++ | + |
| 320 | N-[3-chloro-4-(3-fluoro-4-methoxy-phenyl)phenyl]-7-methyl-2,3-dioxo-1,4-dihydroquinoxaline-6-sulfonamide | +++ | +++ |
| 321 | N-(4-benzyl-3-chloro-phenyl)-7-methyl-2,3-dioxo-1,4-dihydroquinoxaline-6-sulfonamide | + | + |
| 322 | N-[3-bromo-4-(trifluoromethoxy)phenyl]-7-methyl-2,3-dioxo-1,4-dihydroquinoxaline-6-sulfonamide | ++ | ++ |
| 323 | 7-methyl-N-[3-methyl-4-(trifluoromethoxy)phenyl]-2,3-dioxo-1,4-dihydroquinoxaline-6-sulfonamide | ++ | +++ |
| 324 | N-[3-chloro-4-(trifluoromethoxy)phenyl]-7-methyl-2,3-dioxo-1,4-dihydroquinoxaline-6-sulfonamide | ++ | ++ |
| 325 | N-[4-iodo-3-(trifluoromethyl)phenyl]-7-methyl-2,3-dioxo-1,4-dihydroquinoxaline-6-sulfonamide | + | (+) |
| 326 | N-[4-(4-fluorophenyl)-3-(trifluoromethyl)phenyl]-7-methyl-2,3-dioxo-1,4-dihydroquinoxaline-6-sulfonamide | +++ | ++ |
| 327 | N-[4-(4-fluorophenyl)-3-(trifluoromethoxy)phenyl]-7-methyl-2,3-dioxo-1,4-dihydroquinoxaline-6-sulfonamide | + | + |
| 328 | N-(3-chloro-4-imidazo[1,2-a]pyridin-6-yl-phenyl)-7-methyl-2,3-dioxo-1,4-dihydroquinoxaline-6-sulfonamide | + | + |
| 329 | N-[3-chloro-4-(4-methyl-2,3-dihydro-1,4-benzoxazin-7-yl)phenyl]-7-methyl-2,3-dioxo-1,4-dihydroquinoxaline-6-sulfonamide | + | (+) |
| 330 | N-[3-chloro-4-[3-(4-methylpiperazin-1-yl)phenyl]phenyl]-7-methyl-2,3-dioxo-1,4-dihydroquinoxaline-6-sulfonamide | + | + |
| 331 | N-[3-chloro-4-[3-[2-(dimethylamino)ethoxy]phenyl]phenyl]-7-methyl-2,3-dioxo-1,4-dihydroquinoxaline-6-sulfonamide | + | + |
| 332 | 3-[4-[2-chloro-4-[(7-methyl-2,3-dioxo-1,4-dihydroquinoxalin-6-yl)sulfonylamino]phenyl]phenyl]-1,1-dimethyl-urea | + | + |
| 333 | N-[3-chloro-4-[3-(hydroxymethyl)phenyl]phenyl]-7-methyl-2,3-dioxo-1,4-dihydroquinoxaline-6-sulfonamide | ++ | + |
| 334 | N-[3-chloro-4-(1-oxo-3,4-dihydro-2H-isoquinolin-6-yl)phenyl]-7-methyl-2,3-dioxo-1,4-dihydroquinoxaline-6-sulfonamide | + | (+) |
| 335 | N-[3-chloro-4-[3-(dimethylsulfamoyl)phenyl]phenyl]-7-methyl-2,3-dioxo-1,4-dihydroquinoxaline-6-sulfonamide | + | (+) |
| 336 | N-(3-chloro-4-imidazo[1,2-a]pyridin-7-yl-phenyl)-7-methyl-2,3-dioxo-1,4-dihydroquinoxaline-6-sulfonamide | ++ | + |
| 337 | N-[3-chloro-4-([1,2,4]triazolo[1,5-a]pyridin-7-yl)phenyl]-7-methyl-2,3-dioxo-1,4-dihydroquinoxaline-6-sulfonamide | ++ | ++ |
| 338 | N-[3-chloro-4-(2-methylimidazo[1,2-a]pyridin-7-yl)phenyl]-7-methyl-2,3-dioxo-1,4-dihydroquinoxaline-6-sulfonamide | +++ | ++ |
| 339 | N-[3-chloro-4-[3-(trifluoromethoxy)phenyl]phenyl]-7-methyl-2,3-dioxo-1,4-dihydroquinoxaline-6-sulfonamide | ++ | + |
| 340 | N-[3-chloro-4-(1-methylindol-3-yl)phenyl]-7-methyl-2,3-dioxo-1,4-dihydroquinoxaline-6-sulfonamide | + | (+) |
| 341 | N-[3-chloro-4-(1-methylindolin-5-yl)phenyl]-7-methyl-2,3-dioxo-1,4-dihydroquinoxaline-6-sulfonamide | + | (+) |
| 342 | N-[4-[3-(aminomethyl)phenyl]-3-chloro-phenyl]-7-methyl-2,3-dioxo-1,4-dihydroquinoxaline-6-sulfonamide | (+) | + |
| 343 | N-[3-chloro-4-(1,2,3,4-tetrahydroquinolin-6-yl)phenyl]-7-methyl-2,3-dioxo-1,4-dihydroquinoxaline-6-sulfonamide | +++ | + |
| 344 | N-[3-chloro-4-[1-(2-methoxyethyl)pyrazol-4-yl]phenyl]-7-methyl-2,3-dioxo-1,4-dihydroquinoxaline-6-sulfonamide | (+) | (+) |
| 345 | N-[4-(3-fluoro-4-methoxy-phenyl)-3-(trifluoromethyl)phenyl]-7-methyl-2,3-dioxo-1,4-dihydroquinoxaline-6-sulfonamide | +++ | +++ |
| 346 | N-[2-hydroxy-5-[4-[(7-methyl-2,3-dioxo-1,4-dihydroquinoxalin-6-yl)sulfonylamino]-2-(trifluoromethyl)phenyl]phenyl]acetamide | ++ | + |
| 347 | N-[4-indolin-6-yl-3-(trifluoromethyl)phenyl]-7-methyl-2,3-dioxo-1,4-dihydroquinoxaline-6-sulfonamide | +++ | ++ |
| 348 | N-[4-(4-cyanophenyl)-3-(trifluoromethyl)phenyl]-7-methyl-2,3-dioxo-1,4-dihydroquinoxaline-6-sulfonamide | +++ | + |
| 349 | N-[3-chloro-4-(2-methyl-1H-indol-6-yl)phenyl]-7-methyl-2,3-dioxo-1,4-dihydroquinoxaline-6-sulfonamide | +++ | +++ |
| 350 | N-[3-chloro-4-(2-oxoindolin-6-yl)phenyl]-7-methyl-2,3-dioxo-1,4-dihydroquinoxaline-6-sulfonamide | ++ | ++ |
| 351 | N-[3-chloro-4-(7-quinolyl)phenyl]-7-methyl-2,3-dioxo-1,4-dihydroquinoxaline-6-sulfonamide | +++ | + |
| 352 | N-[3-chloro-4-(3-methyl-1H-indol-6-yl)phenyl]-7-methyl-2,3-dioxo-1,4-dihydroquinoxaline-6-sulfonamide | + | + |
| 353 | N-[3-chloro-4-(3,4-dihydro-2H-1,4-benzoxazin-6-yl)phenyl]-7-methyl-2,3-dioxo-1,4-dihydroquinoxaline-6-sulfonamide | ++ | + |
| 354 | N-[3-chloro-4-(4-methyl-2,3-dihydro-1,4-benzoxazin-6-yl)phenyl]-7-methyl-2,3-dioxo-1,4-dihydroquinoxaline-6-sulfonamide | ++ | + |
| 355 | N-[3-chloro-4-(3-oxo-4H-1,4-benzoxazin-6-yl)phenyl]-7-methyl-2,3-dioxo-1,4-dihydroquinoxaline-6-sulfonamide | +++ | +++ |
| 356 | N-[3-chloro-4-(4-methyl-3-oxo-1,4-benzoxazin-6-yl)phenyl]-7-methyl-2,3-dioxo-1,4-dihydroquinoxaline-6-sulfonamide | +++ | ++ |
| 357 | N-[3-chloro-4-(8-fluoro-3,4-dihydro-2H-1,4-benzoxazin-6-yl)phenyl]-7-methyl-2,3-dioxo-1,4-dihydroquinoxaline-6-sulfonamide | +++ | + |
| 358 | N-[3-chloro-4-(6-oxo-1H-pyridin-3-yl)phenyl]-7-methyl-2,3-dioxo-1,4-dihydroquinoxaline-6-sulfonamide | + | ++ |
| 359 | N-[4-(3H-benzimidazol-5-yl)-3-chloro-phenyl]-7-methyl-2,3-dioxo-1,4-dihydroquinoxaline-6-sulfonamide | + | ++ |
| 360 | N-[3-chloro-4-[4-cyano-3-(dimethylamino)phenyl]phenyl]-7-methyl-2,3-dioxo-1,4-dihydroquinoxaline-6-sulfonamide | ++ | + |
| 361 | N-[3-chloro-4-(2-methyl-3H-benzimidazol-5-yl)phenyl]-7-methyl-2,3-dioxo-1,4-dihydroquinoxaline-6-sulfonamide | ++ | +++ |
| 362 | N-(4-cyclobutylphenyl)-7-methyl-2,3-dioxo-1,4-dihydroquinoxaline-6-sulfonamide | + | ++ |
| 363 | 3-fluoro-5-[4-[(7-methyl-2,3-dioxo-1,4-dihydroquinoxalin-6-yl)sulfonylamino]-2-(trifluoromethyl)phenyl]benzamide | ++ | ++ |
| 364 | 7-methyl-N-[4-(2-methyl-1H-indol-6-yl)-3-(trifluoromethyl)phenyl]-2,3-dioxo-1,4-dihydroquinoxaline-6-sulfonamide | +++ | +++ |
| 365 | 7-methyl-2,3-dioxo-N-[4-(7-quinolyl)-3-(trifluoromethyl)phenyl]-1,4-dihydroquinoxaline-6-sulfonamide | +++ | + |
| 366 | N-[4-(3-hydroxyphenyl)-3-(trifluoromethyl)phenyl]-7-methyl-2,3-dioxo-1,4-dihydroquinoxaline-6-sulfonamide | +++ | ++ |
| 367 | 7-methyl-2,3-dioxo-N-[4-(3-oxo-4H-1,4-benzoxazin-6-yl)-3-(trifluoromethyl)phenyl]-1,4-dihydroquinoxaline-6-sulfonamide | +++ | ++ |
| 368 | 2-fluoro-5-[4-[(7-methyl-2,3-dioxo-1,4-dihydroquinoxalin-6-yl)sulfonylamino]-2-(trifluoromethyl)phenyl]benzamide | ++ | + |
| 369 | N-[4-(1H-indol-6-yl)-3-(trifluoromethyl)phenyl]-7-methyl-2,3-dioxo-1,4-dihydroquinoxaline-6-sulfonamide | +++ | ++ |
| 370 | N-[3-chloro-4-(1H-indazol-6-yl)phenyl]-7-methyl-2,3-dioxo-1,4-dihydroquinoxaline-6-sulfonamide | +++ | +++ |
| 371 | N-[3-chloro-4-(3-chloro-4-methoxy-phenyl)phenyl]-7-methyl-2,3-dioxo-1,4-dihydroquinoxaline-6-sulfonamide | | |
| 372 | N-[3-chloro-4-(4-methyl-2-thienyl)phenyl]-7-methyl-2,3-dioxo-1,4-dihydroquinoxaline-6-sulfonamide | | |
| 373 | N-[3-ethynyl-4-(2-methyl-1,3-benzoxazol-5-yl)phenyl]-7-methyl-2,3-dioxo-1,4-dihydroquinoxaline-6-sulfonamide | +++ | +++ |
| 374 | N-[3-bromo-4-(1H-indol-6-yl)phenyl]-7-methyl-2,3-dioxo-1,4-dihydroquinoxaline-6-sulfonamide | +++ | +++ |
| 375 | N-[5-chloro-6-(2-methyl-1,3-benzoxazol-5-yl)-3-pyridyl]-7-methyl-2,3-dioxo-1,4-dihydroquinoxaline-6-sulfonamide | ++ | ++ |
| 376 | N-[3-chloro-4-(4-cyanophenyl)phenyl]-7-methyl-2,3-dioxo-1,4-dihydroquinoxaline-6-sulfonamide | +++ | ++ |
| 377 | N-[3-cyano-4-(4-fluorophenyl)phenyl]-7-methyl-2,3-dioxo-1,4-dihydroquinoxaline-6-sulfonamide | + | + |
| 378 | N-[3-ethynyl-4-(4-fluorophenyl)phenyl]-7-methyl-2,3-dioxo-1,4-dihydroquinoxaline-6-sulfonamide | +++ | +++ |
| 379 | N-[5-chloro-6-(4-fluorophenyl)-3-pyridyl]-7-cyclopropyl-2,3-dioxo-1,4-dihydroquinoxaline-6-sulfonamide | + | + |
| 380 | N-(4-chlorophenyl)-7-methoxy-2,3-dioxo-1,4-dihydroquinoxaline-6-sulfonamide | (+) | + |
| 381 | 7-(methylamino)-2,3-dioxo-N-[4-(trifluoromethoxy)phenyl]-1,4-dihydroquinoxaline-6-sulfonamide | + | + |
| 382 | 7-(cyclopropoxy)-2,3-dioxo-N-[4-(trifluoromethoxy)phenyl]-1,4-dihydroquinoxaline-6-sulfonamide | (+) | + |
| 383 | N-(4-bromophenyl)-7-methoxy-2,3-dioxo-1,4-dihydroquinoxaline-6-sulfonamide | (+) | + |
| 384 | 7-methyl-N-[4-(oxetan-3-yl)phenyl]-2,3-dioxo-1,4-dihydroquinoxaline-6-sulfonamide | (+) | + |
| 385 | N-[5-chloro-6-(triazol-2-yl)-3-pyridyl]-7-methyl-2,3-dioxo-1,4-dihydroquinoxaline-6-sulfonamide | (+) | + |
| 386 | N-[3-chloro-4-(3-hydroxyphenyl)phenyl]-7-methyl-2,3-dioxo-1,4-dihydroquinoxaline-6-sulfonamide | +++ | +++ |
| 387 | N-[3-chloro-4-(4-fluorophenyl)phenyl]-2,3-dioxo-7-(trideuteriomethyl)-1,4-dihydroquinoxaline-6-sulfonamide | +++ | ++ |
| 388 | N-(1-hydroxy-3H-2,1-benzoxaborol-6-yl)-7-methyl-2,3-dioxo-1,4-dihydroquinoxaline-6-sulfonamide | + | (+) |
| 389 | 5-[2-chloro-4-[(7-methyl-2,3-dioxo-1,4-dihydroquinoxalin-6-yl)sulfonylamino]phenyl]-2-fluoro-benzamide | +++ | ++ |
| 390 | 3-[2-chloro-4-[(7-methyl-2,3-dioxo-1,4-dihydroquinoxalin-6-yl)sulfonylamino]phenyl]-5-fluoro-benzamide | +++ | +++ |
| 391 | N-[4-(difluoromethoxy)phenyl]-7-methoxy-2,3-dioxo-1,4-dihydroquinoxaline-6-sulfonamide | (+) | + |
| 392 | N-[5-chloro-6-(4-fluorophenyl)-3-pyridyl]-2,3-dioxo-7-(trideuteriomethyl)-1,4-dihydroquinoxaline-6-sulfonamide | ++ | + |
| 393 | N-[3-chloro-4-(3-cyano-4-fluoro-phenyl)phenyl]-7-methyl-2,3-dioxo-1,4-dihydroquinoxaline-6-sulfonamide | ++ | + |
| 394 | N-[4-(difluoromethoxy)phenyl]-7-methyl-2,3-dioxo-1,4-dihydroquinoxaline-6-sulfonamide | + | ++ |
| 395 | 5-[2-chloro-4-[(7-methyl-2,3-dioxo-1,4-dihydroquinoxalin-6-yl)sulfonylamino]phenyl]-2-methoxy-benzamide | (+) | |
| 396 | 7-methyl-2,3-dioxo-N-[4-(trifluoromethylsulfanyl)phenyl]-1,4-dihydroquinoxaline-6-sulfonamide | + | ++ |
| 397 | N-[4-(4-fluorophenyl)-3-methylsulfonyl-phenyl]-7-methyl-2,3-dioxo-1,4-dihydroquinoxaline-6-sulfonamide | (+) | |
| 398 | N-[3-chloro-4-(4-methylsulfonylphenyl)phenyl]-7-methyl-2,3-dioxo-1,4-dihydroquinoxaline-6-sulfonamide | (+) | (+) |
| 399 | 7-methoxy-2,3-dioxo-N-[4-(trifluoromethylsulfanyl)phenyl]-1,4-dihydroquinoxaline-6-sulfonamide | (+) | + |
| 400 | 7-methoxy-2,3-dioxo-N-[4-(trifluoromethyl)phenyl]-1,4-dihydroquinoxaline-6-sulfonamide | (+) | + |
| 401 | N-(4'-ethynyl-2-(trifluoromethyl)-[1,1'-biphenyl]-4-yl)-7-methyl-2,3-dioxo-1,2,3,4-tetrahydroquinoxaline-6-sulfonamide | | |

Comparison of compounds of the present invention with compounds of formula (I) wherein R² is hydrogen:

| | R² = H | R² =CH₃ |
|---|---|---|
| | Ca²⁺-Influx / LDH Assays EC₅₀ (µM) | Ca²⁺-Influx / LDH Assays EC₅₀ (µM) |
| 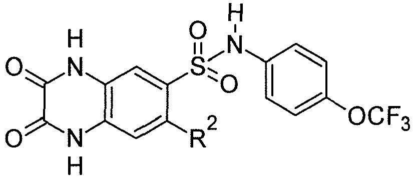 | 0.290 / 0.241 | 0.037 / 0.014 |
| 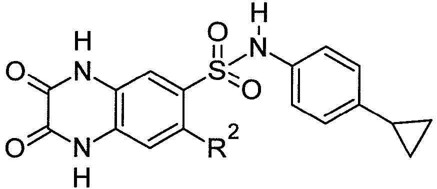 | 0.423 / 0.080 | 0.063 / 0.018 |
| 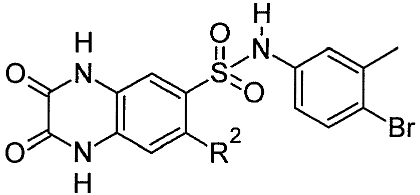 | 1.69 / 0.346 | 0.267 / 0.091 |

As can be taken from the above comparison, the presence of group R² significantly enhances the activity of the compounds of the present invention against *S. aureus.*

All of the pairs shown above show a significant increase in activity upon addition of a substituent at position R².

## Claims

1. A compound of formula (I): 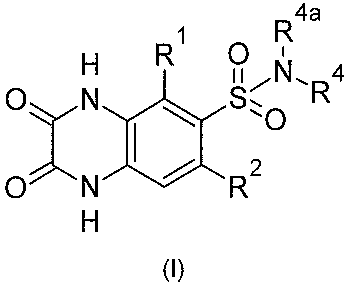 wherein
R¹ is hydrogen, fluorine or a methyl group;
R² is halogen, OH, NO₂, CN or NH₂; or a C₁₋₄ alkyl group, a C₂₋₄ alkenyl group, a C₂₋₄ alkynyl group, a C₃₋₅ cycloalkyl group, an -O-C₃₋₅ cycloalkyl group, a C₄₋₈ alkylcycloalkyl group, or a C₁₋₄ heteroalkyl group;
R⁴ is an optionally substituted phenyl group; an optionally substituted naphthyl group; an optionally substituted heteroaryl group containing 1 or 2 rings and 5 to 10 ring atoms selected from O, S, N and C; an optionally substituted cycloalkyl aryl group comprising a phenyl group and a cycloalkyl group containing 5 or 6 ring atoms; an optionally substituted heterocycloalkyl aryl group comprising a phenyl group and a heterocycloalkyl group containing 5 or 6 ring atoms selected from O, S, B, N and C; an optionally substituted cycloalkyl heteroaryl group comprising a heteroaryl group comprising 5 or 6 ring atoms selected from O, S, N and C and a cycloalkyl group containing 5 or 6 ring atoms; or an optionally substituted heterocycloalkyl heteroaryl group comprising a heteroaryl group comprising 5 or 6 ring atoms selected from O, S, N and C and a heterocycloalkyl group containing 5 or 6 ring atoms selected from O, S, N and C; or an optionally substituted cycloalkyl group containing 1 or 2 rings and 3 to 10 ring atoms; and
R^{4a} is hydrogen; or
R² and R^{4a} together are a group of formula -O-(CH₂)ₙ-, wherein n is 1, 2 or 3, wherein the oxygen is bound to the phenyl ring;
or a solvate, a hydrate or a salt thereof;
wherein the following compounds are excluded:
1. compounds of formula (I) wherein R¹ is H, R² is Me, R^{4a} is hydrogen and R⁴ is selected from the following groups: 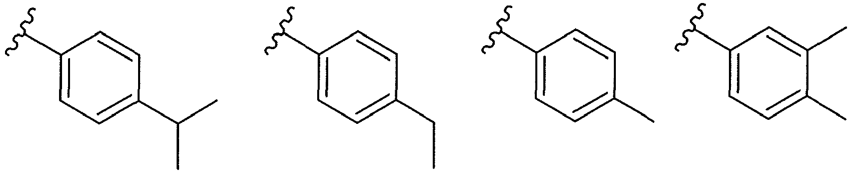 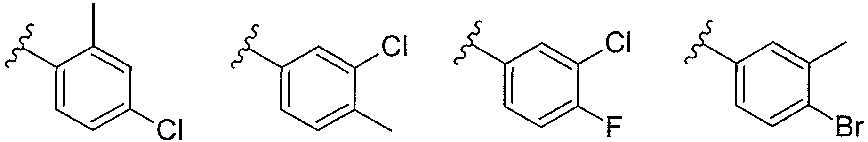 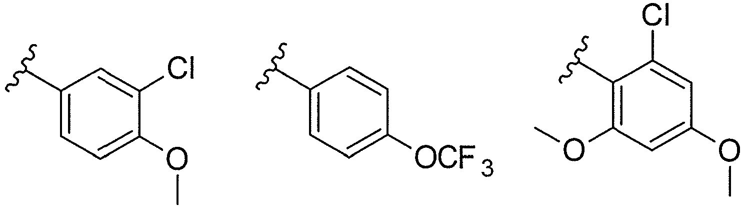 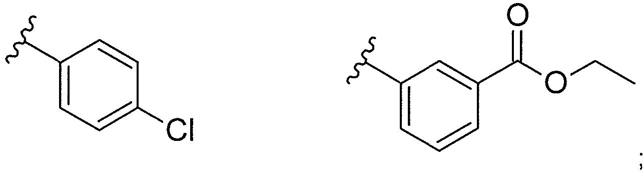 and
2. the compound of the following formula: 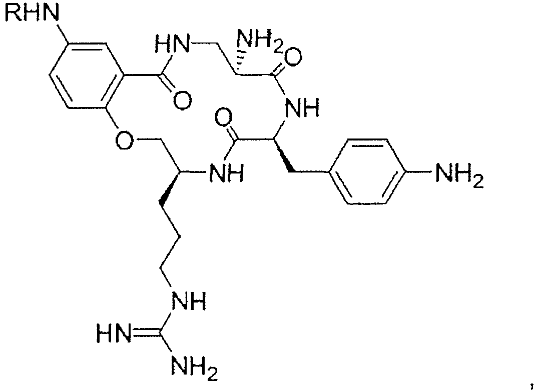
wherein R is a group having the following structure:

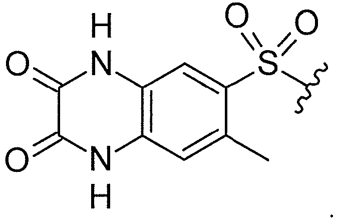

2. A compound according to claim 1, wherein R¹ is hydrogen or fluorine; especially hydrogen.

3. A compound according to claim 1 or 2, wherein R² is F, Cl, Br, a methyl group, an ethyl group, an iso-propyl group, a NO₂ group, a -CF₃ group, a methoxy group, a -O-CF₃ group, a cyclopropyl group, a CN group, a CD₃ group, a -CHF₂ group, a -CH₂F group, a -CH₂OH group, a -NHMe group, an -O-cyclopropyl group, an -O-CH₂CF₃ group, an ethoxy group, an -NHCH₂CH₂OH group, or a - NMe₂ group; especially wherein R² is F, Cl, Br, a methyl group, an ethyl group, an iso-propyl group, a NO₂ group, a -CF₃ group, a methoxy group, a -O-CF₃ group, a cyclopropyl group, a CN group, a CD₃ group, a -CHF₂ group, a -CH₂F group, a -CH₂OH group or a -NMe₂ group; more preferably F, Cl, Br, a methyl group, an ethyl group, iso-propyl group, a methoxy group, a -O-CF₃ group, a NO₂ group, a cyclopropyl group or a dimethylamino group; most preferably, a methyl group.

4. A compound according to any one of the preceding claims, wherein R⁴ is an optionally substituted phenyl group; an optionally substituted naphthyl group; an optionally substituted heteroaryl group containing 1 or 2 rings and 5 to 10 ring atoms selected from O, S, N and C or an optionally substituted cycloalkyl aryl group comprising a phenyl group and a cycloalkyl group containing 5 or 6 ring atoms or an optionally substituted heterocycloalkyl aryl group comprising a phenyl group and a heterocycloalkyl group containing 5 or 6 ring atoms selected from O, S, N and C; especially wherein R⁴ is an optionally substituted phenyl group; or an optionally substituted heteroaryl group containing 5 or 6 ring atoms selected from O, S, N and C.

5. A compound according to any one of the preceding claims, wherein R⁴ has the following formula: 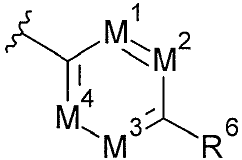 wherein
M¹ is N or CR⁷; M² is N or CR⁵; M³ is N or CR^{5a}; and M⁴ is N or CR^{7a};
R⁵, R^{5a}, R⁷ and R^{7a} are independently selected from hydrogen, halogen, CN, a C₁₋₄ alkyl group, a C₂₋₄ alkenyl group, a C₂₋₄ alkynyl group, or a C₁₋₄ heteroalkyl group; and
R⁶ is halogen, CN, an alkyl group, an alkenyl group, an alkynyl group, a heteroalkyl group, a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁶ is a group of formula -OR^{6a} or -NHR^{6a}, wherein R^{6a} is a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁵ and R⁶ together are part of an optionally substituted phenyl group, an optionally substituted heteroaryl group containing 5 or 6 ring atoms selected from O, S, N and C, an optionally substituted cycloalkyl group containing 5 or 6 ring atoms or an optionally substituted heterocycloalkyl group containing 5 or 6 ring atoms selected from O, S, B, N and C.

6. A compound according to claim 5, wherein R⁷ is hydrogen or methyl; preferably hydrogen; and/or wherein R^{7a} is hydrogen.

7. A compound according to any one of the preceding claims 1 to 4, wherein R⁴ has the following formula: 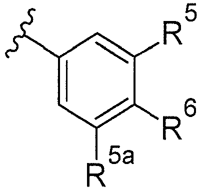 wherein
R⁵ and R^{5a} are independently selected from hydrogen, halogen, CN, a C₁₋₄ alkyl group, a C₂₋₄ alkenyl group, a C₂₋₄ alkynyl group, or a C₁₋₄ heteroalkyl group; and
R⁶ is halogen, CN, an alkyl group, an alkenyl group, an alkynyl group, a heteroalkyl group, a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁶ is a group of formula -OR^{6a} or -NHR^{6a}, wherein R^{6a} is a cycloalkyl group, a heterocycloalkyl group, an alkylcycloalkyl group, a heteroalkylcycloalkyl group, an aryl group, a heteroaryl group, an aralkyl group or a heteroaralkyl group; all of which groups may optionally be substituted; or
R⁵ and R⁶ together are part of an optionally substituted phenyl group, an optionally substituted heteroaryl group containing 5 or 6 ring atoms selected from O, S, N and C, an optionally substituted cycloalkyl group containing 5 or 6 ring atoms or an optionally substituted heterocycloalkyl group containing 5 or 6 ring atoms selected from O, S, B, N and C.

8. A compound according to any one of the preceding claims 5 to 7, wherein R⁵ is hydrogen or methyl; especially hydrogen; and/or wherein R^{5a} is hydrogen, Cl, Br, -CN, methyl, methoxy, -CF₃, -OCF₃, -NMe₂, -C=CH, or -SO₂Me; especially hydrogen, Cl, Br, methyl or methoxy.

9. A compound according to any one of the preceding claims 5 to 8, wherein R⁶ is F, Cl, Br, CN, a C₁₋₆ alkyl group, a C₂₋₆ alkenyl group, a C₂₋₆ alkynyl group, a C₁₋₆ heteroalkyl group, an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

10. A compound according to any one of the preceding claims 5 to 8, wherein R⁶ is CN, a C₂₋₆ alkenyl group, a C₂₋₆ alkynyl group, a C₂₋₆ heteroalkyl group, an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

11. A compound according to any one of the preceding claims 5 to 8, wherein R⁶ is an optionally substituted C₃₋₈ cycloalkyl group, an optionally substituted heterocycloalkyl group containing one or two rings and from 3 to 10 ring atoms selected from O, S, C and N, an optionally substituted phenyl group, an optionally substituted -CH₂-phenyl group, an optionally substituted heteroaryl group containing 5 or 6 to 10 ring atoms selected from O, S, N and C or an optionally substituted heterocycloalkyl aryl group comprising a phenyl group and a heterocycloalkyl group containing 4, 5 or 6 ring atoms selected from O, S, N and C; preferably, R⁶ is an optionally substituted phenyl group or an optionally substituted heteroaryl group containing 5 or 6 ring atoms selected from O, S, N and C.

12. A compound according to any one of the preceding claims 5 to 11, wherein R⁶ is unsubstituted or substituted by 1, 2 or 3 substituents that are independently selected from halogen, CN, OH, NH₂, =O, -P(=O)Me₂, CONH₂, COOH, a C₁₋₄ alkyl group, a C₂₋₄ alkenyl group, a C₂₋₄ alkynyl group, a C₁₋₄ heteroalkyl group, a C₃₋₇ cycloalkyl group, an -O-C₃₋₇ cycloalkyl group or a heterocycloalkyl group containing from 3 to 7 ring atoms selected from O, S, C and N; especially wherein R⁶ is unsubstituted or substituted by 1, 2 or 3 substituents that are independently selected from halogen, CN, COOH, a C₁₋₄ alkyl group, a C₂₋₄ alkenyl group, a C₂₋₄ alkynyl group, a C₁₋₄ heteroalkyl group, a C₃₋₇ cycloalkyl group or a heterocycloalkyl group containing from 3 to 7 ring atoms selected from O, S, C and N.

13. Pharmaceutical composition comprising a compound according to anyone of the preceding claims and optionally one or more carrier substances and/or one or more adjuvants.

14. Compound according to any one of claims 1 to 12 or pharmaceutical composition according to claim 13 for use in the prophylaxis, decolonization and treatment of a *Staphylococcus aureus* infection; especially for use in the prophylaxis and treatment of pneumonia caused by *Staphylococcus aureus.*

15. A compound of formula (I): 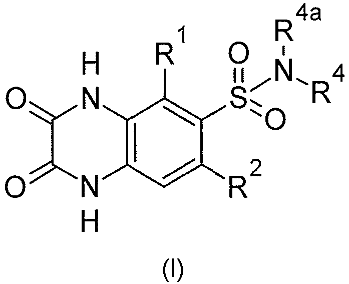 wherein R¹ is H, R² is Me, R^{4a} is hydrogen and R⁴ is selected from the following groups: 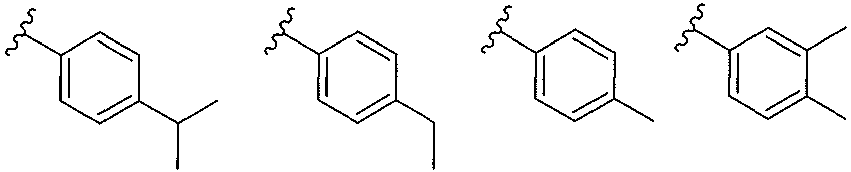 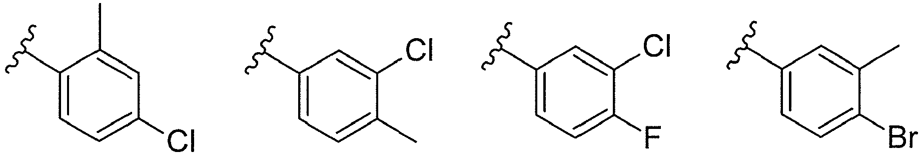 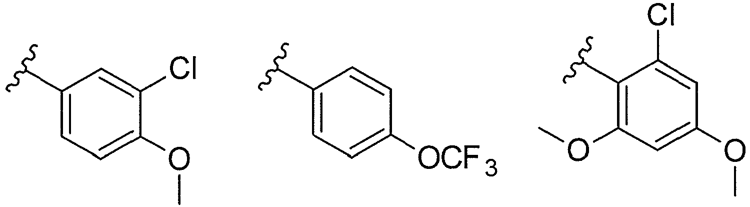 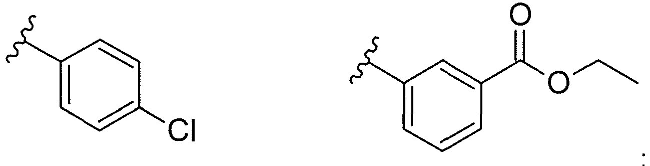 or a solvate, a hydrate or a salt thereof;
for use in the prophylaxis, decolonization and treatment of a *Staphylococcus aureus* infection; especially for use in the prophylaxis and treatment of pneumonia caused by *Staphylococcus aureus.*

## Patentansprüche

1. Verbindung der Formel (I): 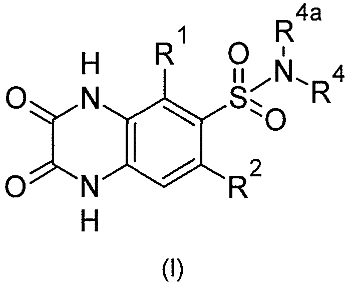 wobei
R¹ Wasserstoff, Fluor oder eine Methylgruppe ist;
R² Halogen, OH, NO₂, CN oder NH₂ ist; oder eine C₁₋₄-Alkylgruppe, eine C₂₋₄-Alkenylgruppe, eine C₂₋₄-Alkinylgruppe, eine C₃₋₅-Cycloalkylgruppe, eine -O-C₃₋₅-Cycloalkylgruppe, eine C₄₋₈-Alkylcycloalkylgruppe oder eine C₁₋₄-Heteroalkylgruppe;
R⁴ eine gegebenenfalls substituierte Phenylgruppe; eine gegebenenfalls substituierte Naphthylgruppe; eine gegebenenfalls substituierte Heteroarylgruppe mit 1 oder 2 Ringen und 5 bis 10 Ringatomen, ausgewählt aus O, S, N und C; eine gegebenenfalls substituierte Cycloalkylarylgruppe, umfassend eine Phenylgruppe und eine Cycloalkylgruppe mit 5 oder 6 Ringatomen; eine gegebenenfalls substituierte Heterocycloalkylarylgruppe, umfassend eine Phenylgruppe und eine Heterocycloalkylgruppe mit 5 oder 6 Ringatomen, ausgewählt aus O, S, B, N und C; eine gegebenenfalls substituierte Cycloalkylheteroarylgruppe, umfassend eine Heteroarylgruppe mit 5 oder 6 Ringatomen, ausgewählt aus O, S, N und C, und eine Cycloalkylgruppe mit 5 oder 6 Ringatomen; oder eine gegebenenfalls substituierte Heterocycloalkylheteroarylgruppe, umfassend eine Heteroarylgruppe mit 5 oder 6 Ringatomen, ausgewählt aus O, S, N und C, und eine Heterocycloalkylgruppe mit 5 oder 6 Ringatomen, ausgewählt aus O, S, N und C, oder eine gegebenenfalls substituierte Cycloalkylgruppe mit 1 oder 2 Ringen und 3 bis 10 Ringatomen ist; und
R^{4a} Wasserstoff ist; oder
R² und R^{4a} zusammen eine Gruppe der Formel -O-(CH₂)ₙ- sind, wobei n 1, 2 oder 3 ist, wobei der Sauerstoff an den Phenylring gebunden ist;
oder ein Solvat, ein Hydrat oder ein Salz davon;
wobei die folgenden Verbindungen ausgeschlossen sind:
1. Verbindungen der Formel (I), wobei R¹ H ist, R² Me ist, R^{4a} Wasserstoff ist und R⁴ aus den folgenden Gruppen ausgewählt ist: 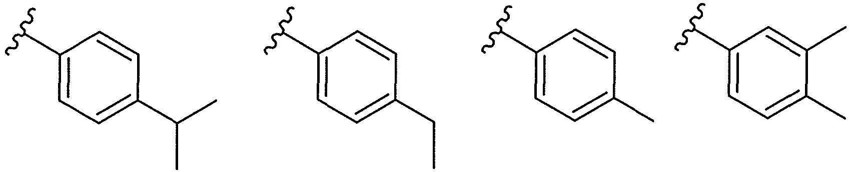 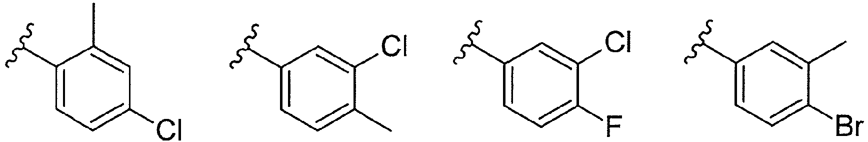 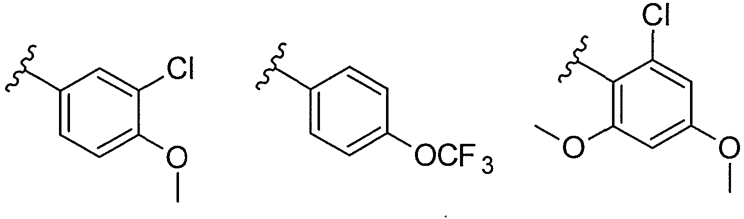 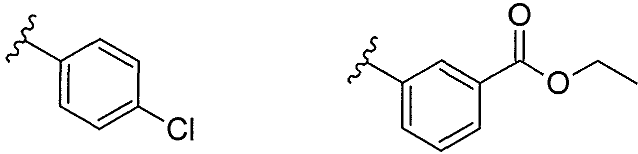 und
2. die Verbindung der folgenden Formel: 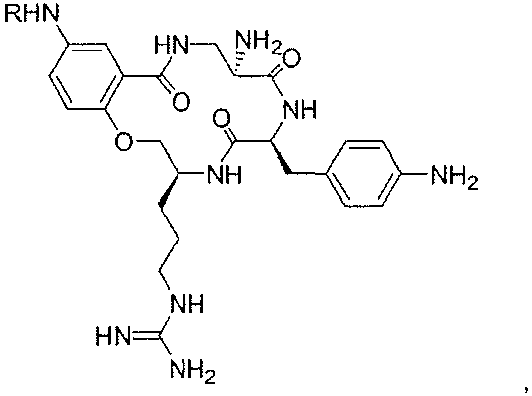
wobei R eine Gruppe mit der folgenden Struktur ist:

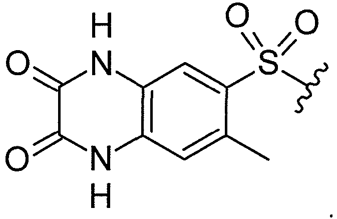

2. Verbindung nach Anspruch 1, wobei R¹ Wasserstoff oder Fluor ist, insbesondere Wasserstoff.

3. Verbindung nach Anspruch 1 oder 2, wobei R² F, Cl, Br, eine Methylgruppe, eine Ethylgruppe, eine Isopropylgruppe, eine NO₂-Gruppe, eine -CF₃-Gruppe, eine Methoxygruppe, eine -O-CF₃-Gruppe, eine Cyclopropylgruppe, eine CN-Gruppe, eine CD₃-Gruppe, eine -CHF₂-Gruppe, eine -CH₂F-Gruppe, eine - CH₂OH-Gruppe, eine -NHMe-Gruppe, eine -O-Cyclopropyl-Gruppe, eine -O-CH₂CF₃-Gruppe, eine Ethoxy-Gruppe, eine -NHCH₂CH₂OH-Gruppe oder eine - NMe₂-Gruppe ist; insbesondere, wobei R² F, Cl, Br, eine Methylgruppe, eine Ethylgruppe, eine Isopropylgruppe, eine NO₂-Gruppe, eine -CF₃-Gruppe, eine Methoxygruppe, eine -O-CF₃-Gruppe, eine Cyclopropylgruppe, eine CN-Gruppe, eine CD₃-Gruppe, eine -CHF₂-Gruppe, eine -CH₂F-Gruppe, eine - CH₂OH-Gruppe oder eine -NMe₂-Gruppe ist; vorzugsweise F, Cl, Br, eine Methylgruppe, eine Ethylgruppe, eine Isopropylgruppe, eine Methoxygruppe, eine -O-CF₃-Gruppe, eine NO₂-Gruppe, eine Cyclopropylgruppe oder eine Dimethylaminogruppe; am meisten bevorzugt eine Methylgruppe ist.

4. Verbindung nach einem der vorhergehenden Ansprüche, worin R⁴ eine gegebenenfalls substituierte Phenylgruppe; eine gegebenenfalls substituierte Naphthylgruppe; eine gegebenenfalls substituierte Heteroarylgruppe, die 1 oder 2 Ringe und 5 bis 10 Ringatome, ausgewählt aus O, S, N und C enthält, oder eine gegebenenfalls substituierte Cycloalkylarylgruppe, die eine Phenylgruppe und eine Cycloalkylgruppe, die 5 oder 6 Ringatome enthält, umfasst, oder eine gegebenenfalls substituierte Heterocycloalkylarylgruppe, die eine Phenylgruppe und eine Heterocycloalkylgruppe umfasst, die 5 oder 6 Ringatome, ausgewählt aus O, S, N und C enthält, ist; insbesondere worin R⁴ eine gegebenenfalls substituierte Phenylgruppe oder eine gegebenenfalls substituierte Heteroarylgruppe mit 5 oder 6 Ringatomen, ausgewählt aus O, S, N und C, ist.

5. Verbindung nach einem der vorangehenden Ansprüche, wobei R⁴ die folgende Formel aufweist: 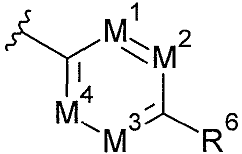 wobei
M¹ N oder CR⁷ ist; M² N oder CR⁵ ist; M³ N oder CR^{5a} ist; und M⁴ N oder CR^{7a} ist;
R⁵ R^{5a}, R⁷ und R^{7a} unabhängig voneinander ausgewählt sind aus Wasserstoff, Halogen, CN, einer C₁₋₄-Alkylgruppe, einer C₂₋₄-Alkenylgruppe, einer C₂₋₄-Alkinylgruppe oder einer C₁₋₄-Heteroalkylgruppe; und
R⁶ Halogen, CN, eine Alkylgruppe, eine Alkenylgruppe, eine Alkinylgruppe, eine Heteroalkylgruppe, eine Cycloalkylgruppe, eine Heterocycloalkylgruppe, eine Alkylcycloalkylgruppe, eine Heteroalkylcycloalkylgruppe, eine Arylgruppe, eine Heteroarylgruppe, eine Aralkylgruppe oder eine Heteroaralkylgruppe ist; wobei alle diese Gruppen gegebenenfalls substituiert sein können; oder
R⁶ eine Gruppe der Formel -OR^{6a} oder -NHR^{6a} ist, worin R^{6a} eine Cycloalkylgruppe, eine Heterocycloalkylgruppe, eine Alkylcycloalkylgruppe, eine Heteroalkylcycloalkylgruppe, eine Arylgruppe, eine Heteroarylgruppe, eine Aralkylgruppe oder eine Heteroaralkylgruppe ist; wobei alle diese Gruppen gegebenenfalls substituiert sein können; oder
R⁵ und R⁶ zusammen Teil einer gegebenenfalls substituierten Phenylgruppe, einer gegebenenfalls substituierten Heteroarylgruppe mit 5 oder 6 Ringatomen, ausgewählt aus O, S, N und C, einer gegebenenfalls substituierten Cycloalkylgruppe mit 5 oder 6 Ringatomen oder einer gegebenenfalls substituierten Heterocycloalkylgruppe mit 5 oder 6 Ringatomen sind, ausgewählt aus O, S, B, N und C.

6. Verbindung nach Anspruch 5, wobei R⁷ Wasserstoff oder Methyl ist; vorzugsweise Wasserstoff; und/oder wobei R^{7a} Wasserstoff ist.

7. Verbindung nach einem der vorangehenden Ansprüche 1 bis 4, wobei R⁴ die folgende Formel aufweist: 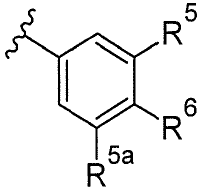 wobei
R⁵ und R^{5a} unabhängig voneinander ausgewählt sind aus Wasserstoff, Halogen, CN, einer C₁₋₄-Alkylgruppe, einer C₂₋₄-Alkenylgruppe, einer C₂₋₄-Alkinylgruppe oder einer C₁₋₄-Heteroalkylgruppe; und
R⁶ Halogen, CN, eine Alkylgruppe, eine Alkenylgruppe, eine Alkinylgruppe, eine Heteroalkylgruppe, eine Cycloalkylgruppe, eine Heterocycloalkylgruppe, eine Alkylcycloalkylgruppe, eine Heteroalkylcycloalkylgruppe, eine Arylgruppe, eine Heteroarylgruppe, eine Aralkylgruppe oder eine Heteroaralkylgruppe ist; wobei alle diese Gruppen gegebenenfalls substituiert sein können; oder
R⁶ eine Gruppe der Formel -OR^{6a} oder -NHR^{6a} ist, wobei R^{6a} eine Cycloalkylgruppe, eine Heterocycloalkylgruppe, eine Alkylcycloalkylgruppe, eine Heteroalkylcycloalkylgruppe, eine Arylgruppe, eine Heteroarylgruppe, eine Aralkylgruppe oder eine Heteroaralkylgruppe ist; wobei alle diese Gruppen gegebenenfalls substituiert sein können; oder
R⁵ und R⁶ zusammen Teil einer gegebenenfalls substituierten Phenylgruppe, einer gegebenenfalls substituierten Heteroarylgruppe mit 5 oder 6 Ringatomen, ausgewählt aus O, S, N und C, einer gegebenenfalls substituierten Cycloalkylgruppe mit 5 oder 6 Ringatomen oder einer gegebenenfalls substituierten Heterocycloalkylgruppe mit 5 oder 6 Ringatomen, ausgewählt aus O, S, B, N und C sind.

8. Verbindung nach einem der vorhergehenden Ansprüche 5 bis 7, wobei R⁵ Wasserstoff oder Methyl ist; insbesondere Wasserstoff; und/oder wobei R^{5a} Wasserstoff, Cl, Br, -CN, Methyl, Methoxy, -CF₃, -OCF₃, -NMe₂, -C=CH oder -SO₂Me ist; insbesondere Wasserstoff, Cl, Br, Methyl oder Methoxy.

9. Verbindung nach einem der vorhergehenden Ansprüche 5 bis 8, wobei R⁶ F, Cl, Br, CN, eine C₁₋₆-Alkylgruppe, eine C₂₋₆-Alkenylgruppe, eine C₂₋₆-Alkinylgruppe, eine C₁₋₆-Heteroalkylgruppe, eine gegebenenfalls substituierte C₃₋₈-Cycloalkylgruppe, eine gegebenenfalls substituierte Heterocycloalkylgruppe, die einen oder zwei Ringe und 3 bis 10 Ringatome umfasst, ausgewählt aus O, S, C und N, eine gegebenenfalls substituierte Phenylgruppe, eine gegebenenfalls substituierte -CH₂-Phenylgruppe, eine gegebenenfalls substituierte Heteroarylgruppe mit 5 oder 6 bis 10 Ringatomen, ausgewählt aus O, S, N und C, oder eine gegebenenfalls substituierte Heterocycloalkylarylgruppe, umfassend eine Phenylgruppe und eine Heterocycloalkylgruppe mit 4, 5 oder 6 Ringatomen, ausgewählt aus O, S, N und C ist, vorzugsweise ist R⁶ eine gegebenenfalls substituierte Phenylgruppe oder eine gegebenenfalls substituierte Heteroarylgruppe mit 5 oder 6 Ringatomen, ausgewählt aus O, S, N und C.

10. Verbindung nach einem der vorhergehenden Ansprüche 5 bis 8, wobei R⁶ CN, eine C₂₋₆-Alkenylgruppe, eine C₂₋₆-Alkinylgruppe, eine C₂₋₆-Heteroalkylgruppe, eine gegebenenfalls substituierte C₃₋₈-Cycloalkylgruppe, eine gegebenenfalls substituierte Heterocycloalkylgruppe, die einen oder zwei Ringe und 3 bis 10 Ringatome, ausgewählt aus O, S, C und N, enthält, eine gegebenenfalls substituierte Phenylgruppe, eine gegebenenfalls substituierte -CH₂-Phenylgruppe, eine gegebenenfalls substituierte Heteroarylgruppe mit 5 oder 6 bis 10 Ringatomen, ausgewählt aus O, S, N und C, oder eine gegebenenfalls substituierte Heterocycloalkylarylgruppe, die eine Phenylgruppe und eine Heterocycloalkylgruppe mit 4, 5 oder 6 Ringatomen, ausgewählt aus O, S, N und C, enthält ist; vorzugsweise ist R⁶ eine gegebenenfalls substituierte Phenylgruppe oder eine gegebenenfalls substituierte Heteroarylgruppe mit 5 oder 6 Ringatomen, ausgewählt aus O, S, N und C.

11. Verbindung nach einem der vorhergehenden Ansprüche 5 bis 8, wobei R⁶ eine gegebenenfalls substituierte C₃₋₈-Cycloalkylgruppe ist, eine gegebenenfalls substituierte Heterocycloalkylgruppe, die einen oder zwei Ringe und 3 bis 10 Ringatome, ausgewählt aus O, S, C und N, enthält, eine gegebenenfalls substituierte Phenylgruppe, eine gegebenenfalls substituierte -CH₂-Phenylgruppe, eine gegebenenfalls substituierte Heteroarylgruppe mit 5 oder 6 bis 10 Ringatomen, ausgewählt aus O, S, N und C, oder eine gegebenenfalls substituierte Heterocycloalkylarylgruppe, die eine Phenylgruppe und eine Heterocycloalkylgruppe mit 4, 5 oder 6 Ringatomen, ausgewählt aus O, S, N und C, umfasst; vorzugsweise ist R⁶ eine gegebenenfalls substituierte Phenylgruppe oder eine gegebenenfalls substituierte Heteroarylgruppe mit 5 oder 6 Ringatomen, ausgewählt aus O, S, N und C.

12. Verbindung nach einem der vorhergehenden Ansprüche 5 bis 11, wobei R⁶ unsubstituiert oder durch 1, 2 oder 3 Substituenten substituiert ist, die unabhängig voneinander ausgewählt sind aus Halogen, CN, OH, NH₂, =O, - P(=O)Me₂, CONH₂, COOH, einer C₁₋₄-Alkylgruppe, einer C₂₋₄-Alkenylgruppe, einer C₂₋₄-Alkinylgruppe, einer C₁₋₄-Heteroalkylgruppe, einer C₃₋₇-Cycloalkylgruppe, einer -O-C₃₋₇-Cycloalkylgruppe oder einer Heterocycloalkylgruppe, die 3 bis 7 Ringatome, ausgewählt aus O, S, C und N, enthält, insbesondere wobei R⁶ unsubstituiert oder mit 1, 2 oder 3 Substituenten substituiert ist, die unabhängig voneinander aus Halogen, CN, COOH, einer C₁₋₄-Alkylgruppe, einer C₂₋₄-Alkenylgruppe, einer C₂₋₄-Alkinylgruppe, einer C₁₋₄-Heteroalkylgruppe, einer C₃₋₇-Cycloalkylgruppe oder einer Heterocycloalkylgruppe, die 3 bis 7 Ringatome, ausgewählt aus O, S, C und N, enthält.

13. Pharmazeutische Zusammensetzung, umfassend eine Verbindung nach einem der vorhergehenden Ansprüche und gegebenenfalls eine oder mehrere Trägersubstanzen und/oder ein oder mehrere Hilfsstoffe.

14. Verbindung nach einem der Ansprüche 1 bis 12 oder pharmazeutische Zusammensetzung nach Anspruch 13 zur Verwendung bei der Prophylaxe, Dekolonisierung und Behandlung einer *Staphylococcus aureus-Infektion;* insbesondere zur Verwendung bei der Prophylaxe und Behandlung einer durch *Staphylococcus aureus* verursachten Lungenentzündung.

15. Verbindung der Formel (I): 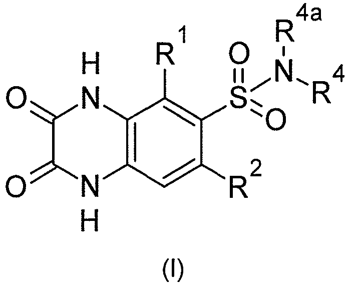 wobei R¹ H ist, R² Me ist, R^{4a} Wasserstoff ist und R⁴ aus den folgenden Gruppen ausgewählt ist: 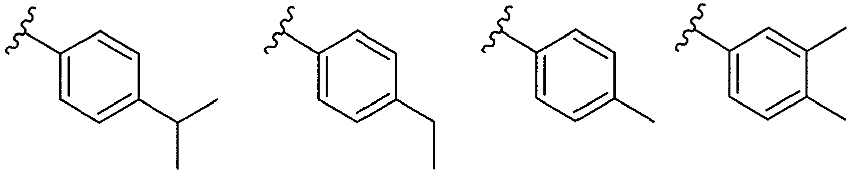 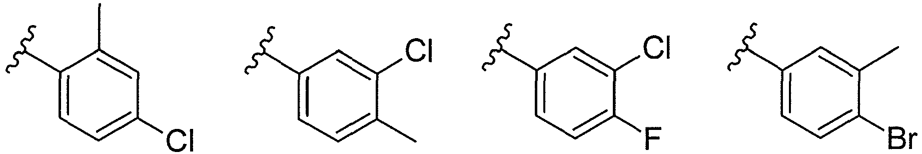 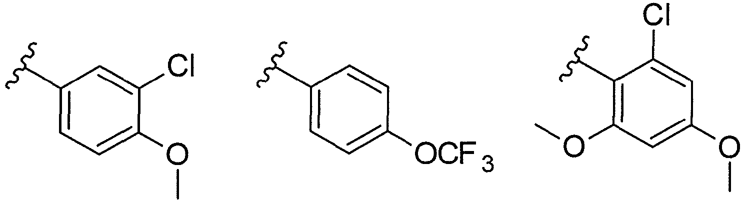 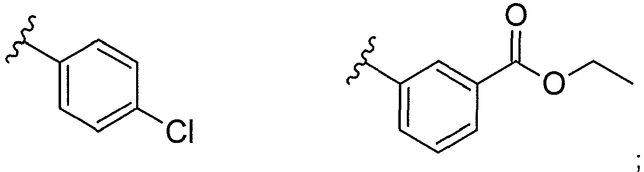 oder ein Solvat, ein Hydrat oder ein Salz davon;
zur Verwendung bei der Prophylaxe, Dekolonisierung und Behandlung einer *Staphylococcus* aureus-Infektion; insbesondere zur Verwendung bei der Prophylaxe und Behandlung einer durch *Staphylococcus aureus* verursachten Lungenentzündung.

## Revendications

1. Composé de formule (I) : 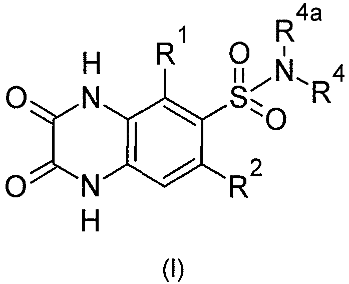 dans lequel
R¹ est hydrogène, fluor ou un groupe méthyle ;
R² est un halogène, OH, NO₂, CN ou NH ; ou un groupe alkyle en C₁₋₄, un groupe alcényle en C₂₋₄, un groupe alcynyle en C₂₋₄, un groupe cycloalkyle en C₃₋₅, un groupe cycloalkyle en -O-C₃₋₅, un groupe alkylcycloalkyle en C₄₋₈, ou un groupe hétéroalkyle en C₁₋₄;
R⁴ est un groupe phényle éventuellement substitué; un groupe naphtyle éventuellement substitué; un groupe hétéroaryle éventuellement substitué contenant 1 ou 2 anneaux et 5 à 10 atomes de cycle choisis parmi O, S, N et C; un groupe cycloalkyle aryle éventuellement substitué comprenant un groupe phényle et un groupe cycloalkyle comprenant 5 ou 6 atomes de cycle; un groupe hétérocycloalkyle aryle éventuellement substitué comprenant un groupe phényle et un groupe hétérocycloalkyle comprenant 5 ou 6 atomes de cycle choisis parmi O, S, B, N et C ; un groupe cycloalkyle hétéroaryle éventuellement substitué comprenant un groupe hétéroaryle comprenant 5 ou 6 atomes de cycle choisis parmi O, S, N et C et un groupe cycloalkyle comprenant 5 ou 6 atomes de cycle; ou un groupe hétérocycloalkyle hétéroaryle éventuellement substitué comprenant un groupe hétéroaryle comprenant 5 ou 6 atomes de cycle choisis parmi O, S, N et C et un groupe hétérocycloalkyle contenant 5 ou 6 atomes de cycle choisis parmi O, S, N et C; ou un groupe cycloalkyle éventuellement substitué comprenant 1 ou 2 anneaux et 3 à 10 atomes d'anneau; et
R^{4a} est un hydrogène; ou
R² et R^{4a} constituent ensemble un groupe de formule -O-(CH₂)ₙ-, dans lequel n est 1, 2 ou 3, l'oxygène étant lié à l'anneau phényle ;
ou un solvant, un hydrate ou un sel de celui-ci;
dans lequel les composés suivants sont exclus:
1. Composés de formule (I) dans lesquelles R¹ est H, R² est Me, R^{4a} est hydrogène et R⁴ est choisi parmi les groupes suivants : 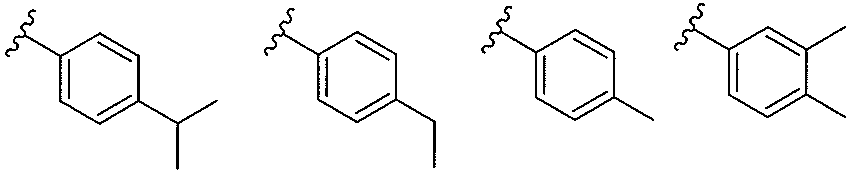 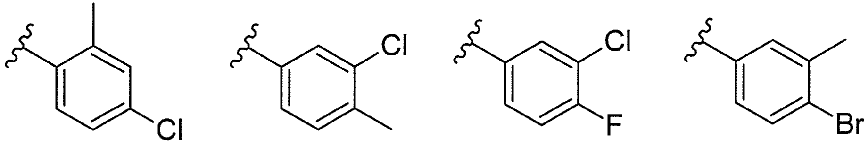 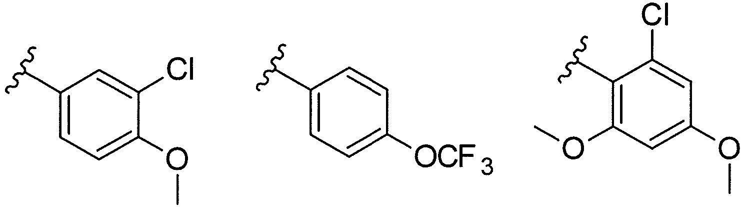 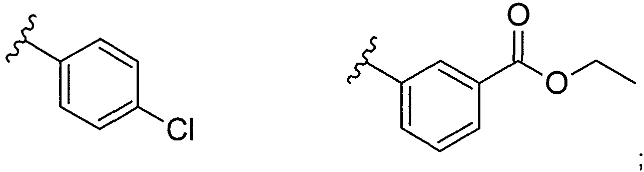 et
2. Composé de formule suivante: 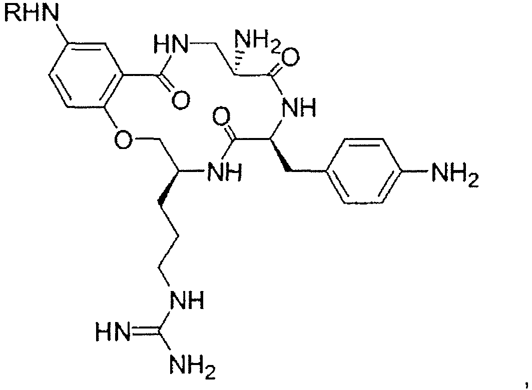
dans lequel R est un groupe ayant la structure suivante :

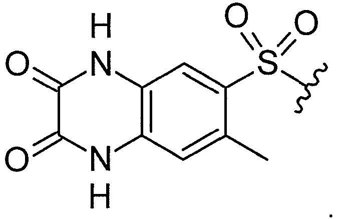

2. Composé selon la revendication 1, dans lequel R¹ est d'hydrogène ou de fluor, en particulier l'hydrogène.

3. Composé selon la revendication 1 ou 2, dans lequel R² est F, CI, Br, un groupe méthyle, un groupe éthyle, un groupe iso-propyle, un groupe NO₂, un groupe - CF₃, un groupe méthoxy, un groupe -O-CF₃, un groupe cyclopropyle, un groupe CN, un groupe CD₃, un groupe -CHF₂, un groupe -CH₂F, un groupe -CH₂OH, un groupe -NHMe, un groupe -O-cyclopropyle, un groupe -O-CH₂CF₃, un groupe éthoxy, un groupe -NHCH₂CH₂OH ou un groupe -NMe₂ ; en particulier dans lequel R² est F, CI, Br, un groupe méthyle, un groupe éthyle, un groupe iso-propyle, un groupe NO₂, un groupe -CF₃, un groupe méthoxy, un groupe - O-CF₃, un groupe cyclopropyle, un groupe CN, un groupe CD₃, un groupe - CHF₂, un groupe -CH₂F, un groupe -CH₂OH ou un groupe -NMe₂; de préférence F, Cl, Br, un groupe méthyle, un groupe éthyle, un groupe iso-propyle, un groupe méthoxy, un groupe -O-CF₃, un groupe NO₂, un groupe cyclopropyle ou un groupe diméthylamino; en préférence, un groupe méthyle.

4. Composé selon l'une quelconque des revendications précédentes, dans lequel R⁴ est un groupe phényle éventuellement substitué; un groupe naphtyle éventuellement substitué; un groupe hétéroaryle éventuellement substitué contenant 1 ou 2 anneaux et 5 à 10 atomes d'anneau choisis parmi O, S, N et C ou un groupe cycloalkyle aryle éventuellement substitué comprenant un groupe phényle et un groupe cycloalkyle contenant 5 ou 6 atomes d'anneau ou un groupe hétérocycloalkyle aryle éventuellement substitué comprenant un groupe phényle et un groupe hétérocycloalkyle contenant 5 ou 6 atomes d'anneau choisis parmi O, S, N et C ; en particulier dans lequel R⁴ est un groupe phényle éventuellement substitué ou un groupe hétéroaryle éventuellement substitué contenant 5 ou 6 atomes d'anneau choisis parmi O, S, N et C.

5. Composé selon l'une quelconque des revendications précédentes, dans lequel R⁴ a la formule suivante : 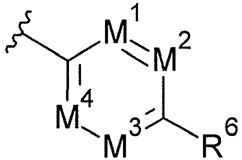 dans lequel
M¹ est N ou CR⁷; M² est N ou CR⁵; M³ est N ou CR^{5a}; et M⁴ est N ou CR^{7a} ;
R⁵, R^{5a}, R⁷ et R^{7a} sont indépendamment choisis parmi l'hydrogène, l'halogène, CN, un groupe alkyle en C₁₋₄, un groupe alcényle en C₂₋₄, un groupe alcynyle en C₂₋₄ ou un groupe hétéroalkyle en C₁₋₄ ; et
R⁶ est un halogène, CN, un groupe alkyle, un groupe alcényle, un groupe alcynyle, un groupe hétéroalkyle, un groupe cycloalkyle, un groupe hétérocycloalkyle, un groupe alkylecycloalkyle, un groupe hétéroalkylecycloalkyle, un groupe aryle, un groupe hétéroaryle, un groupe aralkyle ou un groupe hétéroaralkyle ; tous ces groupes peuvent éventuellement être substitués; ou
R⁶ est un groupe de formule -OR^{6a} ou -NHR^{6a}, dans lequel R^{6a} est un groupe cycloalkyle, un groupe hétérocycloalkyle, un groupe alkylcycloalkyle, un groupe hétéroalkylcycloalkyle, un groupe aryl, un groupe hétéroaryle, un groupe aralkyle ou un groupe hétéroaralkyle ; tous ces groupes peuvent éventuellement être substitués; ou
R⁵ et R⁶ font partie ensemble d'un groupe phényle éventuellement substitué, d'un groupe hétéroaryle éventuellement substitué comprenant 5 ou 6 atomes d'anneau choisis parmi O, S, N et C, d'un groupe cycloalkyle éventuellement substitué comprenant 5 ou 6 atomes d'anneau ou d'un groupe hétérocycloalkyle éventuellement substitué comprenant 5 ou 6 atomes d'anneau choisis parmi O, S, B, N et C.

6. Composé selon la revendication 5, dans lequel R⁷ est un hydrogène ou un méthyle; de préférence un hydrogène ; et/ou dans lequel R^{7a} est un hydrogène.

7. Composé selon l'une quelconque des revendications précédentes 1 à 4, dans lequel R⁴ a la formule suivante : 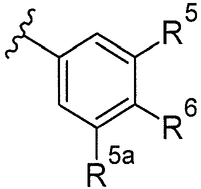 dans lequel
R⁵ et R^{5a} sont indépendamment choisis parmi l'hydrogène, l'halogène, CN, un groupe alkyle en C₁₋₄, un groupe alcényle en C₂₋₄, un groupe alcynyle en C₂₋₄ ou un groupe hétéroalkyle en C₁₋₄ ; et
R⁶ est un halogène, CN, un groupe alkyle, un groupe alcényle, un groupe alcynyle, un groupe hétéroalkyle, un groupe cycloalkyle, un groupe hétérocycloalkyle, un groupe alkylecycloalkyle, un groupe hétéroalkylecycloalkyle, un groupe aryle, un groupe hétéroaryle, un groupe aralkyle ou un groupe hétéroaralkyle ; tous ces groupes peuvent éventuellement être substitués; ou
R⁶ est un groupe de formule -OR⁶ ou -NHR^{6a}, dans lequel R^{6a} est un groupe cycloalkyle, un groupe hétérocycloalkyle, un groupe alkylcycloalkyle, un groupe hétéroalkylecycloalkyle, un groupe aryle, un groupe hétéroaryle, un groupe aralkyle ou un groupe hétéroaralkyle; tous ces groupes peuvent éventuellement être substitués; ou
R⁵ et R⁶ font partie ensemble d'un groupe phényle éventuellement substitué, d'un groupe hétéroaryle éventuellement substitué comprenant 5 ou 6 atomes d'anneau choisis parmi O, S, N et C, d'un groupe cycloalkyle éventuellement substitué contenant 5 ou 6 atomes d'anneau ou d'un groupe hétérocycloalkyle éventuellement substitué comprenant 5 ou 6 atomes d'anneau choisis parmi O, S, B, N et C.

8. Composé selon l'une quelconque des revendications précédentes 5 à 7, dans lequel R⁵ est l'hydrogène ou le méthyle; en particulier l'hydrogène; et/ou dans lequel R^{5a} est l'hydrogène, CI, Br, -CN, le méthyle, le méthoxy, -CF₃, -OCF₃, - NMe₂, -C≡CH, ou -SO₂Me; en particulier l'hydrogène, CI, Br, le méthyle ou le méthoxy.

9. Composé selon l'une quelconque des revendications précédentes 5 à 8, dans lequel R⁶ est F, CI, Br, CN, un groupe alkyle en C₁₋₆, un groupe alcényle en C₂₋₆, un groupe alcynyle en C₂₋₆, un groupe hétéroalkyle en C₁₋₆, un groupe cycloalkyle en C₃₋₈ éventuellement substitué, un groupe hétérocycloalkyle éventuellement substitué contenant un ou deux anneaux et de 3 à 10 atomes d'anneau choisis parmi O, S, C et N, un groupe phényle éventuellement substitué, un groupe -CH₂-phényle éventuellement substitué, un groupe hétéroaryle éventuellement substitué comprenant 5 ou 6 à 10 atomes d'anneau choisis parmi O, S, N et C ou un groupe hétérocycloalkyle aryle éventuellement substitué comprenant un groupe phényle et un groupe hétérocycloalkyle contenant 4, 5 ou 6 atomes d'anneau choisis parmi O, S, N et C; de préférence, R⁶ est un groupe phényle éventuellement substitué ou un groupe hétéroaryle éventuellement substitué contenant 5 ou 6 atomes d'anneau choisis parmi O, S, N et C.

10. Composé selon l'une quelconque des revendications précédentes 5 à 8, dans lequel R⁶ est CN, un groupe alcényle en C₂₋₆, un groupe alcynyle en C₂₋₆, un groupe hétéroalkyle en C₂₋₆, un groupe cycloalkyle en C₃₋₈ éventuellement substitué, un groupe hétérocycloalkyle éventuellement substitué comprenant un ou deux anneaux et de 3 à 10 atomes d'anneau choisis parmi O, S, C et N, un groupe phényle éventuellement substitué, un groupe -CH₂-phényle éventuellement substitué, un groupe hétéroaryle éventuellement substitué contenant 5 ou 6 à 10 atomes d'anneau choisis parmi O, S, N et C ou un groupe hétérocycloalkyle aryle éventuellement substitué comprenant un groupe phényle et un groupe hétérocycloalkyle comprenant 4, 5 ou 6 atomes d'anneau choisis parmi O, S, N et C; de préférence, R⁶ est un groupe phényle éventuellement substitué ou un groupe hétéroaryle éventuellement substitué contenant 5 ou 6 atomes d'anneau choisis parmi O, S, N et C.

11. Composé selon l'une quelconque des revendications précédentes 5 à 8, dans lequel R⁶ est un groupe cycloalkyle en C₃₋₈ éventuellement substitué, un groupe hétérocycloalkyle éventuellement substitué comprenant un ou deux anneaux et de 3 à 10 atomes d'anneau choisis parmi O, S, C et N, un groupe phényle éventuellement substitué, un groupe -CH₂-phényle éventuellement substitué, un groupe hétéroaryle éventuellement substitué contenant 5 ou 6 à 10 atomes d'anneau choisis parmi O, S, N et C ou un groupe hétérocycloalkyle aryle éventuellement substitué comprenant un groupe phényle et un groupe hétérocycloalkyle contenant 4, 5 ou 6 atomes d'anneau choisis parmi O, S, N et C ; de préférence, R⁶ est un groupe phényle éventuellement substitué ou un groupe hétéroaryle éventuellement substitué contenant 5 ou 6 atomes d'anneau choisis parmi O, S, N et C.

12. Composé selon l'une quelconque des revendications précédentes 5 à 11, dans lequel R⁶ est non substitué ou substitué par 1, 2 ou 3 substituants choisis indépendamment parmi l'halogène, CN, OH, NH₂, =O, -P(=O)Me₂, CONH₂, COOH, un groupe alkyle en C₁₋₄, un groupe alcényle en C₂₋₄, un groupe alcynyle en C₂₋₄, un groupe hétéroalkyle en C₁₋₄, un groupe cycloalkyle en C₃₋₇, un groupe cycloalkyle en -O-C₃₋₇ ou un groupe hétérocycloalkyle contenant de 3 à 7 atomes d'anneau choisis parmi O, S, C et N; en particulier dans lequel R⁶ est non substitué ou substitué par 1, 2 ou 3 substituants choisis indépendamment parmi l'halogène, CN, COOH, un groupe alkyle en C₁₋₄, un groupe alcényle en C₂₋₄, un groupe alcynyle en C₂₋₄, un groupe hétéroalkyle en C₁₋₄, un groupe cycloalkyle en C₃₋₇ ou un groupe hétérocycloalkyle comprenant de 3 à 7 atomes d'anneau choisis parmi O, S, C et N.

13. Composition pharmaceutique comprenant un composé selon l'une des revendications précédentes et éventuellement une ou plusieurs substances porteuses et/ou un ou plusieurs adjuvants.

14. Composé selon l'une des revendications 1 à 12 ou composition pharmaceutique selon la revendication 13 pour utilisation dans la prophylaxie, la décolonisation et le traitement d'une infection à *Staphylococcus aureus;* en particulier pour utilisation dans la prophylaxie et le traitement de la pneumonie causée par *Staphylococcus aureus.*

15. Composé de formule (I): 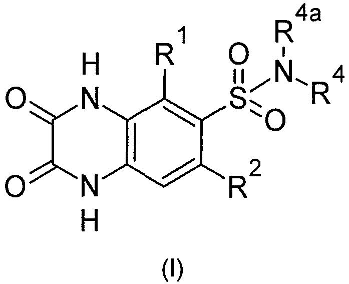 dans lequel R¹ est H, R² est Me, R^{4a} est hydrogène et R⁴ est choisi parmi les groupes suivants : 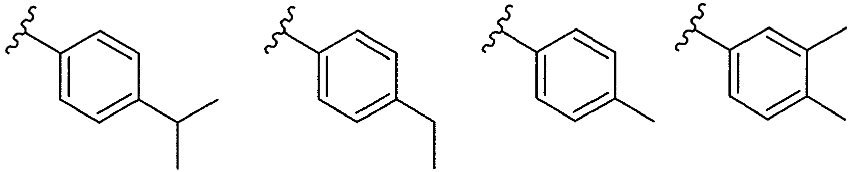 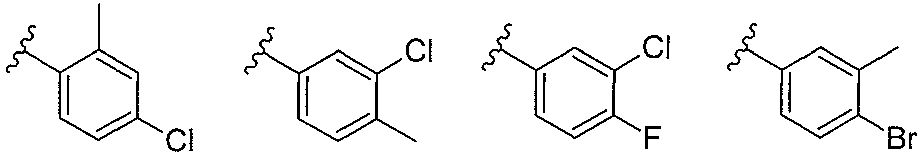 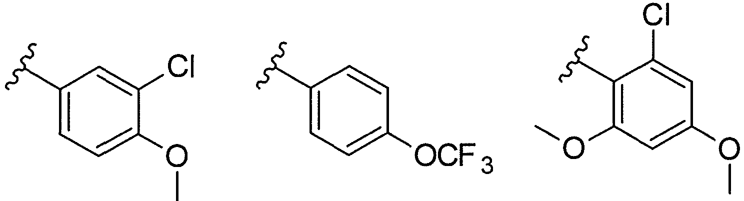 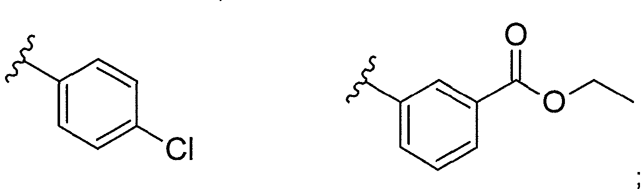 ou un solvant, un hydrate ou un sel de celui-ci ;
pour la prophylaxie, la décolonisation et le traitement d'une infection à *Staphylococcus aureus;* en particulier pour la prophylaxie et le traitement de la pneumonie causée par *Staphylococcus aureus.*
